# EUROPEAN PATENT SPECIFICATION

(11) **EP 4 271 651 B1**
(45) Date of publication and mention of the grant of the patent: **23.10.2024**
(21) Application number: 21848463.2
(22) Date of filing: 29.12.2021
(51) Int. Cl.: B82Y 15/00, G01N 21/64, G01N 33/48, C01B 32/182, C01B 32/198, B82Y 30/00, C12Q 1/00

(54) **PRISTINE GRAPHENE BASED BIOSENSOR FOR BIOMARKER DETECTION AND RELATED CORE PARTICLES, MATERIALS COMPOSITIONS METHODS AND SYSTEMS**
BIOSENSOR AUF BASIS VON REINEM GRAPHEN ZUM NACHWEIS VON BIOMARKERN UND ZUGEHÖRIGE KERNPARTIKEL, MATERIALZUSAMMENSETZUNGEN, METHODEN UND SYSTEME
BIOCAPTEUR À BASE DE GRAPHÈNE PUR POUR LA DÉTECTION DE BIOMARQUEURS ET PARTICULES CENTRALES, MATÉRIAUX, COMPOSITIONS, MÉTHODES ET SYSTÈMES ASSOCIÉS

(30) Priority: 30.12.2020 US 202063131972 P
(43) Date of publication of application: 08.11.2023
(73) Proprietor: Hawkeye Bio, Limited, Dublin D02 A342 (IE); The University of Kansas, Lawrence, KS 66045 (US); Kansas State University Research Foundation, Manhattan, KS 66502 (US)
(72) Inventor: DEMPSEY, Paul W., Westlake Village, California 91361-3074 (US); HANTULA, Spencer, Westlake Village, California 91361-3074 (US); APARICIO, Cristina-Mihaela Sandu, Westlake Village, California 91361-3074 (US); BOSSMANN, Stefan H., Kansas City, Missouri 64112 (US); SORENSEN, Christopher M., Manhattan, Kansas 66505 (US); COVARRUBIAS-ZAMBRANO, Obdulia, Lenexa, Kansas 66215 (US); COVARRUBIAS, Jose, Manhattan, Kansas 66502 (US)
(74) Representative: HGF
(86) International application number: PCT/US2021/065573
(87) International publication number: WO 2022/147171

(56) References cited:
- EP-A1- 3 358 348
- EP-A1- 3 358 348
- WO-A1-2014/090313
- WO-A1-2014/090313
- US-A1- 2012 107 590
- US-A1- 2012 107 590
- SARMANOVA OLGA E ET AL: "A method for optical imaging and monitoring of the excretion of fluorescent nanocomposites from the body using artificial neural networks", NANOMEDICINE: NANOTECHNOLOGY, BIOLOGY, AND MEDICINE, ELSEVIER, AMSTERDAM, NL, vol. 14, no. 4, 12 April 2018 (2018-04-12), pages 1371 - 1380, XP085414611, ISSN: 1549-9634, DOI: 10.1016/J.NANO.2018.03.009
- MING FANG ET AL: "Covalent polymer functionalization of graphene nanosheets and mechanical properties of composites", JOURNAL OF MATERIALS CHEMISTRY, vol. 19, no. 38, 10 August 2009 (2009-08-10), pages 7098, XP055030347, ISSN: 0959-9428, DOI: 10.1039/b908220d
- SARMANOVA OLGA E ET AL: "A method for optical imaging and monitoring of the excretion of fluorescent nanocomposites from the body using artificial neural networks", NANOMEDICINE: NANOTECHNOLOGY, BIOLOGY, AND MEDICINE, ELSEVIER, AMSTERDAM, NL, vol. 14, no. 4, 12 April 2018 (2018-04-12), pages 1371 - 1380, XP085414611, ISSN: 1549-9634, DOI: 10.1016/J.NANO.2018.03.009
- MING FANG ET AL: "Covalent polymer functionalization of graphene nanosheets and mechanical properties of composites", JOURNAL OF MATERIALS CHEMISTRY, vol. 19, no. 38, 10 August 2009 (2009-08-10), pages 7098, XP055030347, ISSN: 0959-9428, DOI: 10.1039/b908220d

## Description

### STATEMENT OF GOVERNMENT GRANT

This invention was made with Government support under grant number 2032751 awarded by the National Science Foundation. The Government has certain rights in the invention.

### FIELD

The present disclosure relates to biomarkers' detection and related biosensor. In particular, the present disclosure relates to pristine graphene biosensors for biomarker detection and related core particles, materials compositions methods and systems.

### BACKGROUND

Detection of biomarkers is central to many fields of investigations such as medical field and basic biological research.

In particular, detection of biomarkers is key to acquiring an understanding of several biological events in various organisms including occurrences of various conditions from cancer to viral infections to additional events such as blood-clotting and inflammation.

For example, Sarmanova et al., in Nanomedicine: Nanotechnology, Biology and Medicine, Volume 14, Issue 4, 2018, Pages 1371-1380 relates to a method for optical imaging and monitoring of the excretion of fluorescent nanocomposites from the body using artificial neural networks,.

Despite progress made in the field, robust reproducible detection of biomarkers in samples at nanomolar or lower concentrations in particular in connection with conditions remains challenging.

### SUMMARY

The present disclosure is related to graphene-based biosensors (herein also graphene biosensor or biosensor) and related core particles, materials, compositions methods and systems, which are so stable in a sample in the sense of the disclosure which in several embodiments allow detection of biomarkers at picomolar or femtomolar concentrations with high reproducibility and signal to noise ratio as will be understood by a skilled person.

The claimed graphene core particle of the present disclosure comprise the features defined in claim 1. Related biosensors comprise the features defined in claim 4. Related multiplex biosensors comprise the features defined in claim 8. Related compositions comprise the features defined in claim 9. Related methods and systems comprise the features defined in claims 10 to 16.

In particular, according to a claimed first set of embodiments a graphene core particle is defined. The graphene core particle comprises more than one pristine graphene nanosheets coated by a coating layer of an organic and/or inorganic compound having a solubility in water of 1% by weight at 25 degree C. In the graphene core particle, the more than one pristine graphene nanosheets and/or the organic and/or inorganic compound of the coating layer are configured to present a functional group capable of binding a corresponding functional group in an aqueous solution. In the graphene core particle, the coating layer, the organic and/or inorganic compound, the pristine graphene and the pristine graphene nanosheet are configured and comprise at least the features indicated in claim 1.

According to a claimed second set of embodiments a biosensor is defined, configured to detect a target biomarker. The claimed biosensor comprises: a graphene core particle of the claimed first set of embodiments , and a detectable moiety capable of emitting a fluorescent signal, the detectable moiety attached to the core particle through a peptide linkage comprising a recognition sequence specific for a target biomarker.

In the biosensor, the coating layer and the peptide linkage are configured to set a quenching distance between the graphene nanosheets and the detectable moiety wherein the graphene nanosheet quenches the fluorescent signal of the detectable moiety.

In the biosensor, the peptide linkage is further configured to set, upon recognition by the target biomarker of the recognition sequence, an emitting distance between the graphene nanosheets and the detectable moiety wherein the graphene nanosheet does not quench the fluorescent signal of the detectable moiety.

In the biosensor, the target biomarker specific peptide linkage and the detectable moiety thus form a target specific detectable component configured to emit a detectable fluorescent signal upon modification of the peptide linkage by the target biomarker.

In the biosensor, the coating layer and the peptide linkage and the detectable moiety are configured and comprise at least the features indicated in claim 4.

According to a claimed third set of embodiments, a multiplex biosensor is defined, comprising: a core particle of the claimed first set of embodiments and a plurality of target specific detectable components attached to the graphene core particle. In the multiplex biosensor, each target specific detectable component is formed by a detectable moiety capable of emitting a fluorescent signal in an excited state and having an excitation spectrum and an emission spectrum, and the detectable moiety attached to a peptide linkage comprising a recognition sequence specific for a target biomarker.

In each target specific detectable component, the peptide linkage is configured to provide in combination with the coating layer of the core particle, a quenching distance between the detectable moiety and the graphene sheet core particle wherein the graphene nanosheet quenches the fluorescent signal of the detectable moiety.

In the biosensor, the recognition sequence for the target biomarker of each detectable component is configured to specifically detect a target biomarker and the plurality of detectable components is selected to specifically detect a plurality of different target biomarkers.

In the plurality of target specific detectable components of the biosensor, each detectable moiety has an excitation spectrum having a minimized overlap with the emission spectrum of another detectable moiety of the plurality of target specific detectable components.

In the multiplex biosensor the graphene core particle and target specific detectable component are configured and comprise at least the features indicated in claim 8.

According to a claimed fourth set of embodiments, a composition is defined, comprising one or more graphene core particles and/or one or more biosensors herein described in combination with a suitable auxiliary agent such as a vehicle diluent, excipient, or the like. In the composition, the one or more graphene core particles, the one or more graphene biosensors and the suitable auxiliary agent are configured and comprise at least the features indicated in claim 9. In some embodiments, the composition is a pharmaceutical composition and the auxiliary agent is a pharmaceutically accepted auxiliary agent. In embodiments where the composition comprises two or more graphene biosensors presenting two or more detectable components specific for different target biomarkers, the excitation spectrum of each detectable moiety of the two or more detectable components has a minimized overlap with the emission spectrum of another detectable moiety of the two or more detectable components.

According to a claimed fifth set of embodiments, a method is defined for making a core particle of the first set of embodiments. The method comprises providing a pristine graphene nanosheet having a graphene surface, and coating the graphene surface with a layer of an organic and/or inorganic molecule as indicated in claim 14.

The claimed method for making a core particle can be performed with a system for making a core particle, comprising pristine graphene nanosheets having a graphene surface and reagents to chemically transform the graphene surface to provide pristine graphene nanosheets coated with a coating layer.

According to a claimed sixth set of embodiments, a method and a system are described for making a biosensor herein described. The method comprises providing a core particle of the claimed first set of embodiments and attaching to the graphene surface a detectable moiety through a peptide linkage, as defined in claim 15.

The system for making a biosensor herein described comprises a pristine graphene nanosheet having a graphene surface, a peptide linkage, a detectable moiety, and reagents for attaching the detectable moiety to the graphene surface through the peptide linkage as defined in claim 16. In the system for making a biosensor herein described the components are comprised in a configuration for simultaneous, combined or sequential use in the method of making a biosensor herein described.

According to a claimed seventh set of embodiments aspect, a method and a system are defined for detecting the activity of a biomarker in a biological sample. The method comprises: contacting a biological sample with any biosensor according to the present disclosure to create a reaction solution and detecting a change in the reaction solution as defined in claim 10.

The system comprises a biosensor herein described and reagents for simultaneous combined or sequential use in a method of detection of the activity of a biomarker in a biological sample herein described as defined in claim 11.

According to a claimed eighth set of embodiments, a method and system are defined for multiplexed detection of the activity of a plurality of biomarkers in a biological sample. The method comprises: contacting a biological sample with a multiplex biosensor according to the present disclosure configured to present detectable components specific for the plurality of the biomarkers, the contacting performed to create a reaction solution; and detecting a change in said reaction solution. In addition, or in the alternative, the method can comprise contacting the biological sample with multiple biosensors each presenting a peptide linkage specific for one biomarker of the plurality of the biomarkers. In the method of the claimed eight set of embodiments the contacting is performed as defined in claim 12.

The system of the claimed eighth set of embodiments, for multiplexed detection of the activity of a plurality of biomarkers in a biological sample comprises a multiplex biosensor of the claimed third set of embodiments and/or biosensors of the claimed second set of embodiments, each presenting a peptide linkage specific for one biomarker of the plurality of the biomarkers as well as optionally a plurality of and reagents for simultaneous, combined, or sequential use in a method of detection, the activity of a biomarker in a biological sample herein described. The system of the claimed eight set of embodiments comprises at least the features defined in claim 12.

In some embodiments of the claimed seventh set of embodiments, and the claimed eight sets of embodiments in methods and systems for detection of a target biomarker, the detecting is preceded by a heating of the sample and/or of the testing solution. In particular, it has been found unexpectedly that pretreating sera and/or running the protease assay at selected temperatures improve signal-to-noise using separate and additive mechanisms.

Biosensors and related graphene core particles, materials, compositions methods and systems herein described provide in several embodiments a graphene-based biosensor highly stable in an aqueous sample which can maintain a percentage of at least 95% particles in suspension with a change in colloidal stability of -24% or less at OD650 for months.

Biosensors and related core particles, materials, compositions methods, and systems herein described allow in several embodiments detection of a biomarker at picomolar or femtomolar concentrations with high reproducibility and in some embodiments, a coefficient of variation in detected signal between different manufactured lots or batches of less than 10%.

Biosensors and related core particles, materials, compositions methods and systems herein described allow in several embodiments detection of a biomarker at picomolar or femtomolar concentrations in an aqueous solution with a signal-to-noise ratio of 25 or higher across multiple sensors as will be understood by a skilled person upon reading of the present disclosure.

Biosensors and related core particles, materials, compositions methods, and systems herein described allow in several embodiments detection of a multiple biomarker in an aqueous sample with high multiplex ability with a number of different detectable components, up to 10 in the UV/Vis spectrum, or up to 50 the entire electromagnetic spectrum, presented on the same graphene particle or within the same solution as will be understood by a skilled person upon reading of the present disclosure.

Biosensors and related core particles, materials, compositions methods and systems herein described allow in several embodiments production of robust biosensors that can be dispersed in aqueous forms, dried, and redispersed for up to 10 cycles of dispersion. Accordingly, the biosensor of the disclosure can be comprised in compositions and systems in a dry form as will be understood by a skilled person.

Biosensors and related core particles, materials, compositions methods and systems herein described can be used in connection with various applications wherein reproducible detection of one or more biomarkers is desired, in particular when present at very low concentrations, such as picomolar or femtomolar concentrations. For example, biosensors and related core particles, materials, compositions methods and systems herein described can be used in medical and diagnostic applications for visualization of the onset of disease pathologies, and responses to drug treatments and therapy, facilitating the development of diagnostic and therapeutic cellular agents, as well as for visualization of biological events such as cellular signaling, homeostasis, cellular migration, responses to external stimuli such as temperature and pH, among other advantages identifiable by a skilled person.

Additional exemplary applications include uses of the biosensors and related core particles, materials, compositions, methods and systems herein described in several fields including basic biology research, applied biology, bio-engineering, aetiology, medical research, medical diagnostics, therapeutics, drug research, and to develop diagnostic and therapeutic approaches and tools in additional fields identifiable by a skilled person upon reading of the present disclosure.

The details of one or more embodiments of the disclosure are set forth in the accompanying drawings and the description below. Other features, objects, and advantages will be apparent from the description and drawings, and from the claims.

### BRIEF DESCRIPTION OF THE DRAWINGS

The accompanying drawings, which are incorporated into and constitute a part of this specification, illustrate one or more embodiments of the present disclosure and, together with the detailed description and example sections, serve to explain the principles and implementations of the disclosure. Exemplary embodiments of the present disclosure will become more fully understood from the detailed description and the accompanying drawings, wherein:

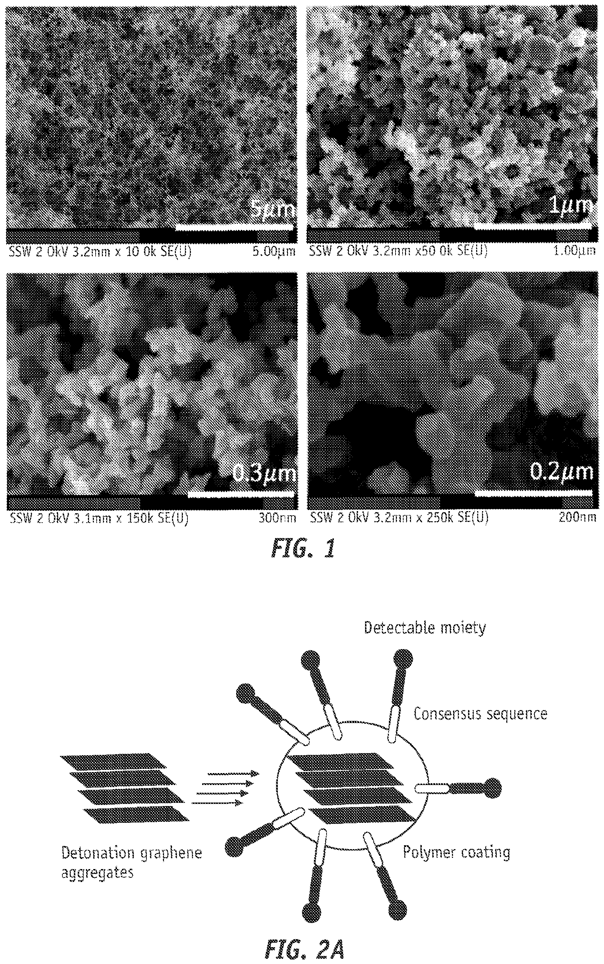

**Figure 1** shows SEM (Scanning Electron Microscopy) of explosion graphene from [1].
**Figure 2A** shows a schematic representation of an exemplary graphene-based biosensor for protease detection of the present disclosure.

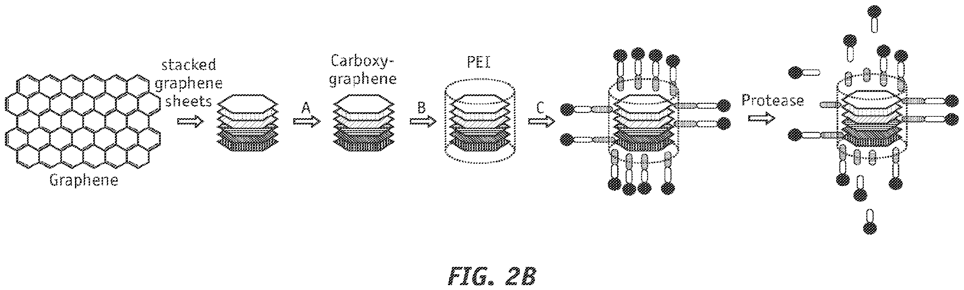

**Figure 2B** shows a schematic representation of an exemplary process to provide biosensors according to the disclosure: A: synthesis of Carboxygraphene; B: Chemical attachment of Polyethylenimine via amide bonds; C: Attachment of consensus sequence (oligopeptide) and fluorescent dye, such as tetra carboxy-phenyl-porphyrin (TCPP).

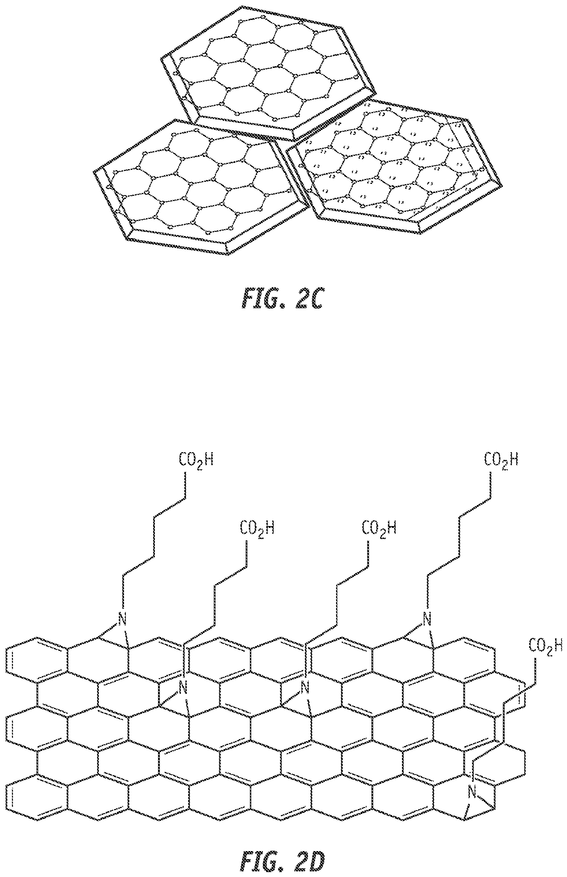

**Figure 2C** shows a schematic representation illustrating thin monolayers of graphene entangled with each other with overlapped edges, or more ordered stacking of nanosheets comprising or consisting of two to three layers.
**Figure 2D** shows a schematic representation of a high density of carboxylic acid groups added across the surface of a graphene nanosheet particulate.

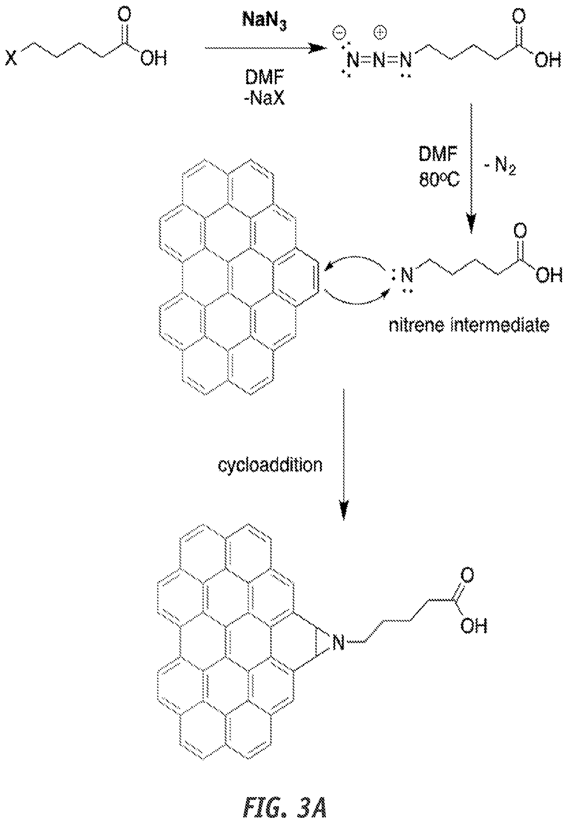

**Figure 3A** shows a reaction scheme for azirane-cycloaddition to add a tailored amount of carboxylic acids to the surface of graphene.

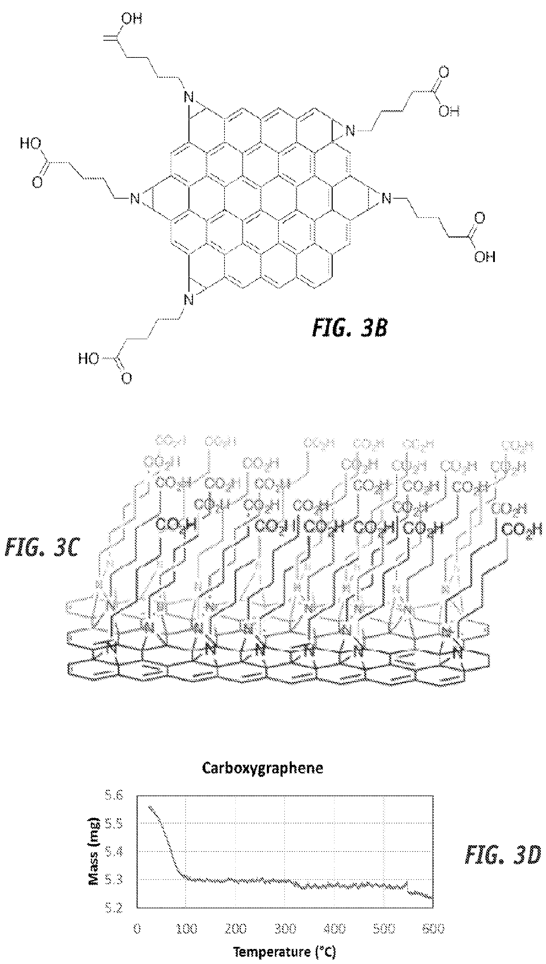

**Figure 3B** shows an exemplary structure of a single nanosheet of carboxygraphene (CG).
**Figure 3C** shows a side-view illustration of the structure of an exemplary carboxygraphene top layer showing the high-density distribution of carboxylic acid groups across the surface.
**Figure 3D** shows Differential Thermogravimetry Analysis (DTA) of synthesized carboxygraphene under N₂.

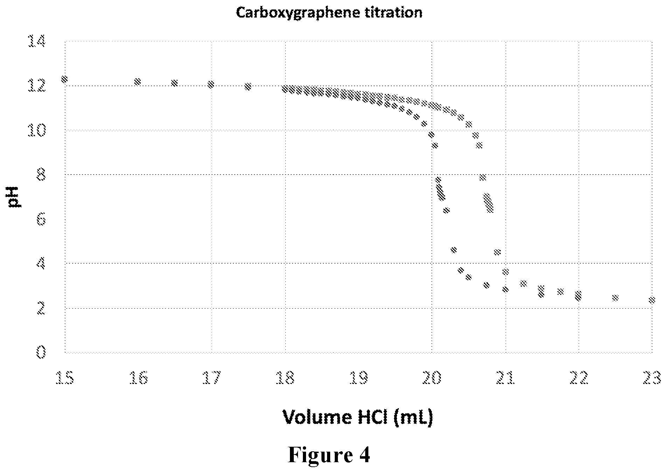

**Figure 4** shows titration curves (pH vs. volume 0.100 M HCl) starting with adding 20 mL of 0.100 M NaOH to 100 mg of carboxygraphene; round dots: titration of carboxygraphene; square dots: reference curve (no GO added).

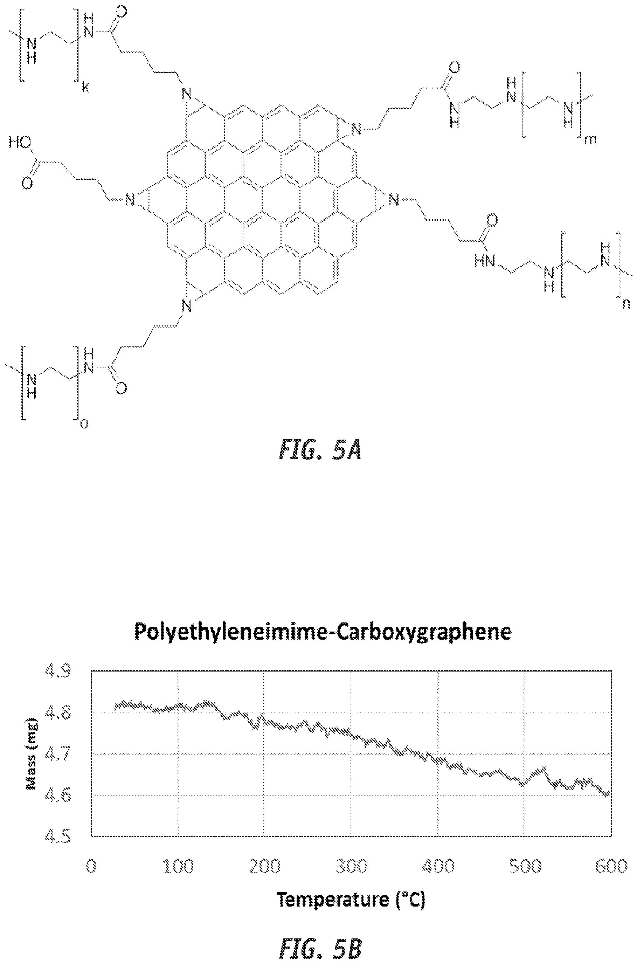

**Figure 5A** shows a structure of carboxygraphene-polyethylenimine, only a single sheet is shown for reference.
**Figure 5B** shows chart reporting the results of Differential Thermogravimetry Analysis (DTA) of exemplary synthesized carboxygraphene-polyethylenimine (G-PEI) under N₂.

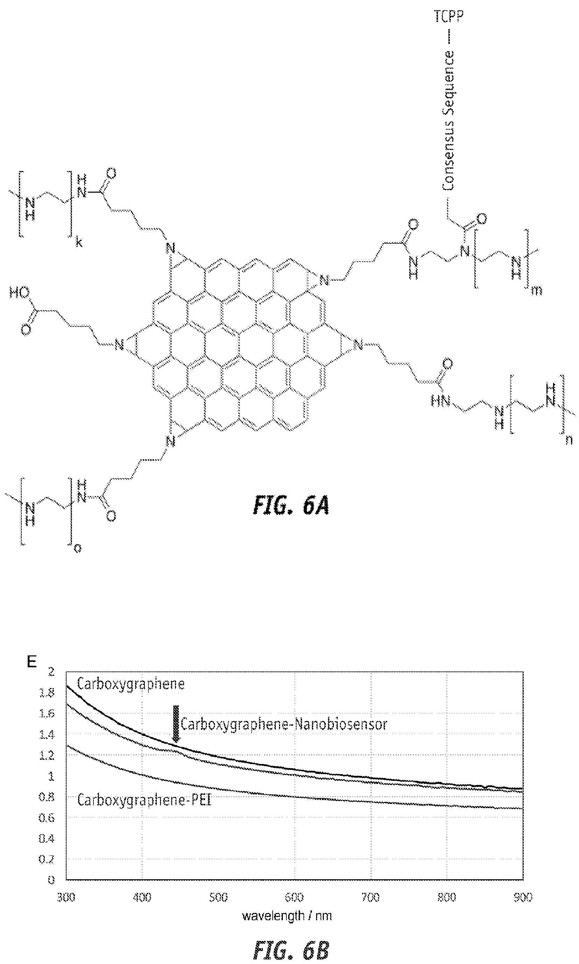

**Figure 6A** shows a structure of an exemplary carboxygraphene-polyethylenimine-based biosensor, in which only a single sheet is shown for reference.
**Figure 6B** shows UV/Vis Spectra of Carboxygraphene (top curve), Carboxygraphene-PEI (bottom curve), and the Carboxygraphene-Biosensor (middle curve) for MMP-1 in water.

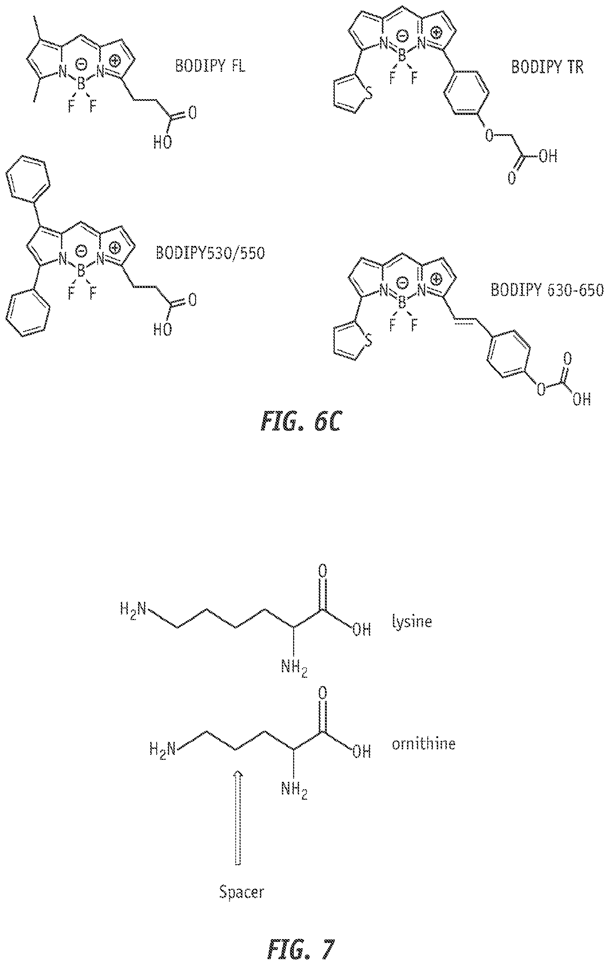

**Figure 6C** shows four exemplary BODIPY dyes with no or only a minimal overlap between the excitation and absorption spectra, and in particular BODIPY FL, BODIPY TR, BODIPY 530/550, and BODIPY 630-650.
**Figure 7** shows a schematic representation of lysine and ornithine as possible spacers for biosensor.

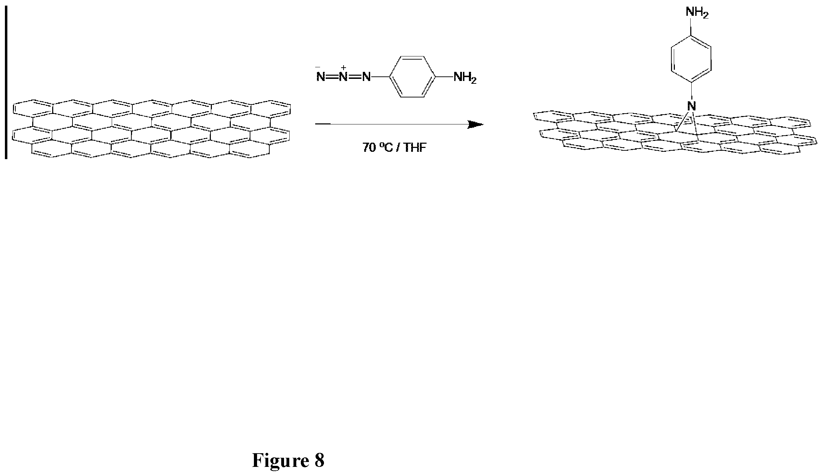

**Figure 8** shows a schematic representation illustrating an aromatic substituted nitrene reaction for chemical amination of a graphene surface.

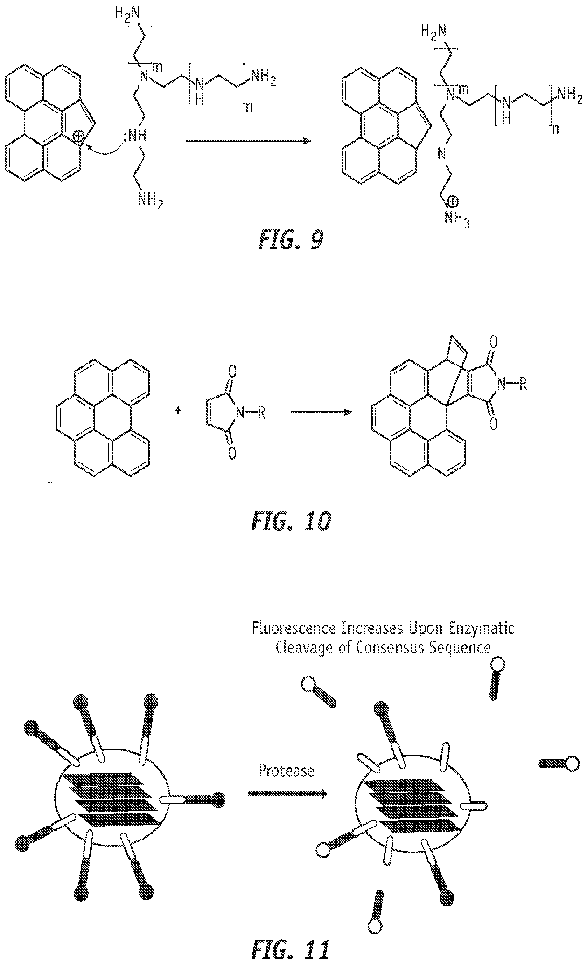

**Figure 9** shows a reaction scheme of graphene-embedded carbocations with secondary amine groups of PEI, leading to the chemical attachment of PEI at the surface of graphene. It is noted that primary amine groups can react as well.
**Figure 10** shows Diels-Alder reaction between a maleimide-derivative and graphene.
**Figure 11** shows a schematic representation of functional principles of graphene-based biosensors of the disclosure used for protease detection.

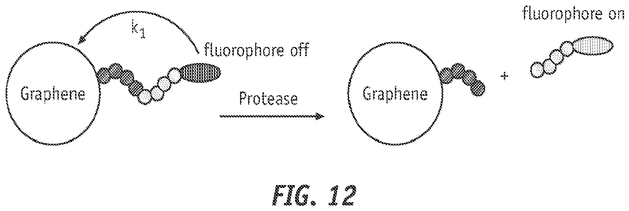

**Figure 12** shows a schematic representation of working principle for an exemplary a graphene-based biosensors configured to detect protease activity.

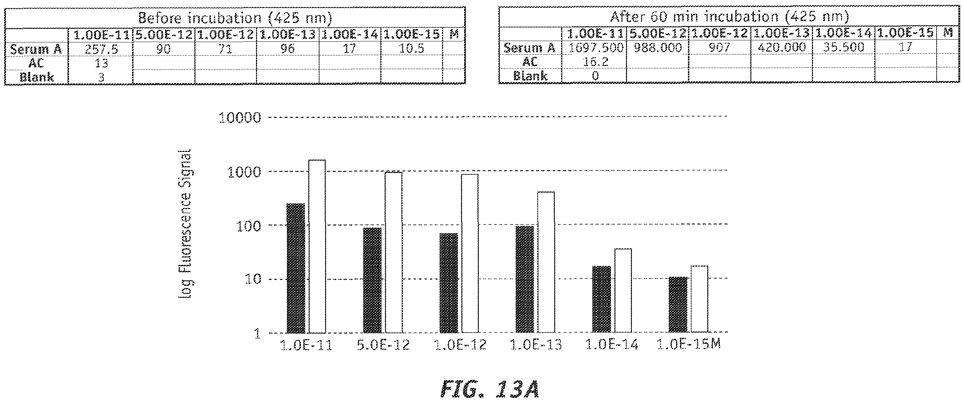

**Figure 13A** shows an estimation of limit of detection (LOD) as indicated by log signal vs. enzyme concentration in an exemplary biosensor of the disclosure; In particular, **Figure 13A** shows a chart in which the left bars of each pair of bars indicates the initial fluorescence signal detected after adding MMP1 in serum before incubation; right bars of each pair of bars show fluorescence signal detected after 60 min. incubation at 37°C. AC (assay control) is the biosensor solution mixed with buffer alone, while blank is the buffer alone; these represent the background signal, or fluorescence signal that is measured from the buffer and biosensors alone.

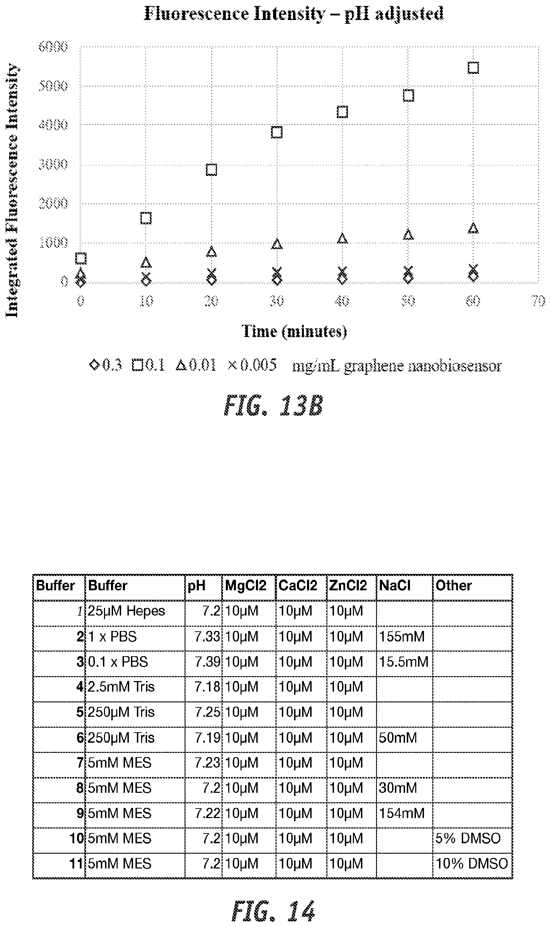

**Figure 13B** shows a chart illustrating the fluorescence signal vs incubation time for different exemplary biosensor concentrations tested. Four different concentrations of graphene biosensor solutions were incubated with serum and measured over 60 min. incubation at 37°C, each concentration is represented with a symbol, indicating that the optimal signal quantified was for biosensor concentrations between 0.01 and 0.1 mg/mL.
**Figure 14** shows a table reporting 11 exemplary buffer formulations for pan gNBS buffering.

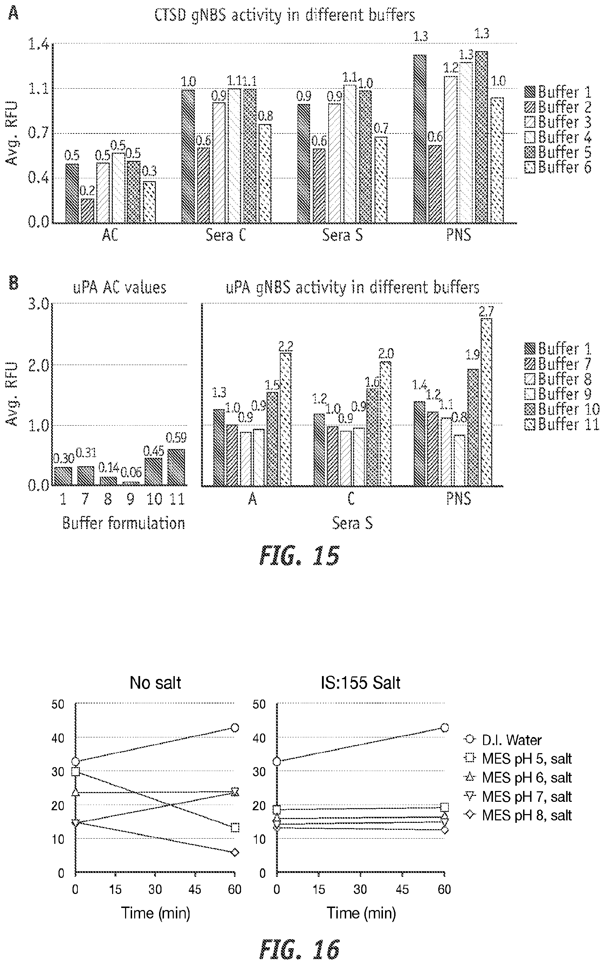

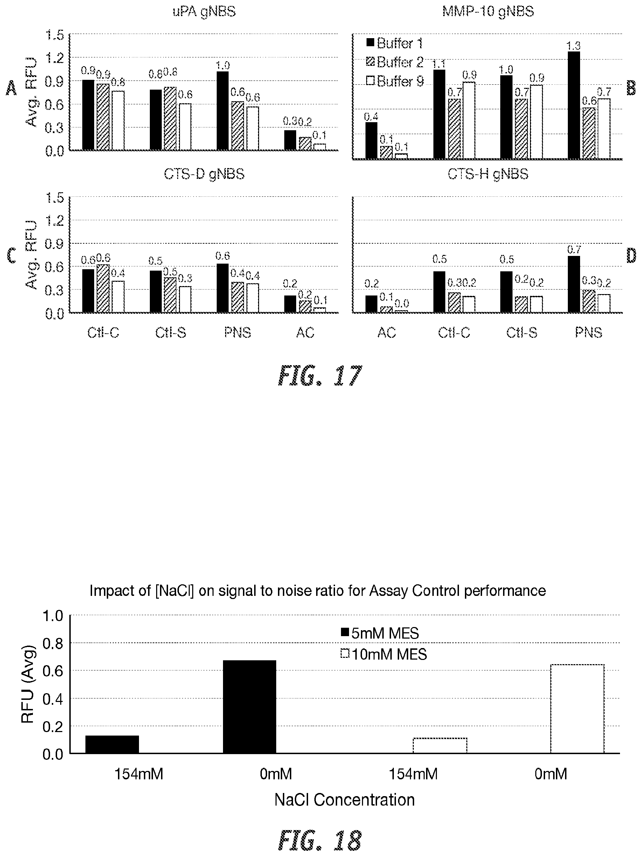

**Figure 15** shows charts reporting results illustrating the performance of Cathepsin D (CTSD gNBS) **(Panel A)** and urokinase (uPA gNBS) **(Panel B)** in the buffers of **Figure 17****.** CTSD and uPA gNBS were suspended at 30µg/ml final concentration in buffers 1-11 of **Figure 14** as indicated. 5µl of sera A, S, or pooled normal sera (PNS) or buffer only (AC) was added in triplicates to the working solution. The fluorescence at 421nm/650nm was measured after incubation at 37°C for 60 minutes.
**Figure 16** shows charts reporting results illustrating the impact of salt on Zeta Potential stability. uPA gNBS was assembled in a working solution of 5mM MES buffer with the indicated pH with **(Panel B)** or without **(Panel A)** 155mM NaCl. A control solution of uPA gNBS was prepared in deionized water.
**Figure 17** shows chart reporting results of experiments illustrating the performance of uPA gNBS **(Panel A),** Matrix Metalloproteinase 10 (MMP10) gNBS **(Panel B),** CTSD gNBS **(Panel C),** and Cathepsin H (CTSH) gNBS(**Panel D**) in isotonic salt buffers. CTSD, uPA, MMP10 and CTSH gNBS were suspended at 30µg/ml final concentration in buffers 1,2 or 9 as indicated. 5µl of sera A, S, or pooled normal sera (PNS) or buffer only (AC) was added in triplicates to the working solution. The fluorescence at 421nm/650nm was measured after incubation at 37°C for 60 minutes.
**Figure 18** shows the impact of NaCl concentration on background noise. uPA gNBS were incubated with 5mM or 10mM MES buffer containing 10µM divalent cations or 154mM NaCl or no salt. The signal obtained (expressed as average RFU) after 90 minutes at 37°C is displayed.

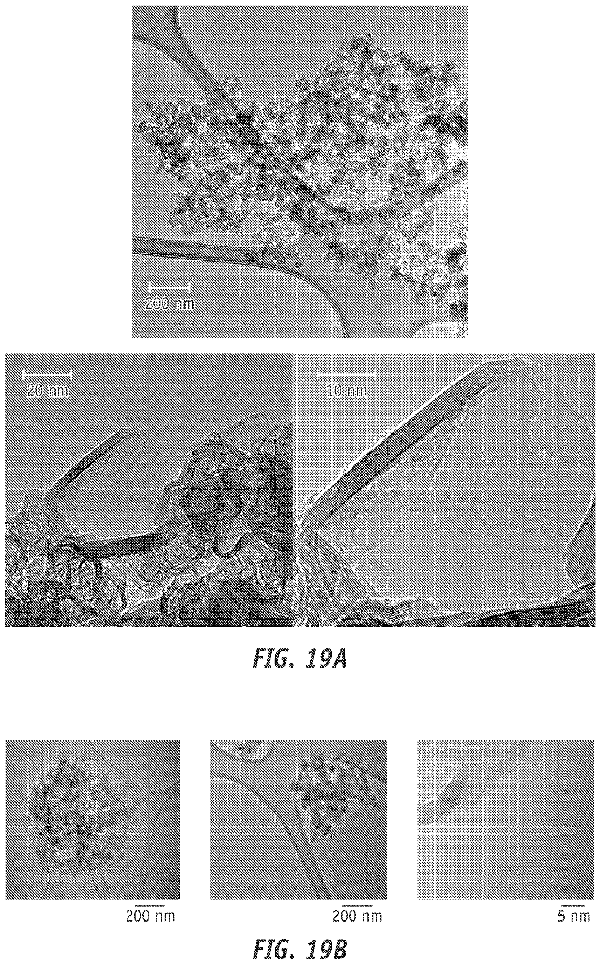

**Figure 19A** shows TEM images of pristine detonation graphene at different magnification scales, in particular, 5 nm: magnification 250,000, 10 nm: magnification 120,000, 20 nm: magnification 70,000, 200nm: magnification 10,000.
**Figure 19B** shows TEM images of graphene biosensor. gNBS were assembled with peptide targets for MMP15. Scanning electron micrographs were collected at 15,000x **(left panel)** and **(middle panel)** 300,000x magnification **(right panel).** Overall size can be seen and the layered structure of the graphene sheets are revealed. Bars indicate 200nm or 5nm.

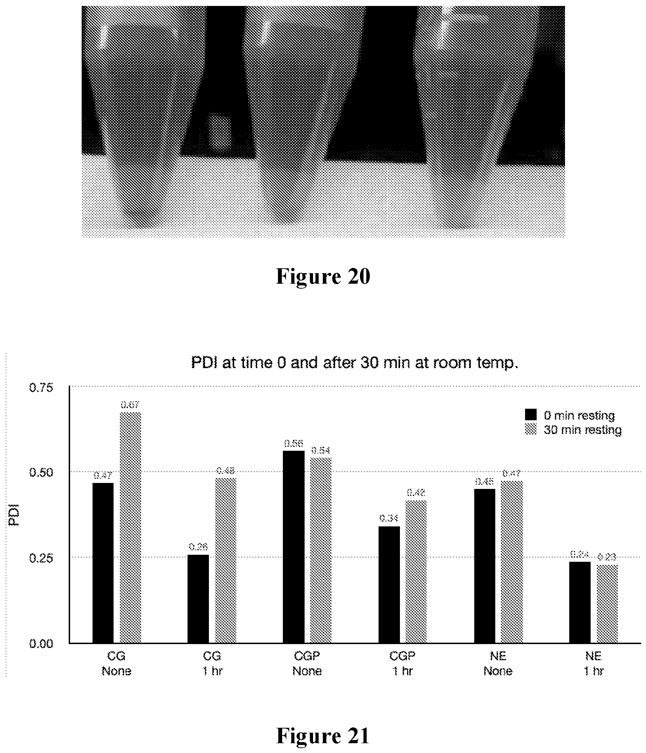

**Figure 20** shows a picture reporting the results of experiment showing the impact of sonication on graphene starting material. The three tubes show samples of KSU Lot 1, HEB100, and HEB101 in order left to right. gNBS were assembled with peptide targets for NE using the original KSU protocol. The only difference was HEB100 was probe sonicated for 10 minutes and HEB 101 was probe sonicated for 60 minutes before assembly of gNBS sensor. After preparation of the gNBS working solution, particles of gNBS can be seen precipitating out of the KSU Lot 1 preparation. A small amount can also be detected in HEB 100. No precipitate is observed in the HEB 101 preparation.
**Figure 21** shows charts reporting the results of experiment showing the impact of sonicating starting material on polydispersity. The graph shows the polydispersity index (PDI) for each formulation when the working solution is first made and dispersed in a sonicating water bath (0 min resting) and then after 30 minutes (30 minutes resting). A significant decrease in the PDI was observed at each stage in manufacture for particles made using the sonicated graphene reflecting a greater uniformity of the particles generated at each stage in the process.

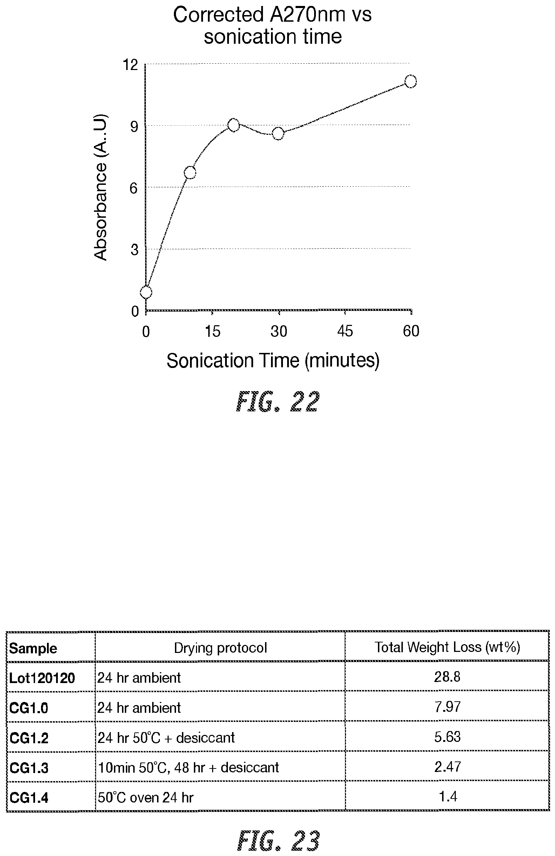

**Figure 22** shows a chart reporting the absorbance at 270nm for graphene with increasing sonication time. A solution of graphene in DMF was probe-sonicated for incremental periods of time. Samples were taken at intervals and the absorbance measured and the related measurement plotted in the chart.
**Figure 23** shows a table reporting the results of Thermogravimetric Analysis (TGA) in terms of total weight loss of original KSU Lot 120120 and CG preparations CG-1, CG-2, CG-3 and CG-4 following different drying protocols.

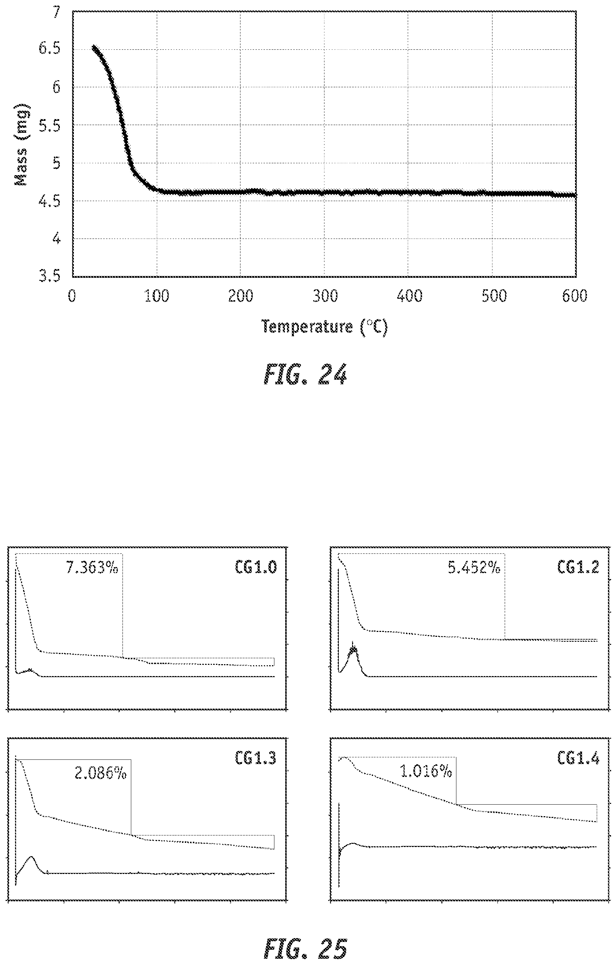

**Figure 24** shows a chart reporting the results of thermogravimetric analysis of a CG preparation featuring 1.6 ± 0.1 x 10⁻⁴ mol/g of carboxylic acid functions, according to titration. Graph shows change in mass as temperature is increased from RT to 600°C. Graphs indicate the mass that is lost during the increase to 100°C. Change in mass after 400°C is minimal.
**Figure 25** shows the results of Thermogravimetric (TG) analysis of CG preparations CG1.1, CG1.2, CG1.3 and CG1.4 of **Figure 23****.** Different preparations of CG were subject to TG analysis. Graphs show change in mass as temperature is increased from RT to 1000°C. Graphs indicate the mass that is lost during the increase to 100°C. Change in mass after 400°C is minimal.

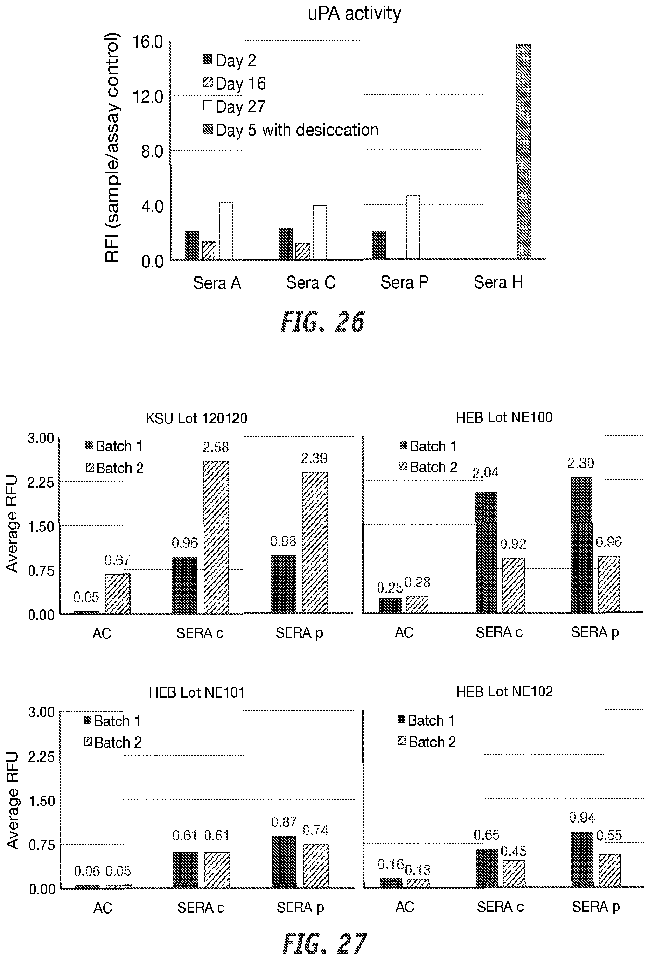

**Figure 26** shows a chart reporting results of experiments showing the impact of removal of solvents on gNBS performance. In particular, uPA gNBS were evaluated in triplicate day 2, 16 or 27 after manufacture using the standard protocol. A separate lot of uPA gNBS were manufactured including ambient drying for 30 minutes and overnight in a 37°C oven. These were evaluated 5 days after manufacture. The chart shows relative fluorescent index calculated by sample/assay control. In the experiments reported in **Figure 26****,** the uPA sensors used, are based on Carboxygraphene/Polyethylenimine.
**Figure 27** shows charts reporting the impact of sonicating starting material on the batch-to-batch variation in signal. Three lots of gNBS for Neutrophil Elastase (NE), HEB Lot NE100, HEB Lot NE 101 and HEB Lot NE 102 were manufactured and compared to the original KSU Lot 120120. gNBS were tested under normal conditions with sera from 2 different normal human donors.

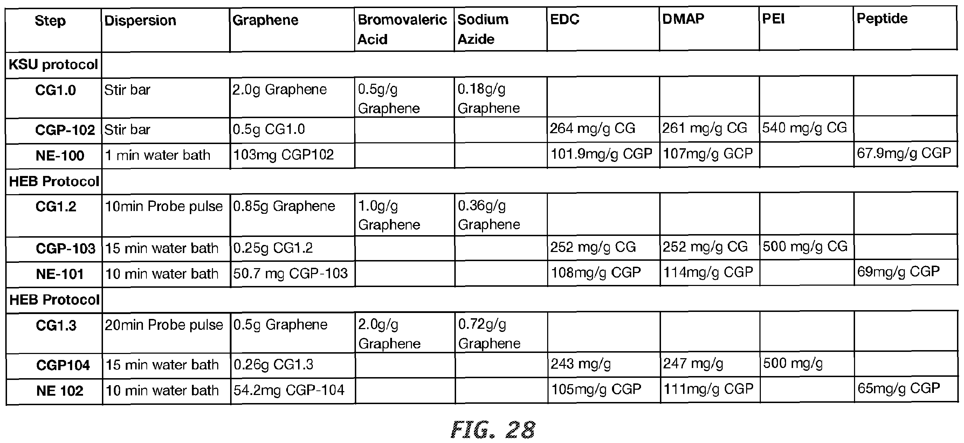

**Figure 28** shows a table reporting the formulation for biosensor manufacture HEB Lot NE-100, HEB Lot NE-101 and HEB Lot NE-102 with increasing dispersion efforts.

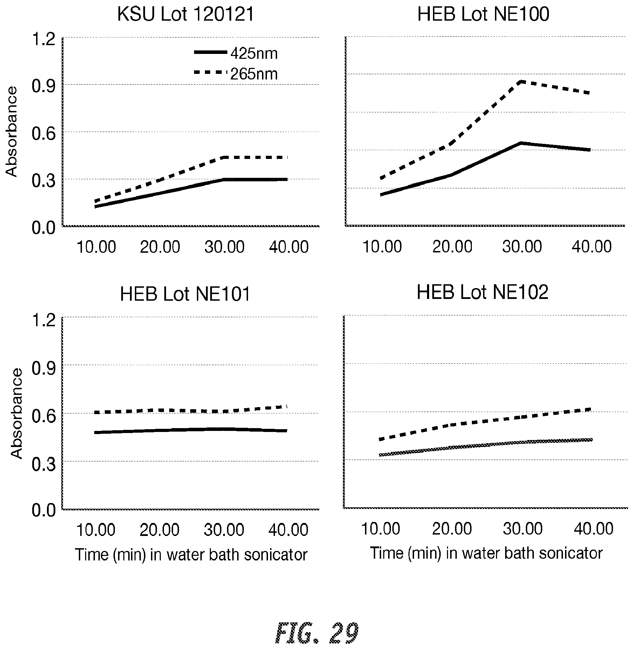

**Figure 29** shows charts reporting results of experiments directed to assess impact of water bath sonication on gNBS absorbance over time. gNBS HEB Lot NE100, HEB Lot NE101 and HEB Lot NE102 and KSU Lot 120120 were resuspended in working buffer and suspended in a sonicating water bath for incremental amounts of time. At intervals, a sample was recovered and the Absorbance at 425nm and 265nm was measured. 4 different Lots of gNBS for NE are shown.

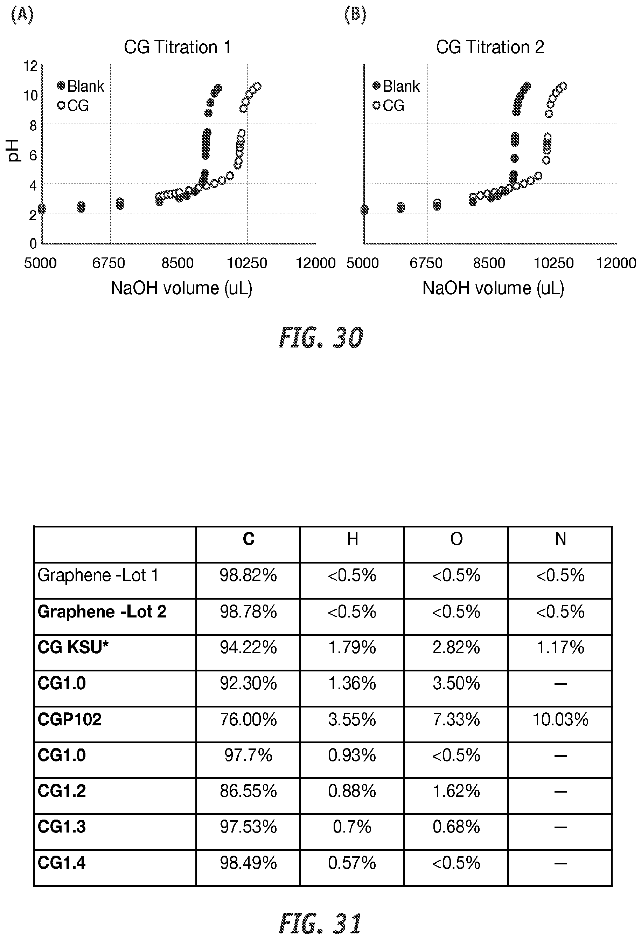

**Figure 30** **panel A** and **Figure 30** **panel B** show charts reporting the results of pH Titration of carboxygraphene. Solution of carboxygraphene prepared using the protocol as described herein was prepared in 50 ml of 0.05M NaOH. After incubation and removal of the CG, the resulting solution was treated with 20mL 0.05M HCl and then neutralized with incremental amounts of 0.05M NaOH and compared to neutralization of a solution of water.
**Figure 31** shows a table reporting results of an Elemental Analysis of different lots of graphene CarboxyGraphene (CG) preparations and CG derivatized with polyethylenimine (CGP) preparations. In particular, two different lots of graphene, 6 different preparations of CG and one preparation of CGP as indicated were subjected to elemental analysis for C, H, O and where results were obtained, N. All results were produced by Galbraith Laboratories other than * data for which were generated by KSU. Results are presented in percent composition. For comparison, CGP102 is PEI derivatized product assembled with CG1.0.

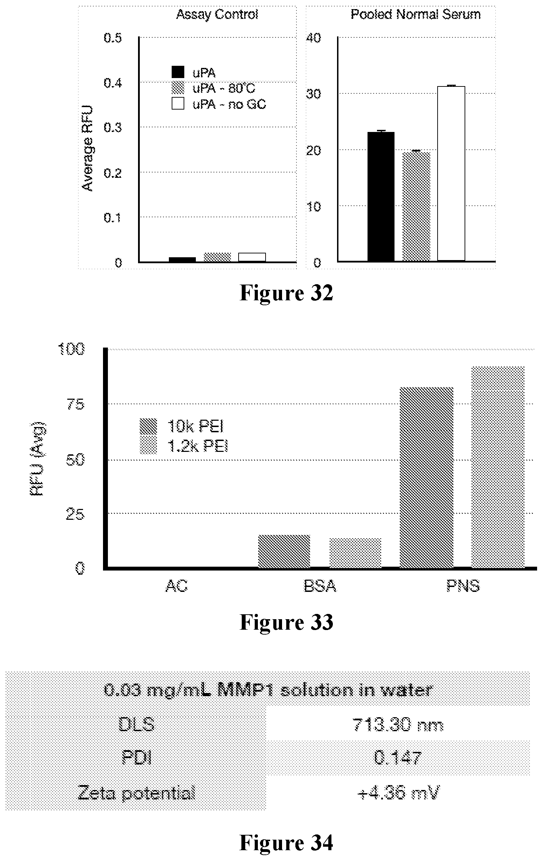

**Figure 32** shows charts reporting the results of experiments directed to the preparation of gNBS without a carboxylation step. uPA gNBS were assembled by attaching PEI to a carboxygraphene backbone (uPA) or directly to a graphene backbone using EDC chemistry, or reacted with PEI without EDC but heating to 80°C. uPA-TCPP was subsequently added to all three backbones and the resulting gNBS stimulated with pooled normal sera (Pool Normal Serum) or buffer (Assay Control) for 90 min at 45°C before being measured on a VarioSkan Lux at 421/653nm.
**Figure 33** shows a chart reporting the results of rapid conjugation of PEI without previous carboxylation. uPA gNBS were prepared by activating 1.2k or 10k PEI with Azidoacetic acid NHS ester and coupling it to graphene at 80°C. Activity of the resulting sensors was assessed by stimulating with buffer, 50mg/mL BSA or pooled normal sera.
**Figure 34** shows a table reporting DLS and zeta potential results for MMP1 G-TEG3amine biosensor.

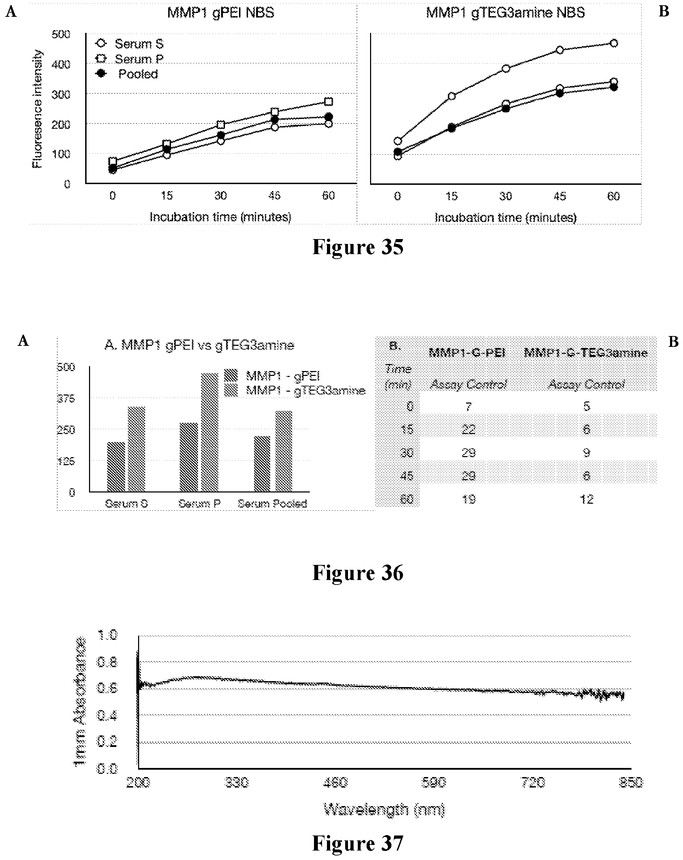

**Figure 35** shows charts reporting results of fluorescence intensity measured for MMP1 G-PEI biosensors **(Panel A)** vs MMP1 G-TEG3amine biosensors **(Panel B).** Fluorescence intensity was measured every 15 min after incubation at 37°C, and these intensities continued increasing as time passed, following almost a linear trend for both. Comparing both biosensors, G-TEG3 amine gave a higher intensity after the 60 min incubation compared to G-PEI.
**Figure 36** shows charts reporting results of fluorescence intensity after 60 min incubation for G-PEI vs G-TEG3amine and assay control. After 60 min incubation **(Panel A),** intensity for G-TEG3amine gave the highest intensity compared to G-PEI showing that for control serum S and P, intensity for G-TEG3amine was almost twice as high compared to G-PEI. **(Panel B)** Fluorescence intensity measured for assay controls for MMP1 G-PEI vs G-TEG3amine biosensors.
**Figure 37** shows a chart reporting the results of absorbance over the visible spectrum of a solution of Neutrophil Elastase gNBS.

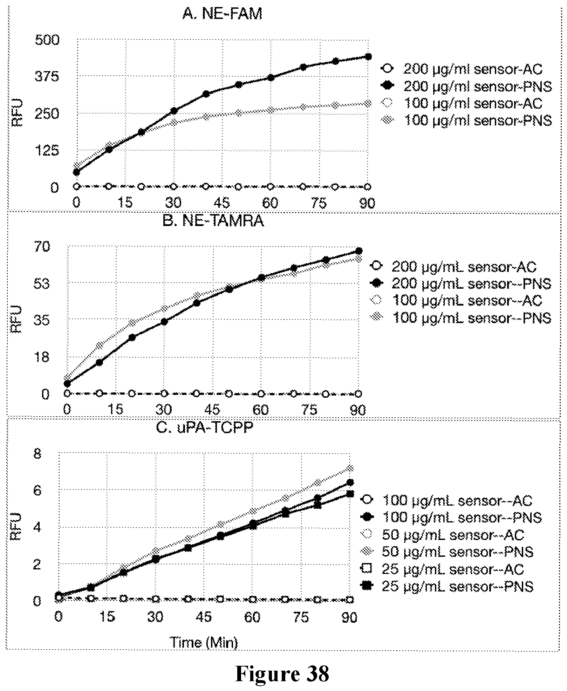

**Figure 38** shows charts reporting the results of experiments showing quenching of multiple different dyes by graphene. gNBS were prepared by coupling NE-FAM **(Panel A),** NE-TAMRA **(Panel B),** or uPA-TCPP **(Panel C)** to sonicated graphene using the standard procedures. Dose responses of each sensor in a 70µL volume were stimulated in triplicate with buffer alone (AC) or with 10µl serum in a 384 well plate. The plates were incubated at 45°C and fluorescence was read at 10 minutes intervals for 90 min.

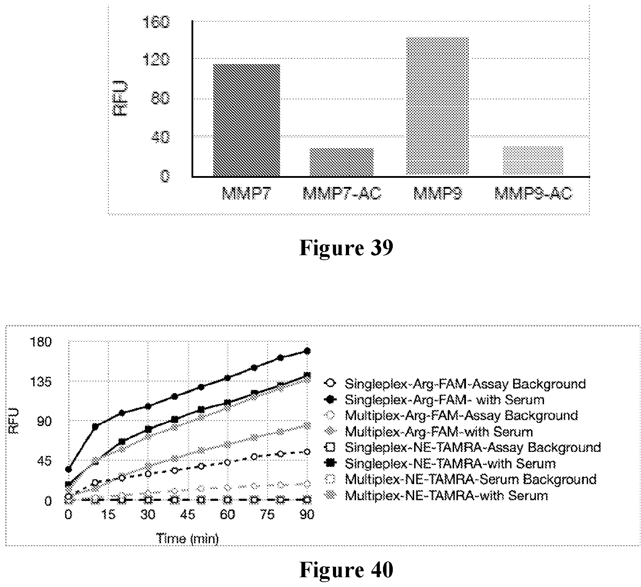

**Figure 39** shows a chart reporting the results of experiments showing quenching of Rhodamine-B by graphene with Rhodamine-B gNBS. MMP7 and MMP9 RhodamineB peptide were coupled to CGP and incubated in the presence of sera or buffer only (AC) for 60 minutes.
**Figure 40** shows a chart reporting results of experiments with multiplex gNBS. gNBS sensors were prepared by coupling Arg-FAM or NE-TAMRA as indicated to sonicated graphene using the standard procedures. 70µl working solutions were prepared with 200µg/ml final concentration of each sensor alone (Singleplex) or both together (Multiplex). Triplicate wells were treated with 10µl buffer or 10µl serum and incubated for 90 minutes at 45°C with measurements taken at 10-minute intervals. Each well was measured at 496/526 (FAM) and 555/586 (TAMRA).

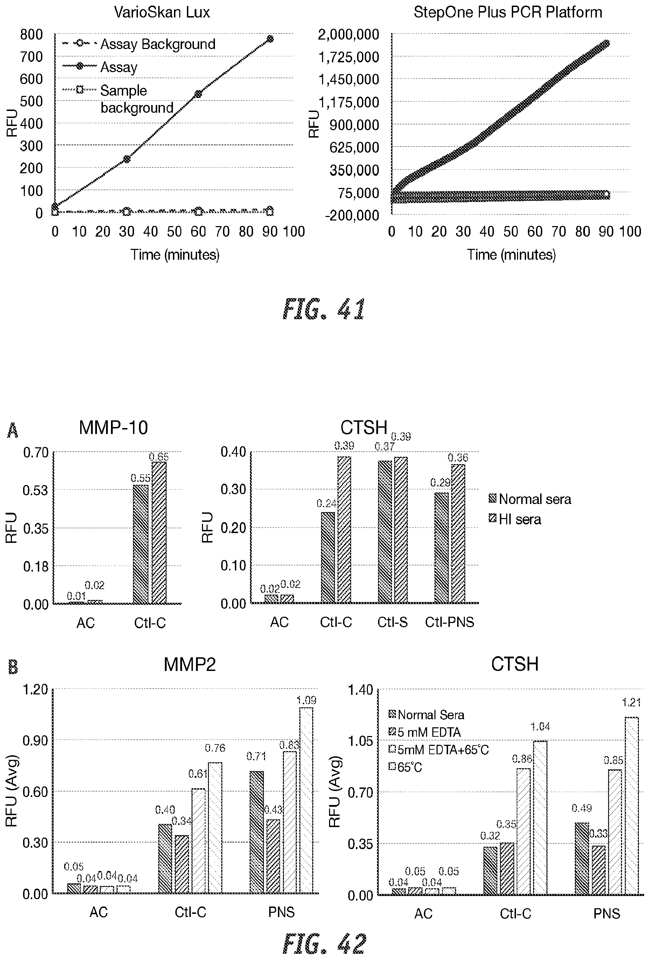

**Figure 41** shows charts reporting results of nanosensor analysis on multiple different platforms. gNBS sensors were prepared by coupling NE-FAM to sonicated graphene using the standard procedures. 125µl working solutions were prepared with 200µg/ml of sensor. Triplicate wells were treated with 5µl buffer (AC) or 5µl serum. An additional well diluting 5µl serum into 125µl of buffer was also prepared (sample background). Each well was measured at 496/526 (FAM) at 30-minute intervals on the Varioskan and 1 minute intervals on the StepOnePlus.
**Figure 42** shows results of experiments directed to detect proteases following heat treatment **(Panel A)** Control sera and pooled normal control sera were used to stimulate MMP10 gNBS and CTSH gNBS in a 125µl working solution of 5mM MES, 10µM cations and IS: 155. An aliquot of each serum was incubated at 55°C for 60 minutes and used to stimulate replicate reactions. Both sets of reactions were incubated at 37°C for 1 hour and the fluorescence measured on a Varioskan Lux. **(Panel B)** MMP2 gNBS and CTSH gNBS were stimulated in a 125µl working solution of 5mM MES, 10µM cations and IS: 155. They were stimulated with 5µl buffer (AC) or in triplicated with 5µl control serum C or pooled normal sera (PNS). Sera were alternatively treated in advance with 65°C for 10 min, EDTA such that the final concentration was 5mM or both. The resulting activity reported in RFU are shown for each sensor.

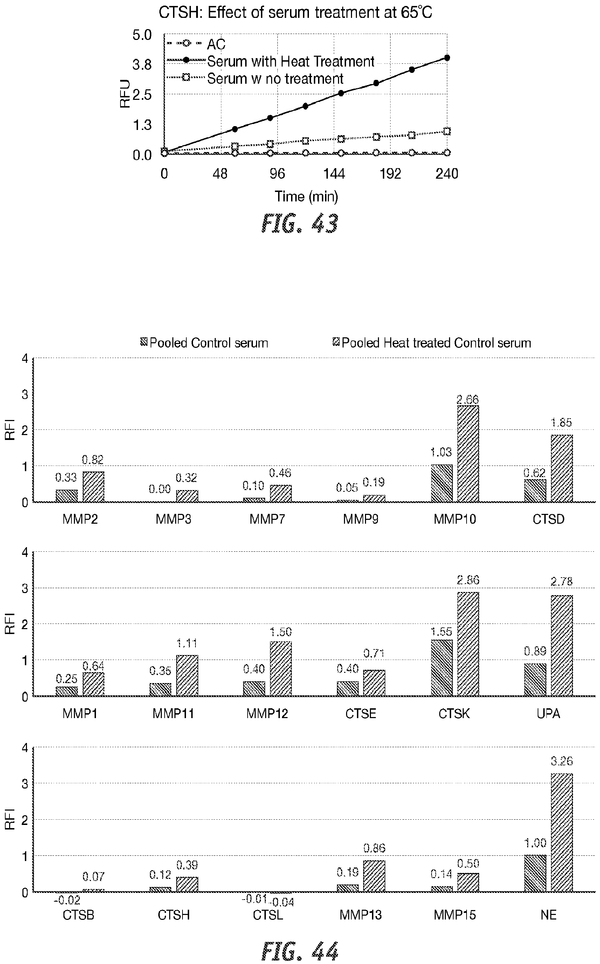

**Figure 43** shows a chart reporting results of experiments including heat pretreatment of sera. CTSH gNBS were stimulated in a 125µl working solution of 5mM MES, 10µM cations and IS: 155. They were stimulated with 5µl buffer (AC) or in triplicate with 5µl PNS that was either previously heat treated at 65°C or not. The activity of CTSH gNBS was monitored in a 37°C incubation at 30 minutes intervals for 6 hours. The chart shows that heat pretreatment results in linear activity.
**Figure 44** shows a chart reporting results of experiments including heat pretreatment of sera. Eighteen gNBS were prepared in a 125µl working solution of 5mM MES, 10µM cations and IS: 155. They were stimulated with 5µl buffer (AC) or in triplicate with 5µl PNS that was either previously heat treated at 65°C or not. The activity of gNBS was measured after 37°C incubation for 90 minutes. Results are presented as RFI where the sample RFU is divided by the assay control (RFI = SM-(AC+SC). The chart shows that heat pretreatment results in increased activity across the sensor panel.

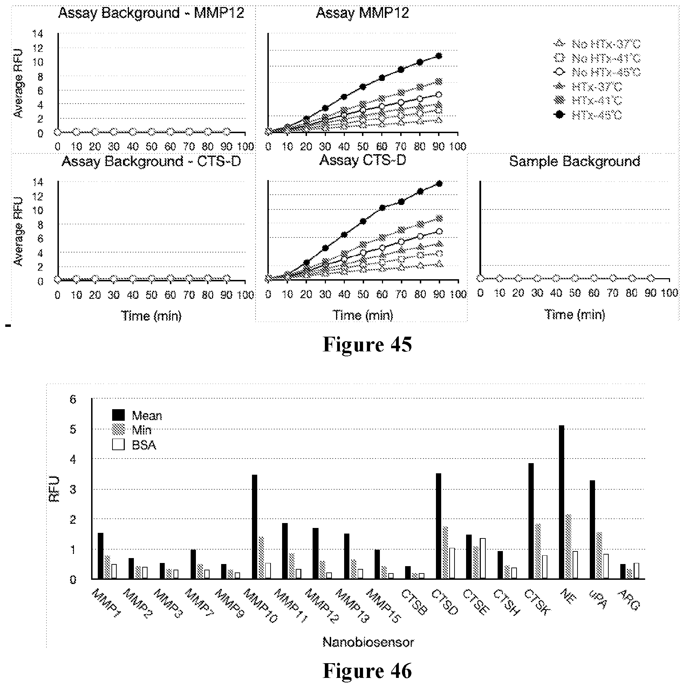

**Figure 45** shows charts reporting results of assays performed at different temperatures. 125µL MMP12 and CTSD gNBS were stimulated with 5µl buffer (Assay background graphs) or in triplicate with 5µl PNS and the assay was incubated at 37, 41 or 45°C (open symbols) in the VarioSkan Lux. Alternatively, serum was heat treated at 65°C for 12 minutes and then 5µL was added in triplicate to 125µL MMP12 or CTSD gNBS in standard MES buffer. Fluorescence was measured at 10 minute intervals for the duration of the incubation. Sample background measurements showed the results from wells that were incubated with 125µL of buffer with no sensor but with 5µL of sera added. The charts show that elevated temperature of incubation mimics heat treatment of serum.
**Figure 46** shows a chart reporting detection of the protein corona of gNBS specific for 18 different target proteases as indicated in the chart. The mean and minimum signal observed across 175 human samples was compared to a 20mg/ml acetylated BSA solution. The graph shows the mean and minimum fluorescent readouts for 175 samples across all 18 sensors.

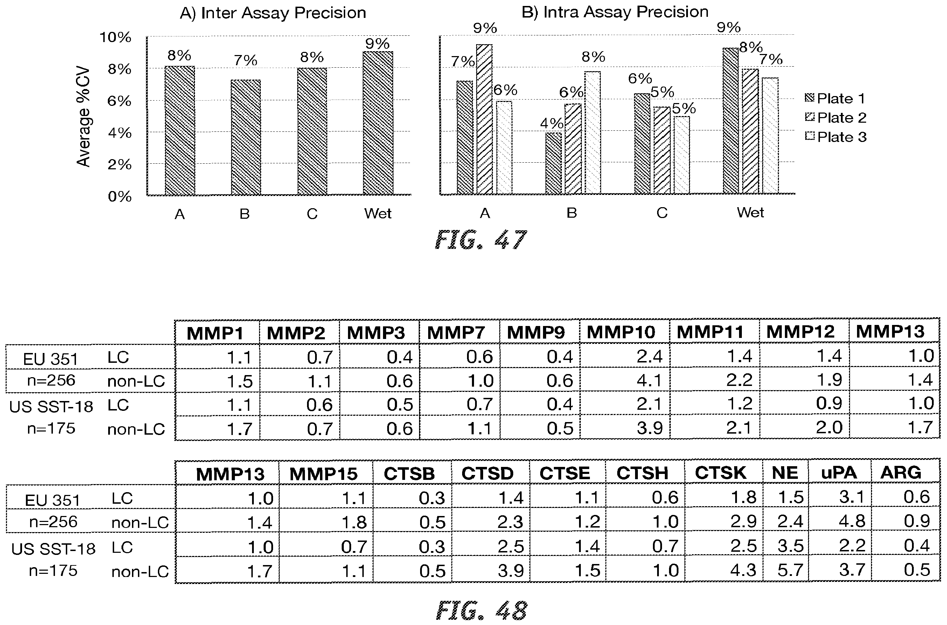

**Figure 47** shows charts reporting results of experiments directed to detect precision of lyophilized gNBS reagents. A selection of gNBS were lyophilized in 384 well plates in one of three different excipient formulations: A, B, or C. Each formulation was compared to the wet reagents prepared from the same batch but not lyophilized. **(Panel A)** Inter Assay Precision: 3 384-well plates tested over 3 separate days, 256 total serum samples and 4 NBS per plate. 64 serum samples were tested per excipient on each plate. %CV measured by averaging the quotient of the sample standard deviation of means and the mean of means for each excipient. **(Panel B)** Intra Assay Precision: 64 serum samples and 4 NBS tested per excipient. Precision measured from the average %CV of each excipient within a 384-well plate run.
**Figure 48** shows tables reporting the average RFU for each NBS assessed on 256 or 175 patients separated into lung cancer and non-lung cancer groups. Two studies referred to as EU 351 and US SST-18 which interrogated 207 and 175 total patients respectively are presented. The numbers indicated the mean RFU produced by each gNBS for the indicated groups. Subjects in each group are separated into pathologically confirmed lung cancer cases or clinically confirmed negative lung cancer cases. All cases are untreated patients.

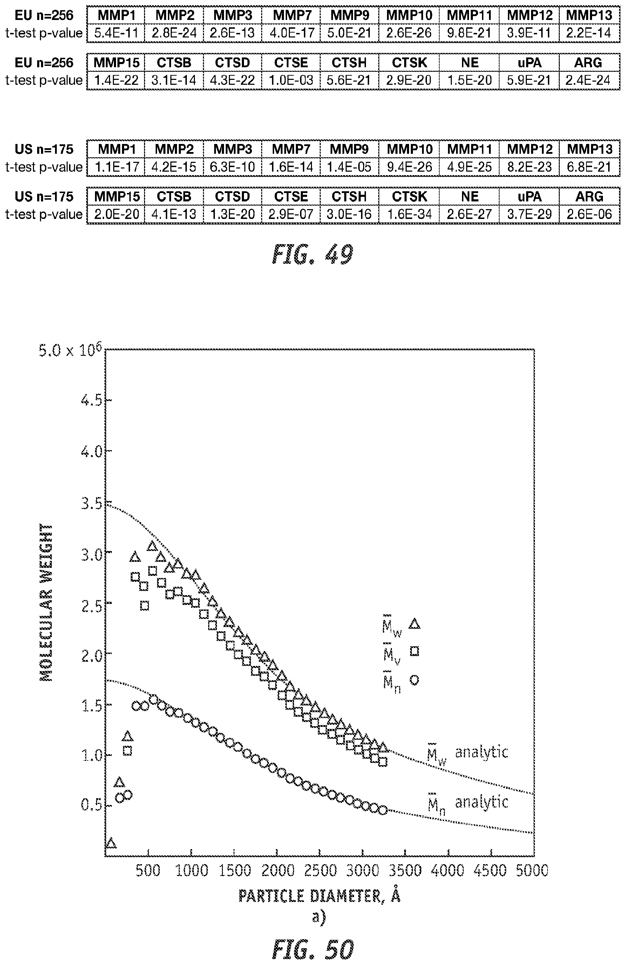

**Figure 49** shows a table of p-values for student's two-tailed, heteroscedastic t-Test comparing RFU of Lung Cancer and non-Lung Cancer samples.
**Figure 50** shows a plot of molecular weight (Da) vs. particle diameter (Ångström). Mₙ: number averaged macromolecular weight, M_{w}: weight averaged macromolecular weight, Mᵥ: average macromolecular mass (corresponding to Figure 1 of Loebach 1975 [2]).

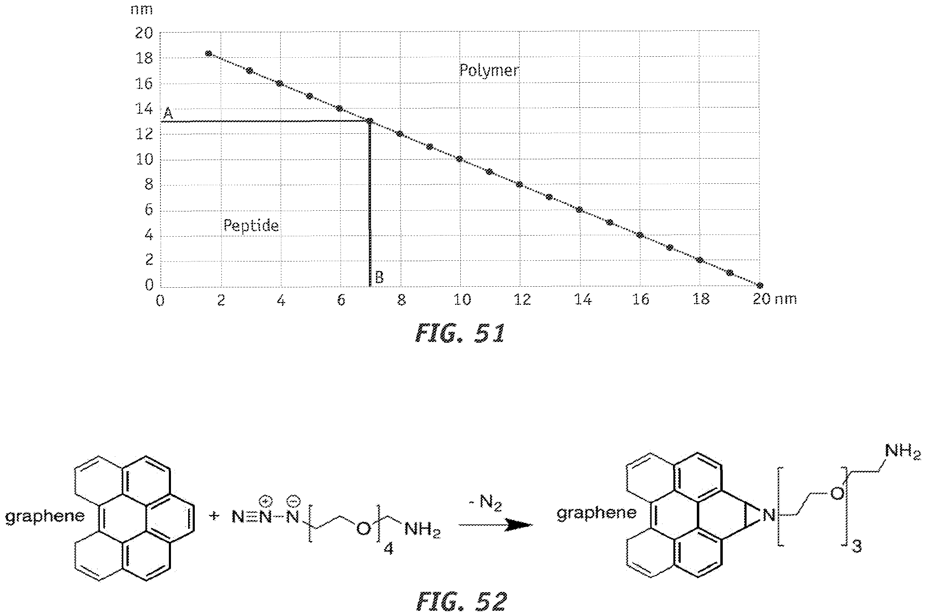

**Figure 51** shows allowable ratio of peptide length (A) and polymer layer thickness (B) in graphene biosensors for protease activity detection.
**Figure 52** shows an exemplary monolayer of water-soluble organic molecules of oligoethylene glycols and the related reaction scheme for related attachment to the surface of graphene

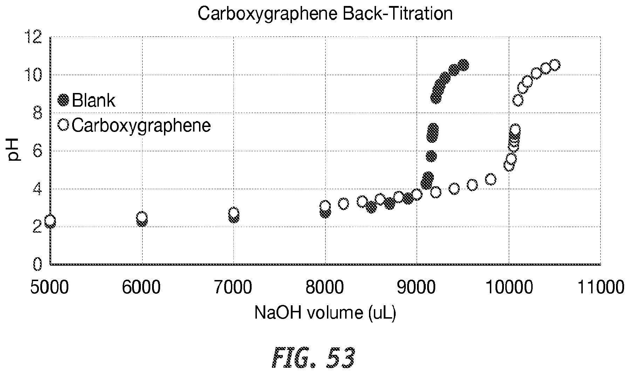

**Figure 53** shows a pH Titration of carboxygraphene in which difference in the volumes of NaOH in the two titration curves for the same value of pH of ~7.00 gives the concentration of the ionized groups (carboxyl groups) per weight increment of Carboxy graphene.

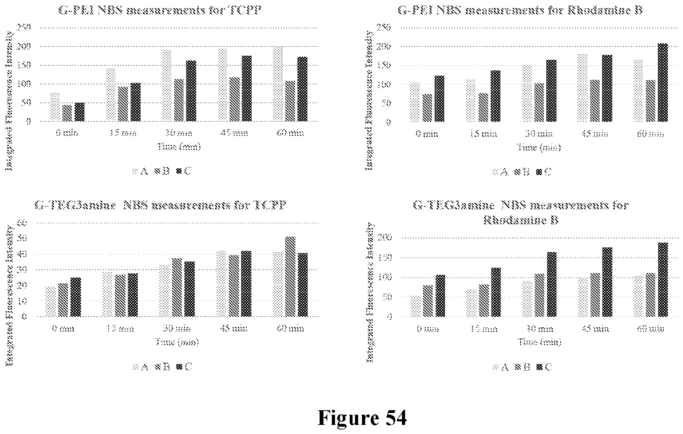

**Figure 54** shows charts illustrating the results of experiments showing detection of target biomarker MMP-9 in 3 different control serum samples with core particle G-PEI and G-TEG3amine presenting different cleavable peptide sequences labeled with two different dyes, in particular MMP-9 (GAGVPLS-LYSGAG)(SEQ ID NO: 132) labeled with TCPP and a MMP-9 (GCDDHAAG-LLGLDG) (SEQ ID NO: 133) labeled with rhodamine B. The fluorescent intensity was measured over time as indicated in the three control samples (marked as A B and C in each chart).

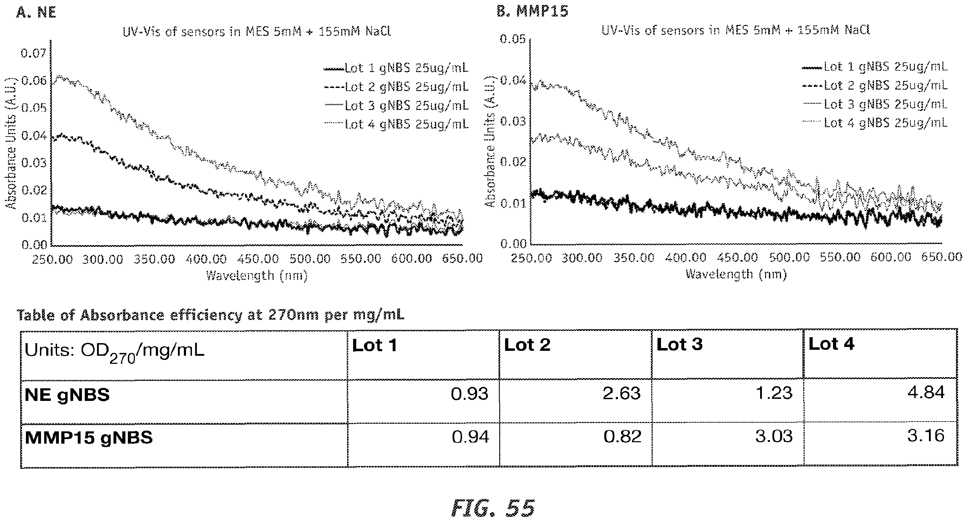

**Figure 55** shows charts illustrating an example impact of dispersal and solvent removal on absorbance efficiency of gNBS. Working solutions of gNBS were prepared in 5mM MES pH 7.4 with 155mM NaCl at a concentration of 25µg/mL. The absorption across 250nm to 650nm was measured for each lot reflecting two lots with no sonication (Lots 1 and 2) and two lots prepared with 1hr sonication (Lots 3 and 4) and solvent removal (Lot 4). Data are presented as spectra or absorbance efficiency at OD270. In particular, **Figure 55****, Panel** A indicates the change in absorbance efficiency for NE biosensor across 250nm to 650nm. **Figure 55****, Panel B** shows the change in absorbance efficiency for MMP15 biosensor across 250nm to 650nm. The table highlights the increase in absorbance efficiency at OD₂₇₀ across 4 lots of each biosensor.

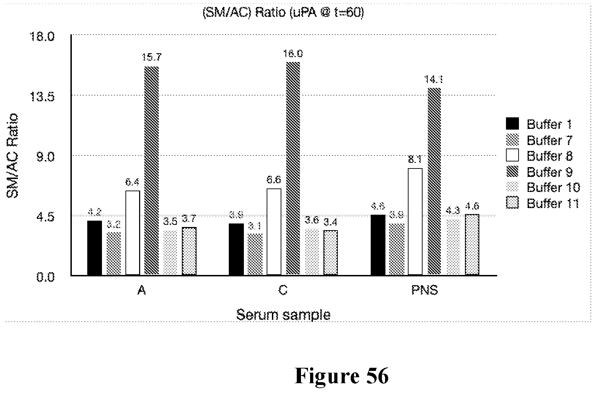

**Figure 56** shows the impact of buffer formulation on Signal to Noise performance. Working solutions of gNBS were prepared in buffers as detailed in **Figure 14****.** Signal to noise was calculated based on the ratio of fluorescence observed in samples/assay control (RFI). Buffer 7 containing MES only performed similarly to Buffer 1 (Hepes). Addition of 30mM NaCl (Buffer 8) or 155mM NaCl (Buffer 9) increases RFI.

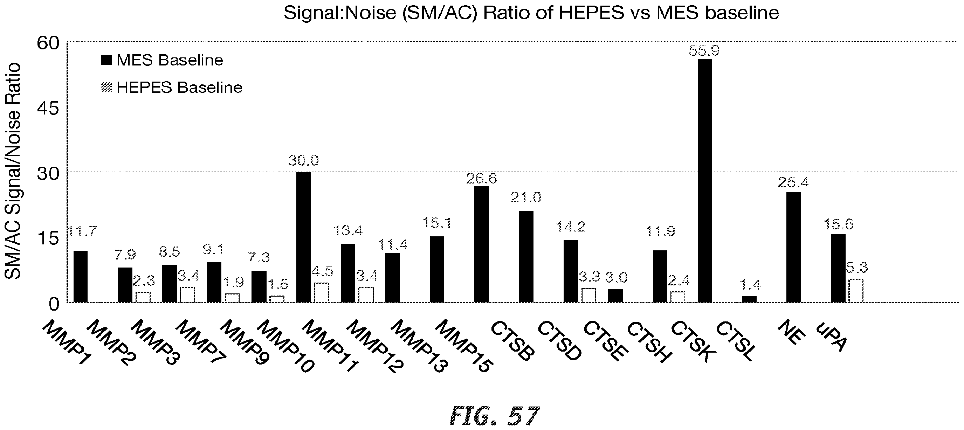

**Figure 57** shows a chart illustrating an exemplary impact of buffer formulation and heat treatment on Signal to Noise performance. Working solutions of gNBS were prepared in HEPES or MES buffer supplemented with 10µM divalent cations and 155mM NaCl. Samples were heat treated at 65°C for 12 min (MES Baseline) or not (HEPES Baseline) and incubated for 90min at 37 °C.

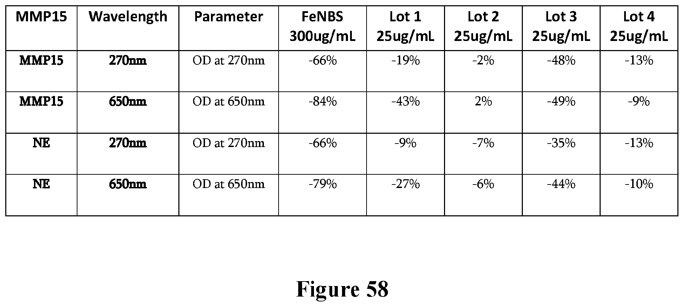

**Figure 58** shows charts illustrating exemplary colloidal stability of gNBS sensors. Working solutions of gNBS were prepared in 5mM MES pH 7.4 with 155mM NaCl at 25µg/mL. The absorption at 250nm to 650nm was measured for each lot upon vortexing the solution. The samples were left to sit for 1 hour at room temperature. A second sample was measured after 60min. The data represent the fold change in Absorbance from time 0. The different samples reflect two gNBS lots with no sonication (Lots 1 and 2) and two lots prepared with 1hr sonication (Lots 3 and 4) and solvent removal (Lot 4). FeNBS (300pg/mL) are included for comparison. Lot 2 had large gNBS particles that did not remain in suspension at all underestimating the colloidal stability.

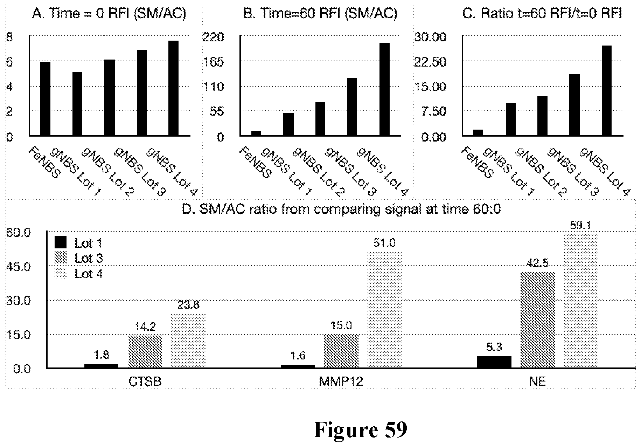

**Figure 59** shows charts illustrating an exemplary evaluation of signal to noise across different manufacturing protocols for gNBS. CTSB was manufactured using the original Iron backbone (FeNBS), using a graphene backbone and the original KSU protocol (gNBS Lot 1), a replicate of the KSU protocol using extended drying procedures (gNBS Lot 2), using probe sonicated gNBS backbone (gNBS Lot 3), and probe sonicated gNBS backbone thoroughly desiccated for solvent removal on manufacture (gNBS Lot 4). The graphs show Signal:Noise as measured by RFI (SM/AC) at time 0 (A), Signal:Noise after 60 min incubation with pooled normal serum (B.) and the Signal:Noise expressed as a ratio of RFI 60/RFI 0 (C). Similar observations were made for CTSB, MMP12 and NE in separate experiments (D).

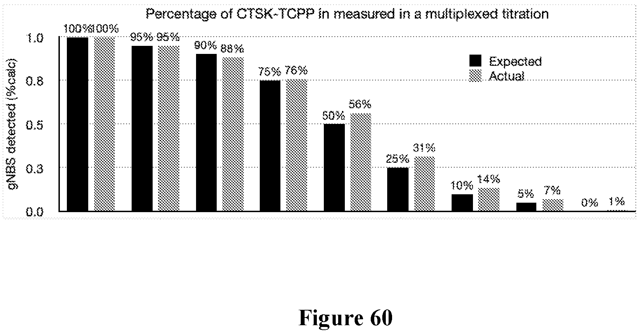

**Figure 60** shows a chart illustrating the results of detection of gNBS in multiplexed setting. Signal is presented as a percentage of the signal obtained at 100%.

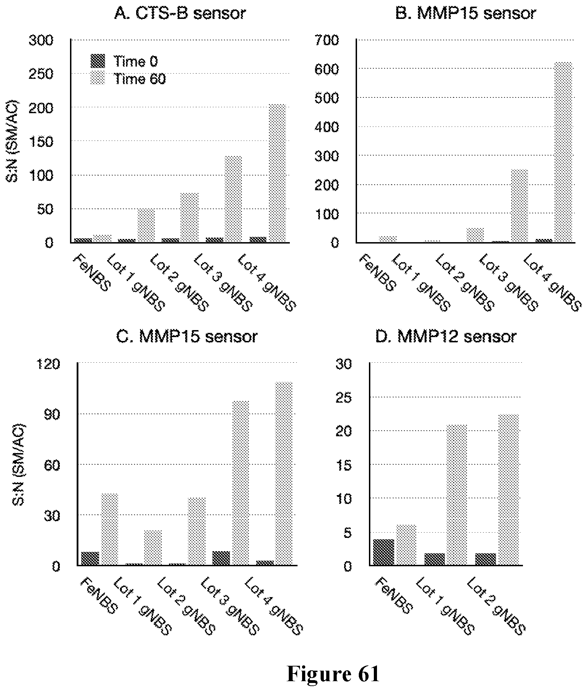

**Figure 61** shows charts illustrating an exemplary evaluation of signal to noise across different manufacturing protocols for Fe and graphene biosensor. Biosensor was manufactured using the original Iron backbone (FeNBS), using a graphene backbone and the original KSU protocol (gNBS Lot 1), a replicate of the KSU protocol using extended drying procedures (gNBS Lot 2), using probe sonicated gNBS backbone (gNBS Lot 3), and probe sonicated gNBS backbone thoroughly desiccated for solvent removal on manufacture (gNBS Lot 4). The graphs show Signal:Noise as measured by RFI (SM/AC) at time 0 and after 60 min incubation with two different normal human sera averaged. Four exemplary biosensors are displayed.

### DETAILED DESCRIPTION

The present disclosure relates to graphene-based biosensors and related core particles, materials, compositions, methods and systems for assays for noninvasive detection and quantification of various biological markers.

The term "graphene" as used herein indicates an allotrope of carbon consisting of a single layer of atoms arranged in a two-dimensional honeycomb lattice nanostructure. The name is derived from "graphite" and the suffix -ene, reflecting the fact that the graphite allotrope of carbon contains numerous double bonds. In particular, graphene refers in the sense of the disclosure to a single layer of at least 1000 carbon atoms in sp² hybridization that are arranged in a hexagonal manner. A graphene aggregate or graphene particle as used herein exchangeable, refers to at least two layers of graphene stacked over one another.

Accordingly, graphene in the sense of the disclosure comprises at least 98% carbon atoms organized in sheets. In a graphene sheet each atom is connected to its three nearest neighbors by a σ-bond, and contributes one electron to a conduction band that extends over the whole sheet. ([3]) (see schematics and properties discussed in **Example 1** and **Example 2).**

In graphene in the sense of the disclosure, a typical apparent static contact angle θ* of a typical graphene surface is θ* = 150 ± 15°, which is borderline super-hydrophobic (θ* ≥150°). Based on this finding and the fact that graphene consists mostly only of carbon (at least 99 ± 1%), it is well established that graphene is among the most hydrophobic materials known. Therefore, its dispersibility in water is < 1 x 10⁻⁸ mg per milliliter [4].

Graphene absorbs light in the UV, visible and near-IR region with absorption coefficients that exceeds graphite[5]. Accordingly, graphene in the sense of the disclosure is a material configured to absorb light of all visible wavelengths. ([3]) Stacked graphene features light absorption over the entire wavelength range from far UV to mid-IR (100 to 1500 nm) [6]. For explosion graphene (5-7 layers), we found absorption coefficients alpha > 5,000 ml mg⁻¹ m⁻¹ at 660 nm, which is in excellent agreement with the literature [7]. In general, graphene from various sources has absorption coefficients alpha from 1,000 to 5,000 at 660 nm.

In graphene in the sense of the disclosure, direct excitation occurs at deep/far ultraviolet, and the surface plasmons of graphene in the visible range are of various intensities. Direct excitation and surface plasmon of graphene are not capable of effectively exciting most detectable moieties capable of emitting a fluorescent signal in an excited state (such as fluorescent dyes or particles). This indication applies to the entire visible range of the electromagnetic spectrum (400 to 730 nm) as will be understood by a skilled person. [6]

Graphene in the sense of the disclosure comprises pristine graphene (graphene in its original, pure, unoxidized form) as well as graphene in an oxidized or surface-modified form, (surface-modified) graphene and additional graphene forms identifiable by a skilled person.

Pristine graphene can be identified by techniques identifiable by a skilled person such as XRD (layer distance), TEM and SEM (morphology), RAMAN (layer distance and turbostratic properties, if present), and FTIR (lack of signature organic functional groups, with the exception of carbon-carbon double bond and aromatic overtones). In particular, FTIR and RAMAN spectra can be used to identify pristine graphene over surface-only oxidized or chemically modified graphene as the related spectra will change in view of the oxidation/chemical modification of the graphene surface as will understood by a skilled person. XRD, TEM, SEM, RAMAN, and FTIR can be used to identify pristine graphene over oxidation/chemical modification that destroys the layer structure of graphene, in view of the changes in the related spectra also identifiable by a skilled person. Additionally, in all cases elementary analysis will confirm that the graphene material is > 98% carbon as will be understood by a skilled person.

In embodiments herein described pristine graphene in the sense of the disclosure can be used as a component of a graphene core particle herein described in which more than one pristine graphene sheet forms a graphene particle which is coated with a coating material configured for attachment of a detectable component specific for a target biomarker.

The term "particle" or "particulate" as used herein indicates a portion of matter with distinct physical or chemical properties, such as size, volume, density, or mass. Exemplary particles of various materials are described in U.S. Provisional Patent Application Serial No. 63/132,058, filed December 30, 2020, entitled "Optical Nanobiosensors for Rapid Identification of Lung Conditions" filed on December 30, 2020 and to corresponding PCT application NoPCT/US2021/065584 entitled "Nanosensors For Rapid Identification of Lung Conditions" filed on December 29, 2021 with docket number P2685-PCT.

Particle in the sense of the disclosure have a size from 1 to 5000 nanometers. In particular, a particle in the sense of the disclosure can have a size from 1 to 250 nanometer, and size from 50 to 150 nm in a nanoscopic mesoscopic/system. Size as used herein is a maximum physical length of any two points of a particle. In a particle in the sense of the disclosure, the size of the particle refers to the longest distance between any two points of the particle. For example, an irregularly shaped graphene particle has a size that is the longest between any two carbons on the irregularly shaped graphene particle. In a spherically shaped particle, the size of the particle is the diameter of the spherically shaped particle.

**.** In a graphene particle of the disclosure the mass distribution of the graphene particles can be detected by centrifugation and measurement of the time required for sedimentation and additional techniques identifiable by a skilled person. The actual size of the graphene particles in air (or N₂, Ar) and a suitable hydrophobic solvent (e.g. chloroform) can be determined by means of Dynamic Light Scattering (DLS). The surface charge of the particles can be determined by means of Zeta Potential Measurements.

A graphene particle can have layers arranged in AB, or Bernal-stacked form, where half of the atoms lie directly over the center of a hexagon in the lower graphene sheet, and half of the atoms lie over an atom. A graphene particle can also have layers arranged in the AA form, in which the layers are exactly aligned in which each carbon atom in one layer is disposed exactly over another carbon atom of an adjacent layer of graphene. In turbostratic particles (AA and AB stacking) the individual layers can move with respect to each other as will be understood by a skilled person.

**In** a graphene core particle of the present disclosure comprises pristine graphene sheets aggregated via van der Waals stacking in configurations comprising more than one graphene layer.

**A** "sheet" or "layer" in the sense of the disclosure indicates a two-dimensional nanostructure with thickness in a scale ranging from 1 to 150 nm. A single layer of carbon atoms with hexagonal lattices of a graphene material is 0.34 nm as will be understood by a skilled person. Since a single layer of graphene absorbs 2.3% of incident light, allowing around 97.7% to pass through, inclusion of 2 or more sheets in the graphene core particle of the disclosure allows enhanced light absorption (blackness) which arises from the electronic interaction of stacked graphene layers in particular when the stacked graphene layers are organized in few layer graphene.

The term "few layer graphene" as used herein indicates a number of graphene layers ranging from about 2 to about 100 graphene sheets, preferably 3 to 50 graphene sheets, and even more preferably 5 to 25 sheets. In a few layer graphene as disclosed herein the lowest number of layers is 2, the maximum number of stacked graphene layers is 100 and the interlayer distance in graphene is 0.348 ± 0.003 nm, compared to 0.337 nm in graphite. [8]. The lower bound for the largest diameter of the stacked graphene flakes is 10 nm, the upper bound is 5,000 nm.

The number of nanosheets in a graphene particle in the sense of the disclosure can be detected by means of Transmission Electron Microscopy (TEM), X-ray diffraction (XRD), Raman spectroscopy and additional techniques identifiable by a skilled person.

**In** a graphene particle of the disclosure the graphene nanosheets typically have a size ranging from about 20 to about 500 nm (e.g. 200 nm or 150 nm size detected through Transmission Electron Microscopy (TEM) images), where the "size" refers to the maximum point to point distance in the graphene core particle (e.g., diameter in the case of spherical particles, or a diagonal distance in a rectangular shape).

**In** preferred embodiments, a graphene particle of the disclosure is a few layer graphene particle having 2 to 100 graphene sheets to allow light absorbance properties which enables efficient quenching of a fluorescent signal emitted by a detectable moiety of the disclosure as will be understood by a skilled person upon reading of the present disclosure.

In preferred embodiments of the disclosure, the graphene particle has further a size of 900 nm or less, preferably, 500 nm or less, more preferably, 350 nm or less and even more preferably between 50 nm and 250 nm to allow formation of a stable core particle of the disclosure in combination with a coating material and detection of target biomarker in picomolar or even femtomolar concentrations.

In particular, pristine graphene sheets forming a graphene particle herein described can be provided according to methods and techniques identifiable by a skilled person.

For example, pristine graphene can be generated via explosion of acetylene/oxygen mixtures in an explosion chamber. The graphene nanosheets so generated aggregate in the gas phase and form aerogels, which can be harvested according to approaches identifiable by a skilled person to provide graphene also identified as Detonation Graphene or Explosion Graphene.

Pristine graphene can also be obtained from graphite via oxidative exfoliation. According to a related method graphite is oxidized under chemically very harsh conditions (e.g. Hummers Methods) and heat, yielding oxidized single layer graphene (named graphene oxide). Graphene oxide is then reduced to few layer pristine graphene using hydrazine or other reductants according to approaches identifiable by a skilled person. [9]

Pristine graphene can also be obtained by performing flash synthesis. Accordingly, Organic materials can be transformed into few layer graphene by applying direct currents according to approaches identifiable by a skilled person to obtain flash graphene.[10, 11]

Pristine graphene can also be obtained through CVD (chemical vapor deposition) which generates graphene monolayers on substrates (not suitable for sensitive optical biosensors) according to approaches identifiable by a skilled person to provide CVD graphene. [9]

In some embodiments of graphene core particles herein described, pristine graphene starting materials are preferably prepared using detonation-synthesized graphene or flash synthesized graphene which can be prepared with processes and approaches identifiable by a skilled person (see e.g. [12], [8], [13], [14]).

Preparation of detonation sized graphene entails, *inter alia,* a one-step process involving the controlled detonation of carbon-containing material(s) as a solid, liquid, or gas, with an oxidizing agent or source of oxygen (e.g., O₂, N₂O, NO) in a reaction vessel at relatively high temperatures to produce pristine graphene nanosheets and ramified fractal aggregates of these nanosheets without the use of catalytic materials. In general, the reaction vessel is loaded with the desired amount of reactants and a spark is used to achieve detonation of the materials. An aerosol is produced which often undergoes gelation to form an aerosol gel comprising graphene particles. ([15] and [16]).

In a scaled-up approach, the apparatus comprises a reaction chamber, a vacuum source operably connected with the reaction chamber, and an ignition assembly. The reaction chamber is operably coupled with a source of a carbon-containing material and a source of an oxidizer. The vacuum source is operable to selectively evacuate at least a portion of the contents of the reaction chamber, especially following generation of the particulate materials. The ignition assembly is also operably connected to the reaction chamber and configured to initiate combustion of a quantity of the carbon-containing material and a quantity of the oxidizer delivered to the reaction chamber from their respective sources. The ignition assembly comprises a pair of electrodes that are operable to generate an ionizing arc therebetween, each electrode is contained within a respective cassette that is removably received within the ignition assembly. A scaled-up apparatus and synthesis process for mass production of pristine graphene is described in co-pending U.S. Serial No. 63/039,087, filed June 15, 2020 and corresponding PCT/US2021/037321, filed June 15, 2021,.

Exemplary carbon-containing materials to use for the reaction include, carbon-rich precursors, gases, gas mixtures, powders, aerosols, and other injectable materials. The carbon-containing material may comprise a single material or compound, or a mixture of carbon-containing compounds. Examples include coal, petroleum coke, biochar, carbon black, biowaste (e.g., food or agricultural waste products), biomass or organic waste (e.g., cellulosic materials, grasses, etc.). It will be appreciated that higher carbon content materials are particularly preferred. The starting material can include any hydrocarbon compound, and in particular a saturated or unsaturated C₁-C₁₂ hydrocarbon compound, with highly saturated C₁-C₁₂ hydrocarbons being particularly preferred. In particular embodiments, preferred hydrocarbon materials include acetylene, ethylene, toluene, butadiene, and/or isoprene. The temperature and/or pressure parameters can be adjusted according to the selected material.

In one or more embodiments, the combustion reaction occurs at a temperature of at least 3000 K, at least 3500 K, or at least 4000 K. In particular embodiments, the combustion reaction occurs at a temperature of between about 3000 K to about 5000 K, between about 3500 K to about 4500K, or about 4000 K. It has been discovered that the combustion of the carbon-containing materials and oxidizer at these temperatures favors the formation of highly ordered graphene particulates as opposed to graphitic soot. Inert gaseous materials such as helium, neon, argon, or nitrogen can be included in the reaction mixture charged into the reaction vessel to assist with temperature control during combustion, if necessary. Also, in certain embodiments, especially in embodiments in which the combustion reaction is a detonation, the combustion of the reaction mixture proceeds very quickly. Detonation typically involves a supersonic exothermic front that accelerates through a medium and eventually drives a shock front propagating directly in front of it. In certain embodiments, the combustion has a duration of between about 5 to about 100 ms, between about 10 to about 75 ms, or between about 20 to about 50 ms.

The ratio of oxidizing agent to carbon-containing material present in the reaction vessel prior to detonation can contribute to the characteristics of the graphene particulates formed upon detonation of the reaction mixture. In certain embodiments, the molar ratio of oxidizing agent to carbon-containing material is 1.5 or less, preferably 1.0 or less, even more preferably 0.5 or less. In particular embodiments, the ratio of oxidizing agent to carbon-containing material is from about 0.1 to about 1.5, from about 0.18 to about 1.2, from about 0.18 to about 1.0, or from about 0.18 to about 0.8, or from about 0.18 to about 0.5. The process permits the bulk synthesis of large quantities of graphene in excellent purities. For preferred purposes of creating graphene nanosheet particulates of the desired size ranges herein, the ratio of oxidizing agent to carbon-containing material is preferably from about 0.2 to about 0.5, with about 0.3, 0.35, or 0.4 being particularly preferred ratios. Preferred particulates of pristine graphene will range from about 20 to about 500 nm in size with approximately 150 nm +/- 20 nm being a target size range. Notably, it is not necessary for the biosensor of the disclosure that the resulting particulates be monodisperse. Rather, polydisperse graphene particulate mixtures can be easily used to prepare the biosensors. In fact, according to light scattering experiments the graphene aggregates feature super-aggregate fractal morphology with a fractal dimension of Df = 2.5 ± 0.1 on micron scales composed of smaller aggregates with fractal dimensions of D_{f} = 1.8 ± 0.1. This behavior has previously not observed for metal, metal-oxide, metal/metal oxide, or semiconductor particles in (nano)biosensors.

Alternative approaches for synthesizing pristine graphene include flash graphene (PCT/US2019/047967, filed August 23, 2019, WIPO publication WO2020/051000,), which uses flash Joule heating of various carbon-based materials or other carbon sources, such as coal, petroleum coke, biochar, carbon black, food waste, rubber tires, and mixed plastic waste to convert the material to graphene. The carbon source is lightly compressed in a reaction vessel between two electrodes, and a high voltage discharge from a capacitor bank brings the carbon source material in the reaction vessel to at least 3000 K in less than 100 ms. The process converts the amorphous carbon in the carbon source into flash graphene. Yields in this process depend heavily on the carbon content of the starting material. In some embodiments, the carbon source material may be mixed with carbon black or another similar conductive material to improve the conductivity of the material. In some embodiments, the flash graphene has an average particle size of less than 20 nm. In some embodiments, the flash graphene is produced in the form of larger, but thin sheets of average size of 0.5 to 1.2 µm. Such thin sheets may be suitable for alternative sensors where graphene nanosheets in single layers are deposited onto a solid surface, instead of used in particulate form.

The pristine graphene that results from these new synthesis processes can adopt various morphologies, but preferably is in the form of ramified fractal aggregates, nanosheets, crystalline flakes, nanoplatelets and platelet chains, as single or multilayer graphene, and can be generally characterized macroscopically as a fluffy or fuzzy black powder or particulate material of high purity (≥98.5% carbon).

The particulates can be observed under an electron microscope as thin monolayers entangled with each other with overlapped edges, or more ordered stacking of nanosheets comprising or consisting of two to three layers, but potentially up to 25 layers, potentially up to 10 layers, for example 2 to 25 layers, and even more preferably about 5 to about 10 layers (with overall sizes ranging from 20 nm to 500 nm).

Thus, references herein to "pristine" graphene are used specifically to denote the highly pure graphene produced by high temperature (e.g., ~>3000K) conversion of a carbon source into graphene (e.g., rapid heating or combustion of amorphous carbon into detonation graphene, flash graphene, etc.) as compared to graphene made via organic synthesis, chemical-vapor deposition, or exfoliation from graphite. In other words, the pristine graphene is preferably essentially free of graphite or graphite oxide. As such, the pristine graphene is highly pure meaning that it is essentially free (less than 0.5% w/w, preferably less than 0.1% w/w) of foreign substances and impurities, with a carbon content of at least about 98.5%, and preferably at least 99% (and conversely an oxygen content of less than 1%). A discussion of the detection of detonation graphene with Transmission Electron Microscopy (TEM) is provided in **Example 21** and related **Figures 19A and 19B** which show exemplary TEM images of pristine detonation graphene.

In some embodiments related to biosensors based upon pristine graphene nanosheet particles and related core particles, materials comprise detonation graphene preferably 0.25, 0.30, or 0.35.

In some most preferred embodiments the graphene nanosheet particulate comprises an aggregate of graphene in fractal form.

The term "fractal" as used herein in connection with an object indicates an object that displays a property known as self-similarity, i.e. a geometric shape that can be reduced to smaller parts, with each smaller part being a reduced copy of the whole. A fractal dimension is a statistical quantity that describes how a fractal appears to fill space. [17]

Accordingly, typically, stacked few layer graphene features in fractal form have fractal dimensions between Df = 2.5 ± 0.3 (upper bound) and Df = 1.8 ± 0.4 (lower bound) as will be understood by a skilled person [8] (see also **Example 1).**

**.** The fractal nature of graphene vs. other nanomaterials used in biosensors can be ascertained by light scattering methods such as SEM (Scanning Electron Microscopy) [18], and additional techniques identifiable by a skilled person, such as light-scattering methods [19] (see also **Example 1).**

In graphene core particles herein described, more than one graphene nanosheets are coated with a coating layer of an organic and/or inorganic material. Accordingly, in order to allow coating, the graphene nanosheets are functionalized or derivatized to present a functional group on the surface capable of binding corresponding functional groups presented on organic and/or inorganic material to allow formation of the coating layer.

The term "functional group" as used herein indicates specific groups of atoms within a molecular structure that are responsible for a characteristic chemical reaction of that structure. Exemplary functional groups include hydrocarbons, groups containing double or triple bonds, groups containing halogen, groups containing oxygen, groups containing nitrogen and groups containing phosphorus and sulfur all identifiable by a skilled person.

The term "corresponding" used in connection with elements such as functional groups identify two or more elements capable of reacting one with another under appropriate conditions. Typically, a reaction between corresponding moieties and in particular functional groups, results in binding of the two elements.

The term "bind", "binding", "conjugation" as used herein indicates an attractive interaction between two elements which results in a stable association of the element in which the elements are in close proximity to each other. If each element is comprised in a molecule the result of binding is typically formation of a molecular complex. Attractive interactions in the sense of the present disclosure includes both non-covalent binding and, covalent binding. Covalent binding indicates a form of chemical bonding that is characterized by the sharing of pairs of electrons between atoms, or between atoms and other covalent bonds. For example, attraction-to-repulsion stability that forms between atoms when they share electrons is known as covalent bonding. Covalent bonding includes many kinds of interaction, including σ-bonding, π-bonding, metal to non-metal bonding, agostic interactions, and three-center two-electron bonds. Non-covalent binding as used herein indicates a type of chemical bond, such as protein-protein interaction, that does not involve the sharing of pairs of electrons, but rather involves more dispersed variations of electromagnetic interactions. Non-covalent bonding includes ionic bonds, hydrophobic interactions, electrostatic interactions, hydrogen bonds, and dipole-dipole bonds. Electrostatic interactions include association between two oppositely charged entities.

Exemplary pairs of functional groups and the corresponding coupling chemistry are shown in the following Schemes (I) to (IX). 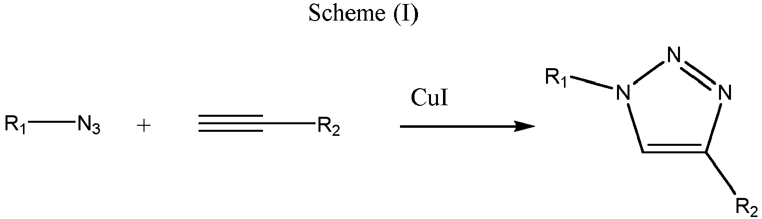 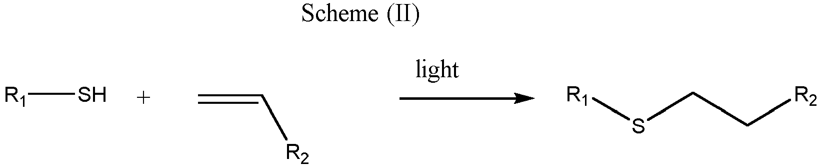 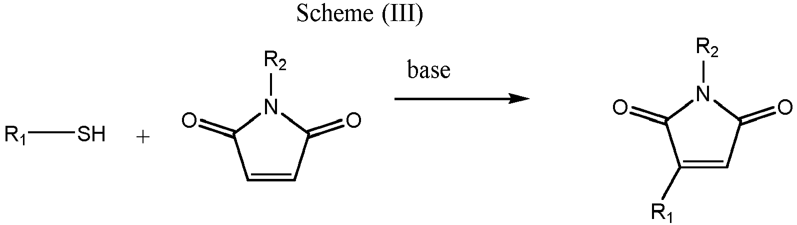 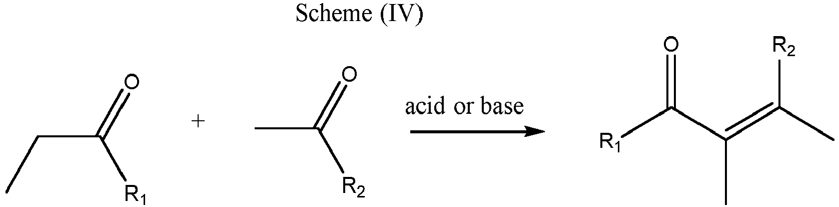 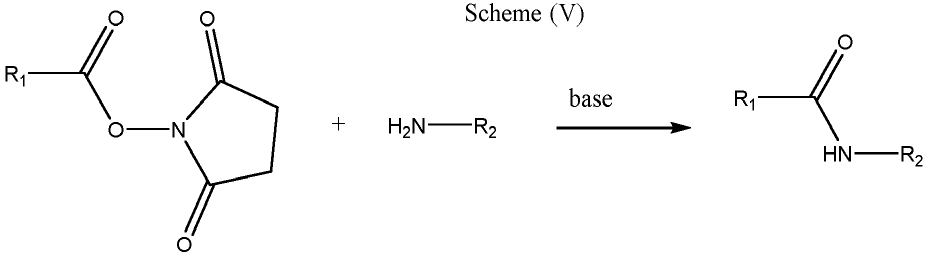 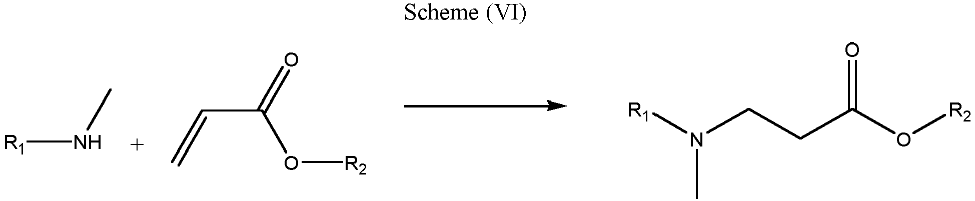 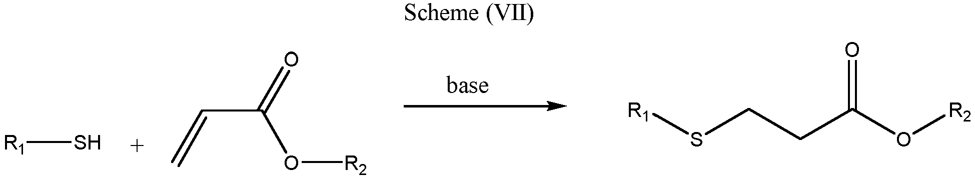 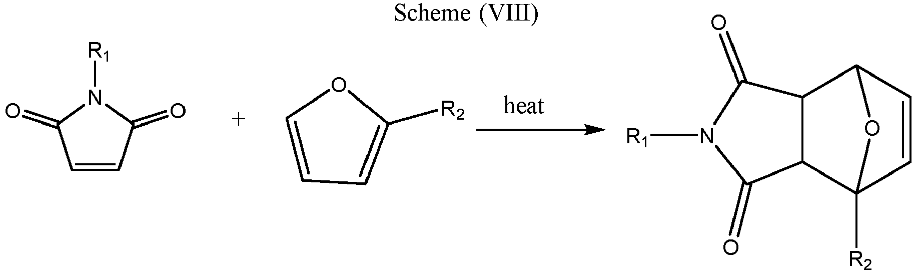 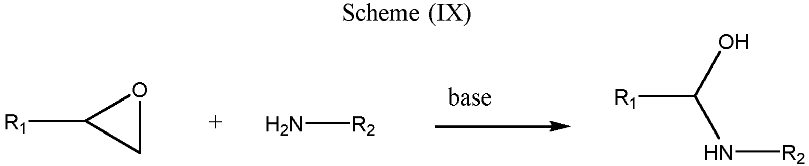 Additional pairs of corresponding functional groups in the sense of the disclosure will be identifiable by a skilled person.

In embodiments herein described, the pristine graphene nanosheet particulates obtained with methods described herein are or can be functionalized with a suitable functional group as described herein to facilitate preparation of derivative compounds suitable for use as the core particle for the biosensors. Graphene can be functionalized using a chemical modification of organic groups via covalent functionalization. Another approach involves a Fenton oxidation process described in detail in PCT/US2020/038055, filed June 17, 2020,. In this process, the surface of stacked graphene is oxidized utilizing ferrous sulfate and hydrogen peroxide as oxidants, resulting in creating > 95% of carboxylic acid functions at the surface. All other possible functional groups, such as hydroxides, aldehydes, ketones, or oxiranes are present at < 5%.

The methods generally comprise reacting pristine graphene particulates with hydrogen peroxide in an aqueous reaction solution at low pH (< 5.0) in the presence of a source of iron. The reaction solution is agitated or stirred for a period of time to react hydroperoxyl radicals generated in the reaction solution with the graphene particulates to oxidize the outer surface of the pristine graphene particulates in solution and yield graphene/graphene oxide particulates. The resulting graphene/graphene oxide particulate comprise a graphene core with a thin graphene oxide surface coating or shell that can be readily modified via various reactions.

**In** a particular embodiment, a preferred graphene derivative having a high density of surface -COOH groups, referred to herein as "carboxygraphene" can be synthesized using pristine graphene and surface functionalization based upon cycloaddition of nitrene which yields a stable azirane anchor. In a particular process, pristine graphene nanoparticulates are dispersed in a suitable organic polar solvent system, such as anhydrous dimethylformamide (DMF), dimethylacetamide (DMA), dimethyl sulfoxide (DMSO), acetone, acetonitrile, or chloroform, at room temperature. After the dispersion is complete, carboxylic acids with a primary or secondary halogen (e.g., chloroacetic acid, 3-iodopropionic acids, 4-bromo-butyric acid, 4-bromovaleric acid, 5-bromovaleric acid, etc.) are added, followed by the addition of anhydrous hydrazoic acid, or its azide derivatives, such as anhydrous sodium azide (NaN₃), lithium azide, and/or potassium azide. The temperature of the reaction solution is then slowly increased (~1°C/minute) to about 80°C. Between 20°C and 40°C, a nucleophilic substitution reaction (S_{N}) occurs, in which an organic azide and solvent-soluble sodium halogenide are formed. The organic azide releases dinitrogen (N₂) and forms a nitrene intermediate at temperatures above ~50°C. Nitrenes feature 6 instead of 8 electrons in the outer shell of nitrogen. This reactive intermediate undergoes a cycloaddition with a Π-Π double bond at the surface of the graphene honeycomb lattice to form a stable azirane anchor (three-membered C-N-C ring). The azirane-cycloaddition offers the opportunity to add a tailored amount of carboxylic acids to the surface of graphene without compromising the optical and electrical properties of the graphene itself. In this case, a high density of carboxylic acid groups can be added across the surface of the graphene nanosheet particulates for further functionalization and/or interaction with a water-soluble polymer coating (see **Examples 2 to 9).**

In some embodiments additional functional modifications of graphene can be described to allow further reaction and formation of a coating layer according to the disclosure. In this connection, although aliphatic linkages are used for functionalization herein, aromatic reactions are also described in the literature for chemical amination of graphene surfaces [20] (see **Example 10).**

Moreover, additional modifications could be carried out to decrease the surface charge of the graphene making it suitable for binding cationic polymers in lieu of or in addition to the functionalizations described above as will be understood by a skilled person upon reading of the disclosure.

In graphene core particles herein described the graphene nanosheets are coated with a coating layer of an organic and/or inorganic material.

The term "coating layer" as used herein indicates a sheet, quantity, or thickness of a material covering a surface or body. In particular a coating layer in the sense of the disclosure indicates a thickness of an organic and/or inorganic material covering the surface of a graphene particle. In a graphene core particle of the disclosure the coating layer has two major functions: a) permit the dispersion of graphene in aqueous buffers and b) maintaining a distance of attached detectable moiety of disclosure of < 20 nm to facilitate effective quenching in their tethered state.

In particular in embodiments herein described a coating layer is applied of a hydrophilic organic and/or inorganic compound having a solubility in water of 1% by weight at 25°C.

The term "organic compound" as used herein indicates any chemical compounds that contain carbon-hydrogen bonds and the ability to facilitate and/or accept hydrogen bonds from water. All or part of the hydrogen atoms in an organic compound can be substituted by other atoms.

The term "inorganic compound" as used herein indicates a chemical to any chemical substance that does not contains C-H bond. Typically an inorganic compound that features at least one element that is heavier than fluorine

In particular, an organic and/or inorganic compound suitable to provide biosensors herein described a solubility in water of at least 10 grams per liter H₂O and is able to be covalently linked to, or in water electrostatically stably attached to, the graphene as described herein as will be understood by a skilled person.

In some embodiments, an organic and/or inorganic compound suitable to provide biosensors herein described feature a consensus log P (or cLogP) < 0.

A cLogP value in the sense of the disclosure refers to the logarithm of its partition coefficient between n-octanol and water log(Coctanol/Cwater) as is known by a person skilled in the art. High lipophilicity corresponds to a high cLogP value. Organic molecules of coatings of the present disclosure can be configured with a combination of moieties and/or substituents to have specific clogP values as will be understood by a skilled person upon reading of the present disclosure.

Coating materials according to the present disclosure are expected to have a range of cLogP between 1 and -2.5, preferably from 0 to -2.

In some embodiments the inorganic compounds comprise mesoporous silica, mesoporous silazanes, aluminum oxide, tin oxide, titanium dioxide, zirconium dioxide, oxides of lanthanides. Alkoxide and alkoxy-amine, and amine- precursors (e.g. Si(NH-CH₃)₄; Si(O-CH₂-CH₃)₃(CH₂-CH₂-CH₂-NH₂); M(O-CH₂-CH₃)₄ with M=Si, Sn, Ti, Zn; or M(O-CH₂-CH₃)₃ with (M=Al or lanthanide(III) cations) react with hydroxyl groups, carboxylic acids, or oxirene rings at the surface of graphene or chemically modified graphene to for stable O-metal bonds. This is followed by hydrolysis of the alkoxy, alkoxy-amine, and amine-bonds around the graphene-attached metals, resulting in semiconductor-oxides and metal-oxides layers surrounding graphene. [21]

In particular, in some embodiments the inorganic compound can be a mesoporous silica which is a form of silica having pores with diameters between 2 and 50 nm. Mesoporous silica can be prepared with starting material, for example, of tetraethyl orthosilicate (TEOS) or (3-mercaptopropyl)trimethoxysilane, often abbreviated to MPTMS. In an exemplary process, the silica precursors will be deposited in tetrahydrofuran/water mixtures (THF/H2O: 99:1 to 95:5 percent by weight) by stirring at room temperature for 24h, followed by centrifugation at 5,000 RPM for 10 min., redispersing in H₂O, followed by collection by means of centrifugation (5,000 RPM for 15 min.). The thickness of the mesoporous silica layers around (stacked) graphene will be determined by the weight ratio between TEOS or MPTMS and graphene (0.001/1.0 to 0.1/1.0) as will be understood by a skilled person.

In embodiments, where the inorganic compound is a mesoporous silica functionalization of the silica surface featuring polar Si-OH groups (silanol) can be achieved, for example, by one of the following steps:
- Silyl ether formation: Si-OH + X-R -> Si-O-R + HX, X=halogen), followed by organic chemistry. For that purpose, the organic group R has to contain a polar functional group.
- Tethering the C-terminal end of a peptide sequence or a carboxylic ester group in its side chain to an amine group contained in the coating by means of a stable amide bond.
- Reacting the mesoporous silica layer (or metal oxide layer) around graphene with SiCl₄, SiHCl₃, SiH₂Cl₂ or Si(CH₃)₂Cl₂, and subsequent chemisorption of the N-terminal end of a peptide to the Si-Cl groups on the surface. This can also be achieved by reacting a polar hydroxyl, thiol, or carboxylic acid group.
- Polar Si-Cl groups on the surface can react with NaN₃ (sodium azide) to form silicon-azides, which undergo click reactions.

**In** some embodiments the organic compounds comprise aliphatic amines, aromatic amines, oligoethylene glycols, aliphatic oligoamines, ethylene oligoimines, aliphatic carboxylic acids, aromatic carboxylic acids, aliphatic alcohols, aliphatic sulfonic acids, aromatic sulfonic acids, and combinations thereof.

In some embodiments an inorganic and/or organic compound forming the coating layer comprises one or more polymers. The term "polymers" as used herein indicates a substance or material consisting of very large molecules, or macromolecules, composed of many repeating subunits. Polymers can be organic or inorganic, synthetic and natural, and are usually created via polymerization of many small molecules, known as monomers. Their consequently large molecular mass, relative to small molecule compounds, produces unique physical properties including toughness, high elasticity, viscoelasticity, and a tendency to form amorphous and semicrystalline structures rather than crystals as will be understood by a skilled person [22])

**In** one or more embodiments, the coating organic polymer can be represented by Formula (I). 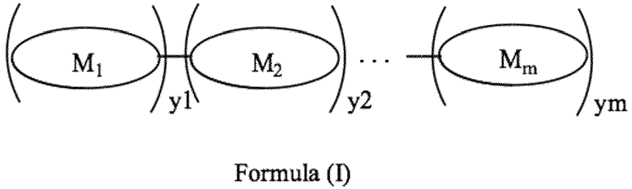 wherein M1 to Mm are polymerized monomeric moieties formed by the same or different polymerizable organic monomers, wherein m ranges from 1 to 10 and y1, y2 to ym are each independently 1 to 1,000.

In particular, exemplary polymerizable organic monomers comprise aziridine propylene oxide, ethylene glycol, acrylic acid, methacrylic acid, 2-hydroxyethyl methacrylate, methacrylamide, N-iso-propylacrylamide, 2-hydroxypropyl methacrylate, N-vinylpyrrolidone, 2-vinylpyridine, 2-vinylpyridine N-oxide, 2-ethyl-2-oxazoline.

Accordingly, in one or more embodiments, one or more water-soluble organic polymers can then be added to the surface of the graphene derivative (e.g., carboxygraphene). Examples for suitable water-soluble polymers of Formula (I) include polyethyleneimine, polycaprolactone, polyethylene glycol, polyvinyl alcohol, polyvinylpyrrolidone, (meth)acrylate- and (meth)acrylamide polymers, polystyrene-carboxylic acid, polystyrene-amine, as well as natural biopolymers (e.g., dextran, pullulan, chitosan, alginate, cellulose, and hyaluronic acid) as well as mixtures or co-polymers thereof.

**In** some embodiments, the polymers comprise or consist of homopolymers such as polyethyleneimine, polycaprolactone, polyvinyl alcohol, polyglycolate, polyvinylpyrrolidone, (meth)acrylate- and (meth)acrylamide polymers, polystyrene-carboxylic acid, polystyrene-amine, polystyrene-sulfonic acid, dextran, pullulan, chitosan, alginate, cellulose, and hyaluronic acid, and mixtures or co-polymers thereof.

In some embodiments, the polymer comprise or consist of block copolymers. In those embodiments when block copolymers are being used, hydrophobic polymers, such as polystyrene, polyisobutylenes, polyvinylchloride, polyethylene, polypropylene and additional can be components of block copolymers as long as the block co-polymer features a consensus log P < 0, or a solubility in water of > 1.0 x 10³ grams per liter H₂O.[23] Hydrophobic polymers are expected to be adsorbed on the surface of pristine graphene, whereas their hydrophilic component will mediate water-dispersibility. In some of those embodiments, where the surface charge of graphene is positive (zeta potential = + 60 mV) such as in explosion graphene [1, 8], this allows the combination of hydrophobic and charge attraction when negatively charged polymers, such as polystyrene-sulfonic acid are used in block copolymers of polystyrene and additional organic co-polymers identifiable by a skilled person.

In some embodiments wherein, hydrophobic-hydrophilic block copolymers are used, the block copolymers will be dissolved in a solvent that also disperses graphene well (e.g. dimethylformamide (DMF)). Under continuing sonication, H₂O is added to the mixture, forcing the hydrophobic end of the block-copolymer to bind to graphene. This bond is strengthened due to the presence of negatively charged groups in the hydrophobic-hydrophilic block copolymers as will be understood by a skilled person.

In some embodiments, preferred organic polymers comprise one or more of polyethylenimine, polycaprolactone, polyethylene glycol, polyvinyl alcohol, polyvinylpyrrolidone, (meth)acrylate- and (meth)acrylamide polymers, as well as polystyrene-carboxylic acid and polystyrene-amine. Other preferred coatings are chemically modified oligoethylene glycols, and oligoimines, and starburst dendrimers.

In some embodiments the graphene particles can be coated with a monolayer of water-soluble organic molecules, for instance oligoethylene glycols, which can be tethered to the surface of graphene (see **Example 32**).

Aliphatic oligoimines, aliphatic thioethers, starburst dendrimers, water-soluble peptides (other than consensus sequences), carbohydrates (e.g. galactose, glucose, mannose, fructose, sialic acid, hyaluronic acid, dextran, glycan-like Gb3 trisaccharides, galabiose). Note that all carbohydrates can be aminated in various positions.

In some embodiments the graphene particles can be coated by an inorganic water-soluble shell (for instance a mesoporous silica nanolayer). Preferred are mesoporous silica, mesoporous aminosilica and titanium dioxide.

In graphene core particles of the disclosure, one or more organic and/or inorganic compounds are reacted with a functional group presented on the surface of the graphene nanosheets (e.g. a carboxylic group see **Examples 3 to 9)** to form a thin and tight layer of polymer around the graphene aggregates (as opposed to a thick layer or halo). (see **Example 30, Example 32, Example 35).** [24]

In embodiment of the disclosure, graphene core particles comprising more than one pristine graphene nanosheets coated by a coating layer of an organic and/or inorganic compound having a solubility in water of 1% by weight at 25 degree C has been surprisingly found to be stable in an aqueous solution and capable of attaching a detectable component specific for a target biomarker.

It has been surprising found that graphene particles with the above features exhibit a stability in aqueous solutions which can exceed 20 or even 30 days. Additionally, it has been found that depending on the concentration, possible sedimentation of graphene particles so coated can be addressed by dispersing the particles e.g. through sonication so that a solution can be obtained comprising more than 95% of particles as will be understood by a skilled person.

In particular, the biosensor as described are chemically stable and physically stable in a buffer suspension defined by a decrease of equal to or less than 20%, preferably equal to or less than 10%, and more preferably equal to or less than 1% over 30 days in the light absorption of the detectable moiety in the buffer suspension.

The term "stable" and "stability" as used herein indicate chemical stability as well as colloidal stability.

The term "chemical stability" as used herein refers to the preservation of the chemical identity of a biosensor. As a skilled person would understand, biosensor as described herein comprises chemical bonds that connect each component of the biosensor together, including graphene, coating material, peptide, and detectable moiety. It is further to be understood that each of graphene, coating material, peptide, and detectable moiety comprises chemical bonding that connects atoms together to form the graphene, coating material, peptide, and detectable moiety. A preservation of chemical identity of a biosensor is meant that none of the chemical bonds that make up the biosensor is broken and no new chemical bond is formed. Such chemical integrity can be ascertained by measurements including but not limited to elemental analysis, UV-vis absorption spectroscopy, magnetic resonance spectroscopy, RAMAN spectroscopy, IR spectroscopy, and fluorescence spectroscopy.

The term "colloidal stability" as used herein refers to the preservation of the physical state of a colloidal suspension of a biosensor, colloidal stability depends on several types of interactions including Van der Walls and electrostatic interactions (classical Derjaguin-Landau-Verwey-Overbeek (DLVO) theory), steric interactions (e.g. polymer adsorption), and hydrophobic effect. Electrophoretic light scattering (ELS) is a common technique used to evaluate the potential of dispersion to remain stable. ELS allows measuring zeta-potential of a dispersion, which provides information about electrostatic interactions and, by extrapolation, to their tendency to agglomerate. The zeta-potential is considered to be a reliable indicator of dispersion stability, but several parameters such as steric effects, sedimentation, or hydrophobic effects, will also have a strong influence.

In some embodiments, herein described core graphene particles and related biosensors are chemically stable and physically stable in a buffer suspension defined by a decrease of equal to or less than 20%, preferably 10%, and more preferably equal to or less than 1% over 30 days in the light absorption of the detectable moiety in the buffer suspension.

Accordingly, in embodiments herein described a stable graphene core particle of the disclosure refers to a graphene particle which when included in suspension in an aqueous solution, results in a solution in which at least 95% of particles remain in suspension with a change in colloidal stability of -24% or less at OD650.

In particular, data have been obtained reflecting changes of colloidal stability are 26% or less, with subranges 22% and 25%, averaging 24% settling reflecting the worst performance of 4 lots of nanobiosensors according to the disclosure. In one representative embodiment, the stability of graphene at 60min measured at OD650 showed a 9% and 10% decrease in absolute colloidal stability for NE and MMP15 nanobiosensors (see Lot 4 in **Example 40).** It is therefore expected less than 10% decrease in colloidal stability in an aqueous solution of graphene biosensors of the disclosure according to preferred embodiments.

In particular, in preferred embodiments herein described, a stable graphene core particle of the disclosure comprise sone or more organic and/or inorganic compounds reacted to form a coating layer of 1-20 nm thickness with a packing density of at least 2.40 ± 0.05 x 10⁻⁵ mol/g, preferably up to 6.0 ± 0.5 x 10⁻⁴ mol/g.

Accordingly, in preferred stable graphene core particle of the disclosure the minimal geometric extension of the attached coating layer is 0.45 nm and can go up to 20 nm and the coating layer covers at least 50%, preferably at least 85% and more preferably at least 90%. Most preferably, a monolayer of polymer is formed around the graphene, preferably fully encapsulating the graphene nanosheets therein. In most preferred embodiments, the graphene particle has a size from 10nm to 5000 nm preferably from 150 to 500nm on average as determined using scanning electron microscopy and digital light scattering measurements.

In those embodiments, stability of the graphene core particles can be in terms of months and even years with examples of graphene particles and related biosensors stable in aqueous solution for more than 12 months as will be understood by a skilled person upon reading of the present disclosure.

The determination of surface coverage is performed by comparing the BET surface. The Brunauer-Emmett-Teller (BET) theory aims to explain the physical adsorption of gas molecules on a solid surface and serves as the basis for an important analysis technique for the measurement of the specific surface area of materials. It measures the adsorption of N₂ onto graphene at 77K. The result of this measurement is the size of the accessible surface (here: 150± 40 m²/g). From titration experiments with polar headgroups (acids or bases), we determine the average density of surface groups. Dynamic Light Scattering is used to measure the size of the polymers that will be attached, as well as the size of graphene+layer+polymer. Depending on the chemical nature of surface coating and polymer, the zeta potential of the surface is continuously changing until complete surface coverage is achieved.

**In** some embodiments herein described graphene core particles of the disclosure are combined with detectable components to provide a graphene biosensor herein described. In particular, the graphene biosensor herein described comprises a detectable moiety attached to the graphene core particle through a peptide linkage comprising a recognition sequence specific for a target biomarker.

The term "biomarker" as used herein indicates a biological molecule found in a biological environment that is associated with a biological event. In particular a biomarker can be associated with the occurrence of physiological events (biological events, associated with the normal functions of living organisms and their parts) or with modification of physiological conditions and in particular occurrence of signs of a normal or abnormal process, or of a condition.

The term "condition" as used herein with respect to a living organism indicates a physical status of the body of the living organism individual (as a whole or as one or more of its parts), that does not conform to a standard physical status associated with a state of complete physical, mental and social well-being for the individual. Conditions herein described include but are not limited to disorders and diseases wherein the term "disorder" indicates a condition of the living individual that is associated with a functional abnormality of the body or of any of its parts, and the term "disease" indicates a condition of the living individual that impairs normal functioning of the body or of any of its parts and is typically manifested by distinguishing signs and symptoms.

Accordingly in embodiments herein described target biomarkers can be selected that are associated with a condition of an individual for diagnostic purposes and/or to see how well the body responds to a treatment for a condition.

Target biomarkers of the disclosure are also molecule configured to interact with peptide linkages to modify the related conformation. In some embodiments, the target biomarkers are proteins configured for specific interaction with peptide recognition sequences.

The term "protein" as used herein indicates a polypeptide with secondary, tertiary, and possibly quaternary structure. The protein's secondary, tertiary, and quaternary structure can occur on a variety of length scales (tenths of Å to nm) and time scales (ns to s), so that in various instances the secondary, tertiary and possibly quaternary structures are dynamic and not perfectly rigid.

The term "polypeptide" as used herein indicates a polymer composed of two or more amino acid monomers and/or analogs thereof wherein the portion formed by the alpha carbon, the amine group and the carboxyl group of the amino acids in the polymer forms the backbone of the polymer. As used herein the term "amino acid", "amino acid monomer", or "amino acid residue" refers to any of the naturally occurring amino acids, any non-naturally occurring amino acids, and any artificial amino acids, including both D and L optical isomers of all amino acid subsets. In particular, amino acid refers to organic compounds composed of amine (-NH2) and carboxylic acid (-COOH), and a side-chain specific to each amino acid connected to an alpha carbon. Different amino acids have different side chains and have distinctive characteristics, such as charge, polarity, aromaticity, reduction potential, hydrophobicity, and pKa. Amino acids can be covalently linked to form a polymer through peptide bonds by reactions between the amine group of a first amino acid and the carboxylic acid group of a second amino acid.

The term "polypeptide" includes amino acid polymers of any length including full-length proteins, as well as analogs and fragments thereof. The polypeptide provides the primary structure of a protein wherein the term "primary structure" of a protein refers to the sequence of amino acids in the polypeptide chain covalently linked to form the polypeptide polymer. A protein "sequence" indicates the order of the amino acids that form the primary structure. Covalent bonds between amino acids within the primary structure can include peptide bonds or disulfide bonds. Polypeptides in the sense of the present disclosure are usually composed of a linear chain of amino acid residues covalently linked by peptide bond. The two ends of the linear polypeptide chain encompassing the terminal residues and the adjacent segment are referred to as the carboxyl terminus (C-terminus) and the amino terminus (N-terminus) based on the nature of the free group on each extremity.

Depending on their size, peptides may be classified as oligopeptides (2-20 residues) and polypeptides (>20 residues). Proteins typically consist of polypeptide chains exceeding 50 residues in length. [25] The average lengths of peptides can be estimated to 0.35 to 0.40 nm per amino acid, depending on the 2D structure of the peptide (alpha-helix or amorphous). [25] Note that peptides forming beta-sheets are not suitable for the design of biosensors, because their release kinetics upon enzymatic cleavage is very slow. A peptide of 50 amino acids has a length of 17.5 to 20 nm, depending on its 2D structure. It should also be noted that non-natural amino acids can be used in these peptides, either for anchoring the peptide to the surface of graphene or graphene-attached organic or inorganic shells, or for the attachment of the fluorophore (organic dye or nanostructure).

As used herein the term "amino acid", "amino acid monomer", or "amino acid residue" refers to organic compounds composed of amine and carboxylic acid functional groups, along with a side-chain specific to each amino acid. In particular, alpha- or α- amino acid refers to organic compounds composed of amine (-NH2) and carboxylic acid (-COOH), and a side-chain specific to each amino acid connected to an alpha carbon. Different amino acids have different side chains and have distinctive characteristics, such as charge, polarity, aromaticity, reduction potential, hydrophobicity, and pKa. Amino acids can be covalently linked to form a polymer through peptide bonds by reactions between the amine group of a first amino acid and the carboxylic acid group of a second amino acid. Amino acid in the sense of the disclosure refers to any of the twenty naturally occurring amino acids, non-natural amino acids, and includes both D an L optical isomers.

**In** preferred embodiments, target biomarkers are proteins with a length > 50 amino acids such as enzymes and chemokines/cytokines. Some of these enzymes have coenzymes and co-factors, which consist of heterocycles binding metals (for instance all matrix metalloproteinases). Some of these proteins are post-translationally modified and are attached to sugars or lipids as will be understood by a skilled person.

In additional embodiments, target biomarkers can comprise many inorganic and organic molecules that are capable of changing the conformation of a peptide linkage or cleaving the peptide linkage. The term "conformation" as used herein in connection with peptides or proteins is defined as the arrangement in space of its constituent atoms which determine the protein structure (the three dimensional arrangement of atoms in a polypeptide) and the protein overall shape of the molecule. The term "cleavage" as used herein in connection with compounds and peptides indicates a breaking of a chemical bond and/or a chemical reaction that involves a breaking of a chemical bond. Cleavage of a peptide linkage typically results in the enzymatic hydrolysis of a peptide bond in the linkage and in the breaking of the linkage down into smaller peptide moieties as will be understood by a skilled person.

A method to identify an organic and/or inorganic biomarker configured to interact with peptide linkages to modify the conformation of the peptide or to cleave a peptide linkage can be performed by
- computationally modeling the tertiary structure of a peptide linkage to provide a computationally modeled peptide linkage
- computationally modeling the tertiary structure of a candidate organic or inorganic biomarker to provide a computationally modeled candidate organic or inorganic biomarker
- determining the target site of interaction of the computationally modeled peptide linkage and computationally modeled candidate organic or inorganic biomarker
- determining changes in the computationally modeled peptide linkage following interaction of the candidate organic or inorganic biomarker in the target site of interaction
- selecting the computationally modeled peptide linkage and organic or inorganic target biomarker when the determined change is a change in the conformation of the peptide linkage or a cleavage of the peptide linkage

In addition or in the alternative the method to identify organic and/or inorganic biomarkers configured to interact with peptide linkages can comprise
- Contacting the candidate organic and/or inorganic biomarker with the peptide linkage for a time and under conditions allowing interaction between the candidate organic and/or inorganic compound and the peptide linkage; and
- Detecting a change in conformation of the peptide linkage and/or cleavage of the peptide linkage, the detecting performed following the contacting to provide a detecting result; and
- Selecting the organic and/or inorganic biomarker when the detecting result is a detected change in the conformation of the peptide linkage or a detected cleavage of the peptide linkage thus providing the identified organic and/or inorganic biomarker.

A biosensor of the disclosure can then be configured to detect the target compound by attaching the peptide linkage to a detectable moiety and to a graphene core particle of the disclosure selected to have a coating layer of a thickness selected to have the detectable moiety at a quenching distance of 20 nm or less with respect to the graphene particle following the attaching. In particular if the target compound has a detected change in the conformation of the peptide linkage resulting in an increase in the length of the peptide linkage, the coating layer is configured so that if the biosensor provided following the attaching of the detectable moiety is at an emitting distance following the increase in the length of the peptide linkage, as will be understood by a skilled person upon reading of the disclosure. The functionality of the biosensor so built can then be tested as will be understood by a skilled person

For example, identification of protein biomarker and corresponding supramolecular recognition sequences was performed according to the following procedure:
1) The primary structure of the target was retrieved from a data bank (e.g., UniProtKB or Protein Data Bank).
2) The tertiary structure of the target was determined and then refined utilizing protein structure predicting software, such as the GalaxyWEB protein structure prediction cluster, developed by Dr. Seok at the Computational Biology Lab, Seoul National University, Korea.
3) The primary structure of a monoclonal antibody (MAB) fragment against the target was retrieved from a data source (e.g. Protein Data Bank, SciFinder, or information provided by the vendors of antibodies). A tertiary structure was generated using Galaxy-WEB.
4) A docking procedure between the target and antibody chain is simulated using the Mobyle platform developed by the Institute Pasteur Biology IT Center, Paris, France. This procedure reveals the "true" epitope of the target.
5) A final "site finder" procedure is then performed on the Mobyle platform docking the epitope of the target to the improved structure of the antibody (Ab) fragment. This structure reveals the peptide sequence of the Ab fragment that is actually responsible for binding to the target in the example of protein-protein interaction.
   F)

In addition or in the alternative to the exemplary method for identification of protein biomarker and corresponding supramolecular recognition sequences, a final "site finder" procedure is then performed on the Mobyle platform docking the protease epitope of the target to the improved structure of the protease enzyme active site. This structure reveals the peptide sequence of the active site that is actually responsible for binding to the target.

In addition or in the alternative to the exemplary method for identification of protein biomarker and corresponding supramolecular recognition sequences, cleavage sites and sequences for protease targeting can be identified from available sites such as MEROPS (a peptidase database).

In the exemplary method for identification of protein biomarker and corresponding supramolecular recognition sequence, if the supramolecular recognition sequence was linear, it was synthesized and used as a recognition sequence for the nanosensors. If it is was not linear, or if the Limit of Detection (LOD) of the resulting nanobiosensor was not at least 10⁻¹⁴ M, the *in silica* procedure was repeated using the structural information from another monoclonal Ab.

In some exemplary embodiments, many organic molecules can also interact with a protein such as an enzymes which are capable of interacting with a peptide and change its conformation. In this regard all enzyme inhibitors can change the conformation of a protein to which it binds. Exemplary enzyme inhibitors include nonpeptidic HIV-1 protease inhibitor tipranavir, diisopropylfluorophosphate (DFP), anti-cancer drug methotrexate, penicillin G an inhibitor for Streptomyces transpeptidase.

In some exemplary embodiments, the interaction between a target biomarker and a peptide linkage can result in cleavage of the peptide linkage. In particular, in some embodiments the target biomarker comprises a protease. Exemplary protease includes Serine proteases, Cysteine, Threonine proteases, Aspartic proteases, Glutamic proteases, Metalloproteases, and Asparagine peptide lyases. In general, all known 553 proteases of the human genome are of potential interest (21 aspartic, 143 cysteine, 186 metallo, 176 serine and 27 threonine proteases), as well as their analogues in rodent models can be detected with a biosensor of the disclosure as will be understood by a skilled person [26].

In some embodiments, the target biomarker comprises enzymes performing posttranslational modifications of peptides, which change their biophysical properties (dynamic, effective lengths, etc.). Examples are arginase (converting arginine to ornithine) and all groups of enzymes attaching or removing any chemical group to a size chain of a peptide. Examples include enzymes capable of (de)phosphorylation (kinases, phosphatases), glycosylating enzymes (N-glycosylation,)-glycosylation, glypiation, C-glycosylation, and phosphoglycosylation), ubiquination, S-nitrosylation, methylaton, N-acetylation, and Lipidation ((C-terminal glycosyl phosphatidylinositol (GPI)) anchor, N-terminal myristoylation, S-myristoylation, S-prenylation).

In some embodiments, the target biomarker comprises proteins (e.g. cytokines/chemokines) capable of binding tailored oligopeptides. The binding events change the biophysical properties of the peptides (dynamic, effective lengths, etc.) Exemplary biomarkers that can be detected comprise all cytokines, chemokines, signaling peptides (e.g. pro-inflammatory factors, anti-inflammatory factors, growth factors) and hormones are of interest as will be understood by a skilled person.

In embodiments herein described peptide linkage that are part of detectable components of graphene biosensors of the disclosure are selected to bind specifically to a target biomarker of interest as will be understood by a skilled person.

The wording "specific" "specifically" or "specificity" as used herein with reference to the binding of a first molecule to second molecule refers to the recognition, contact and formation of a stable complex between the first molecule and the second molecule, together with substantially less to no recognition, contact and formation of a stable complex between each of the first molecule and the second molecule with other molecules that may be present. Exemplary specific bindings are antibody-antigen interaction, cellular receptor-ligand interactions, polynucleotide hybridization, enzyme substrate interactions and additional interactions identifiable by a skilled person. The wording "specific" "specifically" or "specificity" as used herein with reference to a computer supported tool, such as a software indicates a tool capable of identifying a target sequence (such as the one of a marker herein described) among a group of sequences e.g. within a database following alignment of the target sequence with the sequences of the database. Exemplary software configured to specifically detect target sequences comprise Primer-3, PerlPrimer and PrimerBlast simultaneously measure multiple analytes in a single experiment.

In some embodiments, the recognition sequence can be a supramolecular recognition sequence or consensus sequence for the target protein, which means that the linkage recognition has specificity (i.e., contains a selective binding site for the target protein). Thus, it will be appreciated that the recognition sequences avoid non-specific binding motifs. Further, the oligopeptide linkage can be "cleavable," or it can be "non-cleavable." Recognition sequences described herein are preferably cleavable for rapid diagnosis.

**In** embodiments of graphene nanobiosensor of the disclosure peptide linkages can include any variety of recognition sequences or consensus sequences for the biomarker of interest, which means that the linkage sequence has specificity for (i.e., contains a selective binding and/or cleavage site for) the target biomarker. Thus, it will be appreciated that the linkage sequences avoid non-specific binding motifs. The peptide linkages are typically short, linear oligopeptides that position the detectable moiety within suitable proximity of the graphene carrier particle for quenching. In particular embodiments, the peptide linkages can be less than 100 amino acid residues in length, preferably less than 75 amino acid residues in length, more preferably 50 amino acid residues or less. In most preferred embodiments the peptide linkages will be shorter and have less than 50 amino acid residues in length, more preferably less than 30 amino acid residues in length even more preferably 20 amino acid residues or less.

For example, in a graphene biosensor of the disclosure a peptide linkage can range from 4 to 50 amino acid residues, more preferably from 4 to 30 amino acid residues and even more preferably 10 to 20 amino acid residues. As used herein, references to "specific interactions" or "specificity" in connection with recognition intended to differentiate the recognition sequences from non-specific binding or reactions between molecules and means that the set of specific target biomarkers for which the oligopeptide sequence can interact is limited, and in some cases even exclusive, such that neither binding nor enzymatic cleavage occurs at an appreciable rate or frequency with any other molecule except for the target biomarker.

**In** particular, in embodiments herein described specificity of a peptide linkage for a target biomarker results in minimal interactions of the peptide linkage for a biomarker different from the specific biomarker targeted by the peptide linkage as will be understood by a skilled person.

The biosensors can be configured to target a wide variety of biomarkers, depending upon the selected recognition sequence. Examples include numerous enzymes including proteases, kinases, arginases, cytokines/chemokines, dioxygenases (e.g., indoleamine 2,3-dioxygenase 1, and/or tryptophan 2,3-dioxygenase), esterases, and the like, which can then be correlated to a particular health status in the sample being tested. For example, such biomarkers can be indicators of cancer or precancerous cellular activity, bacterial activity, inflammation, etc. and are known in the literature. In one or more embodiments, the recognition sequence comprises, consists essentially, or even consists of a "consensus sequence," which refers to an enzyme substrate (peptide sequence) that undergoes enzymatic cleavage in the presence of the target analyte (e.g. protease, esterase, etc.). Such consensus sequences are described, for example, in U.S. Patent No. 8,969,027 and U.S. Patent No. 9,216,154, U.S. Publication No. 2017/0219548,. The relevant consensus sequences are provided in the table of this disclosure. The guiding paradigm is that for every protease a consensus sequence can be designed in-silico that will be cleaved faster by its target protease than by any other protease.

In one or more embodiments, the recognition sequence comprises, consists essentially, or even consists of a recognition sequence for detection of enzymatic posttranslational modification as described, for example, in WO 2016/149637, filed March 18, 2016, to the extent not inconsistent with the present disclosure. In this embodiment, arginine in GR7G is converted to ornithine GO7G, thus changing the dynamics and geometric extension of the peptide, which enables fluorescence detection in optical Fe/Fe3O4 nanobiosensors. Other posttranslational enzymes that were detected are indeolamine-2,3-dioxygenase and tryptophan 2,3-dioxygenase as will be understood by a skilled person.

In the presence of a target biomarker such as a target enzyme, the enzyme modifies the recognition sequence (without cleavage), and more particularly modifies both the primary structure (amino acid residues and metabolic products) and the secondary structure of the recognition sequence. Such "modification" or "posttranslational modification" as used herein, refers to conversion or transformation of the amino acid residues (and particularly the side chains) in the substrate sequence to a different form and/or different residue. This, in turn, extends, "unfolds," or "unravels" the secondary structure and/or increases and/or decreases the mobility of the recognition sequence. In one or more embodiments, the recognition sequence comprises, consists essentially, or even consists of a supramolecular recognition sequence to detect physical binding without chemical modification or enzymatic cleavage. In particular, the target protein binds to the supramolecular recognition sequence linearly, such that the initial folded secondary structure, such as an alpha-helix and/or beta-sheet structure, is modified and linearly extends or unfolds. In each instance, interaction of the target analyte (e.g., protein, enzyme, etc.) with the recognition sequence gives rise to a detectable change in the biosensor, which is indicative of the presence of the target analyte.

In one or more embodiments, the recognition sequence comprises, consists essentially, or even consists of a recognition sequence for a biomarker of inflammation, infection, and/or bacterial activity as described, for example, in WO2016/018798, filed July 27, 2015, to the extent not inconsistent with the present disclosure. In some of those embodiments, target biomarkers can be enzymes indicative of inflammation, infection, and/or bacterial activity such as MMP-8, MMP-9, MMP-11, MMP-14, and neutrophil elastase in comparison to MMP-1, MMP-2, MMP-3, MMP-7, MMP-12, and MMP-13. Other enzymes target biomarker that are considered partially indicative of inflammation, infection, and/or bacterial activity were Cathepsins -B, -D, -E, -K, and -L and urokinase plasminogen activator as will be understood by a skilled person.

In general, enzymes such as proteases and/or esterases are exemplary biomarkers indicative of inflammation, infection, and/or bacterial activity. The recognition sequence is selected to be cleavable by a protease or esterase either specifically (e.g., using a consensus sequence cleavable only by a target protease) or generally (e.g., using sequences cleavable by any protease or esterase). Upon cleavage of the linkage, the particles are released from one another, giving rise to the spectral change that can be detected in the system.

The recognition sequence is typically provided as part of an oligopeptide linkage between the detectable moiety and the pristine graphene carrier particle. Suitable oligopeptide linkages will comprise (consist essentially or even consist of) the recognition sequence for the target biomarker, typically along with a terminal carboxylic acid group (C-terminus), and a terminal amine group (N-terminus). The oligopeptide can also comprise a thiol group at the C terminus, and a carboxylic acid group at the N terminus. In some embodiments, the oligopeptide linker comprises a hydrophilic region of at least 10 amino acids N-terminal to the recognition sequence, and a linking region C-terminal to the recognition sequence, wherein the C-terminal linking region comprises a thiol reactive group at its terminus. In some embodiments, the C-terminus of the oligopeptide comprises a cysteine residue, lysine, or aspartate. In some embodiments, the N-terminal hydrophilic region of the oligopeptide has an excess positive or negative charge at a ratio of about 1:1. The N-terminal hydrophilic region can also comprise amino acid residues capable of forming hydrogen bonds with each other.

In some embodiments, the C-terminal and N-terminal linking regions can consist of virtually any amino acid sequence that is non-cleavable (e.g., not a recognition sequence): (X)ₙ-[Recognition Sequence]-(X)ₘ, where X is any amino acid (that in sequence does not result in a cleavage sequence), and each of n and m are less than 5.

In some embodiments, the recognition sequences can include one or more spacer residues on the N- and/or C-terminal ends. For example, between 1 and 10 amino acids (any amino acids, naturally and non-naturally, L- and D-, or combinations thereof) can be used at one or both ends as spacers to further extend the oligopeptide chain length depending upon the detectable moieties selected. Examples include N-terminal sequences such as GAG- and C-terminal sequences such as -AG.

Depending upon the target biomarker, a desired recognition sequence can be selected and synthesized. It will be appreciated that various sequences are also available in the literature or can be determined through *in silica* binding studies.

Exemplary recognition sequences are listed in the table below with their relevant biomarker. In some cases, a proposed cleavage point is indicated by the "-". Variants of the listed sequences are also contemplated

**Table 1. Consensus and Recognition Sequences**

| **Biomarker** | **Consensus and Recognition Sequences** | **SEQ ID NO:** |
|---|---|---|
| ADAM 17 | LAQA-VVSS | 41 |
| ADAM 33 | GSQH-IRAE | 42 |
| Cathepsin B | SLLKSR-MVPNFN | 43 |
| | GLAG-LVGP | 108 |
| | DAFK | 109 |
| Cathepsin C | TSEE-IRGG | 110 |
| Cathepsin D | GDSG-LGRA | 44 |
| | SLLIFRSWANFN | 111 |
| | SGKPILFFRL | 112 |
| Cathepsin E | EVAL-VALK | 45 |
| | SGSPAFLAKNR | 113 |
| | SGKPIIFFRL | 114 |
| Cathepsin F | GAFR-MCAG | 115 |
| Cathepsin H | EFVR-SPTP | 116 |
| | QFVR-SPSG | 117 |
| Cathepsin K | LGLE-GANL | 46 |
| | AKLK-AENN | 118 |
| Cathepsin L | AALG-SAPG | 47 |
| | SGVVIA-TVIVIT | 84 |
| Cathepsin S | SLLIFR-SWANFN | 48 |
| | EGLG-QAGN | 119 |
| Cathepsin W | GKKG-AVGE | 120 |
| Cathepsin Z | GFFF-WALG | 121 |
| Granzyme B | VEPN-SLEE | 49 |
| Neutrophil elastase | GEPL-SLLP | 50 |
| | GEPV-SGLP | 87 |
| Gelatinase (bacterial) | GPLGMLSQ | 122 |
| MMP 1 | IPVS-LRSG | 51 |
| | VPMS-MRGG | 88 |
| | GAGVPMS-MRGGAG | 123 |
| MMP 2 | IPVS-LRSG | 51 |
| MMP 3 | RPFS-MIMG | 52 |
| MMP 7 | VPLS-LTMG | 53 |
| MMP 8 | GPSG-LRGA | 54 |
| MMP 9 | VPLS-LYSG | 55 |
| MMP10 | GPSG-LQTG | 124 |
| MMP 11 | GAAN-LVRG | 56 |
| MMP 12 | GVPLS-LTMG | 57 |
| | GPKA-LGLA | 58 |
| MMP 13 | PQGLA-GQRGIV | 125 |
| | GPQGLA-GQRGIV | 126 |
| MMP 14 (MT1-MMP) | GPAG-LRLA | 59 |
| | IPES-LRAG | 127 |
| MMP15 | ESDA-SQTG | 128 |
| ALK | RDIYAAPFFRK | 60 |
| AURKB | AMERRRTSAARRSY | 61 |
| CDK2 | KARAAVSPQKRKA | 62 |
| PKD2 | RARKRRLSAPPLASGD | 63 |
| MAPK1 | AKAGPPLSPRPPHVH | 64 |
| JAK2 | DLFIPDNYLKMKPAP | 65 |
| PLK1 | AELDPEDSMDMDMAP | 66 |
| PLK3 | EDEAEELSDEDEELK | 67 |
| ERK1/MAPK3 | AAGPAPLSPVPPVVH | 68 |
| JunK2/MAPK9 | DASRPPPLSPLPSPRA | 69 |
| ARG I/II | GYRFR-ILGGG | 70 |
| uPA | SGA-SAC | 129 |
| | SG RSA | 130 |
| CCL 2 | CQEQFWW | 1 |
| MCP-1^{a} | PYFPRGSSYQGWN | 2 |
| CCL 3 | CCIQNQ | 3 |
| CCL 4 | AWYQPQFE | 4 |
| CCL 21 | EQQKRN | 5 |
| CXCL 2 | CNHGKFYC | 6 |
| CXCL 5 | NIYCNIAY | 7 |
| CXCL 8 (IL-8) | KAYRWEFI | 8 |
| CXCL 9 | IQNSGAPCH | 9 |
| HSP 27 | WQEAKNANQM | 10 |
| HSP 70 | RHQKTYSF | 11 |
| HSP 90 | XLPPHWAGAL | 12 |
| MIF | XLPPHWAFAL | 13 |
| Calprotectin | LTELEKALNSIIDVYHKYSLIKGNFHAV | 14 |
| CCL20^{b} | GESMNFSDVFDSSEDYFVSVNTSYYSVDSE | 15 |
| | GTQWWVVCQQFG | 16 |
| GCSF^{c} | PGHWSDWSPS | 17 |
| IL-6 | YFPEPVTVSGAGTFPAVLGSGQPPGKGL | 18 |
| | TAVYYCANRAGWGMGDYWGQGTQVT | 19 |
| | TASNYGAGYSTNDRHS | 20 |
| | NRPAQAWMLG | 21 |
| IL-13 | AVYYCQQNNEDPRTFGGGTK | 22 |
| | AGDGYYPYAMDNW | 23 |
| | GWLPFGFILISAG | 24 |
| | YQQKPGQPPKL | 25 |
| | SVNWIRQPPGKALEWLAMIWGDGKIVYNS | 26 |
| | WLPFGFILIS | 27 |
| IL1RL1^{d} | TYYCQQWSGYPYTF | 28 |
| MIP-1^{e} | SHFPYSQYQFWKN | 29 |
| OSTF1^{f} | DMSDTNWWKGTSKGRTGLIPSNYVAEQAESIDNPL | 30 |
| | KGTSKGRTGLIPSNYVAEQAEAG | 131 |
| TIMP-1^{g} | IAGKLQSAGSALWTDQL | 31 |
| TSLP^{h} | SSPKHVRFSWHQDAVTVTC | 32 |
| | AKCCPCQQWW | 33 |

| | | |
|---|---|---|
| ^{a}Monocyte Chemoattractant Protein-1; ^{b}Chemokine (C-C motif) ligand 20 (CCL20) or liver activation regulated chemokine (LARC) or Macrophage Inflammatory Protein-3 (MIP3A); ^{c}Granulocyte colony-stimulating factor; ^{d}Interleukin-1-receptor-like type 1; ^{e}Macrophage Inflammatory Protein; ^{f}Osteoclast Stimulating Factor-1; ^{g}Metalloproteinase Inhibitor 1; ^{h}Thymic stromal lymphopoietin. | | |

In one or more embodiments, the nanosensors can be configured with recognition sequences specific for cytokines detection. In those embodiments, the oligopeptide linkage comprises a linear supramolecular recognition sequence (i.e., paratope) for the receptor/binding site of the cytokine of interest. In the presence of the cytokine, stretching of the tether during cytokine binding leads to a signal change (e.g., fluorescence increase) from the attached detectable particle, which can be detected. The nanosensors can measure cytokine concentrations. Exemplary supramolecular recognition sequences are listed in **Table 2** below.

**Table 2: Supramolecular Recognition Sequences for the Detection of Protein Targets**

| | **Recognition Sequence(s)** | **SEQ ID NO:** | **Accession No.** | **LOD* Moles/L** |
|---|---|---|---|---|
| Cytokine/Chemokine Targets | | | | |
| CCL 2 | CQEQFWW | 1 | P13500 | 10⁻¹⁴ |
| MCP-1^{a} | PYFPRGSSYQGWN | 2 | P13500 | 10⁻¹⁵ |
| CCL 3 | CCIQNQ | 3 | P10147 | 10⁻¹⁵ |
| CCL 4 | AWYQPQFE | 4 | P13236 | 10⁻¹⁴ |
| CCL 21 | EQQKRN | 5 | O00585 | 10⁻¹⁵ |
| CXCL 2 | CNHGKFYC | 6 | P19875 | 10⁻¹⁴ |
| CXCL 5 | NIYCNIAY | 7 | P42830 | 10⁻¹³ |
| CXCL 8 (IL-8) | KAYRWEFI | 8 | P10145 | 10⁻¹⁶ |
| CXCL 9 | IQNSGAPCH | 9 | Q07325 | 10⁻¹⁵ |
| HSP 27 | WQEAKNANQM | 10 | Q5S1U1 | 10⁻¹⁵ |
| HSP 70 | RHQKTYSF | 11 | P0DMV8 | 10⁻¹⁵ |
| HSP 90 | XLPPHWAGAL | 12 | P02829 | 10⁻¹⁵ |
| MIF | XLPPHWAFAL | 13 | P14174 | 10⁻¹⁵ |
| Calprotectin | LTELEKALNSIIDVYHKYSLIKGNFHAV | 14 | S 100A9 | 10⁻¹⁴ |
| CCL20^{b} | GESMNFSDVFDSSEDYFVSVNTSYYSVDSE GTQWWVVCQQFG | 15 16 | P78556 | 10⁻¹⁵ |
| GCSF^{c} | PGHWSDWSPS | 17 | P09919 | 10⁻¹⁵ |
| IL-6 | YFPEPVTVSGAGTFPAVLGSGQPPGKGL | 18 | P05231 | 10⁻¹⁶ |
| | TAVYYCANRAGWGMGDYWGQGTQVT | 19 | | |
| | TASNYGAGYSTNDRHS | 20 | | |
| | NRPAQAWMLG | 21 | | |
| IL-13 | AVYYCQQNNEDPRTFGGGTK | 22 | P35225 | 10⁻¹⁵ |
| | AGDGYYPYAMDNW | 23 | | |
| | GWLPFGFILISAG | 24 | | |
| | YQQKPGQPPKL | 25 | | |
| | SVNWIRQPPGKALEWLAMIWGDGKIVYNS | 26 | | |
| | WLPFGFILIS | 27 | | |
| IL1RL1^{d} | TYYCQQWSGYPYTF | 28 | P14778 | 10⁻¹⁵ |
| MIP-1^{e} | SHFPYSQYQFWKN | 29 | Q9BPZ7 | 10⁻¹⁵ |
| OSTF1^{f} | DMSDTNWWKGTSKGRTGLIPSNYVAEQA | 30 | Q92882 | 10⁻¹⁵ |
| | ESIDNPL | | | |
| TIMP-1^{g} | IAGKLQSAGSAL WTDQL | 31 | P01033 | 10⁻¹² |
| TSLP^{h} | S SPKHVRF S WHQD AVT VTC | 32 | Q960D9 | 10⁻¹⁴ |
| | AKCCPCQQWW | 33 | | |

| Peptide Aptamers - Exosome surface markers | | | | |
|---|---|---|---|---|
| CD-9ⁱ | VQEFYKDTYNKLKTK | 34 | P21926 | 10⁻¹⁴ |
| CD-63ⁱ | LNNHTASILNRMQANF | 35 | P08962 | 10⁻¹⁴ |
| CD-81ⁱ | VGIYILIAVGAVMMFVGFK | 36 | P60033 | 10⁻¹⁵ |

| Exosome content analysis - markers of lung inflammation | | | | |
|---|---|---|---|---|
| 000571 ^{j} | NERNINITKDLLDLL VAH | 37 | 000571 | 10⁻¹⁴ |
| P05187 ^{k} | DINWVKQRPGQGLEWI | 38 | P05187 | 10⁻¹⁵ |
| P54136 ^{l} | VLLQGKNPDITKAWKL | 39 | P54136 | 10⁻¹⁵ |
| Q5T4S7 ^{m} | NLSRSRWFDFPFTREE | 40 | Q5T4S7 | 10⁻¹⁴ |

| | | | | |
|---|---|---|---|---|
| ^{a}Monocyte Chemoattractant Protein-1; ^{b}Chemokine (C-C motif) ligand 20 (CCL20) or liver activation regulated chemokine (LARC) or Macrophage Inflammatory Protein-3 (MIP3A); ^{c}Granulocyte colony-stimulating factor; ^{d}Interleukin-1-receptor-like type 1; ^{e}Macrophage Inflammatory Protein; ^{f}Osteoclast Stimulating Factor-1; ^{g}Metalloproteinase Inhibitor 1; ^{h}Thymic stromal lymphopoietin; ⁱCD: Cluster of Differentiation; ^{j}ATP-dependent RNA helicase DDX3X (from human bronchial airway epithelial cells); ^{k}Alkaline phosphatase, placental type, phosphatase PPB 1; ¹Arginine-tRNA ligase, cytoplasmic; ^{m}E3 ubiquitin-protein ligase UBR4 (small airway epithelial cells); *Estimated limit of detection (LOD). | | | | |

In some embodiments, the supramolecular recognition sequences can include one or more spacer residues on the N- and/or C-terminal ends. For example, between 1 and 10 amino acids (any amino acids, naturally and non-naturally, L- and D-, or combinations thereof) can be used at one or both ends as spacers. Examples include N-terminal sequences such as GAG- and C-terminal sequences such as -AG.

In some embodiments, any other linear peptide sequence of suitable length (e.g., at least 10 amino acid residues) can be used for the supramolecular recognition sequences, with the exception of sequences featuring consensus motifs. If synthesized on a peptide synthesizer, peptide sequences up to about 25 amino acid residues can be used, whereas sequence synthesized in an organism (e.g., E. coli) can be up to about 300 amino acid residues in length. Any designed sequence should be checked for the presence of cleavage sequences, utilizing a data bank, such as MEROPS.

In some embodiments, peptide-aptamers for targeting CD9, CD63, and CD81, which are generally accepted surface markers of exosomes have also been developed. They are capable of binding to the exosomes and permit their isolation directly from collected biospecimens using the nanosensors without prior (ultra)centrifugation. The combined use of CD9, CD63, and CD81 will ensure that virtually all exosomes occurring from the sample, such as from airway cells, will be trapped. After treatment with a lysis buffer, the cargo of the captured exosomes can then be analyzed. Analysis of exosomes presents another existing aspect of the technology. Exosomes occurring from small airway epithelial cells and bronchial airway epithelial cells differ significantly in their proteasomes including the tetraspanin content (CD9, CD63, CD81) in their outer membranes.

**In** some embodiments, the biosensor of the disclosure show flexibility to adapt the underlying nanosensor structure by modifying the particles, oligopeptide linkages, and the like to suit the sensor technology available, and likewise, using a variety of sensor technologies for detecting additional targets. For example, a nanosensor can be prepared for detecting protease activity, in which the graphene core particle of the disclosure are used in connection with the peptide linkages and moieties described in U.S. Patent No. 8,969,027 as will be understood by a skilled person.

In some embodiments, Enzyme consensus sequences that can also be used in nanosensors in combination with one or more above include those in **Table** 3 below, where the cleavage point is indicated by the "-".

**Table 3.**

| **Enzyme** | **Consensus Sequence** | **SEQ ID NO:** | **Accession No.** |
|---|---|---|---|
| ADAM 17 | LAQA-VVSS | 41 | P78536 |
| ADAM 33 | GSQH-IRAE | 42 | Q9BZ11 |
| Cathepsin B | SLLKSR-MVPNFN | 43 | P07858 |
| Cathepsin D | GDSG-LGRA | 44 | P07339 |
| Cathepsin E | EVAL-VALK | 45 | P14091 |
| Cathepsin G | NVLH-SWAV | 82 | |
| Cathepsin H | ALQA-RPGP | 83 | |
| Cathepsin K | LGLE-GANL | 46 | P43235 |
| Cathepsin L | AALG-SAPG | 47 | P07711 |
| Cathepsin L | SGVVIA-TVIVIT | 84 | |
| Cathepsin S | SLLIFR-SWANFN | 48 | P25774 |
| Granzyme B | VEPN-SLEE | 49 | P10144 |
| Human airway trypsin-like peptidase (HAT) | RSAR-GLKG | 85 | |
| TMPRSS2 (Epitheliasin, transmembrane protease, serine 2) | RQSR-IVFG | 86 | |
| NE | GEPL-SLLP | 50 | P08246 |
| NE | GEPV-SGLP | 87 | |
| MMP 1 | IPVS-LRSG | 51 | P03956 |
| MMP 1 | VPMS-MRGG | 88 | |
| MMP 2 | IPVS-LRSG | 51 | P08253 |
| MMP 3 | RPFS-MIMG | 52 | P08254 |
| MMP 7 | VPLS-LTMG | 53 | P09237 |
| MMP 8 | GPSG-LRGA | 54 | P22894 |
| MMP8 | EPYH-LMAL | 89 | |
| MMP 9 | VPLS-LYSG | 55 | P14780 |
| MMP10 | RPLS-LQTG | 90 | |
| MMP 11 | GAAN-LVRG | 56 | P24347 |
| MMP 12 | GVPLS-LTMG | 57 | P34960 |
| MMP 12 | GPKN-LKAP | 91 | |
| MMP 12 | GPAN-LVAP | 92 | |
| MMP 13 | PQGLA-GQRGIV | 58 | P33435 |
| MMP 14 | GPAG-LRLA | 59 | P50281 |
| MMP 14 | LPQG-LRTE | 93 | |
| MMP15 | LPQK-SQHG | 94 | |
| ALK | RDIYAAPFFRK | 60 | Q9UM73 |
| AURKB | AMERRRTSAARRSY | 61 | Q96GD4 |
| CDK2 | KARAAVSPQKRKA | 62 | P24941 |
| PKD2 | RARKRRLSAPPLASGD | 63 | Q504Y2 |
| MAPK1 | AKAGPPLSPRPPHVH | 64 | P28482 |
| JAK2 | DLFIPDNYLKMKPAP | 65 | O60674 |
| PLK1 | AELDPED SMDMDMAP | 66 | P53350 |
| PLK3 | EDEAEELSDEDEELK | 67 | Q9H4B4 |
| ERK1/MAPK3 | AAGPAPLSPVPPWH | 68 | P27361 |
| JunK2/MAPK9 | DASRPPPLSPLPSPRA | 69 | P45984 |
| Proteinase 3 (PR3) | LYYV-SLSP | 95 | |
| Proteinase 3 (myeloblastin) | SYYV-SLSP | 96 | |

| ARG I/II | GRRRRRRRG | 70 | P05089 (ARG1) |
|---|---|---|---|
| | | | P78540 (ARG2) |
| | GAGRRRRRRRAG | 133 | |
| ARG II | RRRRRRR | 97 | |
| KLK-1 | EAFR-STGA | 98 | |
| KLK-2 | GTSR-SAGG | 99 | |
| KLK-5 | YRFR-ILGG | 100 | |
| KLK-12 | IESK-INGG | 101 | |
| KLK-13 | VRSR-IVKG | 102 | |
| KLK-14 | TQPR-IVGG | 103 | |
| KLK-15 | ALGR-KLVG | 104 | |
| Streptococcus pneumoniae IgA1 protease | STPP-TPSP | 105 | |
| Mycosin Protease MycP1 | VKAA-SLGK | 106 | |
| Rv2869c (Mycobacterium tuberculosis)-like peptidase (RIP-1) | TCCA-TCAT-CC | 107 | |

**In** some embodiments, the consensus sequences of a peptide linkage can include an N-terminal spacer (e.g., GAG, GAA, GAP) and a C-terminal spacer (e.g., GA, AG, or EG).

**In** the graphene biosensor of the disclosure the peptide linkages are used to attach detectable moieties or labels to the graphene core particle.

The term "attach" or "attached" as used herein, refers to connecting or uniting by a bond, link, force or tie in order to keep two or more components together, which encompasses either direct or indirect attachment where, for example, a first molecule is directly bound to a second molecule or material, or one or more intermediate molecules are disposed between the first molecule and the second molecule or material.

The terms "detect" or "detection" as used herein indicate the determination of the existence, presence or fact of a target in a limited portion of space, including but not limited to a sample, a reaction mixture, a molecular complex and a substrate. The "detect" or "detection" as used herein can comprise determination of chemical and/or biological properties of the target, including but not limited to the ability to interact, and in particular bind, other compounds, ability to activate another compound and additional properties identifiable by a skilled person upon reading of the present disclosure. The detection can be quantitative or qualitative. A detection is "qualitative" when it refers, relates to, or involves identification of a quality or kind of target or signal in terms of relative abundance to another target or signal, which is not quantified. A detection is "quantitative" when it refers, relates to, or involves the measurement of quantity or amount of the target or signal (also referred as quantitation), which includes but is not limited to any analysis designed to determine the amounts or proportions of the target or signal. A quantitative detection in the sense of the disclosure comprises detection performed semi-quantitatively, above/below a certain amount of target biomarker as will be understood by a skilled person.

The term "detectable moiety" or "label" as used herein refers to a molecule capable of detection, including but not limited to radioactive isotopes, fluorophores, chemiluminescent dyes, chromophores, enzymes, enzyme substrates, enzyme cofactors, enzyme inhibitors, dyes, metal ions, particles, metal sols, ligands (such as biotin, avidin, streptavidin or haptens) and the like. As a consequence, the wording "labeling signal" as used herein indicates the signal emitted from the label that allows detection of the label, including but not limited to radioactivity, fluorescence, chemoluminescence, production of a compound in outcome of an enzymatic reaction and the like.

**In** embodiments herein described, the detectable moiety is capable of emitting a fluorescent signal. The term fluorescent signal indicates a longer wavelength light signal emitted by a compound capable of being electronically excited by a light source of shorter wavelength.

Exemplary detectable labels are compounds capable of being excited by a light source and emitting a fluorescent signal including detectable particles that generate a detectable signal (e.g., optical or spectroscopic), such as fluorescence or color change, which can be perceived visually or measured with an appropriate instrument. Chromophore/luminophores suitable for use in the inventive assays include any organic or inorganic dyes, fluorophores, phosphors, light absorbing particles (e.g., Au, Ag, Pt, Pd), combinations thereof, or the nonmagnetic metalated complexes thereof. Preferably, the selected chromophore/luminophores are smaller than the graphene carrier particle, and typically will have a particle size (maximum edge-to-edge dimension, i.e., diameter) of less than about 20 nm.

Suitable organic dyes are selected from the group consisting of coumarins, pyrene, cyanines, benzenes, N-methylcarbazole, erythrosin B, N-acetyl-L-tryptophanamide, 2,5-diphenyloxazole, rubrene, and N-(3-sulfopropyl)acridinium. Specific examples of preferred coumarins include 7-aminocoumarin, 7-dialkylamino coumarin, and coumarin 153. Examples of preferred benzenes include 1,4-bis(5-phenyloxazol-2-yl)benzene and 1,4-diphenylbenzene. Examples of preferred cyanines include oxacyanines, thiacyanines, indocyanins, merocyanines, and carbocyanines. Other exemplary cyanines include ECL Plus, ECF, C3-Oxacyanine, C3-Thiacyanine Dye (EtOH), C3-Thiacyanine Dye (PrOH), C5-Indocyanine, C5-Oxacyanine, C5-Thiacyanine, C7-Indocyanine, C7-Oxacyanine, CypHer5, Dye-33, cyanines (Cy7, Cy7.5, Cy5.0, Cy5.5, Cy3Cy5 ET, Cy3B, Cy3.0, Cy3.5, Cy2), CBQCA, NIR1, NIR2, NIR3, NIR4, NIR820, SNIR1, SNIR2, SNIR4, Merocyanine 540, Pinacyanol-Iodide, 1,1-Diethyl-4,4-carbocyanine iodide, Stains All, Dye-1041, or Dye-304, as well as all BODIPY dyes.

Cyanine dyes can be attached in a variety of configurations, ideally using a peptide linkage that is shorter than the Förster-radius of the cyanine dye (5-6 nm for Cy3.0 and Cy3.5, 6-7 nm for Cy5.0 and Cy5.5, and approx. 7 nm for Cy7 and Cy7.5). The maximal length of the tether, consisting of any spacers (~2.84 nm) and not more than 12 amino acid residues in the cleavage sequences (up to 4 nm) indicates that shorter cleavage sequences (uPA and MMP's) are suitable for use with Cy3.x and Cy5.x dyes, whereas the cathepsins are preferably linked to Cy5.x and Cy.7.x dyes to permit optimal quenching of the tethered cyanine dyes. For all of the cyanines, their emission maxima are red-shifted with respect to the autofluorescence of human urine. Multiple cyanines can be linked to a single particle to create oligoplexing nanoplatforms, to measure the activity of up to four enzymes simultaneously. All four dyes in the UVA or blue region of the electromagnetic spectrum can be excited simultaneously, or each dye can be excited individually. All cyanine dyes have an excitation maximum, which is blueshifted by 20-25 nm with respect to their emission maximum (typical for fluorescent singlet states). Exemplary emission spectra of: NS-Cy3.0 (λex = 538, λem = 560), NS-Cy5.5 (λex = 639, λem = 660), NS-Cy7.0 (λex = 740, λem = 760) and NS-Cy7.5 (λex = 808, λem = 830).

Suitable inorganic dyes are selected from the group consisting of metalated and non-metalated porphyrins, phthalocyanines, chlorins (e.g., chlorophyll A and B), and metalated chromophores. Preferred porphyrins are selected from the group consisting of tetra carboxyphenyl-porphyrin (TCPP) and Zn-TCPP. Preferred metalated chromophores are selected from the group consisting of ruthenium polypyridyl complexes, osmium polypyridyl complexes, rhodium polypyridyl complexes, 3-(1-methylbenzoimidazol-2-yl)-7-(diethylamino)-coumarin complexes of iridium(III), and 3-(benzothiazol-2-yl)-7-(diethylamino)-coumarin complexes with iridium(III).

Suitable fluorophores and phosphophores are selected from the group consisting of phosphorescent dyes, fluoresceines, rhodamines (e.g., rhodamine B, rhodamine 6G), and anthracenes (e.g., 9-cyanoanthracene, 9,10-diphenylanthracene, 1-Chloro-9,10-bis(phenyl-ethynyl)anthracene).

The broad UV/Vis absorption spectrum of graphene and derivatives permits the attachment of multiple detectable labels with respective (different) recognition sequences to target more than one biomarker at a time. In order to function, there should be no or only a minimal overlap between the excitation and absorption spectra of the co-attached detectable labels, thus minimizing FRET between the particles and/or interference of respective signals. Whereas, principally, any combination of fluorescent dyes can be envisioned, BODIPY dyes are especially suitable, because they feature small Stokes-shifts (the distance between the maxima of lowest excitation and fluorescent bands). An example of this strategy is the parallel use of BODIPY FL (505λex/513λem), BODIPY 530550 (534λex/554λem), BODIPY TR (589λex/61λem), and BODIPY 630650 (646λex/660λem) connected via respective (different) sequences to monitor the activity of four biomarkers independently with a single biosensor. Exemplary BODIPY dyes are shown discussed in **Example** 7.

Alternatively, cyanine dyes 2, 3, 5, and 7 are another possible combination. However, cyanine dyes have much larger Foerster radii and may interfere with each other, even when diluted.

Fluorescent quantum dots and fluorescent particles can also be used. A quantum dot is a semiconductor composed of atoms from groups II-VI or III-V elements of the periodic table (e.g., CdSe, CdTe, InP). The optical properties of quantum dots can be manipulated by synthesizing a (usually stabilizing) shell. Such quantum dots are known as core-shell quantum dots (e.g., CdSe/ZnS, InP/ZnS, InP/CdSe). Quantum dots of the same material, but with different sizes, can emit light of different colors. Their brightness is attributed to the quantization of energy levels due to confinement of an electron in all three spatial dimensions. In a bulk semiconductor, an electron-hole pair is bound within the Bohr exciton radius, which is characteristic of each type of semiconductor. A quantum dot is smaller than the Bohr exciton radius, which causes the appearance of discrete energy levels. The band gap, ΔE, between the valance and conduction band of the semiconductor is a function of the nanocrystal's size and shape. Quantum dots feature slightly lower luminescence quantum yields than traditional organic fluorophores, but they have much larger absorption cross-sections and very low rates of photobleaching. Molar extinction coefficients of quantum dots are about 10⁵-10⁶ M⁻¹cm⁻¹, which is 10-100 times larger than organic dyes.

Core/shell quantum dots have higher band gap shells around their lower band gap cores, which emit light without any absorption by the shell. The shell passivates surface nonradiative emission from the core thereby enhancing the photoluminescence quantum yield and preventing natural degradation. The shell of type I quantum dots (e.g., CdSe/ZnS) has a higher energy conduction band and a lower energy valance band than that of the core, resulting in confinement of both electron and hole in the core. The conduction and valance bands of the shell of type II quantum dots (e.g., CdTe/CdSe, CdSe/ZnTe) are either both lower or both higher in energy than those of the core. Thus, the motions of the electron and the hole are restricted to one dimension. Radiative recombination of the exciton at the core-shell interface gives rise to the type-II emission. Type II quantum dots behave as indirect semiconductors near band edges and therefore, have an absorption tail into the red and near-infrared. Alloyed semiconductor quantum dots (CdSeTe) can also be used, although types I and II are most preferred. The alloy composition and internal structure, which can be varied, permits tuning the optical properties without changing the particles' size.

Particularly preferred quantum dots are selected from the group consisting of CdSe/ZnS core/shell quantum dots, CdTe/CdSe core/shell quantum dots, CdSe/ZnTe core/shell quantum dots, and alloyed semiconductor quantum dots (e.g., CdSeTe). More preferably, the quantum dots are less than about 10 nm in diameter, even more preferably from about 2 nm to about 5.5 nm in diameter, and most preferably from about 1.5 nm to about 4.5 nm in diameter. If different color emission is needed for creating multiple sensors (multiplex detection), this can be achieved by changing the size of the quantum dot core yielding different emission wavelengths. The quantum dots can be stabilized or unstabilized as discussed above regarding particles. Preferred ligands for stabilizing quantum dots are resorcinarenes.

**In** a graphene biosensor of the disclosure advantageously, the graphene core particle acts as a quencher for the detectable moiety's signal, such that there is no need for a co-attached quencher particle to be separately added to the sensor (e.g., directly attached to the carrier particle). Thus, unlike other sensors, the particles (detectable moieties) attached to the graphene core particle interact with the graphene core particle carrier particle, but are typically selected and arranged (e.g., in proximity to one another and the carrier particle) such that they do not interact with one another, and only interact with the graphene core particle in terms of sensing functionality. (see e.g. Characteristic of peptides attached to the graphene particle illustrated and exemplified in the Examples section).

Accordingly in embodiments herein described, core particles and biosensors each comprise a graphene core particle in which at least one detectable moiety is attached to pristine graphene nanosheet particulate, via a peptide linkage. In this configuration, the detectable moiety is at a quenching distance with respect to graphene and the detectable signal of the detectable moiety is quenched and either muted or undetectable due to its proximity to the central graphene nanosheet particulate. The peptide linkage comprises a recognition sequence that is specific for a target biomarker, such that the target biomarker, if present in a tested sample, will interact with (and typically cleave or modify the conformation of) the peptide linkage thereby bringing the detectable moiety at an emitting distance where quenching of the graphene particle does not occur (e.g. by releasing the detectable moiety or by modifying the distance by changes of the related conformation). Once released or brought to an emitting distance through changes in conformation, the detectable moiety is no longer quenched by the central graphene nanosheet particulate and its signal (or a change in its signal) can be detected and thereby indicate the presence and/or activity of the target biomarker in the sample.

**In** most embodiments herein described, in the graphene biosensor of the disclosure the diameter of the attached polymer layer plus co-attached consensus sequence does not exceed 20 nm to allow placement of the detectable moiety at a quenching distance with respect to the graphene particles in which the graphene quenches the signal of the detectable moiety (see Characteristic of Polymers illustrated and exemplified in the Example section).

Accordingly in some embodiments, the coating material in the graphene core particle and/or in biosensor of the disclosure has a thickness equal to or lower than 18 nm to permit a minimum of 2 nm for the attached peptide sequences that feature targeting abilities.

In some embodiments, the coating material in graphene core particle and/or in the biosensor of the disclosure has a thickness from 8 to 17 nm, from 2 to 18 nm, or from 5 to 15 nm depending on the size of the detectable component attached or to be possibly attached to the coating material as will be understood by a skilled person upon reading of the present disclosure.

In some embodiments herein described allowable ratio of peptide length (A) and polymer layer thickness (B) in graphene biosensors for protease activity detection can be detected with procedures exemplified in **Example 31.**

In some embodiments, wherein the target biomarker performs post-translation modification the layer thickness is close to 20nm before binding to their target or posttranslational modification. Both events elongate the peptide sequence bringing the detectable moiety to an emitting distance wherein the graphene particle does not quench the detectable moiety, thus causing a fluorescence increase from a tethered fluorophore.

Biosensors of the disclosure have been surprisingly found to have not only a high stability but also a high reproducibility of the related signal detection in particular in embodiments where the graphene layer comprises 2 to 100 sheets of pristine graphene, more preferably fractal pristine graphene, coated with coating material for at least 90% preferably at least 99% and more preferably up to 100% of the graphene particle. In those embodiments target biomarker can be detected with high reproducibility and in particular with a coefficient of variation in detected signal between different manufactured lots or batches of less than 10%, wherein the signal is the amount of fluorescence above the sensor-only background for each lot.

In embodiments herein described, the reproducibility of nanobiosensor is determined by the ability to reproduce a condition of measurement of a defined biological sample which can be expressed in terms of a coefficient of variation.

Accordingly, the high reproducibility of the biosensor of the disclosure allows a skilled person to formulate a core particle modified to be chemically stable in an aqueous solution and attached to a varied sensor moiety in accordance with the disclosure, in independent assemblies to produce the same amount of signal from a single biological sample. To have value for measuring biological processes, the amount of signal released by a defined biological sample must be reproducible.

**In** embodiments of graphene biosensors herein described where the graphene layer comprises 2 to 100 sheets of pristine graphene, coated with coating material for at least 90%, the difference between different manufactured lots or batches of sensor are expected to display a signal with a coefficient of variation of less than 10% for each build.

In particular order to detect reproducibility the quantity of sensor added to a reaction is performed preferably using absorbance rather than being reliant on mass. In particular, the concentration of particle can be determined by absorbance measurements using a standard curve with R² > 0.99. Under these conditions, the concentration of particles can be adjusted with a CV% <4%.

In embodiments herein described wherein a suspension of particles in which graphene layer comprises a particle from 10 nm and 5000 nm preferably from 150 to 500nm on average as determined using scanning electron microscopy and digital light scattering measurements, coated with coating material for at least 90%, the reproducibility can also be detected in terms of stability of an aqueous suspension comprising the sensors, the reproducibility assessed by assessing the colloidal stability at OD₆₅₀ or OD₂₇₀. within a set amount of time. In embodiments where the particle has a dimension of 10 nm to 5000 and preferably from 150 to 500 nm, the average decrease in OD650 between measurements is of 24% or less, and in preferred embodiments, typically 10% or less.

In those embodiments, the configuration of the sensor allows a formulation in which the sensor remains in an evenly suspended state in an aqueous solution. This characteristic allows even distribution of the particles and the subsequent interaction with rare specific biomarker present in a biological sample present at a concentration as low as femtomolar. Stability is further supported by modification of the few layer graphene with surface modifications that mask the nonpolar nature of the graphene surface. These modifications introduce charges that allow the particles to remain in suspension in an aqueous environment. Introduction of polar modifications are required to support a preparation of sensor in buffered solution of at least 1mg/ml. With a stable solution of at least 1 mg/ml, all sensors can be mixed in functional concentrations with the sample between 5 and 500 µg/mL. Preferably, sensor concentration is held at 25, or 30, or 50 or 100, or 200, or 300 µg/mL. The optimal functional concentration can be determined for each sensor based on the specific components and related features. The combined impact of introducing size and charge modifications is to produce a solution that maintains a stable suspension of particles such that the absorbance of the graphene nanobiosensor does not change over a 1 hour period at room temperature. This solution stability is assessed by assessing the colloidal stability at OD₆₅₀ or OD₂₇₀ over a 60 minute interval at room temperature. Graphene sensors show a change in OD₆₅₀ of -20 to - 26%. Absorbance of graphene sensors at OD₂₇₀ can show a change of -17% to -21%. Therefore, the colloidal suspension of graphene sensor remains in suspension more efficiently than other particles known in the art as will be understood by a skilled person.

**In** preferred embodiments, the graphene particle has a size of 900 nm or less, preferably 350 nm or less, more preferably 250 nm or less, the detection can be performed for target biomarkers present at concentrations as low as femtomolar.

Biosensors of the disclosure have been also surprisingly found to have not only a high stability and a high reproducibility of signal detection, but also a high signal to noise ratio in particular in embodiments where the graphene layer comprises 2 to 100 sheets of pristine graphene, more preferably fractal pristine graphene, coated with coating material for at least 90%, preferably 100% of the surface of the graphene particle to minimize interference with the fluorescence readouts due to adsorption and quenching of formerly cleaved off detectable moieties (such as fluorescent dyes) at the surface of uncoated graphene.

In particular, biosensors with the above features provide a signal to noise performance (S:N) in aqueous solutions of at least 25 or higher. In experiments performed with exemplary biosensor having the above features, the relationship of sample incubated with sensor and serum at time 0 for the biosensor and found the relative fluorescence index (RFI) or sample RFU/Assay control RFU averages 4.3 +/- 1.9. Furthermore, the biosensors maintained in aqueous solution for up to 90 min show no significant change in background signal and integrated S:N increases on average from 25 to 46, to 159, to 312 across multiple sensors as different lots of biosensor are measured. Therefore, the integrated S:N, evaluating both the signal to noise ratio at t=0 and t=60 demonstrates an increase of sensitivity for defined stimulus of between 3-fold and 13-fold as the graphene biosensor size and solvent contamination is stabilized (see representative **Example 44** and corresponding **Figure 59** and **Figure 60****).** This S:N performance is dependent on the sensor as will be understood by a skilled person.

In particular, in those experiments performed with exemplary nanobiosensor herein described a 25-fold improvement was measured averaging 4 different sensors (see **Example 17,** **Figure 20** in Lot 1 formulation, **Figure 59****)** ranging from 8- to 50- as will be understood by a skilled person. The same 4 sensors had improved to 312 SN average (see **Example 40** and **Example 43** Lot 4) with a range from 109- to 623- fold S:N. In comparison iron particle sensors had an average SN of 20 (range 6- to 42-) for the same 4 sensors measured with the same buffer and samples at the same time (see **Example 44).**

In some embodiments, the biosensors can have different lengths and configurations of the sensor within the above configurations. For example in some embodiments, the length of the peptide tether can exceed 50 amino acids. This is possible for example in all cases when back-bending of the peptide chain is envisioned to bring the detectable moiety at an emitting distance.

In some embodiments, the peptide linkage can be attached to the coating layer or to the graphene surface of a graphene core particle of the disclosure and/or to the detectable moiety, indirectly through moieties herein also indicated as spacers or connectors. For example, the exemplary peptide linkages of Table 1 can comprise before the N-termini of each sequence the spacer GAG, and the spacer AG is used at the C-termini. Selection of proper spacers in the graphene biosensor of the disclosure can be performed in view of the target biomarker to be detected and of the quenching distance and emitting distance of the specific biosensor as will be understood by a skilled person (see **Example 9).**

In some embodiments, the peptide linkage are oligopeptides comprising a 3 amino acid spacer GAG, and 4+4 amino acid consensus sequence, and a 2-3 amino acid spacer (G)AG, the length is 5±0.5 nm. In some embodiments, the dimension of the layer is 2nm (functionalized graphene) + 12 nm (coating material such as PEI ) = 14 nm. 14nm + 5 nm peptide linkage comprising a spacer = 19 nm < 20 nm.

**In** some embodiments, the graphene-based biosensors are configured for detection methods based upon surface plasmon resonance and FRET between the graphene carrier particle and the detectable moiety. FRET describes energy transfer between two particles. Surface plasmon resonance is used to excite the particles. A donor particle initially in its excited state, can transfer this energy to an acceptor particle in close proximity through nonradiative dipole-dipole coupling. Briefly, while the detectable particle is bound by the oligopeptide in its initial state, a first emission is observed upon excitation of the donor particle. Once the target biomarker binds and/or cleaves its recognition sequence, FRET change is observed, and the emission spectra changes. Typically, once the distance between the two particles is changed by the biomarker (either by cleaving or lengthening the peptide linkage), light is emitted from the detectable particle and a distinct blue-shift in absorption and emission can be observed. This is because the distance between both particles increases. In some embodiments, the signal intensity from the detectable particle increases as the distance between the two particles increases.

In biosensors herein described, in view of the photophysical properties of graphene plasmonic quenching is dominant contrary to other nanomaterials characterized by an overlay of plasmonic excitation and quenching as will be understood by a skilled person.

In general, excitation of the biosensor is directed towards the particle having a higher energy state (e.g., the donor particle). Since the absorption spectrum of graphene (and carboxygraphene) changes only very little between UV and near-IR, these biosensors are capable of working in a broad optical region. Excitation of the detectable particle can advantageously be performed between about 300 nm and about 1500 nm. Excitation is typically performed with an energy source of appropriate wavelength selected from the group consisting of a tungsten lamp, LED, laser, and/or bioluminescence (e.g., luciferase, renilla, green fluorescent protein). The changes in absorption and/or emission of the particles as the peptide binds, cleaves, or chemically modifies the oligopeptide linkages will be observed over a time period of from about 1 minute to about 120 minutes, preferably from about 1 minute to about 60 minutes, and in some cases from about 1 minute to about 30 minutes, depending upon biomarker activity. In practice, the assay can first be calibrated for the particular biosensor using a control sample with and without known concentrations of the target biomarker.

**In** particular the pristine graphene nanosheet are within a core particle herein described and the attaching is performed by attaching the detectable moiety to the chemically transformed surface of the core particle herein described. In embodiments wherein the core particle comprises a layer of an organic and/or inorganic molecule, the chemically transformed surface is coated with a layer of an organic and/or inorganic molecule and the attachment is performed through binding of the peptide linkage to the layer of an organic and/or inorganic molecule.

In most preferred embodiments, the attaching is preceded by dispersing the pristine graphene nanosheet (e.g. by sonication) and the attaching is performed on the dispersed pristine graphene nanosheet.

In preferred embodiments, dispersion is performed with graphene particle comprising fractal graphene. In those embodiments, dispersal can be desirable to decrease the representation of larger fractals in the population of graphene going into manufacturing. In some embodiments wherein detection of target biomarker at femtomolar concentration, the particle population size of the particles is typically between 10 and 5000nm, optimally between 50 and 500nm to support a homogenous and stable aqueous solution of particle. The use of sonication introduces energy into the system to disrupt the electrostatic bonds and Van der Walls forces holding fractals together. Once disrupted, the coating reaction (e.g. polymerization) captures the population as represented. Sonication represents one mechanism that introduces energy into the system some of which will be used to break larger fractals. This will continue to an equilibrium depending on the power of the sonication. Introducing between 2 and 40kJ or between 10 and 20kJ or more preferably between 12 and 15kJ using a microtip probe sonicator in a 200mL solution of few layer graphene in DMF produces a population of suitable particles. Alternatively, the size of the fractal population can be modified by dispersal using a high shear homogenizer. Alternatively, energy is expected to be possibly supplied by microwave to disrupt the larger fractals, or by extrusion under high pressure. These methods use mechanical methods to disperse large fractals to decrease the overall population size. Alternatively size selection is expectedly performed to select a population representing 50-500nm using size selection techniques such as filtration or size fractionation or sedimentation under centrifugation. The examples include references probe sonication (see **Example 22** and related **Figure 27** **and** **Figure 29**). The effective endpoint is determined by determining the size of the end population after treatment for dispersal by any method such as TEM or light scattering methods. Using Absorbance efficiency measured by OD270/mg/ml efficiencies were observed of >1.0, and >2.0 and >3.0 as dispersal steps are included. (see **Example 40** and related data in the **Table** **Figure 55** representative of the efficiencies for the preferred embodiments).

**In** embodiments herein described, graphene particles of the disclosure are functionalized to present a functional group, FGg, which is selected to allow attachment of a corresponding functional group of the coating material FGcg, Attachment of the coating material through binding of the groups FGg and FGcg is preferably performed following dispersion of the functionalized graphene particle and results in a core graphene particle presenting a functional group FGcp configured to bind a corresponding functional group FGp presented in the peptide linkage.

**In** some embodiment, based on the appropriate coupling chemistry as exemplified in Schemes (I) to (IX), the following scheme for the synthesis of a biosensor is described. 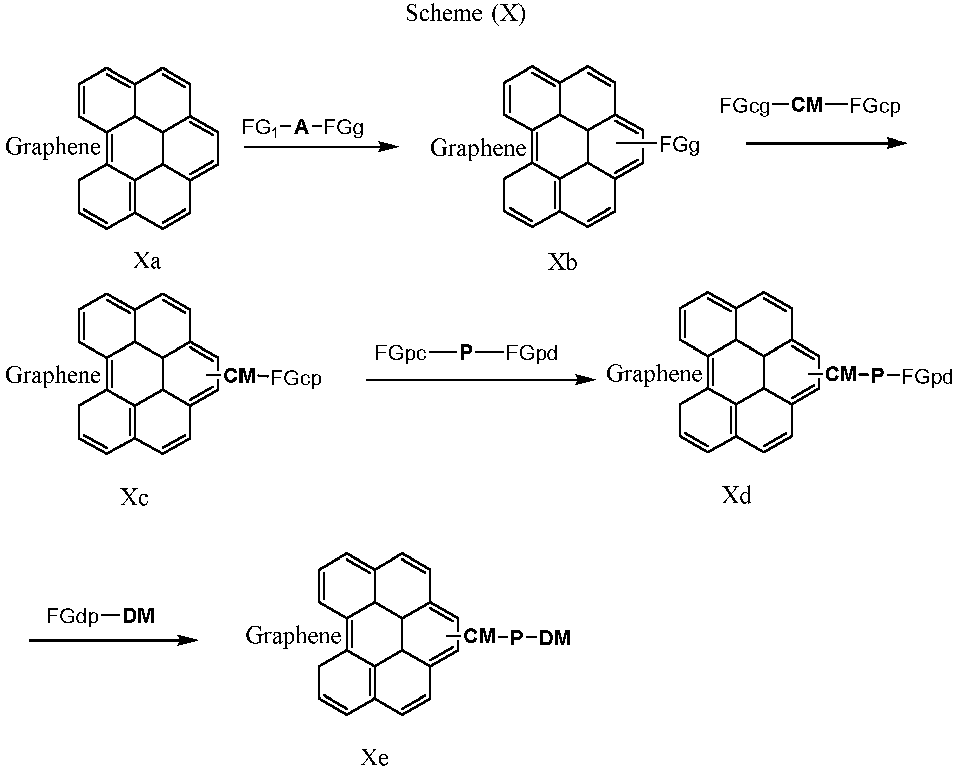 wherein **A** is a reagent configured to functionalize the graphene, **CM** a coating material in the sense of the disclosure, **P** peptide linkage in the sense of the disclosure, and **DM** a detectable moiety.

As shown in Scheme X, graphene of Formula (Xa) is reacted with reagent FG1-**A-**FGg wherein FG1 is a reactive functional group that will react or be made to react with graphene and FGg is a functional group capable of being chemically coupled to a coating material, **A** is a chemical moiety of C2 to C4, or C5 to C12 substituted or unsubstituted linear or branched alkyl group wherein one to 4 carbon atoms may be replaced by one or more of heteroatoms selected from O, S, or NH. Exemplary FG1 includes but are not limited -N₃ (azide) which decomposes to nitrene under heating conditions, maleimide which is a dienophile. Exemplary FGg includes -COOH or NH₂.

The functionalized graphene of Formula (Xb) reacts with functionalized coating material FGcg-**CM**-FGcp, wherein FGcg is a functional group capable of reacting with FGg on the functionalized graphene. Thus, when FGg is -COOH, exemplary FGcg can be NH2 to form an amide bond with FGg.

The coated graphene of Formula (Xc) comprises functional group FGcp, wherein the FGcp is a functional group capable of reacting with FGpc on a functionalized peptide FGpc-**P**-FGpd. Similarly, exemplary FGcp includes -COOH or NH₂. Thus, when FGcp is -COOH, exemplary corresponding Fpc can be NH₂ to form an amide bond with FGcp.

The peptide derivatized graphene of Formula (Xd) comprises functional group FGpd, wherein the FGpd is a functional group capable of reacting with FGdp on a functionalized detecting moiety FGdp-**DM**. In a similar fashion, exemplary FGdp includes -COOH or NH₂. Thus, when FGdp is -COOH, exemplary corresponding Fpd can be NH₂ to form an amide bond with FGdp.

It is to be understood that any of the pair of coupling functional groups herein described can be practiced with any other coupling functional groups, as exemplified in Scheme (I) to Scheme (IX) herein described. Therefore, when one functional group is amine -NH₂, the other functional group can be, besides carboxylic acid, NHS (Scheme V), or oxirane (Scheme IX).

In some embodiments herein described the assembly of the coating on the particle preferably dispersed can be performed by attaching a coating layer to the surface of a graphene particle first to provide a coated particle presenting a functional group of the coating layer FGcp such as amide, amine, ester, ether, thioester, or thioester bond and then by attaching the peptide linkage to the functional group FGcp through a corresponding functional group of the peptide linker FGp. In some embodiments, the linkeris attached to the polymer first and then attached to graphene. The attachment can be performed directly or indirectly and can be through covalent or non-covalent binding depending on the corresponding functional group as will be understood by a skilled person.

In some embodiments, peptide linkages of the disclosure can be attached through a spacer configured to attach the graphene directly such as EPLQLKM[27] (targeting the edges of graphene) and HSSYWYAFNNKT[27] (adsorbing on flat graphene surfaces) to anchor any peptide sequence to pristine graphene. [27] (adsorbing on flat graphene surfaces) to anchor any peptide sequence to pristine graphene.

In some embodiments, peptide linkages of the disclosure can be attached to a terminal cysteine oxidized to a thiyl radical that is attached to graphene.

In some embodiments, non-natural amino acids can be tethered to the N-terminal end of the peptide sequence to perform the linking (all chemistries as described above).

In some embodiments, coating material can comprise oligoethylene groups of < 20 nm, starburst dendrimers with a diameter < 20 nm, and block-copolymers: In some embodiments, when coating material can comprise block copolymers are being used, hydrophobic polymers, such as polystyrene, polyisobutylenes, polyvinylchloride, polyethylene, polypropylene and additional polymers identifiable by a skilled person. can be components of block copolymers in combination with hydrophobic and charge attraction when negatively charged polymers, such as polystyrene-sulfonic acid are used in block copolymers of polystyrene. In those embodiments hydrophobic-hydrophilic block copolymers can be dissolved in a solvent that also disperses graphene well (e.g. dimethylformamide (DMF)). Under continuing sonication, H₂O is added to the mixture, forcing the hydrophobic end of the block-copolymer to bind to graphene. This supramolecular attachment happens spontaneously in the dispersion, due to increasing hydrophobicity decrease due to continuous H₂O addition. Accordingly, as long as this sensor is used in aqueous solvents, this attachment principle is expected to work reliably.

In accordance with the property of graphene biosensors in the sense of the disclosure in aqueous solution, the graphene biosensors have also been found to be surprisingly stable and capable of providing a highly reproducible detection of target biomarkers in an aqueous sample.

The term "sample" as used herein indicates a limited quantity of something that is indicative of a larger quantity of that something, such as fluids from a specimen such as biological environment, cultures, tissues, commercial recombinant proteins, synthetic compounds or portions thereof.

The word "specimen" as used herein indicates a portion of matter from an environment for use in testing, examination, or study. The environment can comprise individuals. When referred to as a noun, the term "individual" as used herein in the context of detection, diagnosis and treatment refers to a single biological organism, including but not limited to, animals and in particular higher animals and in particular vertebrates such as mammals and in particular human beings. In these instances, a specimen can include a portion of tissues, organs or other biological material from the living being such as urethra, urine, cervix, vagina, rectum, oropharynges, conjunctiva, or any body fluids.

**In** particular biological samples can comprise one or more cells of any biological lineage, as being representative of the total population of similar cells in the sampled individual. Exemplary biological samples comprise the following: whole venous and arterial blood, blood plasma, blood serum, dried blood spots, cerebrospinal fluid, lumbar punctures, nasal secretions, sinus washings, tears, corneal scrapings, saliva, sputum or expectorate, bronchoscopy secretions, transtracheal aspirate, endotracheal aspirations, bronchoalveolar lavage, vomit, endoscopic biopsies, colonoscopic biopsies, bile, vaginal fluids and secretions, endometrial fluids and secretions, urethral fluids and secretions, mucosal secretions, synovial fluid, ascitic fluid, peritoneal washes, tympanic membrane aspirate, urine, clean-catch midstream urine, catheterized urine, suprapubic aspirate, kidney stones, prostatic secretions, feces, mucus, pus, wound draining, skin scrapings, skin snips and skin biopsies, hair, nail clippings, cheek tissue, bone marrow biopsy, solid organ biopsies, surgical specimens, solid organ tissue, cadavers, or tumor cells, among others identifiable by a skilled person. Biological samples can be obtained using sterile techniques or non-sterile techniques, as appropriate for the sample type, as identifiable by persons skilled in the art. Some biological samples can be obtained by contacting a swab with a surface on a human body and removing some material from said surface, examples include throat swab, nasal swab, nasopharyngeal swab, oropharyngeal swab, cheek or buccal swab, urethral swab, vaginal swab, cervical swab, genital swab, anal swab, rectal swab, conjunctival swab, skin swab, and any wound swab. Depending on the type of biological sample and the intended analysis, biological samples can be used freshly for sample preparation and analysis, or can be fixed using fixative.

In methods and systems herein described a sample can be split in two or more parts and/or processed through treatment to enrich the target biomarker and/or remove selected materials to increase the quality of the detection. Accordingly, the term sample as used herein comprises samples that are chemically treated or storage, preservation, or further analysis.

Samples used in connection with graphene core particles and related graphene biosensors compositions methods and systems are aqueous samples.

**An** "aqueous sample" in the sense of the disclosure is a sample in which water is the solvent or main component. In particular, an aqueous solution typically includes a buffer component at a concentration capable of maintaining a defined pH in the presence of sensor and sample for the duration of the experiment.

**In** embodiments, herein described, it has been found that graphene biosensors in the sense of the disclosure having 2 to 100 pristine graphene sheets, preferably fractal graphene, coated with at least 90% coating material preferably are surprisingly stable in an aqueous sample and capable of providing a detection characterized by a high reproducibility and signal to noise ratios in particular when having dimension 10 nm to 5000 and preferably from 150 to 500 nm.

**In** particular, in some embodiments, the sensors of the disclosure including these features show an absorbance to replicate sensor concentration (independent batches of a biosensor solution having the same features) with a coefficient of variation CV of <3%. Achievement of this CV is obtained by having a solution with sensors that remain evenly distributed in a reaction determined. The preparation of nanosensors prepared with a method comprising a dispersion step (e.g. using sonication) and solvent removal produced solutions of sensors that maintained colloidal stability with less than 15% settling in a 60-min interval. In particular, in embodiments wherein the particles are provided by a method comprising a particle dispersing step and solvent removing step settling was observed from 9% to 10%.

**In** some embodiments, reproducibility of the sensors of the disclosure can also be detected through a reproducible estimate of both the assay control and the sample as these two values are divided to estimate S:N. Sensors of the disclosure display a CV% between batches of <20%. In this connection, biosensor of the disclosure with the above features combination can provide a batch to batch CV% of between 3% and 10%. Optimally, the CV% between batches **will be <10%**. This is assessed by preparing independent batches of sensor and comparing the SN (SM/AC) under matched conditions with a standard stimulus. (see **Example 22** and related **Figure 27** and **Example 44** and related **Figure 59****).**

In some embodiments, S:N of graphene biosensor of the disclosure can be determined by maintaining a low, stable assay control reading. For example, for biosensors evaluated on a VarioSkan Lux, the AC is between 0.01 and 0.1. The signal is dependent on the sensor and the stimulus. Sample signal for any given sensor using the same stimulus can be improved 15-fold with dispersed, solvent cured sensors. Further, incremental addition of dispersion (6.8-fold average increase in SN) and dispersion and solvent removal (average 15-fold increase in SN) indicates the combined use of the core particle (see **Example 44** and related **Figure 59****),** (1) dispersed using sonication or the equivalent to an Absorbance efficiency of >3.0 OD₂₇₀/mg/ml (see **Example 40** and related **Figure 55****),** and (2) reducing the amount of solvents such that TGA measures a total weight loss of less than 5% (wt%), possibly less than 2%, are associated to this S:N performance (see **Example 21** and related **Figure 25****).**

**In** some embodiments, reproducibility and signal to noise ratio can be increased by performing appropriate buffer selection. Buffer selection can be performed based on a selection from Good's list that reflects the most appropriate pH target for biomarker detection. The addition of monobasic salts increases the S:N performance to an extent dependent on the concentration of salt added as will be understood by a skilled person upon reading of the present disclosure. Monobasic salt decreases the Assay control relative to the sample producing an increased S:N ratio. Inclusion of monobasic salt decreases the assay control to between 0.01 and 0.1 RFU on a VarioSkan Lux on average. Overall, the impact of buffer across all sensors tested increases the S:N 4-fold on average (range 2.5-fold to 6.7-fold) (see **Example 42** and related **Figure 57****).** Certain protease enzymes require buffer co-factors such as divalent cations. In preferred embodiments for protease detection, the buffer includes 10µM Calcium, Magnesium and Zinc divalent ions. Formulations tested focused on pH7.2 as a physiologically relevant pH that was used across all sensors. However, the tumor environment and intracellular compartments often reflect pH below 7.0. So the pH does not have to be one condition except when reaction chambers are shared as in a multiplex reaction. Some enzymes require acidic conditions and for any example, pH7.0 and pH6.0 and 6.4 and 6.8 are expected to support enzymes such as cathepsins or enzymes derived from inflammatory environments. Receptor ligand interactions are preferably measured at physiological conditions so 155mM and pH7.2 is a condition broadly supportive of many sensor formulations as will be understood by a skilled person.

**In** some embodiments the graphene biosensors of the present disclosure comprise a plurality of detectable components each comprising a detectable moiety and recognition sequence specific for a target biomarker.

In some of those embodiments, the recognitions sequences of the detectable components of the plurality of detectable components configured to specifically recognize different target biomarkers.

Accordingly, in those embodiments the graphene biosensors of the disclosure are configured for multiplex detection, and to simultaneously measure multiple analytes in a single experiment.

In particular the terms multiplexing as used herein indicates embodiments where two or more target biomarkers can be detected in a same reaction, using the same reagent mix.

In the plurality of target-specific detectable components of the nanobiosensor, the excitation spectrum of each detectable moiety has a minimized overlap with the emission spectrum of another detectable moiety of the plurality of target-specific detectable components. Accordingly, in those embodiments, each detectable moiety is assigned to a specific biomarker with minimal to no overlap between the excitation and emission spectra of the detectable moieties of the plurality of detectable components to minimize FRET effects among detectable moieties that will distort the readout.

In graphene sensors configured for multiplex detection in view of the properties and features of the graphene sensors, the multiplex ability of sensors having 2 to 100 graphene sheest and coating layer coating at least 90% of the graphene surface, is 5 or higher number of peptide sequences +detectable moiety, possibly up to 10 in the UV/Vis spectrum, and up to 50 for the entire electromagnetic spectrum.

In some embodiments of the graphene biosensor herein described a detectable component comprising a peptide and a detectable moiety (e.g. a fluorophore) can be comprised in multiplex biosensor a concentration up to 1.0 ± 0.25 × 10⁻³ moles per gram. In some embodiments, wherein the fastest signal increase over the concentration range from femtomolar to micromolar of a target biomarkers (e.g. a protease) is desired, the concentration of detectable component can be 1.6 ± 0.2 × 10⁻⁵ moles per gram of graphene-nanobiosensor as will be understood by a skilled person upon reading of the disclosure.

Accordingly, in some of those embodiments where the biosensor is a multiplex biosensor in view of the concentration of detectable component (peptide sequences + detectable moiety of 1.0 ± 0.25 × 10⁻³ moles per gram of nanobiosensor) is to be divided by the number of peptide sequences + detectable moiety that are being used for the purpose of multiplexing. If unequal concentrations of each detectable component are being used, the sum of all partial concentrations for each peptide sequence + detectable moiety has to be equal to 1.0 ± 0.25 × 10⁻³ moles per gram of nanobiosensor, as will be understood by a skilled person.

Additionally, in some of those embodiments where the biosensor are multiplex biosensor, in view of the preferred concentration of tethered peptide sequences + detectable moiety is 1.6 ± 0.2 × 10⁻⁵ moles per gram of nanobiosensor to obtain the fastest signal increase over the concentration range from femtomolar to micromolar of a target biomarker (e.g. a protease), this concentration is to be divided by the number of detectable components that are being used for the purpose of multiplexing. If unequal concentrations are being used, the sum of all partial concentrations for each peptide sequence + fluorophore has to be equal to 1.6 ± 0.2 × 10⁻⁵ moles per gram of nanobiosensor, as will be understood by a skilled person upon reading of the present disclosure.

Accordingly, in some preferred embodiments of multiplexing nanobiosensors herein described, the concentration of the sum of all utilized peptides+fluorophores can range from 1.0 ± 0.25 × 10⁻³ moles per gram to 1.0 ± 0.25 × 10⁻⁷ moles per gram, preferably from 1.0 ± 0.25 × 10⁻⁴ moles per gram to 1.0 ± 0.25 × 10⁻⁶ moles per gram, and even more preferably from 1.0 ± 0.25 × 10⁻⁵ moles per gram to 2.0 ± 0.25 × 10⁻⁵ moles per gram, as will be as will be understood by a skilled person upon reading of the present disclosure.

**A** similar multiplex ability can be achieved by using composition comprising a plurality of biosensors of the disclosure each presenting a detectable component specific for different target biomarker. In those embodiments, the differently assembled sensors can be mixed such that as a fraction of the reaction, each sensor can still produce a signal that is greater than background signal defined as sensor only. In this context, the biosensor specific for a target biomarker can be mixed with 1, or 2, or 3, or 4, or 5, or 6, or 7, or 10 or 20 different sensors and still produce a signal greater than background. At a 1:20 representation, a multiplexed sensor is detectable at 5-7% of the expected value. Therefore multiplexed sensors can be detected at 1 of 20, or 10 or 4 or 2 multiplexed sensors.

In some embodiments, multiplex detection of target biomarkers can be performed in connection with detection of a condition associated with a plurality of target biomarkers. For example, sensors of the disclosure can be used for multiplex detection of MMP-9, CTS-K, MMP-2 and ARG for presence and absence of lung cancer. In another example, sensors of the disclosure can be used for multiplex detection of arginase and IL-13, for detection of anti-inflammatory events and conditions and of IL-6, GCSF, MIP-1, and MCP-1, for detection and diagnosis of pro-inflammatory events and conditions in an individual.

### Exemplary process for making multiplex

In some embodiments, one or more graphene core particle graphene biosensor herein described are comprised in a composition together with a suitable auxiliary agent. The term "auxiliary agent" as used herein indicates any of various media acting usually as solvents, carriers, binders or diluents for the graphene biosensor herein described that are comprised in the composition as an active ingredient. In particular, the composition including the one or more oximes can be used in one of the methods or systems herein described.

In particular, in some embodiments, for storage and/or use, the biosensors would typically be dispersed into a pharmaceutically acceptable solvent system, which could be any type of suitable diluent, excipient, vehicle or the like. As used herein, the term "pharmaceutically acceptable" means not biologically or otherwise undesirable, in that it can be administered to a subject without excessive toxicity, irritation, or allergic response, and does not cause unacceptable biological effects or interact in a deleterious manner with any of the other components of the composition in which it is contained.

In some embodiments, a pharmaceutically acceptable carrier can be selected to minimize any degradation of the biosensor components as would be well known to one of skill in the art and to minimize any adverse side effects in the subject (for in vivo administration), as would be well known to one of skill in the art. Exemplary carriers include non-aqueous solutions, such as diethyl ether, DMF, DMSO, and the like. The biosensors are shelf-stable at room temperature conditions, even up to elevated temperatures up to 100°C without deterioration, and can be stored and shipped without special handling requirements (e.g., without refrigeration).

Pharmaceutically acceptable ingredients include those acceptable for veterinary use as well as human pharmaceutical use, and will depend on the route of administration. For example, compositions suitable for administration via injection are typically solutions in sterile isotonic aqueous buffer. Exemplary carriers include aqueous solutions such as normal (n.) saline (-0.9% NaCl), phosphate buffered saline (PBS), sterile water/distilled autoclaved water (DAW), or other acceptable vehicles as will be understood by a skilled person.

The graphene carrier particles are stable, for an indefinite amount of time based on the data of the present disclosure. The peptide linkages are also stable for long periods of time (years). It will be appreciated that the particular compound selected for the detectable moiety may reduce the shelf stability of the overall biosensors, and the particular sensors could be stored under more careful conditions (e.g., under argon) to preserve delicate detectable moieties for longer periods of time, if desired.

It will also be appreciated that the biosensors can be fabricated in sequential stages where graphene carrier particles can be stored until use, at which time the peptide linkages and detectable moieties are added (e.g., directly before shipping to the end user or added by the end user). Likewise, the graphene carrier particles can be pre-conjugated with selected peptide linkages and stored without attached detectable moieties, which can be added at a later time closer to use. Notably, the graphene carrier particles have a higher tolerance for mislabeling, where a detectable moiety that is accidentally attached directly to the graphene particle itself (and not via a linkage) should not interfere with other correctly attached detectable moieties. That is, the signal of the incorrectly attached carrier particle will simply be permanently quenched due to its proximity of the carrier particle. Accordingly, the biosensors have higher tolerance thresholds during manufacturing further contributing to their advantages.

In embodiments, herein described the composition can comprise components additional to the graphene core particle and/or biosensors of the disclosure in suitable concentrations as will be understood by a skilled person. In particular, compositions in the sense of the disclosure can typically comprise a salt and buffer (a substance that is capable of maintaining the pH of a solution in which it is dissolved) selected to maintain the pH of the solution in which it is dissolved at a controlled pH. In an exemplary preferred embodiment, the composition can comprise 5mM MES with 10µM divalent cations and 155mM NaCl as well as additional combinations of salts and buffers identifiable by a skilled person upon reading of the present disclosure.

**In** one or more embodiments, the biosensors can be pre-distributed into microplate wells and stored/distributed for use. Black chimney well microplates are particularly suited for this purpose. Opaque microplate configurations are preferred for the assay to reduce ambient light interference and noise. Methods for noninvasive detection and quantification of various biological markers are also described herein. These methods utilize biosensors described herein and can be carried out in health are facilities as an integral part of point of care resources, at home or in the field for assessing biological markers indicative of a variety of conditions and diseases. Such biological marker-based diagnostics could have numerous applications in precision medicine, particularly for airway diseases, for example in the differentiation of lung cancer from other lung conditions.

In preferred embodiments the composition can be in lyophilized form. Auxiliary reagents in support of lyophilization can include Trehelose, Dextrose, Sucrose, Glycine, Magnesium Stearate, Microcrystalline Cellulose, Starch (corn), Silicone/Titanum Dioxide, Stearic Acid, Sodium Starch Glycolate, Gelatin, Talc, Sucrose, Calcium Stearate, Povidone, Pregelantinized Starch, HPMC, OPA products, Croscarmellose, Hydroxypropyl Cellulose, Ethycellulose, Calcium Phosphate, or Crospovidone.

In some embodiments, graphene biosensors herein described can be used in a method of detecting the activity of a biomarker in an aqueous sample comprising contacting a biological sample with any biosensor according to the present disclosure to create a reaction solution and detecting a change in the reaction solution.

**In** preferred embodiments the aqueous sample is a biological sample from an individual. In those embodiments, a biological sample can be provided by collecting the sample from an individual and processing the sample for analysis. In those embodiments, the processing of the sample typically results in a processed aqueous sample configured to be used in combination with detection reagents. In particular the biosensors in a reaction solution can be contacted with the processed biological sample for a period of time sufficient for the target biomarker (if present in the sample) to interact with the recognition sequence in the biosensors. Biological samples include, without limitation, blood, urine, saliva, tears, sputum, bronchoalveolar lavage fluid, nasal swab, breath condensate, milk, feces, rectal fluid, vaginal fluid, and the like. In one aspect, a biological sample is collected from a subject and prepared for analysis. The sample can be collected and prepared manually, which includes, for example, manual pipetting or mixing/diluting of the sample, manual protein spot cutting, manual biopsy collection, manual blood draw, and manual microwell loading. Alternatively, an automated process can be used, which includes, for example, use of automated liquid handlers, automated protein spot cutters, automated biopsy collection, automated blood draws, and automated microwell loading. It will be appreciated that the biological sample may contain the biomarker of interest, including various enzymes described herein, which can be used as biological markers indicative of the health status of the subject. Before, during, or after adding the sample to the reaction solution, a pharmaceutically acceptable buffer can be added to adjust the pH to optimal levels depending upon the biomarker of interest as well as the particular detectable moieties used. For example, slightly alkaline buffers such as HEPES can be used to adjust the pH of the reaction mixture within desired ranges. Moreover, the pH can be adjusted to fine-tune the reaction. For example, certain enzymes, such as MMPs and cathepsins, when taken from cancerous samples are more active under slightly acidic pH (lower limit ~6.6) as compared to non-cancerous samples. Thus, the pH is a parameter that can be optimized within the scope of the disclosure to further enhance the output of the assay. The reaction solution can also be spiked with cofactors such as Mg(II), Ca(II), or Zn(II) to further enhance the enzymatic activity for detection.

When the target biomarker is present in the sample, the detectable moieties generate a detectable signal (e.g., optical or spectroscopic), such as fluorescence or color change, which can be perceived visually or measured with an appropriate instrument. A plate reader can be used to detect the results of the assay. As explained when tethered, the central graphene particle quenches the emission of the detectable moiety. In the presence of the protease or other target biomarker, it cleaves the linkage separating the detectable moiety from the central particle or modifies the linkage extending the detectable moiety outside the quenching proximity. The detectable particle generates a detectable signal that changes depending upon its proximity to the carrier particle, which can be detected and correlated with the activity of the target protein. For example, the maximum absorption of the TCPP is near 420 nm, so an inexpensive 420nm LED or any other kind of laser diode can be used for excitation of biosensors using TCPP as the detectable moiety. It will be appreciated that the biosensors are not limited to the TCPP-graphene FRET pair exemplified in the examples. Any FRET sensing pair can be used. If other FRET pairs are used, then the excitation wavelength and detection device can be modified accordingly.

In some embodiments FRET can be performed over the entire range of the electromagnetic spectrum.

**In** some embodiments photoacoustic detection (the photoacoustic response of various fluorophores) can be distinguished.

In some embodiments radioisotopes could be released from the graphene cores and then filtered off.

In some embodiments, MRI probes can be released from graphene coated with a ferromagnetic material (iron oxide) or high-spin d- and f-block metal complex.

In some embodiments EPR probes can be released from graphene coated with a ferromagnetic material (iron oxide) or high-spin d- and f-block metal complex.

Assays using the biosensors can detect biomarkers down to the sub-femtomolar level (<10⁻¹⁵ moles per liter), which is at least two orders of magnitude more sensitive to competing technologies, such as immunoassays. In fact, as demonstrated in the working examples using a non-optimized biosensor, 10⁻¹⁶ limits of detection (LOD) for proteases are possible. A unique advantage of this technology is that different biomarkers (e.g., several proteases, cytokines and kinases) can be measured in one liquid biopsy. That is, the technology permits simultaneous detection of different classes of biomarkers in a single platform. Thus, in multiplexing embodiments, a plurality of different types of detectable moieties are preferably used so that the assays can analyze a plurality of different types of target biomarkers in a single biological sample. Looking at multiple biomarkers permits the differentiation between related diseases (e.g., lung cancer vs. other diseases) and the detection of diseases in very early stages. The latter is, for instance, important for lung cancer detection, because cancer survival significantly increases when it is detected at stages 0 or 1, compared to 3 or 4.

In particular, in multiplexed biosensor herein described the detectable components are selected so that the excitation and emission spectra of the co-attached detectable moiety (e.g. fluorophores) minimize overlapping. More particular, selection of detectable components in multiplexing embodiments of the biosensors herein described is performed to minimize the occurrence of FRET between detectable moieties such as fluorophores. In this respect, for example selection of a detectable moiety such as BODIPY dyes appear to be well suited, because they show bot narrow excitation and emission spectra as will be understood by a skilled person.

In preferred embodiments, the aqueous sample can be heat treated before performing the contacting and/or during the contacting of the sample with one or more of the graphene biosensors herein described.

In particular in some embodiments a sample in absence of a biosensor as described herein can be heat treated at a temperature ranging from 40 °C to 75°C for 0.5 to 3 hours. Preferably, the sample or serum can be heat treated at a temperature ranging from 50 °C to 60°C for 0.5 to 2 hours. In some embodiments, the sample or serum can be heat treated at a temperature of 55 °C for 1 hour.

In some embodiments of the methods for detection of a target biomarker, the detecting is preceded by a heating of the testing solution in the presence of a biosensor as described herein. An assay sample or serum in the presence of a biosensor as described herein can be heat treated at a temperature ranging from 40 °C to 75°C for 0.5 to 3 hours. Preferably, the assay sample or serum can be heat treated in the presence of a biosensor at a temperature ranging from 50°C to 60°C for 0.5 to 2 hours. In some embodiments, the assay sample or serum can be heat treated the in presence of a biosensor at a temperature of 55°C for 1 hour.

In some embodiments, the heating treatment can be performed according to a protocol for heat treatment of sample exampled in **Example 21.** Heat treatment can be applied to the sample before the assay or during the assay, or both. The impact of heat treatment is to specifically increase the Sample reading while having no effect on the Assay Control. S:N therefore increases because of the increased specific signal. Temperatures between room temperature and 75°C are effective. Preferably, a sample can be pretreated at 65°C for 5 to 12 minutes or the assay is conducted at 41 or 45 °C for the duration of incubation.

In some preferred embodiments, the heating is performed on a solution comprising monobasic salt, and/or incubation in buffer solutions holding a pH = 7.2. In some preferred embodiments, the incubation can be performed at 45°C.

In some embodiments, the biosensor and/or related combination therefore can be configured to detect target biomarkers associated with a target condition in an individual as will be understood by a skilled person.

**For** example, in one or more embodiments, the nanosensors can be configured for rapid diagnosis of lung conditions based upon protease activity profiling of two or more proteases (e.g., in a panel). The consensus sequences experience either proteolytic cleavage by their respective proteases, or the chemical constitution of the posttranslational modification sequence is changed. For instance, arginases I + II convert arginine to ornithine without proteolytic cleavage of the oligopeptide. Thus, methods described herein can also be adapted for use with other nanosensors, such as those described in WO 2016/149637 for detection of enzymatic posttranslational modification as will be understood by a skilled person. The nanobiosensors and methods can be used to detect target biomarkers and thereby diagnose numerous lung conditions, including lung diseases, cancers, and infections.

In one aspect, nanobiosensors for chronic inflammatory lung disorders (asthma, COPD, fibrosis) target the proteases reported in **Table 4** together with corresponding consensus sequences for peptide linkage:

| **Table 4: Target proteases and consensus sequence for peptide linkage** | | |
|---|---|---|
| **Host Protease** | **Consensus Sequence*** | **SEQ ID NO:** |
| Proteinase 3 (PR3) | **LYYV-SLSP** | 95 |
| Cathepsin G (CTG) | **NVLH-SWAV** | 82 |

| | | |
|---|---|---|
| *except where noted, sequences further include N-terminal GAG and C-terminal AG spacers. | | |

In some embodiments, nanobiosensors for pulmonary hypertension (PAH) can be configured to target the proteases reported in **Table 5** together with corresponding consensus sequences for peptide linkage:

| **Table 5: Target proteases and consensus sequence for peptide linkage** | | |
|---|---|---|
| **Host Protease** | **Consensus Sequence*** | **SEQ ID NO:** |
| MMP-2 (Matrix Metalloproteinase-2) | **IPVS-LRSG** | 51 |
| MMP-9 | **VPLS-LYSG** | 55 |
| MMP-14 | **LPQG-LRTE** | 93 |

| | | |
|---|---|---|
| *except where noted, sequences further include N-terminal GAG and C-terminal AG spacers. | | |

PAH can lead to secondary complications, such as COPD (Chronic obstructive pulmonary disease), asthma, chronic bronchitis, and others. The activity of three MMPs is enhanced in PAH, MMP-2, -9, and -14 (MT1-MMP-1). MMP-2, and MMP-9 are capable of cleaving basement membrane-associated collagen IV, which accelerates the remodeling of the pulmonary vasculature. Although MMP-14 is membrane-bound, it can be detected in liquid biopsies (serum, BALF, and potentially EBC) in the state of disease.

**In** some embodiments, nanobiosensors can be designed for the detection of influenza virus infection targeting the protease reported in **Table 6** together with corresponding consensus sequences for peptide linkages.

| **Table 6: Target proteases and consensus sequence for peptide linkage** | | |
|---|---|---|
| **Host Protease** | **Consensus Sequence*** | **SEQ ID NO:** |
| TMPRSS2 (Epitheliasin, transmembrane protease, serine 2) | **RQSR-IVFG** | 86 |
| Human airway trypsin-like peptidase (HAT) | **RSAR-GLKG** | 85 |
| Granzyme B | **VEPN-SLEE A** | 49 |

| | | |
|---|---|---|
| *except where noted, sequences further include N-terminal GAG and C-terminal AG spacers. | | |

In some embodiments, biosensor of the disclosure can be configured for detection of other proteases in the lung, such as TMPRSS2 (epitheliasin) and Human airway trypsin-like peptidase (HAT) which facilitate influenza virus infections H1N1 (A/Memphis/14/96), H2N9 (A/Mallard/Alberta/205/98), andH3N2 (A/Texas/6/96) upon cleavage of haemagglutinin (HA) in those conditions, the viral pathogens are able to stimulate protease expression in an "Influenza-cytokine-protease cycle." The enzymes, expressed as zymogens, are then activated by the network of proteases in the lung. Granzymes are also known to activate H1N1. Therefore, we have included granzyme B in this panel. The advantage of the latter is that it can be detected in EBC as will be understood by a skilled person.

In some embodiments, nanobiosensors can be configured for the detection of viral infections to target the activity of kallikreins in the lung. Kallikreins (KLK's) are a family of proteases consisting of 15 closely related, secreted serine proteases with either trypsin-like or chymotrypsin-like specificity. KLK-2, KLK-3, KLK-4, KLK-5, KLK-6, KLK-12, and KLK-15 are poorly expressed in the lung in healthy individuals. This expression pattern changes in response to viral infections. In case of an influenza infection, the expression of KLK-1, KLK-2 and KLK-5 increases, whereas the expression of KLK-13 and KLK-14 decreases. KLK-13 is increased in virtually all coronavirus (SARS and MERS types) infections. An exemplary kallikreins and related corresponding exemplary consensus sequences for the peptide linkages are reported in **Table 7.**

| **Table 7: Target proteases and consensus sequence for peptide linkage** | | |
|---|---|---|
| **Host Protease** | **Consensus Sequence*** | **SEQ ID NO:** |
| KLK-1 | **EAFR-STGA**** | 98 |
| KLK-2 | **GTSR-SAGG***** | 99 |
| KLK-5 | **YRFR-ILGG** | 100 |
| KLK-12 | **IESK-INGG** | 101 |
| KLK-13 | **VRSR-IVKG** | 102 |
| KLK-14 | **TQPR-IVGG** | 103 |
| KLK-15 | **ALGR-KLVG** | 104 |

| | | |
|---|---|---|
| *except where noted, sequences further include N-terminal GAG and C-terminal AG spacers. ** includes N-terminal GAG spacer and C-terminal GA spacer ***includes N-terminal GAA spacer and C-terminal AG spacer. | | |

In some embodiments, biosensors can be configured for the detection of *Streptococcus pneumococcus* infection target bacterial protease can: An exemplary protease which is a target biomarker for *Streptococcus pneumococcus* and related corresponding exemplary consensus sequences for the peptide linkages are reported in **Table 8.**

| **Table 8: Target proteases and consensus sequence for peptide linkage** | | |
|---|---|---|
| **Bacterial Protease** | **Consensus Sequence** | **SEQ ID NO:** |
| Streptococcus pneumoniae IgA1 protease | **STPP-TPSP**** | 105 |

| | | |
|---|---|---|
| ** includes N-terminal GAG spacer and C-terminal GA spacer | | |

**S.** *pneumococcus* is the most prevalent bacterial infection in the lung and the vast majority if not the totality of streptococci express *Streptococcus pneumoniae* IgA1 protease as will be understood by a skilled person. Accordingly this protease is among the most active proteases known and only present in case of a *Streptococcus* infection. It will permit a rapid test (< 10 min.) for a bacterial lung infection.

In one aspect, nanobiosensors for the detection of *Mycobacterium tuberculosis* infection target the following proteases. An exemplary protease which is a target biomarker for *Mycobacterium tuberculosis* and related corresponding exemplary consensus sequences for the peptide linkages are reported in **Table 9.**

| **Table 9: Target proteases and consensus sequence for peptide linkage** | | |
|---|---|---|
| **Bacterial Protease** | **Consensus Sequence** | **SEQ ID NO:** |
| Mycosin Protease MycP 1 | **VKAA-SLGK* *** | 106 |
| Rv2869c *(Mycobacterium tuberculosis*)*-*like peptidase (RIP-1) | **TCCA-TCAT-CC**** | 107 |

| | | |
|---|---|---|
| ** includes N-terminal GAG spacer and C-terminal GA spacer | | |

Additional secreted *Mycobacterium tuberculosis* proteases which can be used as target biomarker are MycP1 and Rv2869c (*Mycobacterium tuberculosis*)*-*like peptidase (RIP-1), which are both specific for MtB as will be understood by a skilled person.

In some embodiments, nanobiosensors of the disclosure can be configured for the detection of Chronic obstructive pulmonary disease (COPD). An exemplary protease which is a target biomarker for COPD and related corresponding exemplary consensus sequences for the peptide linkages are reported in **Table 10.**

| **Table 10: Target proteases and consensus sequence for peptide linkage** | | |
|---|---|---|
| **Host Protease** | **Consensus Sequence** | **SEQ ID NO:** |
| Neutrophil elastase (NE) | **GEPV-SGLP** | 87 |
| MMP-7 (matrilysin) | **VPLS-LTMG** | 53 |
| MMP-8 | **EPYH-LMAL****** | 89 |
| MMP-12 | **GPAN-LVAP******* | 92 |
| Cathepsin G | **NVLH-SWAV** | 82 |
| Proteinase-3 (myeloblastin) | **SYYV-SLSP** | 96 |

| | | |
|---|---|---|
| *except where noted, sequences further include N-terminal GAG and C-terminal AG spacers. ****includes N-terminal GAP spacer and C-terminal AG spacer. **** includes N-terminal GAA spacer and C-terminal EG spacer | | |

Additional target biomarkers for COPD can be identified and selected based on the fact that endogenous serine protease inhibitors are responsible for pulmonary tissue protection against serine proteases in healthy human subjects. Inherited inhibitor deficiencies are considered the most important reason for the development of COPD, followed by smoking. In the absence of effective inhibitors, uncontrolled proteolysis and, subsequently, lung damage are observed. The most important proteases for COPD detection are neutrophil elastase, neutrophil-derived MMP-8, macrophage-derived MMP-12, cathepsin G and proteinase 3. These proteases are capable of damaging ECM components, such as collagen, laminin, fibrillin, and elastin. Especially the cleavage of lung elastin causes emphysema, which is a driver for COPD pathophysiology. MMP-7 degrades the extracellular proteoglycan decorin, which leads to the subsequent release of decorin-bound transforming growth factor-β (TGF-β). Therefore, the co-activation of all six proteases (NE, MMP-7, -8, -12, CTSG, and proteinase 3) permits the detection of COPD.

In some embodiments, nanobiosensors can be configured for the detection of lung cancer and the differentiation between Small Cell Lung Cancer (SCLC) and Non-Small Cell Lung Cancer (NSCLC) target using a panel of proteases: An exemplary protease which is a target biomarker for SCLC and NSCLC and related corresponding exemplary consensus sequences for the peptide linkages are reported in **Table 11.**

| **Table 11: Target proteases and consensus sequence for peptide linkage** | | |
|---|---|---|
| **Host Protease** | **Consensus Sequence** | **SEQ ID NO:** |
| MMP-1 | **VPMS-MRGG** | 88 |
| MMP-2 | **IPVS-LRSG** | 51 |
| MMP-10 | **RPLS-LQTG** | 90 |
| MMP-12 | **GPKN-LKAP******** | 91 |
| MMP-15 | **LPQK-SQHG** | 94 |
| Cathepsin B | **SLLKSR-MVPNFN** | 43 |
| Cathepsin H | **ALQA-RPGP** | 83 |
| Cathepsin L | **SGVVIA-TVIVIT** | 84 |
| Arginase II | **RRRRRRR** | 97 |
| Neutrophil Elastase | **GEPV-SGLP** | 87 |

| | | |
|---|---|---|
| *except where noted, sequences further include N-terminal GAG and C-terminal AG spacers. * * includes N-terminal GAA spacer and C-terminal AG spacer. | | |

**In** some embodiments, the relevant datasets for the selection of proteases for the detection of SCLC and NSCLC vs. non-cancerous health conditions, as well as the differentiation between SCLC and NSCLC can be obtained from the NCBI GEO database (see the website www.ncbi.nlm.nih.gov/geo/ at the filing date of the present disclosure) as will be understood by a skilled person. Datasets included in the analysis were taken from human cancers with datasets that contained both primary tumor samples and healthy human tissue. Exemplary calculated p-values for SCLC and NSCLC are reported in **Table 12** below.

**Table 12: Calculated p-values from NCBI GEO ID's**

| **ID** | **P-Value** | **logFC** | **Gene Symbol** | **Gene Description** |
|---|---|---|---|---|
| **Both, SCLC and NSCLC vs. Tumor Control** | | | | |
| NM_002421_at | 2.04E-04 | 0.960344 | MMP 1 | matrix metallopeptidase 1 (interstitial collagenase) |
| NM_002425_at | 1.14E-03 | 0.921643 | MMP10 | matrix metallopeptidase 10 (stromelysin 2) |
| NM_002426_at | 3.64E-21 | 3.321265 | MMP12 | matrix metallopeptidase 12 (macrophage elastase) |
| NM_002428_at | 5.58E-22 | -0.75712 | MMP15 | matrix metallopeptidase 15 (membrane-inserted) |
| NM_004530_at | 1.41E-20 | -1.9972 | MMP2 | matrix metallopeptidase 2 (gelatinase A, 72kDa gelatinase, 72kDa type IV collagenase) |
| NM_147783_at | 1.33E-06 | -0.75068 | CTSB | cathepsin B |
| NM_004390_at | 2.96E-27 | -2.71018 | CTSH | cathepsin H |
| NM_001333_at | 1.59E-12 | 1.221857 | CTSL2 | cathepsin L2 |
| NM_001172_at | 7.34E-18 | 1.582521 | ARG2 | arginase, type II |
| NM_001972_at | 2.26E-11 | -0.41571 | ELA2 | elastase 2, neutrophil |

| **NSCLC vs. SCLC** | | | | |
|---|---|---|---|---|
| 204475_at | 9.96E-01 | -3.84E-03 | MMP1 | matrix metallopeptidase 1 |
| 205680_at | 7.62E-04 | 2.16 | MMP10 | matrix metallopeptidase 10 |
| 204580_at | 4.87E-02 | 1.04 | MMP12 | matrix metallopeptidase 12 |
| 203365_s_at | 2.19E-01 | -1.58E-01 | MMP15 | matrix metallopeptidase 15 |
| 201069_at | 7.13E-02 | -5.65E-01 | MMP2 | matrix metallopeptidase 2 |
| 200839_s_at | 1.27E-02 | -6.27E-01 | CTSB | cathepsin B |
| 202295_s_at | 4.80E-05 | -1.19 | CTSH | cathepsin H |
| 202087_s_at | 7.48E-02 | -4.50E-01 | CTSL | cathepsin L |
| 203945_at | 9.67E-01 | -5.25E-03 | ARG2 | arginase 2 |
| 206871_at | 2.71E-01 | -7.17E-02 | ELANE | Elastase 2, neutrophil |

Based on these gene expression data, the selected panel of enzymes should be able to differentiate between SCLC and NSCLC.

Differentiation between SCLC and NSCLC is important, because of different treatment approaches to each type of cancer. The main subtypes of NSCLC are adenocarcinoma, squamous cell carcinoma, and large cell carcinoma. These subtypes, which start from different types of lung cells are grouped together as NSCLC because their treatment and prognoses (outlook) are often similar. A smaller subset of all lung cancers are SCLC. This subset is more sensitive to chemotherapy and radiation treatment, although their overall mortality is higher.

Furthermore, in analogy to pancreatic cancer, detection of NSCLC at stages 0 and 1 by means of liquid biopsies will be possible because of distinctly changing protease signatures during the transition from non-cancerous to cancerous. Principally, the same selection of proteases/arginase can differentiate between SCLC and healthy tissue, based on the gene expression differences, MMP10 and Cathepsin H can be selected as suitable proteases to distinguish between SCLC and NSCLC.

Similar gene expression analysis can be used to determine the proteases that are overexpressed in other solid tumors, such as pancreatic cancer, including using databases, such as NCBI GEO, Entrez Gene ID, Unigene ID and Gene Symbol. This strategy is able to select enzyme candidates that have a high probability of being proximal biomarkers for pancreatic cancer from the human genome.

These target panel sequences and detection platforms can be configured into a number of sensors of the disclosure, including solution-based assays, microfluidics assays, microwell assays, high throughput assays, and the like, including in those described in U.S. Patent Nos. 8,969,027; 9,682,155; 9,731,034; and 9,216,154, , as well as in co-pending WO 2017/165800 (US 2020/0300849), filed March 24, 2017.

In a further preferred embodiment, inflammatory biological markers can be detected along with the detection of specific viral, bacterial, or mold infections. For example, nanosensors could be prepared using a peptide aptamer against Capsid B (AISGSGGSTYYANSVLG (SEQ ID NO:71), recognition sequence for detecting *Haemophilus influenzae* B, or recognition sequences for *Haemophilus influenzae* NT (TNLGILHSMVARAVGNNTQG (SEQ ID NO:72)), or *Moraxella catarrhalis* (GIITYALSGGEIKILAG (SEQ ID NO:73)). Likewise, nanosensors for viral infections can be prepared using protease consensus sequences for HIV detection (SAVL-LEAT (SEQ ID NO:74), or SQNY-PIVQ (SEQ ID NO:75)).

It will be appreciated that each of the foregoing sensors described above can be designed in an alternative configuration where the graphene core particle is attached via a modifiable recognition sequence, a protease consensus sequence, a post-translationally modifiable sequence or bond that interacts with the target biomarker.In such embodiments, while the detectable particle remains relatively stationary and affixed to the carrier particle, the quencher particle is instead either cleaved or moved away from the detectable particle and carrier particle, to effect the change in distance between the particles, as described above. As noted, the increase in distance between the graphene core particle and the detectable particle (from cleavage or elongation of the quencher particle tether) results in a detectable change in the signal from the nanosensor.

**In** one or more embodiments, sensors in accordance with the disclosure can be used to diagnose lower respiratory tract infections by detecting markers associated with infected lower airway epithelial cell infection. These markers include CCL20, TSLP, and CCL3-L1. As noted herein, exosome contents can be used to determine whether the exosomes originated from the upper or lower airway, and further assist in localization of where the infection or other condition may originate from in the subject's body. The sensors can also be used for environmental risk assessment in patients with chronic lung disease. Exposure to air pollutants, ozone, particulates, acetaldehydes, acroleine, formaldehyde, tobacco smoke and other compounds triggers inflammation of the respiratory tract. Detection of markers such as cytokines, proteases, and/or kinases can be used to identify the stage at which lower respiratory tract inflammation is manifest, allowing for personalized environmental assessment. The nanosensors can also be used for measurement of lower respiratory tract inflammation in the real-time management of inflammation in asthma. Details regarding biological markers and understanding of protein expression patterns associated with severe asthma are described in U.S. Patent No. 8,053,199,. Currently anti-inflammatory (corticosteroids, IL-13 antibodies and others) are given and monitored on the basis of symptoms and exacerbations. By testing for lower airway markers such as cytokines, proteases, and/or kinases, patients and caregivers can track inflammation in real time and adjust treatments accordingly. Similarly, the nanosensors can be used for measurement of lower airway remodeling in severe asthma and chronic obstructive lung disease (COPD). Remodeling refers to the process of fibrosis of the respiratory tract, a process linked to progressive decline in lung function in a subset of patients with severe asthma and COPD. There is no treatment or diagnostic currently available. New therapies directed towards epigenetic remodeling are currently being developed. Our method will enable the development and approval of remodeling inhibitors for clinical use by detecting and measuring the presence of fibronectin, IL6, or vimentin, as indicative of progressive airway remodeling.

**In** some embodiments the sensors of the disclosure can be used in connection with detection of opportunistic infections. Opportunistic infections are also a significant complication in patients being treated for cancer, immunosuppressed through treatment of rheumatoid arthritis or inflammatory bowel disease, or those with HIV. Pneumocystis pneumonia (PCP) is the most common infection of HIV patients, and can only be diagnosed currently by invasive bronchoscopy. Invasive aspergillosis is also an opportunistic lung infection occurring in patients undergoing chemotherapy for leukemia. The nanosensors can be used to indicate the presence of distinct host response proteins when the fungus invades the airway in patients immunosuppressed from their leukemia treatment.

In some embodiments, the sensors of the disclosure can also be used for monitoring for transplant rejections or host versus graft disease in lung transplants. Lung transplant patients are treated with intensive immunosuppressive therapy and their physicians tread a fine line between too much immunosuppression (get opportunistic infections), or too little, where they would reject the organ. If a transplant patient could monitor their immune profiles on a real time basis, this would make management of the transplant much easier. It will also be appreciated that the nanosensors can be used for monitoring response to lung cancer treatment. Mobile biosensing would detect cancer signatures, such as cytokines, proteases, and/or kinases (e.g., MMPs, EMT, etc.), in the airway or circulating in the blood. This would include free proteins and those contained within microparticles (exosomes).

In some embodiments, sensors of the disclosure can be used in conjunction with protein sample collection, processing, profiling, and detection, embodiments described herein can also be utilized in conjunction with traditional PCR/RT-PCR, real-time quantitative PCR/RT-PCR, and isothermal PCR/RT-PCR methodologies to assess bacterial, viral, and mold infections that are associated with increased or decrease protein level expression. Thus, assays according to the invention can be used as a stand-alone technology, and/or in conjunction with traditional approaches, especially in risk groups that have been pre-identified as being at-risk by genetic testing.

In general, detection performed by sensors of the disclosure involves contacting the prepared sample with the nanosensor (and generally a plurality of nanosensors) to create a reaction mixture. The nanosensors can then be probed or excited using the appropriate energy source. The wavelength used will depend upon the particles used in the nanosensors. The changes in absorption and/or emission of the particles are then detected over a period of time as the target biomarker interacts with the nanosensors (e.g., such as through binding and extension of the recognition sequence).

Embodiments described herein also rely on nanosensors integrated with microfluidic and smart device platforms, which in turn, can be read by a simple but robust fluorescence or optical reader. "Microfluidic" refers to techniques manipulating, controlling, and/or analyzing small volumes of (fluid) samples, generally ranging from microliters (10⁻⁶) to picoliters (10⁻¹²). In one aspect, microfluidic technology is employed to introduce rapid, high-throughput sample collection, sample processing, sample profiling and target detection. In a further preferred embodiment, the microfluidic device will interface with a mobile computing device such as, but not limited to, a smart phone or smart tablet. Mobile computing devices including tablets, smartphones, handheld computers, laptops, etc. that are capable of optically scanning a sample and transmitting the information to other computing devices. This integrated platform will revolutionize the current "traditional" protocols, which use multiple instruments with tedious, manual sample-handling steps. In a further preferred embodiment, a stand-alone, battery-powered diagnostic platform with rapid analysis times and multiplexing capabilities will achieve statistical significance of measured quantities, with minimum consumption of human fluids (< 5µl per sampling) using a hand-held smartphone-based system that can be used as a diagnostic tool by patients and medical professionals.

In some embodiments assays performed with biosensors of the disclosure can utilize a combination of two or more of the various sensors discussed above. Importantly, nanosensors utilized in accordance with the disclosure can be designed to include a supramolecular recognition sequence, a protease consensus sequence, a post-translationally modifiable sequence, that gives rise to a specific interaction with a biological marker. As used herein, references to "specific interactions" are intended to differentiate the interaction between recognition sequences of the sensors from non-specific binding or reactions between molecules, and means that the set of specific target analytes for which the oligopeptide sequence can interact is limited, and in some cases even exclusive, such that neither binding nor enzymatic cleavage occurs at an appreciable rate with any other molecule.

Methods of the present disclosure can be performed with corresponding systems comprising various combinations of layered graphene, organic and/or inorganic molecule, detectable moieties, peptide linkage, detectable components, biosensors, reagents to perform chemical reactions herein described as well as additional components identifiable by a skilled person.

Exemplary optical detection techniques are described. However, it should be understood that other methods of analyzing the assay can be employed. In general, the methods involve exposing the reaction solution (e.g., in the microwell) to an appropriate energy source. The wavelength used will depend upon the detectable labels used in the biosensors. Both the position/presence and concentration/intensity of the signal from the detectable moiety can then be sensed or detected with the appropriate sensing or detecting instrument for both quantitative and qualitative assessment of the detectable moiety.

The systems herein disclosed can be provided in the form of kits of parts. In kit of parts for performing any one of the methods herein described to make the biosensors, the graphene particles can be included in the kit alone or in the presence of one or more buffers as well as reagents to perform chemical transformation of the graphene particles. In a kit for detection of the biomarkers, one or more biosensors herein described can be included in the kit alone or in the presence of one or more buffers as well as of reagents to perform detection of one or more biomarkers. Additional components identifiable by a skilled person can also be included.

**In** a kit of parts, the graphene particles, biosensors and the reagents are comprised in the kit independently possibly included in a composition together with suitable vehicle carrier or auxiliary agents. For example, one or more biosensors can be included in one or more compositions together with reagents for detection also in one or more suitable compositions.

Additional components can include labels, reference standards, and additional components identifiable by a skilled person upon reading of the present disclosure.

In embodiments herein described, the components of the kit can be provided, with suitable instructions and other necessary reagents, in order to perform the methods here disclosed. The kit will normally contain the compositions in separate containers. Instructions, for example written or audio instructions, on paper or electronic support such as tapes, CD-ROMs, flash drives, or by indication of a Uniform Resource Locator (URL), which contains a pdf copy of the instructions for carrying out the assay, will usually be included in the kit. The kit can also contain, depending on the particular method used, other packaged reagents and materials (i.e. wash buffers and the like).

Further details concerning the identification of the suitable auxiliary agent of the compositions, and generally manufacturing and packaging of the kit, can be identified by the person skilled in the art upon reading of the present disclosure.

### EXAMPLES

The following examples set forth methods in accordance with the instant disclosure. It is to be understood, however, that these examples are provided by way of illustration and nothing therein should be taken as a limitation upon the overall scope of the disclosure.

### Example 1: Graphene and metal photochemical properties

Graphene contains at least 98% carbon and shows minimal reaction with water or aqueous buffers as well as with thiols functional groups (e. g. presented on proteins and glutathione) in aerated aqueous buffers. These properties are opposite to the ones of metal/metal oxide material.

Graphene UV/V's absorption in the Ultra Violet (UV), visible and near-Infra Red (IR) region exceeds the absorption of graphite. [28] In particular, stacked graphene features light absorption over the entire wavelength range from far UV to mid-IR (100 to 1500 nm). [6]

Although direct excitation occurs at deep/far ultraviolet, surface plasmons of graphene in the visible range are of low intensity and not capable of effectively exciting attached organic fluorescent dyes or nanoparticles. [6]

In contrast, many nanoparticles (especially Au, Ag, but also Fe in Fe/Fe₃O₄) show strong plasmons in the visible range of the electromagnetic spectrum, which contribute to both, fluorophore excitation and quenching.[29, 30] In graphene, plasmon excitation in the visible range of the spectrum can occur by means of energy transfer from nearby attached fluorescent organic dyes and is expected from metal nanostructures and quantum dots as well. [31]

Because of the photophysical properties of graphene, there is no need for a FRET acceptor per fluorophore to be integrated in the design. This permits multitasking and avoids overcrowding at the surface of the nanobiosensor, which would kinetically slow down the nanobiosensors significantly.

Because of the photophysical properties of graphene, when using graphene within a FRET pair, there is no need for a FRET acceptor to be integrated in the design to complement a FRET donor (such as a fluorophore). These graphene properties allow a user to minimize overcrowding at the surface of the nanobiosensor, which would kinetically slow down the nanobiosensors significantly as will be understood by a skilled person.

In addition to the above, some forms of graphene such as explosion graphene and some fractions from flash graphene possess a super-aggregate fractal morphology which can be ascertained by light scattering methods such as SEM (Scanning Electron Microscopy) and Light Scattering. [18, 19]

Exemplary SEM (Scanning Electron Microscopy) images of fractal graphene comprising 7 layers are reported in Figure 1. [8]

As can be seen in the illustration of Figure 1, stacked layers of graphene feature fractal dimensions between Df = 2.5 ± 0.3 (upper bound) and Df = 1.8 ± 0.4 (lower bound). In general, any fractal dimensions between Df = 2.9 ± 0.09 and Df = 2.1 ± 0.09 can be attained, depending on the size and surface modification of explosion-graphene.

The fractal geometry of explosion graphene causes enhanced light absorption because light gets "trapped" within the structure. Whereas in flat graphene sheets, light passes through graphene only once, light reflection and diffraction from within the fractal morphology causes multiple absorption events as will be understood by a skilled person.

### Example 2: Overview biosensors and method of making

A schematic representation of the main components of biosensors herein described is provided in **Figure 2A** related to exemplary embodiments where the graphene is provided by detonation graphene and the coating of the core particle is provided by a polymer coating.

In the exemplary configuration schematically illustrated in **Figure 2A****,** nanosheets of detonation graphene aggregates are used to provide the graphene component of a core particle coated by a monolayer of a polymer coating. Detection components formed by a peptide linkage comprising a consensus sequence and a detectable moiety are further attached to the core particle through attachment to the polymer coating.

The biosensors with the structure schematically illustrated in **Figure 2A** can be provided with an exemplary process schematically shown in **Figure 2B****.** In the schematic illustration of **Figure 2B****,** the graphene surface is functionalized with, e.g., a plurality of carboxylic acid groups and/or coated with a thin polymer monolayer. In the exemplary illustration of **Figure 2B****,** a plurality of detectable moieties can be attached to the same carrier particle via respective oligopeptide linkages. For example, two or more different detectable moieties can be attached to the same carrier particle via respective oligopeptide linkages with specificity for respective target biomarkers as schematically shown in **Figure 2B****.**

In particular, the schematic illustration of **Figure 2B** shows the design of novel biosensors: A: synthesis of Carboxygraphene; B: Chemical attachment of Polyethylenimine via amide bonds; C: Attachment of consensus sequence (oligopeptide) and fluorescent dye (TCPP). TCPP is attached to the N-terminal end of the oligopeptide while on resin. After cleavage from the resin and HPLC-purification (if necessary), the consensus sequence+TCPP is attached by means of its C-terminal end to the polyethylenimine. Due to kinetic reasons, consensus sequences+TCPP are attached via the C-terminal end of the oligopeptide. In the presence of the correct protease, the consensus sequence is proteolytically cleaved and the fluorescent dye TCPP+covalently attached half of the consensus sequence is released (light switch effect).

A further schematic illustration of thin monolayers of graphene entangled with each other with overlapped edges, or more ordered stacking of nanosheets comprising or consisting of two to three layers is reported in **Figure 2C****.**

A schematic illustration of a high density of carboxylic acid groups added across the surface of a graphene nanosheet particulate is reported in **Figure 2D****.**

Additional details concerning process for carboxylation of graphene and attachment of detectable components through PEI polymer coating are reported in **Example 3** to **Example 9.**

### Example 3: Synthesis of Carboxygraphene - General process

To synthesize a graphene derivative referred to herein as "carboxygraphene," detonation graphene (0.2-0.5) nanoparticulates (nanosheet particulates) are dispersed in anhydrous dimethylformamide (DMF) at room temperature.

After the dispersion is complete, a carboxylic acid with a primary or secondary halogen is added, followed by the addition of anhydrous sodium azide (NaN₃). The temperature will then be slowly increased (l°C/minute) to 80°C. Between 20°C and 40°C a nucleophilic substitution reaction (S_{N}) occurs, in which an organic azide and DMF-soluble sodium halogenide are formed. The organic azide releases dinitrogen (N₂) and forms a nitrene intermediate at temperatures > 50°C. Nitrenes feature 6 instead of 8 electrons in the outer shell of nitrogen. This reactive intermediate undergoes a cycloaddition with a II-II double bond at the surface of graphene to form a stable azirane anchor.

The azirane-cycloaddition offers the opportunity to add a tailored amount of carboxylic acids to the surface of graphene without compromising the optical and electrical properties of graphene.

An exemplary reaction scheme and protocol are outlined in **Example 4.**

### Example 4: Graphene derivatization (Carboxygraphene (CG))

An example reaction scheme for synthesis of carboxygraphene from detonation graphene in a one-pot reaction is shown in **Figure 3A****.**

The protocol starts with detonation-synthesized graphene (0.40) nanosheet particulates having an elementary composition of: 98.9% C, 0.08% H, and 1.02% O. The material is then reacted with bromovaleric acid and sodium azide to yield carboxygraphene (CG) as follows.

1.0 g of graphene nanosheet (GN) particulates were suspended in 20 mL DMF at 20°C in a 250-mL three-necked round bottom flask. Next, at 40°C, 0.50 g of 5-bromovaleric acid (0.0028 mol) were added to the GN suspension, as well as 0.18 g NaN₃ crystals. After NaN₃ was dissolved, the suspension was slowly heated up to 80°C (about 1°C/min.) and stirred for 1 hr. The release of N₂ was observed. Finally, after 1 hour, the suspension was cooled down to room temperature, and derivatized GN was collected via centrifugation (5 min @ 7,000 rpm) and washed 5 times with DMF and then 3 times with anhydrous diethyl ether. The resulting CG was dried for characterization at 50°C for 1h and then stored under argon. Yield: 82% (by weight, assuming total reaction). Elementary analysis of carboxygraphene is 94.22% C, 1.79% H, 1.17% N, and 2.82% O. The incorporation of N indicates that the addition reaction was successful.

A schematic structure of Carboxygraphene in which only a single graphene nanosheet is shown is reported in **Figure 3B****,** while a Side-view illustration of Carboxygraphene top layer showing high-density distribution of carboxylic acid groups across the surface is reported in **Figure 3C****.**

As shown in **Figure 3D****,** Differential Thermogravimetry Analysis (DTA) indicates a weight loss of 4.5 ± 0.5 percent by weight when heated beyond 100°C. At temperatures beyond 100°C the material is virtually stable, as this can be expected from graphene. The initial weight loss arises from the partial loss of attached valeric acid units in the outer shell of carboxygraphene.

### Example 5: Titration of Carboxygraphene (CG)

100 mg of Carboxygraphene (CG) were suspended in 20 mL of 0.100 M NaOH. After stirring the suspension for 5 min at 300K, 0.100 M HCl solution was added in incremental steps. At each step the pH of the solution was recorded using a pH meter after making sure equilibrium had been reached (1-5 min.), before addition of next amount of HCl. The same procedure was used with the same volume of NaOH but without the addition of Carboxygraphene.

The difference in the volumes of HCl in the two titration curves for the same value of pH of -7.00 gives the concentration of the ionized groups (carboxyl groups) per weight increment of Carboxygraphene, as shown in **Figure 4****.** This titration curve indicates that we have a 6.1*10⁻⁴ mol/g of acidic groups (-COOH) (i.e., surface density of 4 -COOH groups per nm²). For comparison, this is 4 times higher compared to our Fenton-oxidized graphene oxide which possesses 1.7*10⁻⁴ mol/g (i.e., surface density of 1 -COOH group per nm²). This density of -COOH groups at the surface of carboxygraphene corresponds to an average space per - COOH group of 0.27 nm² in the synthesized carboxygraphene. This corresponds to a very high labeling density with surface -COOH groups, contributing to a much higher water dispersibility for these carboxygraphene nanosheet particulates.

It will be appreciated that the synthesis of carboxygraphene is even further simplified as compared to Fenton-oxidized graphene oxide, with faster reaction times. The process also does not involve addition of an iron salt, which would somewhat interfere with the photophysical properties of the attached fluorophore in biosensors described herein.

### Example 6: Carboxygraphene-polyethylenimine (G-PEI)

An exemplary synthetic procedure for tethering polyethylenimine to carboxygraphene to produce polyethylenimine-derivatized carboxygraphene is described below.

100 mg of carboxygraphene synthesized above was suspended in 20 mL anhydrous DMF in a 50 mL round bottom flask at room temperature. Next, 50 mg of polyethylenimine, branched, molecular weight 10,000 (PEI), 25 mg of 1-Ethyl-3-(3-dimethylaminopropyl) carbodiimide (EDC), and 25 mg of 4-Dimethylaminopyridine (DMAP) were added to the carboxygraphene solution. The reaction was stirred at room temperature overnight. Afterwards, carboxygraphene-polyethylenimine (G-PEI) was collected via centrifugation (5 min @ 7000 rpm) and washed twice with DMF and three times with diethyl ether. Material was collected via centrifugation after each washing step. Yield: 65% (by weight, assuming total reaction).

A schematic representation of the structure of resulting carboxygraphene-polyethylenimine, is shown in **Figure 5A** where only a single sheet is shown for reference. The Elementary analysis of the resulting carboxygraphene-PEI: 82.58% C, 4.65% H, 10.65% N, and 2.12% O. The significant increase in N content indicates that the addition reaction of PEI was successful. Based on the N-content we estimate that the resulting carboxygraphene-PEI contains 27±2% PEI.

A Differential Thermogravimetry Analysis (DTA) shown in shown in **Figure 5B****,** indicates a weight loss of 4.5 ± 0.5 percent by weight starting at 150 °C and extending through the entire temperature range. This behavior is clearly different from carboxygraphene and consistent with the stabilization of the graphene nanosheets by the linkage of polyethylenimine.

### Example 7: Synthesis of PEI coated Explosion Graphene-Based Biosensors

A detectable component formed by Tetra(4-carboxyphenyl)porphyrin (TCPP) and a peptide cleavage sequence for MMP-1 (GAG**VPMS-MRGG**AG) was attached to a PEI coated graphene based biosensor according to the following protocol.

50 mg of carboxygraphene-PEI synthesized above was suspended in ~8.1 mL DMF in a small glass vial. Next, 5 mg DMAP and 5 mg EDC were added to the vial along with ~3.5 mg of TCPP pre-conjugated with a peptide cleavage sequence for MMP-1 (GAG**VPMS-MRGG**AG). The sample was sonicated for 5 min. to ensure everything was suspended in DMF. The reaction was then stirred overnight at room temperature.

The biosensor was collected via centrifugation (5 min @ 7000 rpm) and washed with DMF and three times with diethyl ether. **Figure 6A** shows the structure of carboxygraphene-polyethylenimine-based biosensor, only a single sheet is shown for reference.

The synthesized materials were analyzed via spectroscopy. As shown in **Figure 6B****,** the UV/Vis Spectra of carboxygraphene, carboxygraphene-PEI, and the carboxygraphene-biosensor for MMP-1 in water were analyzed. The Soret band of the TCPP detectable label is clearly discernible at ~420 nm. TCPP is tethered to the polyethylenimine layer via the peptide containing the consensus sequence for MMP-1. Based on an estimated absorption coefficient of 135,000 M⁻¹ cm⁻¹, the TCPP concentration is 1.63 × 10⁻⁵ moles per gram carboxygraphene.

Additional exemplary detectable moiety that can be added with the process schematically shown above are reported in **Figure 6C** which shows BODIPY dyes with no or only a minimal overlap between the excitation and absorption spectra, including BODIPY FL, BODIPY TR, BODIPY 530/550, and BODIPY 630-650.

### Example 8: Additional functionalization of graphene surface

In the previous examples, functionalization othe f graphene surface was performed to tether carboxylic acid groups to the surfaces of the graphene. Polyethyleneimine (or, principally, other polymers) were then bound via stable amide bonds to the carboxylic acid functions.

Additional functional groups can be presented on the surface of graphene for example through modification of the carboxylic group. A skilled person will be able to perform such a functionalization using the whole toolbox of Organic Chemistry[32] for carboxylic acid reactions which are applicable to tether polymers or monomers to the surface of graphene. Reference is made for instance to the following exemplary reactions:
- Conversion of carboxylic acids into alcohols and formation of stable ethers
- Conversion of carboxylic acids into esters
- Conversion of carboxylic acids into aldehydes and then into double bonds (Wittig-type reactions)
- conversion of carboxylic acids into carbon centers with effective leaving groups, followed by nuclear substitution reactions.

Additional reactions can be used on graphene with or without previous functionalization to present a functional group configured to bind a corresponding functional group on an organic or inorganic compound, or directly on a peptide linkage as will be understood by a skilled person. These reactions comprise:
- Click reactions[33, 34] (especially Diels-Alder reactions[35, 36]). These can be performed directly with the surface of graphene or to connect a peptide sequence and a tether that is already linked to the surface.
- Additions of radicals (e.g. carbon, nitrogen, oxygen, and sulfur-centered) to the surface of graphene[37, 38]
- Additions of electrophiles (e.g. carbon, nitrogen, oxygen, and sulfur-centered) to the surface of graphene[38]

**In** embodiments where functionalization is performed to provide carboxygraphene according to titration experiments of carboxygraphene and molecular modelling, the highest possible packing density of linear molecules attached to the surface of graphene is: 6.0 ± 0.5 × 10⁻⁴ mol/g. The lower bound at which water dispersibility still can observed is 2.40 ± 0.05 × 10⁻⁵ mol/g.

Additional maximum and minimum packing density can be identified for different functionalization as will be understood by a skilled person. In the case of synthesizing asymmetrical nanobiosensors featuring a surface region of unsubstituted (pristine) graphene, this region is not to be larger than 10 relative percent to minimize interference of with the fluorescence readouts of the particle due to adsorption and quenching of formerly cleaved off fluorescent dyes at the surface of graphene.

### Example 9: Direct and indirect attachment of peptides to carboxylated graphene

In an alternative attachment process for detectable component, instead of utilizing polyethylenimine or other water-soluble polymers, the peptide sequences that are linked to the fluorescent dye can also be directly linked to carboxyl groups of carboxygraphene.

In some instances, in addition or in place of direct attachment to the graphene surface a peptide linkage can be indirectly attached through a spacer or connector presenting an amine group for binding to the carboxy. For that purpose, lysine, ornithine, or any non-natural amino acid featuring a spacer and an accessible extra primary amine can be utilized to facilitate attachment as shown by the schematic illustration of **Figure** 7.

Spacers can be used by a skilled person in combination with a desired peptide linkage while controlling the particle dimensions to obtain a desired distance between particles and detectable moiety.

A special class of "connectors" between a central graphene-based nanostructure and detectable component of the biosensors herein described (e.g. consensus sequences with attached fluorophore) are supramolecular systems. A plethora of supramolecular host-guest systems are published in the literature. [39] [40] The following host-guest systems are noteworthy:
a) Biotin/streptavidin [41]
b) Cyclodextrines and a variety of hosts [42]
c) Stoddart-cyclophanes and hydrophilic hosts [43]
d) Cucurbiturils and tailored hosts [44]
e) (structurally modified) DNA assemblies [45]
f) Nucleic Acid/Peptide Hybrids [46]
g) Supramolecular binding of designer peptides [47]

### Example 10: Chemical amination of graphene surfaces and attachment of peptide linkage

An exemplary additional chemical amination of the graphene surfaces such as an aromatic substituted nitrene reaction for chemical amination of a graphene surface [20] is schematically illustrated in **Figure 8****.**

According to an exemplary protocol 1.0 g of graphene nanosheet (GN) particulates were suspended in 20 mL DMF at 20 °C in a 250-mL three-necked round bottom flask. Next, at 40°C, 0.50 g of 1-amino-4-bromo-butane (0.0033 mol) were added to the GN suspension, as well as 0.20 g NaN₃ crystals. After NaN₃ was dissolved, the suspension was slowly heated up to 80°C (about 1°C/min.) and stirred for 1 hr. The release of N₂ was observed. Finally, after 1 hour, the suspension was cooled down to room temperature, and derivatized GN was collected via centrifugation (5 min at 7,000 rpm) and washed 5 times with DMF and then 3 times with anhydrous diethyl ether. The resulting grapheneamine (GA) was dried for characterization at 50°C for 1h and then stored under argon. Yield: 80% (by weight, assuming total reaction). Elementary analysis of carboxygraphene is 95.48% C, 1.86% H, 2.38% N, and 0.28% O. The incorporation of N indicates that the addition reaction was successful.

In order to add a detectable component TCPP-GAG RPF S-MIMG AEG, 1.00 g of GA was dispersed in 30mL DMF in a flask with a stir bar. 0.54g EDC and 0.53g DMAP were added and stirred until dissolved at room temperature. 0.20g TCPP-GAG RPFS-MIMG AEG was added and the reaction stirred for 21 hours. The CGP particles were washed 4-times with DMF using centrifugation as above, and 3-times with ethyl-ether. The GA-consensus sequence for MMP 3-TCPP labeled particles were dried by flushing with Argon and holding at room temp for 48 hours loosely covered. This reaction can be performed with every peptide designed and configured to be a peptide linkage in the sense of the disclosure (consensus sequence, posttranslational modification and supramolecular binding).

### Example 11: Reaction of graphene-embedded carbocations with secondary amine groups of PEI

Additional attachment reaction of PEI can be performed through binding of secondary amine groups with carbocations in explosion graphene.

In explosion graphene 0.3, numerous "holes" (carbocations) are embedded in the structure, thus causing a zeta potential of +60 mV. [8] Holes near the surface can react with polyethylenimine, as shown above. It is noteworthy that a nucleophilic addition reaction can occur between explosion graphene and virtually all (polymeric, monomeric, and small molecule) alcohols, thiols, amines, phosphines, and - potentially - carboxylic acids.

In conventional few-layer graphene, hydroxyl groups and oxirene functions can react with PEI. Hydroxyl groups form carbocations after elimination, which then react according to the scheme in **Figure 9****.** Oxirene (oxygen-containing three-membered rings) react with amines under nucleophilic addition. These reactions will covalently attach PEI to the graphene layers. Another possibility of directly attaching PEI to graphene presents itself if defects (carbocations) are present in graphene. This permits a direct nucleophilic attachment of PEI to graphene surfaces.

### Example 12: Electrostatic attraction at the surface of graphene

Making use of the strongly positive charge of explosion graphene, anionic polymers featuring sulfonic acids, aminosulfonic acids, and organic sulfates can be adsorbed at the surface of graphene. The driving force is the electrostatic attraction.

Note that when block copolymers are being used, hydrophobic polymers, such as polystyrene, polyisobutylenes, polyvinylchloride, polyethylene, polypropylene etc. can be components of block copolymers. They will be adsorbed on the surface of pristine graphene, whereas their hydrophilic component will mediate water-dispersibility. Note that the surface charge of explosion-graphene is positive (zeta potential = + 60 mV). [8] This permits the combination of hydrophobic and charge attraction when negatively charged polymers, such as polystyrene-sulfonic acid are used in block copolymers of polystyrene etc.

### Example 13: "Click Chemistry" of graphene

A further reaction allowing attachment of molecule on the surface of graphene nanosheets of the core particles herein described can be performed by click chemistry.

"Click Chemistry" can be performed at the surface of explosion (and few layer) graphene. A typical reaction is the Diels-Alder reaction, because it does not require a copper-catalyst. **Figure 10** depicts a scheme of Diels-Alder reaction between a maleimide-derivative and graphene. However, principally any click reaction, including the addition of thiols on the surface can be conducted as described for example in Li et al. 2016 [48] , Namvari et al. 2014 [49] and Farivar et al 2021 [50] as well with additions of radicals (e.g. carbon, nitrogen, oxygen, and sulfur-centered) to the surface of graphene [37, 38], additions of electrophiles (e.g. carbon, nitrogen, oxygen, and sulfur-centered) to the surface of graphene [38] and other methods identifiable by a skilled person (see also **Example 8).**

### Example 14: Use of biosensors and related functional principles

The functional principle of the biosensor here described for the related use is schematically illustrated in **Figure 11****.**

In the schematic illustration of **Figure 11** it is shown that upon interaction with a biological sample containing the target biomarker, the peptide linkage is changed (e.g., cleaved or elongated) resulting in a characteristic change in a detectable signal of the detectable moiety used in the sensor, such as a change in the fluorescence spectrum. The observable change in fluorescence (increase, decrease, shift, etc. depending on the detectable moiety) is a function of biomarker presence and/or activity.

In the exemplary embodiment schematically shown in **Figure 11****,** upon proteolytic cleavage of the consensus sequence (oligopeptide), the attached fluorophore is released and escapes the quenching by the graphene core of the biosensor. Fluorescence increase and intensity is observed that is a function of protease activity. Instead of particle-based biosensors, the biosensors can be constructed using similar components, except that the graphene is first immobilized on a solid support (e.g., planar substrate), onto which the other elements, e.g., peptide linkage, and then detectable moieties, are attached to create a planar sensor, e.g., lateral flow type sensor or stationery microwell sensor. The sensor would otherwise operate the same way, where the sample is added to the solid support, and binding or cleavage by the target biomarker in the sample would yield a detectable change in the assay on the solid support as would be understood by a skilled person upon reading of the disclosure.

### Example 15: Multiplex Biosensors

Whereas the classic biosensor design permits the use of only one protease-cleavable fluorescent dye or quantum dot per particle, the broad UV/Vis absorption spectrum of graphene and derivatives permits the attachment of several biosensors for different proteases.

**Figure 12** shows a working example of a detonation-graphene based biosensor for Matrix-Metalloproteinase-1. The working principle for graphene-based biosensors for protease activity is shown herein.

As shown in **Figure 12****,** the protease (if present in the tested sample) cleaves its consensus (cleavage) sequence and releases the fluorophore. The latter escapes the quenching of the graphene, resulting in a measurable increase in fluorescence ("fluorophore on"). The prototype biosensor for MMP1 was tested in a sample with an increasing concentration of enzyme.

It can be seen that the intensity of the detected signal is proportional to concentration of enzyme, as shown in **Figure 13A** and **13B****.** The estimated limit of detection (LOD) for this prototype biosensor under even non-optimal experimental design still shows that femtomolar protease activities can be detected. When TCPP is bound, it shows virtually no fluorescence due to strong carboxygraphene quenching. It is then released during 60min. and its fluorescence is detectable.

### Example 16: Buffering and a standard reaction

Protease enzymes are protein digesting enzymes that operate in physiological conditions. The Matrix Metalloproteinase family involve a complexed divalent cation while Cathepsin proteases are primarily activated in an acidic lysosomal environment. The operation of the Fe-NBS protocol as originally designed was prepared in a 25µM HEPES buffer with 10µM of divalent cations.

This system was altered to achieve more robust signal though improved pH control, by including isotonic conditions to support the protease activity while also supporting the divalent cation needs. It was also sought to demonstrate it was possible to build a buffer compatible with multiple different gNBS sensors at once to allow for future multiplexing.

To this end, a series of buffers were designed that all included 10µM final concentrations of MgCl₂, CaCh and ZnCh. HEPES buffer was evaluated as well as MES, TRIS and phosphate buffered saline (Table in **Figure 14**).

Solutions of uPA gNBS and CTSD gNBS were prepared at 10x working concentration of 300µg/ml in water. They were sonicated for 10 minutes in a sonicating water bath. The gNBS were diluted 1:10 in each of the different buffer formulations reported in **Figure 14****.**

125µl gNBS solution was dispensed in replicates in a black 96 well plate. 5µL serum was added and the plates were incubated at 37°C for 60 min. Released fluorescence was read on a VarioSkan Lux plate reader with 421nm excitation and 650nm emission. The assays included an assay control where 5 µl of the appropriate buffer was added in place of the serum. The gNBS were incubated with two different normal health donor sera samples or a pooled normal sera (PNS). Buffer 1 was the original formulation. Protease activity was observed in all the buffering conditions. The results are reported in **Figure 15****, Panel A** (CTDS gNBS) and **Figure 15****, Panel B** (uPA gNBSS).

**A** much more stable pH was observed in the PBS and MES buffered formulations with the pH holding steady for multiple days. Only DMSO significantly increased the RFU observed under stimulated conditions. However, of note, the buffers that included NaCl significantly lowered both the background and serum signal. Decreasing the AC serves to increase the signal to noise performance of the gNBS. Buffers 2, 3, 6, 8 and 9 all containing NaCl, had significantly reduced AC performance in comparison to the original buffer.

Buffer 9 was selected as the buffer with the best signal to noise performance. This performance supported the conclusion that the impact of physiological ion concentrations was stabilizing the zeta potential and enabling the natural conformation of the peptide-TCPP construct. Thus the zeta potential stabilization which contributes to the stability of the particle solution, also supports optimal enzyme conditions. The impact of NaCl on zeta potential can be observed in **Figure 16****.** To confirm these observations, the experiment was repeated on additional gNBS using Buffers 1, 2 and 9. Evaluation of gNBS for uPA, CTSD, MMP10, and CTSH all showed decreasing AC signal and highest signal to noise for buffers containing isotonic salt concentration with Buffer 9 outperforming Buffer 2 (**Figure 17**).

These experiments have demonstrated the role of ions for the efficient function of a gNBS used to detect protease activity in addition to the features of biosensors of the disclosure.

In particular, the functionality of a sensor of the present disclosure relies on the ability to build repeatedly with the same specifications and allow the measurement of activated proteases or other suitable biomarker in a normal serum sample such that different batches and lots of the sensors can be manufactured to produce the same signal.

In addition, buffer and manufacturing conditions can be selected to generate an optimal signal to noise ratio. It is observed in this experiment that the inclusion of monobasic salt ions contributes to a significant reduction in the noise as reflected by a reduced buffer only signal (see AC values **Figure 15** and **Figure 17**).

The impact of monobasic salt ions was also noticed to stabilize the zeta potential of the particles across a range of pH from pH5.0 to ph8.0. This range reflects the natural working environments for certain protease family members that are normally expressed in lysosomes or other acidic environments. Protease activity can occur across a range of pH conditions that reflect natural biological environments from pH2.0 to pH10.0. Depending on the pH requirements, MES buffer may be replaced with a buffer whose pKa is better suited to the pH target such as PIPES, MOPS, HEPES, TRIS, Phosphate buffer, Bikini, CHES or other buffers commonly found on Good's list.

The above observations supported the conclusion that the inclusion of salt decreases the AC activity of the sensors. The effective salt concentrations can be observed as a ratio less than 1.0 in comparison with the same sensor in the same buffer but absent any monobasic salt ions (shown in **Figure 18**). Monobasic salt ions of between 15.5mM and 155mM decrease the ratio of AC of salt/no-salt conditions. Hypertonic salt conditions may similarly increase the signal to noise performance by reducing AC. The salt concentrations in physiological conditions reflecting high salinity environments may be as great as 600mM, or as high as 1M.

### Example 17: Preparation and performance of biosensor prepared with sonication step

Few layers graphene can be produced in a detonation reaction using gas phase detonation of a hydrocarbon substrate in the presence of a defined ratio of oxygen. The resulting graphene is made up of ramified fractal aggregates that are on average between 50 and 500nm in size when detonated with a molar oxygen ratio of 0.3.

Electron microscopy of the resulting particles shows graphene aggregates variously composed of chains, sheets, aggregates and individual platelets as illustrated by the exemplary pictures of **Figure 19A****.**

The spectrum of sizes and shapes is evident from analysis of the transmission electron microscopy performed on gNBS (**Figure 19B**). The particles imaged in **Figure 19B** were prepared as described in the KSU disclosure of 30 Dec 2020 (Lot 120120). They show irregular shaped particles of many different sizes and the number of layers on higher magnification reflect the expected 5-25 layers of graphene.

As a fractal, there is no defined size or shape for the individual particles. They are made up of aggregates that are formed in the detonation process so are random in size and shape within the spectrum.

Exemplary SEM (Scanning Electron Microscopy) of explosion graphene are shown in **Figure 1****.** [8]To prepare a solution of gNBS for an assay, the gNBS is massed out and resuspended in buffer to a defined concentration by mass. This solution is then dispersed in a sonicating water bath. The time in the water bath is not defined in the original disclosure and has the potential to lead to different levels of disruption of the particles. The impact of this variance is seen in **Figure 20****.** When working solutions of gNBS are generated, the KSU Lot 120120 preparation results in some particles precipitating out of solution. This is due to the absence of efficient dispersion of graphene during the initial manufacturing process leading to an unstable final product. In contrast, when graphene is prepared by probe sonication during manufacturing and before assembly of the gNBS, the particles remain more polydisperse.

To evaluate further the impact of sonication on the distribution of the resulting gNB S population, the dispersion properties of the particles was analyzed on a Malvern Instruments ZetaSizer running v7.02 analysis software. Detonation graphene was either dissolved in DMF directly (no sonication) or actively dispersed by sonicating with a Fisherbrand FB705 Sonic Dismemberator for 1 hour in DMF.

The resulting graphene solution was assembled into a gNBS using the standard protocol by first reacting with bromovaleric acid and sodium azide to yield carboxygraphene (CG) (**Figure 21****: Panel A**). It is further modified by tethering Polyethylenimine to the carboxygraphene using EDC and DMAP to yield Polyethylenimine-derivatized carboxygraphene (**Figure 21****, Panel B**). Finally, peptide-TCPP is added using EDC chemistry with DMAP catalyst to yield the sensor; in this case using a peptide recognized by the protease neutrophil elastase (NE). Solutions of each intermediate, CG, and CG derivatized with polyethylenimine (CGP) were prepared and processed in a sonicating water bath for 10 minutes to disperse the solution for efficient mixing and redistribution of particles and to ensure effective surface modification and water solubility. PDI measurements were taken immediately after sonication and then again after settling for 30 minutes.

The results of the polydispersity index (PDI) measurements are shown in **Figure 21****.** PDI is a measure of the uniformity of the particles in a solution. The data show the uniformity of the particle sizes is greater (a smaller PDI number) after sonication of the starting graphene material for 1 hour before assembly of the gNBS. This uniformity was apparent for the CG and the CGP intermediate, as well as the NE gNBS final product.

Further to this, a solution of 1.0g Graphene 0.3 (O-C Ratio) was dissolved in 20mL DMF without further processing. The Absorbance at 270nm was measured on a NanoDrop 1000C spectrophotometer. The solution was then dispersed using a probe sonicator using 20s on and 10s off pulses at 35 intensity level for up to 60 minutes. A sample was collected at intervals and the ABS₂₇₀ₙₘ was measured out to 1 hour. As the probe sonication was continued, the slurry of graphene got thicker to the point it had to be diluted to perform an ABS₂₇₀ₙₘ reading and the reading corrected for dilution. **Figure 22** shows the increment in absorbance with increasing sonication time. The absorbance increases because the graphene fractals are broken into smaller particles as more energy is applied.

As the number of particles increases from the energy applied during sonication, the overall particle size decreases resulting in Mie or Rayleigh scattering of light which is observed as a decrease in transmission on the UV-vis spectra [51]. Therefore, as the graphene is sonicated, the input mass is decreased into the manufacturing process but can maintain the same or greater numbers of peptide-TCPP particles attached due to an overall increase in particle surface area.

Proteases are very sensitive to environmental changes such as pH and chemicals in the buffers. It was checked that the chemicals used during washing and manufacture of the biosensors were not remaining on the sensors and contributing to batch to batch variation we observed. A thermogravimetric analysis (TGA) was performed on 4 preparations of CG using a TGA Instrument TGA5500 and incrementing the temperature from room temperature (20°C) to 1000°C at 20°C/min in a nitrogen environment.

It was observed an overall decrease in Mass Loss Percentage in lots of manufactured nanosensor that had made an effort to completely dry the particles (Table in **Figure 23**).

Specifically, the TGA for the original KSU lot (120110) indicated the majority of the mass was lost in the temperature interval up to 100°C, matching the boiling points of the solvents used in manufacturing and indicating residual solvent contamination. As the protocol was modified to include the efforts to dry using temperature and desiccants, the percentage of mass loss decreased (**Figure 24** and **Figure 25**).

It was observed that sensors that do not have solvents removed have to go through a curing period before being fully functional. For the initial lots of nanosensor made by KSU, this period was determined to be 2 weeks where the signal/noise ratio was insufficient to produce significant signal due chiefly to an elevated AC. In contrast, a nanosensor prepared with efforts to remove residual solvents, including a 37°C overnight drying incubation post manufacture, generated robust signal within 5 days of manufacture (**Figure 26**). Desiccation of the final sensor product is considered important to remove residual solvents and reveal optimal activity.

Residual solvents impact the performance of the gNBS by inhibiting optimal activation of the sensors revealed as a reduced signal to noise performance. The removal of manufacturing solvents is therefore characterized using TGA. TGA provides a measurement of the quantity of solvent released as a percentage of total mass. Specifically, the solvents are removed between 20°C and 200°C or 20°C and 400°C. As a contribution to the total mass of the sensor, the preparation of gNBS should result in a contribution of thermally transitioned mass of less than 30%. Optimally the mass loss measured by TGA should be less than 10%, or 5%. Optimally the mass loss as measured by TGA should be less than 1%.

### Example 18: Batch to batch variation and the impact of sonication

The manufacture protocol as defined by the original disclosure was a three-step process.

First graphene was modified to generate Carboxygraphene (CG). This was achieved by dispersing 2.0g detonation synthesized graphene 0.3 (O-C Ratio) in 40mL DMF in a 250mL Erlenmeyer flask suspended in a room temperature silicone oil bath with a magnetic stir bar. After dispersion, the absorbance of the solution was measured on a NanoDrop 2000C spectrophotometer. The temperature of the reaction was raised to 40°C and 1.0g of 5-Bromovaleric acid was added to the graphene suspension followed by slow addition of 0.36g Sodium Azide crystals. Once all the reagents were dissolved, the temperature was ramped slowly to 80°C and incubated for 1hr at 80°C and then allowed to cool to room temperature. The suspension was washed by centrifugation at 10,000 × g for 5 minutes 5-times with DMF and 3-times with ethyl-ether before drying.

Second, for manufacture of the polyethylenimine-derivatized carboxygraphene (CGP) intermediate, 1g of CG was dispersed in 30mL DMF in a flask with a stir bar. 0.54g EDC and 0.53g DMAP were added and stirred until dissolved at room temperature. 1.2g polyethylenimine was added and the reaction stirred for 21 hours. The CGP particles were washed 4-times with DMF using centrifugation as above, and 3-times with ethyl-ether. The CGP particles were dried by flushing with Argon and holding at room temp for 48 hours loosely covered.

The last step is the conjugation of the sensor peptide-TCPP. 103mg of CGP was dispersed in 16mL DMF using a sonicating water bath for 1 minute. 10.5mg of EDC and 11mg of DMAP was added to the CGP with stirring at room temperature. 7mg of TCPP labelled peptide was then added and the reaction stirred for 20hrs at room temperature. The resulting gNBS was washed 3-times with DMF and 3-times with ethyl-ether before flushing with Argon The final gNBS product was stored at -20°C. This protocol mirrors the original KSU protocol.

It was observed that preparations of gNBS made with graphene using the initial protocol presented with large variances in performance between lots, even those batches prepared on the same day (**Figure 27** compare Lots 120120 and NE100 with NE101 and NE102). The possibility was considered that in the preparation of working solution, the dispersal of sensor using a sonicating water bath may generate a different population size and particle number with every sonication. So unless the particle size was optimized before manufacture, the repeated sonication of the gNBS working solutions would regularly alter the result of an analysis.

To determine the impact of sonication on the starting material, the manufacture was modified as outlined in Table in **Figure 28** The starting graphene material was used without sonication as described in the original protocol, being dispersed in DMF with a stir bar only, or the starting material was dispersed with a probe sonicator for 10 or 20 minutes (Table in **Figure 28**). After dispersion, the input quantity of graphene was reduced due to the increased numbers of particles and surface area, but the quantity of peptide TCPP input to the conjugation reaction remained the same. One Lot of NE gNBS was manufactured using the KSU protocol and two Lots using 10 and 20 minutes probe sonication of the starting graphene.

All three were compared to the Lot manufactured at KSU (**Figure 27**). KSU Lot 120120 and HEB Lot NE100 were manufactured using the same protocol and dispersion procedures. HEB Lots NE101 and NE102 were modified by the inclusion of probe sonication. The gNBS were evaluated under standard conditions using normal human sera compared to the assay control (AC) containing sensor and buffer but no serum. It was observed a 244% average difference between batch 1 and 2 for Lot 120120 and HEB Lot NE100. In contrast the probe sonicated Lots (NE101 and NE102) showed a 132% average difference between batches (**Figure 27**). It was concluded that dispersion through sonication before manufacture led to more consistent particle sizes in the graphene starting material, a smaller and more acceptable PDI (<0.5) (**Figure 21**), and produced more repeatable activity in the final gNBS sensor.

To confirm the impact on particle size in the final preparation, we evaluated the impact of sonication in a water bath on the finished particles. A significant ABS increase was observed in NE-gNBS that had undergone no sonication during manufacture with an average increase in ABS of 259% at the ABS wavelength for graphene (265nm) and TCPP (425nm).

In contrast, the change in ABS over time for the Lots prepared including sonication showed on average a 121% change in ABS over time at the same wavelengths (**Figure 29**). Our conclusion was that preparation of the particles including a probe sonication step produced a more homogenous suspension that was not impacted by the water bath sonication steps used to prepare working solutions.

### Example 19: Preparation and performance of biosensors

The preferred protocol for assembly of the particles includes adjusting size distribution of particles using sonication or physical disruption. The graphene is derivatized by addition of a carboxyl moiety to the surface using an azirane anchor. The surface is then modified by addition of a polymer such as polyethylenimine (PEI), followed by conjugation of a peptide dye complex.

With the set of experiments reported in the present examples, it was sought to find the boundaries of these steps and components by substituting other components to evaluate the modifications that could be considered for alternate manufacturing protocols.

In particular, experiments were repeated to determine the amount of carboxy labelling that occurs during the first step of synthesis. Solutions of 0.5M NaOH and HCl were prepared using new high purity HPLC grade water. The 0.5M NaOH solution was degassed with Argon for 2 hours before dispersing CG. After degassing, 250mg of CG was dispersed in 50mL of 0.05M NaOH. The sample was sonicated for 10 minutes in a water bath and then stirred for 24hr at room temperature. After 24hrs, the CG suspension was filtered out to remove the CH< and solutions, filtered 0.05M NaOH, 0.05M NaOH, and 0.05M HCl, were degassed overnight. Next, 20mL of 0.05M HCl was added to a 10mL aliquot of the CG treated and filtered NaOH solution. The pH was recorded after every addition of NaOH once the pH had stabilized and before the next addition. For blank, a 10mL solution of 0.05M NaOH was prepared the same way by addition of 0.05M HCl and back titration with 0.05M NaOH. Degassing was maintained during titration. Using this protocol, CG preparations required more NaOH than blank for neutralization implying the presence of acidic functional groups. Replicate experiments calculated a concentration of 1.765 × 10⁻⁴ and 1.8 × 10⁻⁴ mol/g acidic groups. Although lower than the original estimate, it supported the addition of acidic groups to the graphene (**Figure 30** **Panel A** and **Panel B**).

In support of this observation, elemental analysis of unmodified graphene and carboxygraphene showed undetectable levels of oxygen and hydrogen in the starting graphene material (Table in **Figure 31**). After carboxylation, there were detectable levels of H and O observed between 0.5% and 3.5%. Decreased oxygen content was noted in comparison to the original KSU protocol (KSU and CG1.0) so concluded some of that oxygen content is derived from solvents. In support of this, preparations CG1.2, CG1.3, and CG1.4 (Refer to Table in **Figure 28**) were prepared with sonication and included drying protocols and showed lower resulting percentages of H and O detected. For comparison, CG product that was derivatized with PEI (CGP102) produced significantly higher contributions of H, O and N. CGP102 was assembled using CG1.0 (Table in **Figure 31**).

On this basis it was concluded that the original pH titration overestimated the numbers of carboxylic acid groups added to the graphene and was more accurately estimated using Argon degassing and back titration. This was supported by the elemental analysis that showed a lower estimate of oxygen content in CG preparations where efforts were made to eliminate residual solvent than in the product generated with original protocols. Despite differences in elemental analysis, all the protocols as described were still able to annotate graphene with H and O and to produce functional biosensors.

To determine the steps for the biosensors assembly, the addition of PEI to graphene was tested in the absence of a previous carboxylation step. In the first instance, control uPA gNBS was assembled using the standard protocol by carboxylation of graphene followed by PEI polymerization. In a second example, 0.3 graphene was substituted for CG with the same addition of 500mg/g PEI using 243 mg/g EDC and 247 mg/g DMAP as outlined for CGP104 in Table in **Figure 28** (uPA-no GC).

This reaction was incubated with stirring at RT for 90 minutes. In a third instance, 0.3 graphene was dispersed into DMF with the addition of 550mg/g PEI but no EDC or DMAP catalyst were added. Instead, the reaction was heated to 80°C for 1 hour (uPA - 80°C). On completion of the addition of PEI, all three preparations were washed at least 5 times with 200 proof Ethanol and resuspended in DMF. All three preparations were then conjugated to Peptide TCPP using the uPA-TCPP sequence. All three constructs were treated with 105mg/g EDC, 11 1mg/g DMAP and 65 mg/g uPA-TCPP with stirring at RT for 90 min. The resulting particles were washed with Ethanol and dried overnight in a 37°C oven. The sensors were tested by preparing solutions of 200µg/ml in 10mM MES, 10µM CaCl₂, MgCl₂, ZnCl₂, and IS:155. 70µl of each working solution was incubated with 10µl of pooled normal sera at 45°C for 90 minutes.

The fluorescence was measured on a VarioSkan Lux plate reader with 425nm excitation and 653nm emission. All three preparations produced assay control values less than 0.05 and had RFU from pooled normal serum between 19 and 31 (in **Figure 32**). All three sensors were insensitive to adjustment of the reaction to 10mM KOH, a treatment that reveals non-covalently bound TCPP (Data not shown).

It was thus concluded that since the elemental analysis of graphene has a limit of detection for oxygen of 0.5%, as the quantity of oxygen increases to the detectable levels with production of CG, the targets for addition of PEI also become detectable. However, given the functional sensors generated by heat based condensation at 80°C or adding PEI with catalyst directly to graphene, evidence is obtained that 0.3 graphene has some ability to attach the small numbers of PEI molecules necessary to assemble a sensor implying the presence of oxygen in the detonation graphene.

Additional methods of coupling PEI to graphene which might require fewer steps or be more efficient were also evaluated. It was identified surrogates for the bromovaleric acid linker step such as a modified tetra ethylene glycol (TEG), Azidoacetic acid NHS ester, or Azidoacetic Acid. These molecules provide the azido- group and the carboxylate or amine group in place of the Bromovaleric acid + azide.

An experiment was performed where 125 mg graphene was probe sonicated into 22.5mL DMF and heated to 60°C. PEI was prepared by adding 60mg 10k MW PEI to 3 ml DMF and then adding 270mg Azidoacetic acid NHS Ester. The mixture was heated to 60°C and incubated for 30 minutes. The activated PEI solution was then added to the graphene and the temperature ramped over 10 minutes from 60°C to 80°C. The reaction was continued for 1 hour and purified as using Ethanol washes and desiccation. A second preparation was made the same way but substituting the 10k MW PEI for 1.2k MW PEI. Both constructs were subsequently treated with 105mg/g EDC, 111mg/g DMAP and 65 mg/g uPA-TCPP with stirring at RT for 90 min. The resulting particles were washed with ethanol and dried overnight in a 37°C oven. The sensors were tested by preparing solutions of 200µg/ml in 10mM MES, 10µM CaCl₂, MgCl₂, ZnCl₂, and IS: 155. 70µl of each working solution was incubated with 10µl of buffer, 50mg/mL acetylated BSA, or pooled normal sera at 45 °C for 90 minutes in a black 384 well plate.

The fluorescence was measured on a VarioSkan Lux plate reader with 425nm excitation and 653nm emission (**Figure 33**). Similar activation of the uPA sensor made with either PEI preparation was observed. This observation indicates it is possible to manipulate the distance of a peptide and the associated dye from the graphene backbone - by selecting polymers of different molecular weights therefore decreasing the distance between the graphene quencher and fluor. This will likely become necessary as we change to more efficient fluorescent dyes that have shorter Förster radius or are more efficient fluorophores. This experiment also confirms the ability to use anchors other than bromovaleric acid.

Currently, assembly of graphene-based biosensors consists of a series of surface modification reactions, which are performed using the following modifications: graphene to carboxygraphene to carboxygraphene-PEI to graphene biosensors. In order to reduce this complex synthetic procedure, a single surface modification of graphene prior to the assembly of the biosensor was investigated, which is described below.

A small scale reaction was started by dispersing 1g of 0.3 detonation graphene in 50 mL of distilled and degassed *N*,*N*-Dimethylformamide (DMF) (Fisher Scientific Cat#: D119-20) in a round bottom flask at room temperature. This dispersion solution was then heated up to 40°C and 556 µL (0.0028 mol) of Azido-TEG3-amine (BroadPharm Cat#: BP-20580) was added slowly. Then, the temperature was increased to 80°C and the reaction solution was stirred for 1 hour. After 1 hour, the reaction was cooled down to room temperature and sample was collected via centrifugation (5 minutes at a speed of 8000 rpm). Finally, the G-TEG3amine product was washed 5 times with DMF and 3 times with anhydrous diethyl ether. Resulting G-TEG3amine was further dried with argon and stored at -20°C.

The next step was to assemble MMP1 biosensor using G-TEG3amine, which was assembled following the same optimized conditions established for G-PEI biosensors. For this, two solutions were prepared: 1) 3.75 mg of TCPP-MMP1 peptide, 5 mg of EDC, and 5 mg of DMAP were dissolved in 2 mL of distilled and degassed DMF and 2) 50 mg of G-TEG3 amine were dispersed in 2 mL of distilled and degassed DMF. Both solutions were sonicated in a sonicating water bath (3 minutes) and vortexed until completely dissolved. Solution 1) and 2) were then mixed, and solutions were further sonicated (3 minutes) to ensure everything remained suspended in DMF. The solution was then stirred overnight at room temperature. After 24hr, biosensor was collected via centrifugation (8 minutes at 8000 rpm), washed three times with DMF and three times with diethyl ether. Lastly, biosensor was dried with argon and stored and stored at -20°C.

Once biosensors were assembled, dynamic light scattering (DLS) and zeta potential measurements were taken using the NanoBrook 90Plus PALS. For this, a 0.03 mg/mL solution of MMP1 was prepared using highly pure water (HPLC water, Fisher Cat#: W7-4). Average size (DLS), polydispersity index, and zeta potential surface charge are shown (Table in **Figure 34**). Results demonstrated that biosensor has a slightly positive charge of 4.36 mV, an average diameter size of 713.30 nm, and PDI of 0.147.

The following experiment was a side-by-side comparison of both biosensors, G-PEI MMP1 vs G-TEG3amine MMP1. For this experiment, a 0.03 mg/mL solution of each MMP1 biosensor was prepared using HEPES Buffer (25 µmol/L HEPES buffer enriched with 10 µmol/L of Ca²⁺, Mg²⁺, Zn²⁺, pH 7.20). After preparing the biosensor solutions, pH increased to 7.40 for G-TEG3amine and 7.52 for G-PEI, therefore, pH was then adjusted to 7.20 for both solutions using a 0.1 M HCl solution.

Three solutions were prepared (as described below) and plated in duplicates in a 96-well plate (Greiner Bio-one, microplate 96 well, PS, F-bottom, Black non-binding; item #: 655900).
- Solution 1: Sample control (125 µL of HEPES buffer + 5 µL of collected control serum - S, P, or Pooled)
- Solution 2: Assay control (125 µL of biosensor solution in HEPES buffer + 5 µL of HEPES buffer)
- Solution 3: Assay (125 µL of biosensor solution in HEPES buffer + 5 µL of collected control serum - S, P, or Pooled).

After the solutions were plated, the 96-well plate was incubated at 37°C for 1 hour, and fluorescence intensities were measured every 15 min during the 1 hr incubation. Fluorescence intensity was measured using a BioTek Synergy H1 plate reader (_{exc} = 425 ±10 nm, ₑₘ: 650 ±20 nm). **Figure 35** shows the fluorescence intensities measured every 15 min for MMP1 G-PEI and MMP1 G-TEG3 amine biosensor for each control sera (S, P, and Pooled). The highest intensity measured after 1 hr incubation for G-TEG3amine was higher for all 3 control sera tested compared to G-PEI biosensor.

Also, intensity increased over time following an almost linear trend for both biosensors tested with all 3 control sera samples. **Figure 36** **panel (A)** shows the intensity measured after the 1 hr incubation with both biosensors tested with each control sera. Here, the differences in intensities can be noticed comparing G-PEI vs G-TEG3 amine, which was almost twice as high for G-TEG3amine as for control serum S and P compared to G-PEI. It should be also mentioned that the intensity for assay controls were lower for G-TEG3amine compared to G-PEI, which are summarized in **Figure 36** **panel (B).** Overall, G-TEG3amine based biosensor produces improved signal/noise measurements with lower assay control and greater specific signal for serum.

In view of the above experiments, it was concluded that since explosion synthesized graphene is hydrophobic and virtually not dispersible in water or aqueous buffers (and many surfactants interfere with the fluorescence quantum yield of organic dyes), addition of a hydrophilic coating is to be performed to obtain the desired stability.

This can be achieved by attaching carboxylic acid groups to the surface of the graphene stacks via nitrene addition [52]. Alternatively, amine-terminated oligoethylene glycol can be attached to the surface of explosion graphene via nitrene addition.

To this carboxylic acid, polyethylenimine is attached. Alternatively, amine groups can be attached to the surface of explosion graphene, and then a polymer displaying carboxylic groups.

Alternatively, the precursor can be bound for graphene addition bearing an azide group to the polymer, and then anchor the polymer to the graphene surface.

The attached organic coating (polymer, oligoethylene glycol) has the following functions:

Without hydrophilic coating the graphene nanobioensors would not be dispersible in aqueous buffers. The achievable concentration of the biosensor strongly depends on the chemical nature of the coating and the mass ratio of the coating to graphene.

The coating is responsible for the stability of the biosensor when dispersed in aqueous buffers and the graphene cores of the biosensor are extremely stable.

The size and structure of the PEI is responsible for the observed increase of fluorescence upon proteolytic cleavage of the consensus sequence. In general, Förster Resonance Energy Transfer (FRET) is occurring between graphene (acceptor) and attached organic fluorophore. The efficacy of FRET is distance dependent. Therefore, the structure of PEI (branched vs. linear) matters. However, graphene (and especially graphene stacks) efficiently absorbs light from the UV into the near-infrared region. Theoretical studies by Bian et al. (2016) suggest that the quenching sphere around a graphene monolayer extends approx. 20 nm [53]. Within this "Förster Volume" strong quenching occurs, which very strongly decreases TCPP fluorescence. Upon escape from the FörsterVolume, TCPP starts emitting.

### Example 20: Multiplex graphene biosensor

The possibility was considered of running multiple different sensors in the same reaction well. Graphene has a broad-spectrum absorbance of light (**Figure 37**). Accordingly, multiple different peptide-dye sequences can be assembled on a single graphene particle or multiple sensors each labeled with a different single peptide-dye could be mixed together.

The multiplex capability of the biosensor of the disclosure was evaluated using the later system - mixing two independently labeled particles. Peptides for Neutrophil Elastase (NE) and Arginase (ARG) were variously labeled with FAM or TAMRA. Peptide-FAM or Peptide-TAMRA were directly substituted into the manufacturing process for the Peptide-TCPP with the only modification being conjugation at half the density due to the increased fluorescent efficiency of these dyes. So the peptides were added at 35mg/g CGP (Reference Table in **Figure 28**). gNBS working solutions were prepared by massing outNE-FAM, NE-TAMRA, or uPA-TCPP gNBS into assay buffer comprised of 10mM MES, 10µM CaCl₂, MgCl₂, ZnCl₂, IS:155. 125µl of working solution with incremental quantities of each gNBS were dispensed in black 96 well plates and stimulated in a single well with buffer for the assay control (AC) or in triplicate with 5µl of pooled normal sera. The plates were incubated in the VarioSkan Lux at 45°C (**Figure 38** and **Figure 39**). Fluorescence was observed for each reaction over 90 minutes using Ex₄₉₆/Em₅₂₆ for FAM and Ex₅₅₅/Em₅₈₆ . Alternatively, MMP7 and MMP9 peptides were conjugated to Rhodamine-B and conjugated to CGP at 70mg/g using the KSU manufacture protocol as above. gNBS working solutions were prepared with a final concentration of 30µg/mL gNBS in 10mM MES, 10µM CaCl₂, MgCl₂, ZnCl₂, IS: 155 and 125µL reactions were set up in triplicate in black 96 well plates and incubated with 5µL sera or buffer only (AC) for 60min at 37°C. Fluorescence was measured at Ex₅₃₅/Em₅₇₀ on a BioTek Synergy H1 FL800 plate reader (**Figure 39**).

These observations support the model that graphene can act as an effective quencher for multiple different dyes. Having demonstrated that multiple different sensors can be built with different dyes and these dyes can be successfully quenched by the graphene backbone in separate reactions, it was sought to demonstrate that multiple dyes could be observed in the same reaction.

Arg-FAM and NE-TAMRA gNBS were prepared again halving the input peptide dye contribution to 35mg/g CGP for the gNBS manufacture protocol as above. gNBS working solutions were prepared by massing out 400 µg/ml Arg-FAM or NE-TAMRA gNBS into assay buffer comprised of 10mM MES, 10µM CaCl₂, MgCl₂, ZnCl₂, IS: 155. 80µl reactions were set up in 384w black plates with 35µl single sensor and 35µl buffer or 35µl of each sensor mixed for a 200µg/ml final concentration of either sensor. 10µl pooled normal sera or buffer was added to each sensor solution.

The sensors were all stimulated in triplicate and incubated for 90 minutes at 45°C with measurements taken at 10-minute intervals. Each well was measured at 496/526nm (FAM) and 555/586nm (TAMRA) (**Figure 40**).

It was noted that the individual sensor and the multiplexed sensors can both be detected and there was no detection of signal from one sensor into the other channel although there is evidence for some quenching when both sensors are present (Compare singleplex to multiplex fluorescence in either channel). It was also noted that the Arg-FAM presented with an elevated background in the singleplex and multiplex reactions. It was interpreted this as an indication that the amount of peptide conjugated when high efficiency dyes are being used may exceed the capacity of the graphene backbone to quench.

This noise can be alleviated by titrating down the level of labeling. Alternatively, a shorter linking chemistry between the FAM dye and the graphene would bring the FAM closer to the FRET partner and improve quenching. In this context, it was considered substituting the 10k MW PEI with the 1.2K MW PEI as described in **Figure 32** as a viable solution.

Using a combination of polymerization as with PEI or direct conjugation as by TEG or Azidoacetic acid NHS ester or equivalent was considered to conjugate dye-labelled peptide to a graphene backbone, such that the graphene backbone effectively quenches the attached dye.

Variations on this chemistry are expected to be effective as exampled above so long as the peptide-dye conjugate is maintained within 20nm of the graphene [53] [54]. So the signal to noise performance is modified by altering the distance between the dye conjugate and the graphene backbone by optimizing the polymerization or conjugation chemistry to optimize the efficient quenching of the dye depending on the quantum efficiency of the dye selected. This conjugation selection must also contribute sufficient charged groups such as carboxylic acid, amino, or alcohol side chains to allow secondary conjugation of the peptide-dye complex and to support solubilization of the graphene particles.

Finally, a NE-FAM gNBS reaction was set up in duplicate with one assay being conducted in a 96w black plate and analyzed in a VarioSkan Lux platform and the other assay using NE-FAM gNBS in a 96-well PCR plate and analyzed in a StepOnePlus PCR platform (**Figure 31**). The absence of background signal and the linear relationship between incubation time and fluorescence is apparent on both platforms and raises the possibility that these types of gNBS assays could be run on laboratory equipment even more widely distributed than fluorimeters, such as quantitative PCR platforms.

### Example 21: Heat Treatment of Serum

An experiment was designed to demonstrate the specificity of these reactions by heat inactivating the protease enzymes. To this end, samples of two different control sera were heat treated at 55°C for 1 hour. The heat treated sera and the untreated sera were incubated with MMP10 and CTSH gNBS using the standard conditions. With both sera and for both sensors, a modest elevation in the RFU was unexpectedly observed. (**Figure 42** **Panel A**). The experiment was repeated with treatment of the two control sera at 65°C for 10 minutes (any longer interval caused precipitation of the sera). In addition, we treated the sera with EDTA such that the final EDTA concentration in the sensor reaction was 5mM. We also substituted MMP2 for MMP10 gNBS to see how generalizable this observation was (**Figure 42** **Panel B**). Treatment of sera with EDTA produced a modest decrease in activity consistent with other reports [55]. In contrast, heat treating the sera at 65°C gave a significant increase in activity in both sera and both sensors. The addition of EDTA modestly decreased the elevated activity as it did for the untreated sera and this effect was more pronounced for the MMP2 sensor. Therefore, surprisingly treatment of sera at elevated temperatures reveals additional protease activity that can increase the gNBS signal. To determine if this change is the result of additional activation events occurring as zymogens become activated, we stimulated CTSH gNBS with pooled normal sera or replicate sample that was heat treated to 65°C for 10 minutes. The reaction was measured at 30 minute intervals after the first hour for over 240 minutes. The activity of the untreated sera and heat treated sera were both maintained as linear reactions for the duration (**Figure 43**). If additional zymogens were being activated, an increase in kinetics would be expected over time. However, no evidence was seen of a change in kinetics.

In support of this conclusion, the impact of heat treatment on pooled normal sera sensor activity was evaluated in 18 protease biosensors (**Figure 44**). Every sensor that has a signal significantly above the assay control (RFI >0.05) demonstrated an increase in activity of on average 3.2-fold when 65 °C heat treated sera were used.

Lastly, MMP12 and CTSD gNBS were prepared in 125µl working solution of 5mM MES, 10µM cations and IS: 155. They were stimulated with 5µl buffer (Assay background) or in triplicate with 5µl PNS. Replicate plates were assembled and each replicate was incubated at 37°, 41° or 45°C in the VarioSkan Lux. Fluorescence was measured at 10 minute intervals for the duration of the incubation. Sample background measurements showed the results from wells that were incubated with 125µL of buffer (no sensor) with 5µL of sera added. In this arm of the experiment, the serum was not heat treated prior to the measurement. Instead, the reactions were carried out at incremental temperatures.

Each temperature increment revealed additional activity so for both MMP12 and CTSD gNBS, the 41°C incubation produced more fluorescence than 37°C and 45°C more than 41°C (**Figure 45**). For MMP12 and CTSD, in serum that was not previously heat treated, increasing the assay temperature to 41°C nearly doubled the RFU (1.7x and 1.9x respectively). Increasing the assay temperature to 45 °C tripled the RFU (3.1x). There was no elevation in the background signal of the sensors or the sample background under these conditions. For comparison, we also pretreated sera at 65°C of 5 minutes and compared the impact on signal to noise when both pretreatment with 65°C and elevated assay temperature were applied (compare open and closed symbols in **Figure 45**).

Pretreating sera at 65°C increases the signal 2.3-fold over heat-treated sera. It also 2.3x and 2.0x signal for assays subsequently run at 41°C and 45°C respectively. Therefore, pretreating sera and running the protease assay at elevated temperature improve signal to noise using separate and additive mechanisms.

With respect to thermal stability of protease enzymes, active MMP2 enzyme has been reported to have two irreversible unfolding temperatures at 70°C and 78°C; substantially above the temperatures used herein [56]. The possibility was considered that the family of inhibitors designed to regulate protease activity express greater temperature sensitivity and operating these reactions at higher temperatures reduces their inhibitor impact. The impact of elevated temperature was concluded to alter multiple aspects of the assay that together contribute to significant improvements in signal:noise ratio.

It is expected that the stability of protease enzymes and the relative instability of inhibitor proteins to temperature increases will alter the ratio of activity to inhibited enzymes. Elevated temperature will result in elevated enzyme kinetics at elevated temperature offer improved detection of protease activity. Furthermore, it is expected that increased temperature will modify the protein corona observed around particles and described herein (**Figure 46**).

A gradient of temperature dependence was expected that balances improvements to protease activity with temperatures that will denature other proteins in serum before causing precipitation. Incubating sera before the assay or during the assay are both effective at increasing the signal to noise ratio. It has been considered 41°C, or 45°C or 50°C or 55°C or 60°C or 65°C or 70°C or 75°C or intervals between these targets to be effective for the desired impact.

### Example 22: Negative Controls

There is well described literature describing the corona formed when proteins aggregate around particles [57]. It was wanted to test whether a solution of protein devoid of any activity could be shown to generate and contribute to background signal. Recall that the majority of these sensors require a protease specific cleavage.

Arginase is the exception as an enzyme that post-translationally modified targets. In the gNBS system, Arginase will convert arginine to ornithine and urea. This modification alters the structure and causes the peptide to position the terminal TCPP dye outside the Förster radius of graphene. So the contribution of a general protein corona to background performance is important to understand. In order to understand the signal-to-noise using protein as a negative control, it was evaluated using 175 human sera samples on 18 gNBS. Each gNBS was massed out in a MES Assay buffer (10mM MES, 10µM Cations, 155mM NaCl, pH 7.2). 125µL of each gNBS working solution was added to three wells. 5µL sera was added to duplicate wells and 5µL buffer to the third well.

A sample-only control was also included with 5µL of sera added to 125µL of buffer with no sensor. In addition, each set of sensors was also stimulated with 5µl of a solution of 20mg/mL acetylated BSA. The plates were loosely covered and incubated at 45°C for 90 minutes before fluorescence was measured at Ex/Em 425/653nm for 500ms on a VarioSkan Lux plate reader (**Figure 24**). With a single exception (Arg gNBS), the mean fluorescence obtained by all the gNBS was significantly greater than the BSA reaction. Only one sensor had serum samples with signal that was at or below the BSA control. The BSA control was significantly greater than the assay control which has been used to measure the amount of fluorescence presented by the sensor in buffer only. We interpret this negative control as a much more valuable reflection of non-specific performance of the gNBS. The impact of temperature on the activity of the Arg gNBS may prove optimal temperatures for this sensor are lower than 45°C.

### Example 23: Lyophilization

Maintaining a performance that is repeatable and reproducible is central to maintaining an effective signal to noise. Preparations of uPA, CTSE, CTSK, and NE gNBS in MES working buffer were provided and lyophilized plates were prepared using three different excipient formulations. The plates were tested on different days using the same lot of normal sera and compared to the wet chemistry batch added to the same plate.

An improvement in inter-assay and intra-assay precision was observed when compared to wet reagents as measured by decreased coefficients of variation in both settings (**Figure 47**). Thus, maintaining greater precision allows measurements of protease activity to produce a more repeatable signal across different measurements. This precision is necessary to maintain a constant signal to noise performance in an assay setting. With consistent, repeatable and reproducible measurements of protease activity, a relationship between protease activity across a panel of protease targets can be assembled to distinguish sera derived from normal patients from patients with a spectrum of different inflammatory conditions.

### Example 24: Clinical Performance of graphene biosensors

The performance of gNBS was evaluated on clinical samples. In the first cohort, 256 subjects were accumulated from European sites and classified as lung cancer (n=89) or non-lung cancer (n=167). The serum samples were heat treated for 12 minutes at 65°C in a thermal cycler and cooled to 15°C. MES Assay Buffer (5mM MES, 10µM cations, pH 7.20, 154mM NaCl) was used to make a working solution with each gNBS adjusted to a final ABS₄₂₅ₙₘ of 0.1-0.15 depending on the sensor. This preparation was sonicated for in a water bath for 10 minutes and 125 µL dispensed in replicate wells. 5 µl heat treated sera was added to duplicate wells and a single sera only control. A sensor only assay control was included for each plate.

The plates were covered with loose foil and placed in a 37°C incubator for 90 minutes. Fluorescence was read on the VarioSkan Lux taking readings at Ex/Em 425/653nm. The mean fluorescence observed for the LC and non-LC subjects was reported (Table in **Figure 48**). A general diminishment of protease activity is recorded in lung cancer patients vs non-lung cancer patients. The stage of patients and sub-type of disease did not impact this observation. Similarly, non-LC subjects included 9 patients with breast cancer, colorectal cancer and hematologic malignancies that did not confound the non-LC analysis although those subjects are not included in this analysis. A second cohort of 175 subjects were recruited as part of the "US SST-18" cohort.

In this experiment, all the non-LC control were age matched donors with >20 pack year smoking habits, a case controlled group for the LC cohort. In the LC cohort, the majority of the LC samples were from untreated Stage I subjects. In this experiment, we tested a second Lot of sensors, a different VarioSkan Lux fluorimeter, samples derived from the US rather than the EU. gNBS were massed out and solutions adjusted to a final ABS₄₂₅ₙₘ of 0.1-0.15 depending on the sensor in MES Assay Buffer (10mM MES, 10µM cations, pH 7.20, 155mM NaCl). 125µl of Assay Buffer or Assay buffer containing each gNBS were loaded into a 96w black plate.

The plates were covered with loose foil and incubated at 45°C for 90 minutes. The fluorescence was read on the VarioSkan Lux using a one-time endpoint at Ex/Em 425/653nm (500ms). The same striking decrease in overall protease activity in the LC samples was observed (Table in **Figure 48**). A Student's two-tailed heteroscedastic t-test comparing the RFU of lung cancer with the varied non-lung cancer cohorts was calculated. The present calculations gave p-values between 2.6e-6 and 2.6e-27 across both cohorts and all sensors. The impact of this observation is the possibility that these sensors could be configured in many different arrangements to achieve a diagnostic potential with a very powerful ability to detect the presence of disease.

**Figure 49** shows a table of p-values for student's two-tailed, heteroscedastic t-Test comparing RFU of Lung Cancer and non-Lung Cancer samples.

### Example 25: Exemplary method and system to make a biosensors

Exemplary methods for assembling a particle can comprise the following steps:
1) Dispersing a mass of few layered graphene in Dimethyl Formamide (DMF) to obtain a solution.
2) Sonicating the solution for up to 1 hr using a probe sonicator using 10-15kJ per g of graphene over 1 hour.
3) Functionalizing the graphene surface to derive carboxylated surface by using a nucleophilic substitution reaction with sodium azide and bromovaleric acid as described herein to obtain a carboxylated graphene (CG).
4) Washing the CG with DMF or Ethanol to remove reactants.
5) For core particle assembled with PEI, resuspending the CG in DMF to obtain a CG/DMF solution.
6) Distributing the CG/DMF using sonication.
7) In case of exemplary Polyethylenimine (PEI) conjugating the PEI using 1-ethyl-3-(-3-dimethylaminopropyl) carbodiimide hydrochloride, (EDC) and 4-Dimethylaminoyridine (DMAP) at a defined ratio for 1-2 hours at RT.
8) Washing the product extensively with Ethanol to obtained a core particle.
9) Drying the core particle at ambient temperature for 30min. and further drying the core particle for 24-48 hours at 37°C.

A corresponding system can comprise
a) Layered graphene;
b) Reagents for modification of the graphene, attachment of the polymer and subsequently the sensor;
c) Devices for Sonication, probe sonication, physical dispersal using a high shear mixer, dispersal (e.g. a microwave);
d) A tool for measuring absorbance to standardize the concentration of graphene used in assembly (e.g. e a Nanodrop spectrophotometer); and/or
e) Devices for sensor manufacture to dry the sensor (e.g. ovens complemented in some cases by vacuum or desiccation chambers).

### Example 26: Exemplary system to detect a biomarker with biosensors of the disclosure

Methods to detect one or more target biomarkers according to the disclosure can be performed with systems comprising the following components
1) The nanosensor: This can be a single sensor or multiple sensors. The sensors can be provided as a solution and adjusted to a working concentration using defined absorbance for the working solution for each sensor. Or the sensors can be provided previously lyophilized in an opaque 96 or 384 well plate.
2) A buffer: The sensor activity is supported by an optimal buffer. This buffer may be provided as a solution or as a lyophilized cake in a multi-well plate. The buffer includes a component to stabilize the solution at a defined pH. In the current configuration this pH is 7.2. The optimal pH can vary depending on the sensors present and the optimal pH required to support enzyme health and activity.

Preferably, the buffer will also contain monobasic ions. Monobasic solutions support enzyme health and provide ion buffering for the particles to support dispersion. Also, peptide configuration will be impacted by the mono basic salt concentration.

In general, a buffer will contain necessary ions for enzyme activity. In the example of proteases enzymes, MMP are divalent cation requiring enzymes so buffers are supplemented with a selection of divalent cations.

Additional components comprise for example
3) A standard curve comprised of the included dyes at a range of concentrations. These standard curves would be used to convert a relative fluorescent unit (which is dependent on the fluorimeter used) to a concentration of dye. The standard curve could contain multiple different dyes for a multiplexed reaction. Therefore, a standard curve allows the kit to be used on many different platforms. Alternatively, the relative fluorescence may be used.

In addition or in the alternative the system can also comprise
4) Controls to demonstrate the presence of functional sensors. These controls might include serum samples with known and demonstrated activity. They might be purified enzymes with known activity. They might be chemistries designed to hydrolyze a peptide thus releasing the dye non-enzymatically.

The system can further comprise
5) An incubator capable of maintaining the multi-well plate at constant temperature for the duration of the experiment. For some fluorimeters, this incubation capability is included. The plates need to be maintained at 25°C or 37°C or 41°C or 45°C or 50°C or 55°C or 60°C or 65 °C for the duration of the experiment or for up to 5 minutes.
6) A fluorimeter to determine the amount of fluorescence released during the reaction. This fluorescence is typically measured as an endpoint but may also be measured kinetically such that the rate of change is measured at intervals of 1min or 5 min or 15 min or 30 min or 60 min.
7) An algorithm specific for a defined application. The integration of sensor activities to describe a diagnostic status will require an algorithm to interpret the defined activities and assign a classification to the sample. This algorithm will be application and sensor specific.

In particular components 1 to 4 and 7) can be included in kit of parts as will be understood by a skilled person.

For multiplex detection, additional considerations are: Dye selection, combinations of sensor with multiple dyes are calibrated to ensure there is no FRET between dyes. In addition, depending on whether the sensors are attached to a single particle or attached to separate particles and mixed, the final concentration of particles has to be optimized such that there are sufficient particles to interact with reactants and drive the reaction but not so much graphene particles that they quench released signal. Furthermore, for mixed sensors, there should be a buffer that compromises for the optimal activity of both enzyme targets. The reproducibility of the signal is dependent on reproducing the environment for every reaction. So providing a buffer with stable pH and defined salt concentrations as well as spectrophotometrically defined concentration of sensor will duplicate the environment and support batch to batch stability (see **Figure 27**).

### Example 27: Stability, reproducibility signal to noise ratio and multiplex capability of the biosensors in a testing solution

During the manufacture of gNBS sensors, few layered graphene is covalently bound to the sensor reagents. The graphene particles being covalently assembled are not subject to disassembly in aqueous solutions. As a result, the particles are observed not to cause pH drift in an aqueous solution and as described herein, they are dispersible in aqueous solutions.

**A** second aspect of the structural advantage of a graphene based sensor is reflected in the signal to noise performance of each family of nanosensors in aqueous solutions in the presence of serum. At time 0, one would not expect significant release of any fluorescent signal as at t=0 there has been no time for the enzyme to cleave and release any fluorescence. Moreover, upon addition of serum, the increase in signal is dependent on both the sensitivity of the system and a consistently low noise so that the signal can be distinguished. The relationship of sample incubated with sensor and serum at t=0 for both FeNBS and gNBS was evaluated and two differences were found.

The Assay Control, that is sensor in the presence of buffer only, shows a slight increase in signal over a 60 min period depending on the sensor for FeNBS. This increase is on the order of 2-fold. In contrast, the gNBS maintained in aqueous solution for up to 90 min shows no significant change in background signal. However, the S:N performance of gNBS that is neither prepared with sonication nor has solvents removed, while the S:N is reduced at t=0, also generate a lower signal over time. So the performance of the gNBS sensors is dependent on better S:N at t=0 and improved S:N detection on incubation with stimulus.

More significantly, when the signal:noise for graphene NBS att=0 is calculated, a low signal to noise is observed as measured by dividing sample fluorescence by Assay Control fluorescence. The gNBS sensors present smaller difference between Assay Control and Sample at t=0. The S:N at t=0 is 4.1 +/- 1.6 (1 S.D) for multiple different graphene sensors in Lots that were not manufactured using probe sonication or extensive drying (in these examples, CTSB, uPA, CTSK, CTSD, CTSE, MMP12, MMP13, MMP15, NE, MMP3, MMP9).

In contrast, as sonication and desiccation are included, the S:N at t=0 increases to 6.9-7.6; not significantly different from FeNBS. However, after stimulation for 60min with serum, for an example FeNBS sensor CTSB, the S:N at t=60 was 2.0. For CTSB made with CG KSU Lot 120120, the SN at t=60 was 9.8. For HEB CTSB100, the SN was 12.0, for HEB CTSB101 SN=18.5 and CTSB102, SN=27. Thus, as solvent removal and sonication are included in the manufacturing, the amount of enzyme activity revealed with a single sample of sera increase from 2.0 to 27 in this example.

The S:N performance difference is manifest in both the t=0 performance and the relative signal generated over time for a given sample. So S:N does not rely on the different performance of any individual sensor. Rather it is generalizable to the backbone and not to any particular enzyme activity. FeNBS to gNBS were compared in a single experiment with different lots of gNBS reflecting the inclusion of solvent removal and sonication. The S:N performance of the gNBS increments with each iteration of the manufacture protocol in reflecting solvent removal and sonication.

In defining the testing solution, the optimal formulation coordinates the best S:N performance being a measure of the stability of the sensor, with the best kinetic increase in activity over time, which reflects the provision of a healthy environment for enzyme activity.

This balance has been observed to be optimized with a pH environment of 7.2 using MES. Inclusion of monobasic salts has maintained a physiological environment for enzyme activity when included at 155mM. However, a range of monobasic salt concentrations from 0mM to 1M also impact the S:N of the sensor. As demonstrated by the results reporting in **Figure 14** increasing mono basic salt concentration reduces the assay control signal at 60min. Thus the "noise" ascribed to the particle only is lower as the concentration of salt increases. 155mM was used as a concentration that both reduces the particle noise and supports enzyme activity at physiological conditions.

### Example 28: Stability, reproducibility of signal to noise ratio and multiplex capability of the biosensors in a testing solution, in embodiments where there is a heating step

An impact of heat treatment has been observed when applied to serum in advance of incubation or when the assay is carried out at temperatures elevated above 37°C. For a typical sensor, in this example MMP10, heat treatment in advance of the assay by incubation at 65°C for 5 min, the SM/AC S:N is 1.2 without heat treatment and 1.3 with heat treatment. Thus the low signal to noise is not increased by heat treatment. After incubation in the presence of serum, the signal of untreated serum increased to 14.1 SM/AC after 90 minutes. In contrast, the heat treated sample SM/AC increased to 26.8, a 190% increase in signal strength. So the impact of heat treatment is not manifest for the t=0 signal, rather both heat treatment (**Figure 44**) and increased assay temperature (**Figure 45**) provide more enzyme activity which results in greater signal over the kinetic interval measured. Enzyme activity is increased on average 3.2-fold when pretreated with increased temperature (65°C). Similarly, the signal was increased 3.1-fold on average when the assay was incubated at 45°C and 1.7- to 1.9-fold when incubated at 41°C. Such changes were observed for all sensors and the increase in activity measured was dependent on temperature. So 41°C revealed more activity than 37°C. 45°C revealed more activity than 41°C.

The temperature was established and maintained in an oven or in a fluorimeter that had a temperature regulating function. Elevated temperature will result in elevated enzyme kinetics at elevated temperature offer improved detection of protease activity. Furthermore, it is expected that increased temperature will modify the protein corona observed around particles and described herein (**Figure 46**).

A gradient of temperature dependence that balances improvements to protease activity with temperatures is expected, that will denature other proteins in serum before causing precipitation. Incubating sera before the assay or during the assay are both effective at increasing the signal to noise ratio. Temperatures of 41°C, or 45°C or 50°C or 55°C or 60°C or 65°C or 70°C or 75°C or intervals between these targets were considered to be effective for the desired impact.

### Example 29: Explosion Graphene PEI Core Particle

The exemplary experimental procedures below are discussed using explosion graphene [12] [58]. However, virtually any few layer graphene can be used for the synthesis of biosensors biomarker detection. in this connection, the utilized graphene sheets or aggregates are of a size that is dispersible in water (< 1 micrometer in diameter).

Explosion graphene typically consists of 5-7 layers of stacked graphene. The approximate diameter of each layer is 150 nm. [8] The surface reaction forming carboxygraphene is only targeting the two outer surfaces of the graphene stack and potentially some of the edges.

Polyethylenimine can be covalently bound to the carboxylic acid groups at the surface of carboxygraphene (density of carboxylic acid groups: 1.76 ± 0.6 × 10⁻⁴ moles -COOH groups per gram, according to pH titrations).

### Example 30: Characterizing Features of the Polymer or Other Organic or Inorganic Structures attached to the graphene particle

Polyethylenimine is very hydrophilic (Consensus log P = -1.94, solubility in water > 1.2 × 10⁵ grams per liter H₂O, which is required to disperse the graphene particles in aqueous buffers. [59]

Principally, all polymers of the correct size can be attached that feature a consensus log P < 0, or a solubility in water of > 1.0 × 10³ grams per liter H₂O. [23] The photophysical properties of graphene [53] permit the quenching of attached organic fluorophores, metal particles, and quantum dots over a distance of up to 20 nm, which is again larger than any other known energy transfer distance FörsterResonance Energy Transfer (FRET) and plasmonic quenching. [53]

Consequently, the diameter of the attached polymer layer plus co-attached consensus sequence does not exceed 20 nm. Otherwise, the fluorescence of the attached fluorophore will not increase upon enzymatic release. According to the plot of molecular weight vs. particle diameter (Loebach (1975), Figure 1 [60]) for emulsion polymers herein reported as **Figure 50****,** which is applicable for this estimation, the allowable mass range of polymers featuring a diameter of up to 20 nm is from 0.5 to 1.7 × 10⁶ (Mₙ) and 0.5 to 3.0 × 10⁶ (M_{w}). However, smaller polymers may be used since their diameters can add up to 20 nm.

### Example 31: Attachment of peptides

Depending on their size, peptides may be classified as oligopeptides (2-20 residues) and polypeptides (>20 residues). Proteins consist of polypeptide chains exceeding 50 residues in length. [61] The average lengths of peptides can be estimated to 0.35 to 0.40 nm per amino acid, depending on the 2D structure of the peptide (alpha-helix or amorphous). [61] Note that peptides forming beta-sheets are not suitable for the design of biosensors, because their release kinetics upon enzymatic cleavage is very slow. A peptide of 50 amino acids has a length of 17.5 to 20 nm, depending on its 2D structure. It should also be noted that non-natural amino acids can be used in these peptides, either for anchoring the peptide to the surface of graphene or graphene-attached organic or inorganic shells, or for the attachment of the fluorophore (organic dye or nanostructure).

**Figure 51** shows the allowable ratio of peptide length (A) and polymer layer thickness (B) in graphene biosensors for protease activity detection. The maximal layer thickness (peptide+polymer) cannot exceed 20 nm. In graphene biosensors for cytokine/chemokine and arginase detection, the layer thickness is close to 20nm before binding to their target or posttranslational modification. Both events elongate the peptide sequence, thus causing fluorescence increase from a tethered fluorophore.

Principally, the graphene particles can be surrounded with an inorganic water-soluble shell (for instance a mesoporous silica nanolayer). [62] In that case, the thickness requirements for organic and inorganic nanolayers are the same: the maximal diameter of mesoporous silica shell + peptide sequence has to be smaller or equal 20nm.

### Example 32: Attachment of organic molecule

Organic and inorganic compounds presenting a functional group can be attached to a graphene surface chemically modified to present one or more corresponding functional groups.

For example, a monolayer of water-soluble organic molecules, for instance oligoethylene glycols presenting an OH functional group, can be tethered to the surface of graphene modified by adding a nitrene group to obtain modified graphene and presenting an NH2 functional group.

Instead of a polymer, a monolayer of water-soluble organic molecules, for instance oligoethylene glycols, can be tethered to the surface of graphene as shown in **Figure 52****.** Principally, the requirements for log P and water solubility are the same as for organic polymers.

Note that the greatest diameter of Amino-tetraethylene glycol azide (NH₂-TEG-N₃) is 1.5nm, according to molecular modelling (Chemdraw 3D). Accordingly, in those exemplary embodiments the size of a tetraethylene glycol unit is 1.4 nm. Therefore, the number of glycol units (-O-CH₂-CH₂-) is less than 58, not to exceed a total length of 20 nm.

When coating a graphene particle to configure a sensor, the length of the oligopeptide will have to be subtracted from the length of the oligoethylene oxide tether.

**.** Note that, principally, any linear or branched molecule that can be attached to the surface of graphene and that complies with the required solubility in water (log P < 0 or solubility in water > 1.0 × 10³ grams per liter H₂O [23] can be used, as long as it complies with the 20nm energy transfer quenching distance requirement.

A skilled person will be able to modify the above example for a different organic or inorganic material presenting functional groups corresponding to functional groups of the graphene surface. In particular, a skilled person will understand that any linear or branched molecule that can be attached to the surface of graphene and that achieves target dispersibility in aqueous solvents (> 0.1mg per mL), and the related graphene core particle can be used to provide a biosensor of the disclosure as long as the configuration of the coating material and peptide linkage complies with the 20nm energy transfer quenching distance requirement

### Example 33: Titration of Carboxygraphene (CG)

250 mg of Carboxygraphene (CG) were dispersed in 50 mL of 0.05 M NaOH (NaOH solution prepared using HPLC water and degassed with argon for 2 hours). Carboxygraphene suspension was sonicated for 10 minutes and stirred for 24 hours at 300K. After the 24-hour stirring, suspension was filtered to remove the carboxygraphene, and all solutions (carboxygraphene filtered solution, 0.05 MHCl, and 0.05 M NaOH) were degassed with argon overnight. Before titrations were started the following day, pH meter was calibrated with standard pH solutions. 20 mL of 0.05 M HCl was added to a 10 mL aliquot from the carboxygraphene filtered solution and back-titrated with 0.05 M NaOH (added in incremental steps). The pH was recorded after each addition of NaOH after the pH meter had stabilized before the next addition of NaOH. For the blank titration, a 10 mL of 0.05 M NaOH was prepared in the same way as carboxygraphene, adding 20 mL of 0.05 M HCl and back-titrated with 0.05 M NaOH.

The difference in the volumes of NaOH in the two titration curves for the same value of pH of -7.00 gives the concentration of the ionized groups (carboxyl groups) per weight increment of Carboxygraphene, as shown in **Figure 53****.**

It should be mentioned that degassing with argon was maintained during titration for both the carboxygraphene filtered solution and blank.

The difference in the volumes of HCl in the two titration curves for the same value of pH of -7.00 gives the concentration of the ionized groups (carboxyl groups) per weight increment of carboxygraphene. This titration curve indicates that we have between 1.765x10' ⁴ and 1.8x10-4 mol/g of acidic groups (-COOH) (i.e., surface density of 4 -COOH groups per nm²).

### Example 34: Signal to Noise ratio

Structural features responsible for increasing the signal-to-noise ratio comprise, configuring the biosensor to include fractal graphene and a coating and peptide linkage configured to maintain a distance of less than 20nm from the graphene surface of the tethered detectable moiety fluorescent dye (TCPP, as well as other dyes with higher fluorescence quantum yields).

The majority of the consensus sequences feature 13 amino acid residues, which results in 5 ± 1 nm in length. The thickness of the covalently attached PEI layer is 10 nm. Consequently, the average position of the TCPP fluorophores is well below the threshold of 20 nm.

Additional examples of signal to noise ratios achievable with the biosensors herein described are illustrated in **Example 19** (see **Figure 33****,** and **Figure 35** and related portions of the specification) **Example 28** (see **Figure 46** and related portions of the specification) and **Example 46** (see **Figure** 54 and related portions of the specification). These examples show the operability that all embodiments that maintain a distance between graphene surface and tethered fluorophore of < 20 nm as will be understood by a skilled person.

### Example 35: Method of making a core graphene particle for biosensor

An exemplary procedure for making a core particle according to the disclosure:
Step 1: selecting detonation graphene or few layer graphene to secure the required UV/Vis absorption behavior (strong emission from UV to mid-IR (100 to 1500 nm).
Step 2: Performing a chemical transformation of the graphene surface e.g. by including an organic or inorganic shell, to make the graphene particle dispersible in aqueous buffers.

The resulting organic or inorganic shell has a size of less than 20nm in diameter due to the quenching properties of graphene.

The main characterization steps of the resulting particle are:
Step 1: UV/Vis absorption spectroscopy, range 200 to 1500nm in chloroform; RAMAN and XRD to confirm that the material is graphene, elemental analysis to confirm that the material is > 98% carbon, TEM to ascertain the size distribution of the graphene nanoflakes, SEM to ascertain the fractal nature of graphene.
Step 2: FTIR to characterize functional groups of the organic or inorganic shell around graphene, RAMAN and XRD to confirm that the material is graphene, elemental analysis to confirm the chemical composition of the graphene core + shell, TEM to ascertain the size distribution of the graphene nanoflakes, SEM to ascertain the fractal nature of graphene. Solid state NMR shows the chemical makeup of the shell alone when cross-polarization is utilized, because there are no hydrogen atoms within graphene. [63]

### Example 36: Method of making a biosensor of the disclosure

An exemplary procedure to make a biosensor of the disclosure comprises the following steps
Step 1: selecting detonation graphene or few layer graphene to secure the required UV/Vis absorption behavior (strong emission from UV to mid-IR (100 to 1500 nm).
Step 2: Performing a chemical transformation of the graphene surface to make it dispersible in aqueous buffers by including an organic and/inorganic shell. The resulting organic or inorganic shell should be less than 20nm in diameter due to the quenching properties of graphene.
Step 3: Synthesizing the required peptide sequences and add a fluorophore to the N-terminal end of the peptide sequence. This is followed by cleavage of the peptide+fluorophore from the resin.
Step 4: attaching the peptide+fluorophore to the organic or inorganic shell.

### Example 37: Method of detecting a biomarker with a biosensor of the disclosure

An exemplary method to detect a biomarker according to methods of the disclosure comprises
1) Selecting the optimal range of attached fluorophore to graphene. The optimal concentration of peptide+TCPP was 7.3 mg for 100 mg of carboxygraphene. This results in 1.6 ± 0.2 × 10⁻⁵ moles peptide+TCPP (fluorophore) per gram carboxygraphene-PEI. At loading higher than that, the biosensors react significantly slower due to steric interference with the enzyme kinetics. The upper bound (when activity in the presence of 1 × 10⁻¹⁰ moles L⁻¹ of active protease is not detectable within an hour) is 1.0 ± 0.25 × 10⁻³ moles peptide+TCPP (fluorophore) per gram carboxygraphene-PEI. The lower bound (disappearance of signal) is 1.0 ± 0.25× 10⁻⁷ moles peptide+TCPP (fluorophore) per gram carboxygraphene-PEI.
2) Dispersing of the biosensor in the defined aqueous buffer. The optimal concentration range of the biosensor solution is from 0.1 to 0.03 mg per mL, having 0.3 mg per mL yielding the highest fluorescence intensity.
3) Adding the liquid biopsy (blood, plasma, serum, saliva, Bronchoalveolar lavage, exhaled breath condensate) in defined buffer. The concentration of the targeted enzyme or protein should be in the range of sub-femtomolar (10⁻¹⁶ moles L⁻¹) to micromolar (10⁻⁶ moles L⁻¹)
4) Incubating of the dispersed biosensor + liquid biopsy at defined temperature for defined time or recording of the increase in fluorescence over time at a defined temperature or temperature interval.

All biosensors for proteolytic activity measurements feature a highly selective consensus sequence. The biosensor for arginase measurements relies on post translational modification (post synthetic modification: arginine to ornithine), which elongates the peptide. Binding of targeting peptides to protein receptors elongates the former as well. All distance increases between fluorophore and graphene lead to an increase in fluorescence (and subsequent detection of activity) if the distance of 20 nm from graphene is exceeded. Otherwise, fluorescence quenching will be dominant.

### Example 38: Method of multiplex detecting a biomarker with a biosensor of the disclosure

Principally, the concentration ranges of dispersed biosensors and the preparation of the liquid biopsy are the same for the detection of one or multiple species. Due to kinetic aspects (the enzyme has to reach the peptide sequence), we cannot increase the maximal density of peptide+fluorophore at the surface of graphene.

As discussed above, the highest possible concentration of peptide+TCPP (fluorophore) is 1.0 ± 0.25 × 10⁻³ moles per gram, the optimal concentration (fastest signal increase over the concentration range from femtomolar to micromolar protease act) is in 1.6 ± 0.2 × 10⁻⁵ moles peptide+TCPP (fluorophore) per gram carboxygraphene-PEI. When several peptide sequences+different fluorophores are used to achieve multitasking, their fractions have to add up to 1.6 ± 0.2 × 10⁻⁵ moles peptide+TCPP (fluorophore) per gram carboxygraphene-PEI to ensure optimal reaction kinetics. For slower sensors, the total concentration (all fractions) of peptides+different fluorophores can reach is 1.0 ± 0.25 × 10⁻³ moles per gram.

Methods of attaching more than peptide+fluorophore to the shell around graphene comprise: 1) stepwise addition, 2) addition of all peptides+fluorophors in parallel. For either approach, the chemical addition conditions have to be optimized.

After chemically attaching several peptides+fluorophors and several washing procedures (three times redispersion in DMF, followed by centrifugation, followed by three times redispersion in diethyl ether plus centrifugation, followed by drying under argon at 40°C for 24h). The multitasking biosensor is dispersed in the buffer used for analysis and the concentrations of attached fluorophores are measured by means of UV/Vis absorption spectroscopy using calibration curves of the same dyes in the analytical buffer.

Moreover, additional modifications can be carried out to decrease the surface charge of the graphene making it suitable for binding cationic polymers in lieu of or in addition to the functionalizations described above.

### Example 39: Features of graphene particles and additional components of biosensors

Any surface reaction that makes graphene water-dispersible and/or permits the anchoring or either organic ligands or polymers of sufficient water-solubility (consensus log P < 0, or a solubility in water of > 1.0 × 10³ grams per liter H₂O.[23]) is suitable. Alternative, inorganic shells, such as mesoporous silica, can be used to disperse graphene as well.

The most important factors for organic or inorganic coating are water solubility, the presence of functional groups to attach the peptide+detecable moiety component, and the requirement of less than 20 nm thickness around graphene to facilitate quenching of the fluorophore when attached.

With respect to the peptide linkage, the specificity arises from the peptide sequences used. Different fluorophores are attached to the N-terminal position of the peptide while on resin. Alternatively, the fluorophores could also be attached to the C-terminal end, but this approach is more complicated and expensive (but doable).

Multiplexing is achieved by the interaction of the enzymes and proteins in the liquid biopsy with each co-attached peptide, because cleavage and subsequent fluorescence increase occurs much faster if there is a match with protease and its respective consensus sequence. The same is true for successful binding events of targeting peptide sequences and their respective protein targets.

In addition to the fluorescence from attached dyes, additional detectable signals comprise:
1) Phosphorescence from attached nanostructures and dyes
2) Principally, excitation could be achieved by multiphoton excitation
3) Photoacoustic signatures from attached nanostructures and dyes
4) Radioisotopes that will be cleaved off a graphene surface and analyzed.
5) Stable radicals (EPR probes) that will be released from graphene that is covered with an oxide possessing a high-spin d- or f-block metal, or co-tethered to another stable radical.
6) MRI probes that will be released from graphene that is covered with an oxide possessing a high-spin d- or f-block metal cation, or a supramolecularly bound high-spin d- or f-block metal cation.

### Example 40: impact of dispersal and solvent removal on absorbance efficiency of graphene biosensors

Solutions of graphene biosensors reflecting lots 1, 2, 3 and 4 of nanosensor reflecting the KSU protocol, the HEB replication of the KSU protocol, a sonicated protocol (Lot 3) and a sonicated protocol that eliminates solvent contamination (Lot 4) respectively were prepared as working solutions in 5mM MES pH7.4 containing 155mM NaCl. The sensors were prepared at concentrations of 25µg/mL. The working solutions were prepared and sonicated in a water bath for 1min per mL of solution. Samples were vortexed once immediately before sampling 2µL three times, The absorbance was established by measuring the absorbance across 250nm to 650 on a Nanodrop. The samples were then allowed to sit for 1 hour without any agitation. After 60 min, 2µL samples were taken with a pipette tip submerged to 50% depth in the sensor solution. The absorbance spectrum was repeated on this t=60 sample. The graphs show the absorbance spectrum for each of 4 Lots of NE and MMP15 biosensor. The absorbance efficiency is displayed on the table calculated as absorbance at 260nm per mg/ml of biosensor. The analysis shows the increasing absorbance of sensor as it is sonicated during preparation and dried to remove solvents. This observation is not dependent on the biosensor measured. (see **Figure 55**).

### Example 41: Impact of buffer formulation on Signal to Noise performance of graphene biosensor

Solutions of uPA nanosensor were prepared at 10x working concentration of 300µg/ml in water. They were sonicated for 10 minutes in a sonicating water bath. The nanosensor was diluted 1:10 in each of the different buffer formulations reported in **Figure 14****.** 125µl biosensor solution was dispensed in replicates in a black 96 well plate. 5µL serum was added and the plates were incubated at 37°c for 60 min. Released fluorescence was read on a VarioSkan Lux plate reader with 421nm excitation and 650nm emission. The assays included an assay control where 5 µl of the appropriate buffer was added in place of the serum. The biosensor was incubated with two different normal health donor sera samples or a pooled normal sera (PNS). Buffer 1 was the original formulation. Protease activity was observed in all the buffering conditions. The S:N are reported in **Figure 56** (uPA biosensor) showing the SM/AC ratio as an estimate of S:N. It was noted the buffers that included NaCl significantly lowered both the background and serum signal. Decreasing the AC serves to increase the signal to noise performance of the gNBS. Buffers 7 and 8 containing NaCl, had significantly reduced AC performance in comparison to the original buffer. This yielded an increased SN performance.

### Example 42: Impact of buffer formulation and heat treatment on Signal to Noise performance of graphene biosensors

Working solutions of 18 different biosensors were prepared in HEPES or MES buffer supplemented with 10µM divalent cations and 155mM NaCl. Samples were heat treated at 65°C for 12 min (MES Baseline) or not (HEPES Baseline) and incubated for 90min at 37 °C. After 90 minutes, fluorescence release was evaluated on a VarioSkan Lux and the S:N performance was calculated by SM/AC. The impact of protocol changes to add monobasic salt ions, more robust pH control with MES buffer demonstrated an average reduction of AC on the VarioSkan platform to 0.10RFU or lower. The more stable pH control in addition to the lower AC produced a S:N calculation that was on average 4-fold increased over the HEPES baseline (see **Figure 57**).

### Example 43: Colloidal stability of graphene biosensors

Solutions of NE or MMP15 graphene biosensors reflecting lots 1, 2, 3 and 4 of the KSU protocol, the HEB replication of the KSU protocol, a sonicated protocol (Lot 3) and a sonicated protocol that eliminates solvent contamination (Lot 4) respectively were prepared as working solutions in 5mM MES pH7.4 containing 155mM NaCl. The sensors were prepared at working concentrations of 25µg/mL. As a comparator, a preparation of Fe biosensor was also prepared at a working concentration of 300µg/mL using the same buffer. The working solutions were prepared and sonicated in a waterbath for 1 min per mL of solution. Samples were vortexed once immediately before sampling 2µL three times. The absorbance was established by measuring the absorbance across 250nm to 650 on a Nanodrop. The samples were then allowed to sit for 1 hour without any agitation. After 60 min, 2µL samples were taken with a pipette tip submerged to 50% depth in the sensor solution. The absorbance spectrum was repeated on this t=60 sample. The performance is displayed as a percentage change in absorbance efficiency at OD₂₇₀ or OD₆₅₀.

The analysis shows the increasing stability of sensor as it is sonicated during preparation and dried to remove solvents reflected in the decreasing change in absorbance over 60 minutes with each Lot preparation. This observation is not dependent on the biosensor measured. (**Figure 58**).

### Example 44: Evaluation of signal to noise across different manufacturing protocols for graphene biosensors

Solutions of NE, CTSB or MMP12 graphene biosensors reflecting lots 1, 2, 3 and 4 of the KSU protocol, the HEB replication of the KSU protocol, a sonicated protocol (Lot 3) and a sonicated protocol that eliminates solvent contamination (Lot 4) respectively were prepared as working solutions in 5mM MES pH7.4 containing 155mM NaCl. and 10µM divalent cations. The sensors were prepared at working concentrations of 25µg/mL. As a comparator, a preparation of Fe biosensor was also prepared at a working concentration of 300µg/mL using the same buffer. Biosensors were stimulated with the same sera on the same day (**panel A, B and C,** **Figure 59****, and Panel D,** **Figure 59**).

The fluorescence released after 60min at 45°C was measured on a VarioSkan Lux. Across four biosensors on different days, the impact of sonication (Lots 3 & 4) and solvent removal (Lot 4) clearly demonstrate and increase t=60 S:N. (See t=60 RFI (**Figure 59****, Panel B and** **Figure 61**). To capture the stability of unstimulated graphene nanosensor vs Fe nanosensor, we also calculated the ratio of S:N at time 0 and time 60. For three different biosensors on three different measurements, the t=60/0 S:N ratio was significantly increased for Lot 3 and Lot 4 (**Figure 59****, Panel C and D**). All the arrangements were noted to produce stronger S:N performance than Fe biosensor.(**Figure 61**)

### Example 45: evaluation of signal to noise across different manufacturing protocols for graphene biosensors

Solutions of graphene biosensors for CTS-K labeled with TCPP and NE labelled with the fluorescent dye FAM were made up at 50µg/ml each in 10mM MES solution with 10µM divalent cations and 155mM NaCl, pH 7.25. A solution of CTSK-TCPP biosensor was mixed in volume ratios to produce mixtures of 5% NE-FAM, 10%, 25%, 50%, 75%, 90% or 100% NE-FAM biosensor. 70µL of each mixture was stimulated on a 384 well plate in triplicate with 10µL pooled normal serum and incubated at 45°C for 90 minutes before fluorescence at 422/650 and 496/526nm was measured to detect TCPP and FAM respectively.

The data were analyzed to determine what percentage of signal was detectable at each dilution of sensor based on the known mixture of sensors. The graph shows the expected signal and the observed signal for TCPP (**Figure 60**).

A very efficient detection of signal from TCPP or FAM labeled sensors was observed, even when the sensors were diluted 1:20 in a competing sensor that was independently generating a separate signal for a different sensor.

These results support the conclusion that one can reliably detect sensors present as 1:20 mixtures in multiplexed reactions as it will be understood by a skilled person.

### Example 46: Synthesis of Graphene-Based Biosensors: G-PEI and G-TEG3amine

Two different cleavable peptide sequences were labeled with two different dyes, MMP-9 (GAGVPLS-LYSGAG) (SEQ ID NO: 132) was labeled with TCPP and a second MMP-9 (GCDDHAAG-LLGLDG) (SEQ ID NO: 132) was labeled with rhodamine B. These were attached to graphene-based biosensors, G-PEI and G-TEG3amine according to the following protocol.

25 mg of carboxygraphene-PEI or graphene-TEG3amine were sonicated and suspended in 4 mL of DMF in a small glass vial. Then, for each vial 2.5 mg of DMAP, 2.5 mg of EDC, and ~1.75 mg of TCPP and rhodamine B labeled peptides were added. Sample was sonicated for 5 minutes and the reaction was stirred overnight at room temperature.

The next day, each biosensor was collected via centrifugation (5 min @ 7000 rpm) and washed twice with DMF and three times with diethyl ether.

Each biosensor was incubated with 3 different control serum samples to measure fluorescence intensity for both TCPP (425 nm excitation and 650 nm emission) and rhodamine B (535 nm excitation and 570 nm emission) over 60 min. incubation at 37°C.

**Figure 54** shows the fluorescence intensity measured every 15 min. for both biosensors within the 60 min incubation period. Results demonstrated that fluorescence intensity increased for both corresponding fluorophores as the incubation time increased, reaching the highest intensity at 60 min. for both G-PEI and G-TEG3amine biosensors incubated with each control serum sample. These results demonstrated successful assembly of both biosensors using 2 different fluorophores.

### Example 47: Method of making multiplex sensor

Multiplex graphene biosensors can be prepared by stepwise addition or competitive synthesis.

The maximal number in the UV/Vis/Near IR range is 10, due to the requirement of virtual non-overlapping excitation and emission spectra.

The optimal density of oligopeptides tethered to the shell surrounding graphene is 1.6 ± 0.2 × 10⁻⁵ moles peptide+fluorophore per gram of biosensor. At loading higher than that, the biosensors react significantly slower due to steric interference with the enzyme kinetics. The upper bound (when activity in the presence of 1 × 10⁻¹⁰ moles L⁻¹ of active protease is not detectable within an hour) is 1.0 ± 0.25 × 10⁻³ moles peptide+fluorophore per gram biosensor. The lower bound (disappearance of signal) is 1.0 ± 0.25× 10⁻⁷ moles peptide+fluorophore per gram of biosensor.

The synthesis of a multitasking sensor can be achieved as follows:
1) Competitive binding of up to 10 different peptides+fluorophores to the shell around graphene. The bonds form during the same time interval. The resulting distribution of peptides+fluorophores is statistical.
2) Each of the peptide+fluorophore is added separately, followed by purification.

Both approaches can be optimized in terms of the concentrations of added reagents and reaction time. Competitive synthesis is less time-consuming.

### Example 48: General protocol to identify a peptide linkage specific for a target biomarker:

The original information about consensus sequences for proteases is obtained from the database MEROPS [64]. For all proteases, up to 25 different candidates for consensus sequences are built. By using CAPS-dock, a computational service provided by the Kolinski research group at the University of Warsaw, Poland, we were able to select the consensus sequences that are excellent fits for the active centers of the human proteases [65].

CABS-dock performs docking searches and determination of preferred binding sites allowing for full flexibility of the peptide and small fluctuations of the receptor backbone. CABS-dock simulations are carried out using a coarse-grained protein model, which is reverted to Protein Data Bank (PDB)-compatible coordinates once the optimization is completed.

For kinases, the relevant peptide sequences in the vicinity of the phosphorylation site are available from UNIPROT. For cytokines/chemokines, the structures of cytokine/chemokine receptors are available from UNIPROT. Docking experiments with CAPS-dock are able to guide point mutations of the relevant amino acids in these receptors for finding stronger binding oligopeptides. All *in silico* derived oligopeptides are tested in biosensors.

In summary, provided herein are graphene biosensors and related core particles, compositions methods and systems in which more than one pristine graphene sheet is coated with a coating layer of an organic or inorganic material to provide a core graphene particle, to which detectable components comprising a detectable moiety and a peptide linkage are attached through binding of the peptide linkage.

The examples set forth above are provided to give those of ordinary skill in the art a disclosure and description of how to make and use embodiments of the biosensors, core particles, materials, compositions, methods and systems of the disclosure, and are not intended to limit the scope of what the inventors regard as their disclosure. Those skilled in the art will recognize how to adapt the features of the exemplified biosensors, core particles, materials, compositions, methods and systems based on the target compound removing agents, nucleic acid removing agents, solid matrices, and devices according to various embodiments and scope of the claims.

All patents and publications mentioned in the instant specification inclusive of Background, Summary, Brief description of the Drawings, Detailed Description and Examples are indicative of the levels of skill of those skilled in the art to which the disclosure pertains.

The citation of references relates to the entire disclosure of each document cited (including webpages patents, patent applications, journal articles, abstracts, laboratory manuals, books, or other disclosures) in the instant disclosure inclusive of Background, Summary, Brief description of the Drawings, Detailed Description and Examplesis . All references cited in this disclosure inclusive of Background, Summary, Brief description of the Drawings, Detailed Description and Examples are to be considered to the same extent as if each reference had been referred in its entirety individually. However, if any inconsistency arises between a cited reference and the present disclosure, the present disclosure takes precedence.

Further, the computer readable form of the sequence listing of the ASCII text file "P2672-PCT-2021-12-28-SequenceListing_ST25" created on December 28, 2021 is incorporated herein by reference in its entirety.

The terms and expressions which have been employed in the instant disclosure are used as terms of description and not of limitation, and there is no intention in the use of such terms and expressions of excluding any equivalents of the features shown and described or portions thereof, but it is recognized that various modifications are possible within the scope of the materials, compositions, systems and methods of the disclosure claimed. Thus, it should be understood that although the biosensors, core particles, materials, compositions, methods and systems of the disclosure have been specifically described by embodiments, exemplary embodiments and optional features, modification and variation of the concepts herein described can be resorted to by those skilled in the art, and that such modifications and variations are considered to be within the scope of this disclosure in the instant disclosure as defined by the appended claims.

It is also to be understood that the terminology used herein is for the purpose of describing particular embodiments only and is not intended to be limiting. As used in this specification and the appended claims, the singular forms "a," "an," and "the" include plural referents unless the content clearly dictates otherwise. The term "plurality" includes two or more referents unless the content clearly dictates otherwise. Unless defined otherwise, all technical and scientific terms used herein have the same meaning as commonly understood by one of ordinary skill in the art to which the disclosure pertains.

When a Markush group or other grouping is used in the instant disclosure, all individual members of the group and all combinations and possible subcombinations of the group are intended to be individually included in the disclosure. Every combination of components or materials described or exemplified herein can be used to practice the materials, compositions, systems and methods of the disclosure, unless otherwise stated. One of ordinary skill in the art will appreciate that methods, device elements, and materials other than those specifically exemplified can be employed in the practice of the materials, compositions, systems and methods of the disclosure without resort to undue experimentation. All art-known functional equivalents, of any such methods, device elements, and materials are intended to be included in the instant disclosure.

The present description also uses numerical ranges to quantify certain parameters relating to various embodiments of the present disclosure. Whenever a range is given in the specification, for example, a temperature range, a frequency range, a time range, or a composition range, all intermediate ranges and all subranges, as well as, all individual values included in the ranges given are intended to be included in the disclosure. In particular, it should be understood that when numerical ranges are provided, such ranges are to be construed as providing literal support for claim limitations that only recite the lower value of the range as well as claim limitations that only recite the upper value of the range. For example, a disclosed numerical range of about 10 to about 100 provides literal support for a claim reciting "greater than about 10" (with no upper bounds) and a claim reciting "less than about 100" (with no lower bounds).

Any one or more individual members of a range or group disclosed herein can be excluded from a claim of this disclosure. The disclosure illustratively described herein suitably can be practiced in the absence of any element or elements, limitation or limitations, which is not specifically disclosed herein.

As used herein, the phrase "and/or," when used in a list of two or more items, means that any one of the listed items can be employed by itself or any combination of two or more of the listed items can be employed. For example, if a composition is described as containing or excluding components A, B, and/or C, the composition can contain or exclude A alone; B alone; C alone; A and B in combination; A and C in combination; B and C in combination; or A, B, and C in combination.

"Optional" or "optionally" in the instant disclosure means that the subsequently described circumstance may or may not occur, so that the description includes instances where the circumstance occurs and instances where it does not according to the guidance provided in the present disclosure. For example, the phrase "optionally substituted" means that a non-hydrogen substituent may or may not be present on a given atom, and, thus, the description includes structures wherein a non-hydrogen substituent is present and structures wherein a non-hydrogen substituent is not present. It will be appreciated that the phrase "optionally substituted" is used interchangeably with the phrase "substituted or unsubstituted." Unless otherwise indicated, an optionally substituted group may have a substituent at each substitutable position of the group, and when more than one position in any given structure may be substituted with more than one substituent selected from a specified group, the substituent may be either the same or different at every position. Combinations of substituents envisioned can be identified in view of the desired features of the compound in view of the present disclosure, and in view of the features that result in the formation of stable or chemically feasible compounds. The term "stable", as used herein, refers to compounds that are not substantially altered when subjected to conditions to allow for their production, detection, and, in certain embodiments, their recovery, purification, and use for one or more of the purposes disclosed herein.

A number of embodiments of materials, compositions, systems and methods of the disclosure have been described. The specific embodiments provided herein are examples of useful embodiments of the materials, compositions, systems and methods of the disclosure and it will be apparent to one skilled in the art that the materials, compositions, systems and methods of the disclosure can be carried out using a large number of variations of the devices, device components, methods steps set forth in the present in the instant disclosure. As will be obvious to one of skill in the art, methods and devices useful for the present methods can include a large number of optional composition and processing elements and steps.

In particular, it will be understood that various modifications may be made within the scope of the following claims.

### REFERENCES

1. Wright, J.P., et al., Synthesis of nanoscale graphene via chamber detonation of oxygen/acetylene mixtures. Materials Chemistry and Physics, 2021. electronic preprints**.**
2. Loebach, D.V., The relationship between particle size and molecular weight in emulsion polymerization, in Chemical Engineering. 1975, Iowa State University: Ames, IA. p. 129.
3. Wikipedia. Graphene. 2021 [cited 2021 December 19]; Available from: https://en.wikipedia.org/wiki/Graphene#cite_note-geim2007-2.
4. De Nicola, F., et al., Wetting properties of graphene aerogels. Scientific reports, 2020. 10(1): p. 1916.
5. Güler, Ö., et al., Production of graphene layer by liquid-phase exfoliation with low sonication power and sonication time from synthesized expanded graphite. Fullerenes, Nanotubes and Carbon Nanostructures, 2016. 24(2): p. 123-127.
6. Cui, L., J. Wang, and M. Sun, Graphene Plasmon for Optoelectronics. Reviews in Physics, 2021. 6: p. 100054.
7. Nuvoli, D., et al., The production of concentrated dispersions of few-layer graphene by the direct exfoliation of graphite in organosilanes. Nanoscale research letters, 2012. 7(1): p. 1-7.
8. Wright JP, S.S., Corkill S, Covarrubias J, LeBan L, Nepal A, Li J, Bossmann SH, Sorensen CM, Synthesis of nanoscale graphene via chamber detonation of oxygen/acetylene mixtures. Materials Chemistry and Physics, 2021. electronic preprints**.**
9. Backes, C., et al., Production and processing of graphene and related materials. 2D Mater., 2020. 7(2): p. 022001.
10. Luong, D.X., et al., Gram-scale bottom-upflash graphene synthesis. Nature (London, U. K.), 2020. 577(7792): p. 647-651.
11. Stanford, M.G., et al., Flash Graphene Morphologies. ACS Nano, 2020. 14(10): p. 13691-13699.
12. Nepal, A., et al., One-step synthesis of graphene via catalyst free gas-phase hydrocarbon detonation. Nanotechnology, 2013. 24(24): p. 245602.
13. Luong, D.X., et al., Gram-scale bottom-up flash graphene synthesis. Nature, 2020. 577(7792): p. 647-651.
14. Sorensen, C., A. Nepal, and G.P. Singh. US 9,440,857.
15. Dhaubhadel, R., et al., Aerosol gelation: Synthesis of a novel, lightweight, high specific surface area material. Aerosol science and technology, 2007. 41(8): p. 804-810.
16. Sorensen, C.M., et al. US 7,691,909
17. Daniel, D. Fractal Dimension. 2011; Available from: http://soft-matter.seas.harvard.edu/index.php/Fractal_Dimension.
18. Sorensen, C., Light scattering by fractal aggregates: a review. Aerosol Science & Technology, 2001. 35(2): p. 648-687.
19. Sorensen, C.M., Light Scattering by Fractal Aggregates: A Review. Aerosol Science and Technology, 2001. 35(2): p. 648-687.
20. Qin, H., et al., Chemical amination via cycloaddition of graphene for use in a glucose sensor. Journal of nanoscience and nanotechnology, 2016. 16(5): p. 5034-5037.
21. Ma, M., et al., Rational design, synthesis, and application of silica/graphene-based nanocomposite: A review. Mater. Des., 2021. 198: p. 109367.
22. Wikipedia. Polymer. 2021 [cited 2021 December 20]; Available from: https://en.wikipedia.org/wiki/Polymer.
23. Daina, A., O. Michielin, and V. Zoete, iLOGP: a simple, robust, and efficient description of n-octanol/water partition coefficient for drug design using the GB/SA approach. Journal of chemical information and modeling, 2014. 54(12): p. 3284-3301.
24. Covarrubias, J.J., Rational Chemical Applications of Explosion-Graphene, in Chemistry. 2021, Kansas State University: Manhattan, KS, USA. p. 110.
25. Ainavarapu, S.R.K., et al., Contour Length and Refolding Rate of a Small Protein Controlled by Engineered Disulfide Bonds. Biophysical journal, 2007. 92: p. 225-33.
26. Puente, X.S., et al., Human and mouse proteases: a comparative genomic approach. Nature Reviews Genetics, 2003. 4(7): p. 544-558.
27. Kim, S.N., et al., Preferential Binding of Peptides to Graphene Edges and Planes. J. Am. Chem. Soc., 2011. 133(37): p. 14480-14483.
28. Guler, O., et al., Production of graphene layer by liquid-phase exfoliation with low sonication power and sonication time from synthesized expanded graphite. Fullerenes, Nanotubes, Carbon Nanostruct., 2016. 24(2): p. 123-127.
29. Wu, Y., G. Li, and J.P. Camden, Probing Nanoparticle Plasmons with Electron Energy Loss Spectroscopy. Chemical Reviews, 2018. 118(6): p. 2994-3031.
30. Zhang, B., et al., Preparation of aramid nanofiber and its application in polymer reinforcement: A review. Eur. Polym. J., 2020. 139: p. 109996.
31. Lange, P., et al., Evidence for graphene plasmons in the visible spectral range probed by molecules. arXiv:1404.6518 Condensed Matter > Nanoscale Physics, 2014. arXiv:1404.6518**.**
32. Wade, L. and J. Simek, Organic Chemistry (Mastering Chemistry) 9th Edition. 2017, Essex CM20 2JE England: Pearson.
33. Castelain, M., et al., Effect of Click-Chemistry Approaches for Graphene Modification on the Electrical, Thermal, and Mechanical Properties of Polyethylene/Graphene Nanocomposites. Macromolecules, 2013. 46(22): p. 8980-8987.
34. Li, W., Y. Li, and K. Xu, Azidated graphene: direct azidation from monolayers, click chemistry, and bulk productionfrom graphite. Nano letters, 2020. 20(1): p. 534-539.
35. Sarkar, S., et al., Diels-Alder Chemistry of Graphite and Graphene: Graphene as Diene and Dienophile. Journal of the American Chemical Society, 2011. 133(10): p. 3324-3327.
36. Sarkar, S., E. Bekyarova, and R.C. Haddon, Chemistry at the Dirac Point: Diels-Alder Reactivity of Graphene. Accounts of Chemical Research, 2012. 45(4): p. 673-682.
37. Salavagione, H.J., Modification of graphene with polymers via addition chemistry, in Graphene Science Handbook: Mechanical and Chemical Properties, M. Aliofkhazraei, Editor. 2016, Institute of Polymer Science and Technology (CSIS)
   Madrid, Spain: Department of Polymer Physics, Elastomers, and Energy Applications
38. Park, J. and M. Yan, Covalent functionalization of graphene with reactive intermediates. Accounts of chemical research, 2013. 46(1): p. 181-189.
39. Atwood, J.L. and J.W. Steed, Encyclopedia of Supramolecular Chemistry, . 2004, Boca Raton, FL: Taylor & Francis, 1639.
40. Huang, B., et al., Recent advances and perspectives on supramolecular radical cages. Chemical Science, 2021. 12(41): p. 13648-13663.
41. Knoll, W., et al., Streptavidin arrays as supramolecular architectures in surface-plasmon optical sensor formats. Colloids and Surfaces A: Physicochemical and Engineering Aspects, 2000. 161(1): p. 115-137.
42. Biedermann, F., W.M. Nau, and H.J. Schneider, The Hydrophobic Effect Revisited-Studies with Supramolecular Complexes Imply High-Energy Water as a Noncovalent Driving Force. Angewandte Chemie International Edition, 2014. 53(42): p. 11158-11171.
43. Liu, Z., S.K.M. Nalluri, and J.F. Stoddart, Surveying macrocyclic chemistry: from flexible crown ethers to rigid cyclophanes. Chemical Society Reviews, 2017. 46(9): p. 2459-2478.
44. Shetty, D., et al., Can we beat the biotin avidin pair?: cucurbit [7] uril-based ultrahigh affinity host-guest complexes and their applications. Chemical Society Reviews, 2015. 44(23): p. 8747-8761.
45. McLaughlin, C.K., G.D. Hamblin, and H.F. Sleiman, Supramolecular DNA assembly. Chemical Society Reviews, 2011. 40(12): p. 5647-5656.
46. Higashi, S.L., et al., Supramolecular architectures of nucleic acid/peptide hybrids. International Journal of Molecular Sciences, 2020. 21(24): p. 9458.
47. Zhu, H., et al., Supramolecular peptide constructed by molecular Lego allowing programmable self-assembly for photodynamic therapy. Nature communications, 2019. 10(1): p. 2412.
48. Li, J., et al., Click and patterned functionalization of graphene by Diels-Alder reaction. Journal of the American Chemical Society, 2016. 138(24): p. 7448-7451.
49. Namvari, M. and H. Namazi, Sweet graphene I: toward hydrophilic graphene nanosheets via click grafting alkyne-saccharides onto azide-functionalized graphene oxide. Carbohydrate research, 2014. 396: p. 1-8.
50. Farivar, F., et al., Highly Water Dispersible Functionalized Graphene by Thermal Thiol-Ene Click Chemistry. Materials, 2021. 14(11): p. 2830.
51. Sandhya, M., et al., Ultrasonication an intensifying tool for preparation of stable nanofluids and study the time influence on distinct properties of graphene nanofluids A systematic overview. Ultrasonics sonochemistry, 2021. 73: p. 105479.
52. Strom, T.A., et al., Nitrene addition to exfoliated graphene: a one-step route to highly functionalized graphene. Chemical communications, 2010. 46(23): p. 4097-4099.
53. Bian, T., R. Chang, and P.T. Leung, Förster resonance energy transfer between molecules in the vicinity of graphene-coated nanoparticles. Plasmonics, 2016. 11(5): p. 1239-1246.
54. Zhou, J., et al., Two-dimensional nanomaterials for Förster resonance energy transfer-based sensing applications. Nanophotonics, 2020. 9(7): p. 1855-1875.
55. Gendron, R., et al., Inhibition of the activities of matrix metalloproteinases 2, 8, and 9 by chlorhexidine. Clinical Diagnostic Laboratory Immunology, 1999. 6(3): p. 437-439.
56. Meraz-Cruz, N., et al., Thermal stability of human matrix metalloproteinases. Heliyon, 2020. 6(5): p. e03865.
57. Blume, J.E., et al., Rapid, deep and precise profiling of the plasma proteome with multi-nanoparticle protein corona. Nature communications, 2020. 11(1): p. 3662
58. Sorensen C, N.A., Singh GP, inventors; Kansas State University Research Foundation, USA . assignee. , Process for high-yield production of graphene via detonation of carbon-containing material U.S. Patent PGPub No. US20140335010A1, 2014.
59. Daina A, M.O., Zoete V. , SwissADME: a free web tool to evaluate pharmacokinetics, drug-likeness and medicinal chemistry friendliness of small molecules. Sci Rep. 2017;7:42717. Epub 20170303. doi: 10.1038/srep42717. PubMed PMID: 28256516; PMClD: PMC5335600, 2017.
60. Loebach, D.V., THE RELATIONSHIP BETWEEN PARTICLE SIZE AND MOLECULAR WEIGHT IN EMULSION POLYMERIZATION. 1975: Iowa State University.
61. Ainavarapu, S.R.K., et al., Contour length and refolding rate of a small protein controlled by engineered disulfide bonds. Biophysical journal, 2007. 92(1): p. 225-233.
62. Liu, J., et al., Advances in Multicompartment Mesoporous Silica Micro/Nanoparticles for Theranostic Applications. Annual Review of Chemical and Biomolecular Engineering, 2018. 9: p. 389-411.
63. Mollica, G., et al., Structural Investigations of Polymer Materials by Dynamic Nuclear Polarisation Solid-state NMR. NMR Methods for Characterization of Synthetic and Natural Polymers, 2019: p. 533-554.
64. EBI. MEROPS. 2021; Available from: https://www.ebi.ac.uk/merops/.
65. Kolinski, A. Multiscale molecular modeling. 2021; Available from: http://biocomp.chem.uw.edu.pl/.

## Claims

1. A graphene core particle comprising
at least two pristine graphene nanosheets having a pristine graphene nanosheet surface stacked over one another to form a pristine graphene aggregate, and
a coating layer covering at least 50 % of a pristine graphene nanosheet surface,
said coating layer comprising an organic and/or inorganic compound, wherein the organic and/or inorganic compound has a solubility in water of at least 10 grams per liter H₂O at 25 degrees C and is able to be covalently linked to, or in water electrostatically stably attached to, pristine graphene,
wherein the organic and/or inorganic compound is attached to a pristine graphene nanosheet to form said coating layer,
wherein the pristine graphene of the at least two pristine graphene nanosheets has a carbon content of at least 99%, an oxygen content of less than 1% and is pure, such that the pristine graphene is free of foreign substances and impurities,
wherein the pristine graphene nanosheet and/or the organic and/or inorganic compound of the coating layer is configured to present a functional group capable of binding a corresponding functional group in an aqueous solution.

2. The graphene core particle of claim 1, wherein:
(a) the graphene core particle has an average size from 10 nm to 5000 nm, and the coating layer covers at least 85% of the surface of the graphene particle;
(b) the graphene core particle comprises 2 to 100 pristine stacked graphene sheets and the particle has an average size of 900 nm or less, preferably 350 nm or less, more preferably 250 nm or less; or
(c) the graphene core particle comprises 2 to 100 pristine stacked graphene sheets and the coating layer covers at least 90% of the surface of the graphene particle.

3. The graphene core particle of claim 1 or 2, wherein:
(i) the graphene core particle comprises 5 to 25 pristine stacked graphene sheets, optionally 5 to 7 pristine stacked graphene sheets;
(ii) the stacked pristine graphene nanosheets form a particle having an average size of 150 nm;
(iii) the coating layer comprises an organic polymer, optionally wherein the organic polymer comprises one or more of polyethyleneimine, polycaprolactone, polyethylene glycol, polyvinyl alcohol, polyvinylpyrrolidone, (meth)acrylate- and (meth)acrylamide polymers, polystyrene-carboxylic acid and polystyrene-amine;
(iv) the coating layer covers at least 99% of the surface of the graphene sheets;
(v) the coating layer has a thickness equal to or lower than 18 nm to permit a minimum of 2 nm for an attached peptide sequence that features a targeting ability; and/or
(vi) the coating layer has a thickness from 8 to 17 nm, from 2 to 18 nm, or from 5 to 15 nm.

4. A graphene biosensor configured to detect a target biomarker, the graphene biosensor comprising:
a particle of any one of claims 1 to 3, and a detectable moiety capable of emitting a fluorescent signal, the detectable moiety is covalently bonded to the graphene core particle through a peptide linkage comprising a recognition sequence specific for the target biomarker,
wherein, the coating layer and the peptide linkage are configured to set a quenching distance between the graphene nanosheets and the detectable moiety in which the graphene nanosheets quench the fluorescent signal of the detectable moiety,
wherein the peptide linkage is further configured to set, upon recognition by the target biomarker of the recognition sequence, an emitting distance between the graphene nanosheets and the detectable moiety wherein the graphene nanosheets do not quench the fluorescent signal of the detectable moiety, and
wherein the target biomarker specific peptide linkage and the detectable moiety thus form a target specific detectable component configured to emit a detectable fluorescent signal upon modification of the peptide linkage by the target biomarker, and optionally wherein:
(i) said recognition sequence is specific for a biomarker selected from the group consisting of proteases, kinases, arginases, cytokines/chemokines, dioxygenases, esterases, and combinations thereof, and optionally wherein said protease is selected from the group consisting of serine, aspartyl, cysteine, metalloprotease, caspase, urokinase, cathepsin, caspase, and combinations thereof;
(ii) said biomarker is indicative of a condition selected from the group consisting of cancerous or precancerous cellular activity, bacterial activity, infection, inflammation, and combinations thereof.

5. The graphene biosensor of claim 4, wherein said detectable moiety is a chromophore/luminophore selected from the group consisting of organic dyes, inorganic dyes, fluorophores, phosphophores, light absorbing particles, quantum dots, combinations thereof, and the metalated complexes thereof, optionally wherein:
(a) said chromophore/luminophore is an organic dye selected from the group consisting of coumarins, pyrene, cyanines, BODIPY dyes, benzenes, N-methylcarbazole, erythrosin B, N-acetyl-L-tryptophanamide, 2,5-diphenyloxazole, rubrene, and N-(3-sulfopropyl)acridinium;
(b) said chromophore/luminophore is an inorganic dye selected from the group consisting of porphyrins, phthalocyanines, chlorins, and metalated chromophores, further optionally wherein said porphyrins are selected from the group consisting of tetra carboxyphenyl-porphyrin (TCPP) and Zn-TCPP;
(c) said chromophore/luminophore is a fluorophore or phosphor selected from the group consisting of phosphorescent dyes, fluoresceins, rhodamines, and anthracenes; or
(d) said quantum dot is selected from the group consisting of CdSe/ZnS core/shell quantum dots, CdTe/CdSe core/shell quantum dots, CdSe/ZnTe core/shell quantum dots, and alloyed semiconductor quantum dots.

6. The graphene biosensor of claims 4 or 5, wherein:
(i) said coating layer comprises a polymer monolayer selected from the group consisting of polyethyleneimine, polycaprolactone, polyvinyl alcohol, polyvinylpyrrolidone, (meth)acrylate- and (meth)acrylamide polymers, polystyrene-carboxylic acid, polystyrene-amine, dextran, pullulan, chitosan, alginate, cellulose, and hyaluronic acid, and mixtures or co-polymers thereof; and/or
(ii) the graphene biosensor comprises at least two different detectable moieties attached to said central carrier particle via respective peptide linkages that each have a recognition sequence specific for different target biomarkers.

7. The graphene biosensor of any one of claims 4 to 6, wherein:
(a) the graphene core particle has a size of from about 50 nm to about 500 nm; or
(b) the graphene biosensor comprises a plurality of detectable components attached to the graphene core particle via respective peptide linkages.

8. A multiplex graphene biosensor, comprising: the graphene core particle of any one of claims 1 to 3 and a plurality of target specific detectable components attached to the graphene core particle,
wherein each target specific detectable component is formed by a detectable moiety capable of emitting a fluorescent signal, the detectable moiety attached to a peptide linkage comprising a recognition sequence specific for a target biomarker,
wherein each target specific detectable component the peptide linkage is configured to provide in combination with the coating layer of the graphene core particle, a quenching distance between the detectable moiety and the graphene core particle, wherein the graphene nanosheet quenches the fluorescent signal of the detectable moiety, and
wherein the recognition sequence for the target biomarker of each detectable component is configured to specifically detect a target biomarker and the plurality of detectable components is selected to specifically detect a plurality of different target biomarkers, and optionally wherein:
(a) the multiplex graphene biosensor comprises up to 10 target specific detectable components, each detectable component presenting a detectable moiety, detectable over the whole range of the UV/Vis/NIR spectrum; and/or
(b) the multiplex graphene biosensor comprises target specific detectable components oligopeptides at a density of 1.6 ± 0.2 × 10⁻⁵ moles per gram of biosensor.

9. A composition comprising one or more graphene core particles of any one of claims 1 to 3 and/or one or more graphene biosensors of any one of claims 4 to 8 in combination with a suitable auxiliary agent, and optionally wherein the composition is a pharmaceutical composition and the auxiliary agent is a pharmaceutically acceptable auxiliary agent.

10. A method of detecting the activity of a biomarker in a biological sample comprising:
contacting a biological sample with the graphene biosensor of any one of claims 4 to 8 to create a reaction solution; and
detecting a change in said reaction solution;
and optionally wherein:
(a) the method further comprises:
exposing said reaction solution to an excitation light source; and
detecting a change in an absorption or emission spectrum of the detectable moiety as a function of activity of said biomarker in said sample;
(b) said change comprises a blue-shift in the absorption or emission maximum of said detectable moiety after said contacting relative to the absorption or emission spectrum prior to said contacting;
(c) said change comprises the appearance of a new visible color or luminescence band relative to the absorption or emission spectrum of said detectable moiety prior to said contacting;
(d) said contacting comprises incubating said sample with said biosensor for a period of time of less than 90 minutes, preferably less than 60 minutes;
(e) said contacting comprises providing a microplate comprising a plurality of microwells therein, one or more of said microwells comprising a plurality of said biosensors distributed therein, and adding said biological sample to said microwells to create respective reaction solutions in each of said microwells; and/or
(f) the detecting is preceded by a heating of the sample and/or of the testing solution.

11. A system for detecting the activity of a biomarker in a biological sample, the system comprising a biosensor of any one of claims 4 to 8 and reagents for simultaneous combined or sequential use in a method of detection the activity of a biomarker in a biological sample of claim 10.

12. A method for multiplexed detection of the activity of a plurality of biomarker in a biological sample, the method comprising:
contacting a biological sample with a multiplex biosensor according to claim 8 configured to present detectable components specific for the plurality of the biomarkers, the contacting performed to create a reaction solution; and detecting a change in said reaction solution and/or
contacting the biological sample with multiple biosensors according to any one of claims 4 to 6 each presenting a peptide linkage specific for one biomarker of the plurality of the biomarkers;
and optionally wherein:
(a) the detecting is preceded by a heating of the sample and/or of the testing solution; and/or
(b) the sample comprises a plurality of samples and the contacting is performed with the plurality of samples in parallel.

13. A system for multiplexed detection of the activity of a plurality of biomarkers in a biological sample, the system comprising
a multiplex biosensor of claim 8 and/or biosensor each presenting a peptide linkage specific for one biomarker of the plurality of the biomarkers as well as optionally a plurality of reagents for simultaneous combined or sequential use in a method of multiplex detection of the activity of a biomarker in a biological sample of claim 12.

14. A method for making the graphene core particle of any one of claims 1 to 3, the method comprising
providing at least two pristine graphene nanosheets having a pristine graphene surface stacked over one another to form a pristine graphene aggregate; and
coating a graphene surface with a coating layer of an organic and/or inorganic molecules.

15. A method for making a biosensor of any one of claims 4 to 6, the method comprising providing the graphene core particle of any one of claims 1 to 3 and attaching to the graphene surface a detectable moiety through a peptide linkage, and optionally wherein the attaching is preceded by dispersing the graphene core particle of any one of claims 1 to 3, and the attaching is performed by contacting the dispersed graphene core particle with the detectable component comprising the detectable moiety attached to the peptide linkage.

16. A system for making a biosensor of anyone of claims 4, 5, 7 or feature (ii) of claim 6, the system comprising a graphene core particle of any one of claims 1 to 3, a peptide linkage, a detectable moiety and reagents for attaching the detectable moiety to a surface of the particle through the peptide linkage.

## Patentansprüche

1. Graphenkernpartikel, umfassend
mindestens zwei Nanoblätter aus unberührtem Graphen mit einer Nanoblattoberfläche aus unberührtem Graphen, die übereinander gestapelt sind, um ein Aggregat aus unberührtem Graphen zu bilden, und
eine Beschichtungsschicht, die mindestens 50 % einer Nanoblattoberfläche aus unberührtem Graphen bedeckt,
wobei die Beschichtungsschicht eine organische und/oder anorganische Verbindung umfasst, wobei die organische und/oder anorganische Verbindung eine Wasserlöslichkeit von mindestens 10 Gramm pro Liter H₂O bei 25 °C aufweist und in der Lage ist, kovalent mit unberührtem Graphen gekoppelt oder in Wasser elektrostatisch stabil daran angelagert zu werden,
wobei die organische und/oder anorganische Verbindung an ein Nanoblatt aus unberührtem Graphen angelagert ist, um die Beschichtungsschicht zu bilden,
wobei das unberührte Graphen der mindestens zwei Nanoblätter aus unberührtem Graphen einen Kohlenstoffgehalt von mindestens 99 %, einen Sauerstoffgehalt von weniger als 1 % aufweist und rein ist, sodass das unberührte Graphen frei von Fremdsubstanzen und Verunreinigungen ist,
wobei das Nanoblatt aus unberührtem Graphen und/oder die organische und/oder anorganische Verbindung der Beschichtungsschicht dazu konfiguriert ist, eine funktionelle Gruppe zu präsentieren, die in der Lage ist, eine entsprechende funktionelle Gruppe in einer wässrigen Lösung zu binden.

2. Graphenkernpartikel nach Anspruch 1, wobei:
(a) das Graphenkernpartikel eine durchschnittliche Größe von 10 nm bis 5000 nm aufweist und die Beschichtungsschicht mindestens 85 % der Oberfläche des Graphenpartikels bedeckt;
(b) das Graphenkernpartikel 2 bis 100 unberührte gestapelte Graphenblätter umfasst und das Partikel eine durchschnittliche Größe von 900 nm oder weniger, bevorzugt 350 nm oder weniger, weiter bevorzugt 250 nm oder weniger, aufweist; oder
(c) das Graphenkernpartikel 2 bis 100 unberührte gestapelte Graphenblätter umfasst und die Beschichtungsschicht mindestens 90 % der Oberfläche des Graphenpartikels bedeckt.

3. Graphenkernpartikel nach Anspruch 1 oder 2, wobei:
(i) das Graphenkernpartikel 5 bis 25 unberührte gestapelte Graphenblätter, optional 5 bis 7 unberührte gestapelte Graphenblätter, umfasst;
(ii) die gestapelten Nanoblätter aus unberührtem Graphen ein Partikel mit einer durchschnittlichen Größe von 150 nm bilden;
(iii) die Beschichtungsschicht ein organisches Polymer umfasst, wobei optional das organische Polymer eines oder mehrere von Polyethylenimin, Polycaprolacton, Polyethylenglycol, Polyvinylalkohol, Polyvinylpyrrolidon, (Meth)acrylat- und (Meth)acrylamidpolymeren, Polystyrolcarbonsäure und Polystyrolamin umfasst;
(iv) die Beschichtungsschicht mindestens 99 % der Oberfläche der Graphenblätter bedeckt;
(v) die Beschichtungsschicht eine Dicke von gleich oder weniger als 18 nm aufweist, um ein Minimum von 2 nm für eine angelagerte Peptidsequenz zu gestatten, die über eine Zielfähigkeit verfügt; und/oder
(vi) die Beschichtungsschicht eine Dicke von 8 bis 17 nm, von 2 bis 18 nm oder von 5 bis 15 nm aufweist.

4. Graphenbiosensor, der dazu konfiguriert ist, einen Zielbiomarker nachzuweisen, wobei der Graphenbiosensor Folgendes umfasst:
ein Partikel nach einem der Ansprüche 1 bis 3 und einen nachweisbaren Anteil, der in der Lage ist, ein fluoreszierendes Signal zu emittieren, wobei der nachweisbare Anteil durch eine Peptidkopplung, die eine für den Zielbiomarker spezifische Erkennungssequenz umfasst, kovalent an das Graphenkernpartikel gebunden ist,
wobei die Beschichtungsschicht und die Peptidkopplung dazu konfiguriert sind, einen Löschabstand zwischen den Nanoblättern aus Graphen und dem nachweisbaren Anteil festzulegen, in dem die Nanoblätter aus Graphen das fluoreszierende Signal des nachweisbaren Anteils löschen,
wobei die Peptidkopplung ferner dazu konfiguriert ist, bei Erkennung der Erkennungssequenz durch den Zielbiomarker einen Emissionsabstand zwischen den Nanoblättern aus Graphen und dem nachweisbaren Anteil festzulegen, wobei die Nanoblätter aus Graphen das fluoreszierende Signal des nachweisbaren Anteils nicht löschen, und
wobei die zielbiomarkerspezifische Peptidkopplung und der nachweisbare Anteil somit eine zielspezifische nachweisbare Komponente bilden, die dazu konfiguriert ist, bei Modifikation der Peptidkopplung durch den Zielbiomarker ein nachweisbares fluoreszierendes Signal zu emittieren, und wobei optional:
(i) die Erkennungssequenz für einen Biomarker spezifisch ist, der ausgewählt ist aus der Gruppe bestehend aus Proteasen, Kinasen, Arginasen, Cytokinen/Chemokinen, Dioxygenasen, Esterasen und Kombinationen davon, und wobei optional die Protease ausgewählt ist aus der Gruppe bestehend aus Serin, Aspartyl, Cystein, Metalloprotease, Caspase, Urokinase, Cathepsin, Caspase und Kombinationen davon;
(ii) der Biomarker auf einen Zustand hinweist, der ausgewählt ist aus der Gruppe bestehend aus kanzeröser oder präkanzeröser Zellaktivität, bakterieller Aktivität, Infektion, Entzündung und Kombinationen davon.

5. Graphenbiosensor nach Anspruch 4, wobei der nachweisbare Anteil ein Chromophor/Luminophor ist, der ausgewählt ist aus der Gruppe bestehend aus organischen Farbstoffen, anorganischen Farbstoffen, Fluorophoren, Phosphophoren, lichtabsorbierenden Partikeln, Quantenpunkten, Kombinationen davon und den metallierten Komplexen davon, wobei optional:
(a) der Chromophor/Luminophor ein organischer Farbstoff ist, der ausgewählt ist aus der Gruppe bestehend aus Cumarinen, Pyren, Cyaninen, BODIPY-Farbstoffen, Benzolen, N-Methylcarbazol, Erythrosin B, N-Acetyl-L-tryptophanamid, 2,5-Diphenyloxazol, Rubren und N-(3-Sulfopropyl)acridinium;
(b) der Chromophor/Luminophor ein anorganischer Farbstoff ist, der ausgewählt ist aus der Gruppe bestehend aus Porphyrinen, Phthalocyaninen, Chlorinen und metallierten Chromophoren, wobei ferner optional die Porphyrine ausgewählt sind aus der Gruppe bestehend aus Tetracarboxyphenylporphyrin (TCPP) und Zn-TCPP;
(c) der Chromophor/Luminophor ein Fluorophor oder Phosphor ist, der ausgewählt ist aus der Gruppe bestehend aus phosphoreszierenden Farbstoffen, Fluoresceinen, Rhodaminen und Anthracenen; oder
(d) der Quantenpunkt ausgewählt ist aus der Gruppe bestehend aus CdSe/ZnS-Kern/Schale-Quantenpunkten, CdTe/CdSe-Kern/Schale-Quantenpunkten, CdSe/ZnTe-Kern/Schale-Quantenpunkten und legierten Halbleiter-Quantenpunkten.

6. Graphenbiosensor nach Anspruch 4 oder 5, wobei:
(i) die Beschichtungsschicht eine Polymermonoschicht umfasst, die ausgewählt ist aus der Gruppe bestehend aus Polyethylenimin, Polycaprolacton, Polyvinylalkohol, Polyvinylpyrrolidon, (Meth)acrylat- und (Meth)acrylamidpolymeren, Polystyrolcarbonsäure, Polystyrolamin, Dextran, Pullulan, Chitosan, Alginat, Cellulose und Hyaluronsäure und Gemischen oder Copolymeren davon; und/oder
(ii) der Graphenbiosensor mindestens zwei unterschiedliche nachweisbare Anteile umfasst, die über jeweilige Peptidkopplungen, die jeweils eine für unterschiedliche Zielbiomarker spezifische Erkennungssequenz aufweisen, an das zentrale Trägerpartikel angelagert sind.

7. Graphenbiosensor nach einem der Ansprüche 4 bis 6, wobei:
(a) das Graphenkernpartikel eine Größe von etwa 50 nm bis etwa 500 nm aufweist; oder
(b) der Graphenbiosensor eine Vielzahl von nachweisbaren Komponenten umfasst, die über jeweilige Peptidkopplungen an das Graphenkernpartikel angelagert ist.

8. Multiplex-Graphenbiosensor, umfassend: das Graphenkernpartikel nach einem der Ansprüche 1 bis 3 und eine Vielzahl von zielspezifischen nachweisbaren Komponenten, die an das Graphenkernpartikel angelagert ist,
wobei jede zielspezifische nachweisbare Komponente durch einen nachweisbaren Anteil gebildet ist, der in der Lage ist, ein fluoreszierendes Signal zu emittieren, wobei der nachweisbare Anteil, der an eine Peptidkopplung angelagert ist, eine für einen Zielbiomarker spezifische Erkennungssequenz umfasst,
wobei jeder zielspezifischen nachweisbaren Komponente die Peptidkopplung dazu konfiguriert ist, in Kombination mit der Beschichtungsschicht des Graphenkernpartikels einen Löschabstand zwischen dem nachweisbaren Anteil und dem Graphenkernpartikel bereitzustellen, wobei das Nanoblatt aus Graphen das fluoreszierende Signal des nachweisbaren Anteils löscht, und
wobei die Erkennungssequenz für den Zielbiomarker jeder nachweisbaren Komponente dazu konfiguriert ist, einen Zielbiomarker spezifisch nachzuweisen, und die Vielzahl von nachweisbaren Komponenten so ausgewählt ist, dass sie eine Vielzahl von unterschiedlichen Zielbiomarkern spezifisch nachweist, und wobei optional:
(a) der Multiplex-Graphenbiosensor bis zu 10 zielspezifische nachweisbare Komponenten umfasst, wobei jede nachweisbare Komponente einen nachweisbaren Anteil präsentiert, der über den gesamten Bereich des UV/Vis/NIR-Spektrums nachweisbar ist; und/oder
(b) der Multiplex-Graphenbiosensor zielspezifische nachweisbare Komponenten Oligopeptide in einer Dichte von 1,6 ± 0,2 x 10⁻⁵ Mol pro Gramm Biosensor umfasst.

9. Zusammensetzung, die ein oder mehrere Graphenkernpartikel nach einem der Ansprüche 1 bis 3 und/oder einen oder mehrere Graphenbiosensoren nach einem der Ansprüche 4 bis 8 in Kombination mit einem geeigneten Hilfsmittel umfasst und wobei optional die Zusammensetzung eine pharmazeutische Zusammensetzung ist und das Hilfsmittel ein pharmazeutisch unbedenkliches Hilfsmittel ist.

10. Verfahren zum Nachweisen der Aktivität eines Biomarkers in einer biologischen Probe, umfassend:
Kontaktieren einer biologischen Probe mit dem Graphenbiosensor nach einem der Ansprüche 4 bis 8, um eine Reaktionslösung zu erzeugen; und
Nachweisen einer Änderung in der Reaktionslösung;
und wobei optional:
(a) das Verfahren ferner Folgendes umfasst:
Aussetzen der Reaktionslösung einer Anregungslichtquelle; und
Nachweisen einer Änderung in einem Absorptions- oder Emissionsspektrum des nachweisbaren Anteils als eine Funktion der Aktivität des Biomarkers in der Probe;
(b) die Änderung eine Blauverschiebung im Absorptions- oder Emissionsmaximum des nachweisbaren Anteils nach dem Kontaktieren relativ zum Absorptions- oder Emissionsspektrum vor dem Kontaktieren umfasst;
(c) die Änderung das Erscheinen eines neuen sichtbaren Farb- oder Lumineszenzbandes relativ zum Absorptions- oder Emissionsspektrum des nachweisbaren Anteils vor dem Kontaktieren umfasst;
(d) das Kontaktieren Inkubieren der Probe mit dem Biosensor für einen Zeitraum von weniger als 90 Minuten, bevorzugt weniger als 60 Minuten, umfasst;
(e) das Kontaktieren Bereitstellen einer Mikroplatte, die eine Vielzahl von Mikronäpfchen darin umfasst, wobei eines oder mehrere der Mikronäpfchen eine Vielzahl der Biosensoren darin verteilt umfassen, und Hinzufügen der biologischen Probe zu den Mikronäpfchen, um jeweilige Reaktionslösungen in jedem der Mikronäpfchen zu erzeugen, umfasst; und/oder
(f) dem Nachweisen ein Erwärmen der Probe und/oder der Prüflösung vorausgeht.

11. System zum Nachweisen der Aktivität eines Biomarkers in einer biologischen Probe, wobei das System einen Biosensor nach einem der Ansprüche 4 bis 8 und Reagentien zur gleichzeitigen kombinierten oder sequentiellen Verwendung in einem Verfahren zum Nachweis der Aktivität eines Biomarkers in einer biologischen Probe nach Anspruch 10 umfasst.

12. Verfahren zum gemultiplexten Nachweis der Aktivität einer Vielzahl von Biomarkern in einer biologischen Probe, wobei das Verfahren Folgendes umfasst:
Kontaktieren einer biologischen Probe mit einem Multiplex-Biosensor nach Anspruch 8, der dazu konfiguriert ist, für die Vielzahl der Biomarker spezifische nachweisbare Komponenten zu präsentieren, wobei das Kontaktieren durchgeführt wird, um eine Reaktionslösung zu erzeugen; und Nachweisen einer Änderung in der Reaktionslösung und/oder
Kontaktieren der biologischen Probe mit mehreren Biosensoren nach einem der Ansprüche 4 bis 6, von denen jeder eine für einen Biomarker der Vielzahl der Biomarker spezifische Peptidkopplung präsentiert;
und wobei optional:
(a) dem Nachweisen ein Erwärmen der Probe und/oder der Prüflösung vorausgeht; und/oder
(b) die Probe eine Vielzahl von Proben umfasst und das Kontaktieren mit der Vielzahl von Proben parallel durchgeführt wird.

13. System zum gemultiplexten Nachweis der Aktivität einer Vielzahl von Biomarkern in einer biologischen Probe, wobei das System Folgendes umfasst
einen Multiplex-Biosensor nach Anspruch 8 und/oder Biosensor, von denen jeder eine für einen Biomarker der Vielzahl der Biomarker spezifische Peptidkopplung präsentiert, sowie optional eine Vielzahl von Reagentien zur gleichzeitigen kombinierten oder sequentiellen Verwendung in einem Verfahren zum Multiplex-Nachweis der Aktivität eines Biomarkers in einer biologischen Probe nach Anspruch 12.

14. Verfahren zum Herstellen des Graphenkempartikels nach einem der Ansprüche 1 bis 3, wobei das Verfahren Folgendes umfasst
Bereitstellen von mindestens zwei Nanoblättern aus unberührtem Graphen mit einer Oberfläche aus unberührtem Graphen, die übereinander gestapelt sind, um ein Aggregat aus unberührtem Graphen zu bilden; und
Beschichten einer Graphenoberfläche mit einer Beschichtungsschicht aus organischen und/oder anorganischen Molekülen.

15. Verfahren zum Herstellen eines Biosensors nach einem der Ansprüche 4 bis 6, wobei das Verfahren Bereitstellen des Graphenkernpartikels nach einem der Ansprüche 1 bis 3 und Anlagern eines nachweisbaren Anteils an die Graphenoberfläche durch eine Peptidkopplung umfasst und wobei optional dem Anlagern Dispergieren des Graphenkernpartikels nach einem der Ansprüche 1 bis 3 vorausgeht und das Anlagern durch Kontaktieren des dispergierten Graphenkernpartikels mit der nachweisbaren Komponente, die den an die Peptidkopplung angelagerten nachweisbaren Anteil umfasst, durchgeführt wird.

16. System zum Herstellen eines Biosensors nach einem der Ansprüche 4, 5, 7 oder Merkmal (ii) nach Anspruch 6, wobei das System ein Graphenkernpartikel nach einem der Ansprüche 1 bis 3, eine Peptidkopplung, einen nachweisbaren Anteil und Reagentien zum Anlagern des nachweisbaren Anteils an einer Oberfläche des Partikels durch die Peptidkopplung umfasst.

## Revendications

1. Particule centrale de graphène comprenant
au moins deux nanofeuilles de graphène vierge comportant une surface de nanofeuille de graphène vierge empilées l'une sur l'autre pour former un agrégat de graphène vierge, et
une couche de revêtement recouvrant au moins 50 % d'une surface de nanofeuille de graphène vierge,
ladite couche de revêtement comprenant un composé organique et/ou inorganique, ledit composé organique et/ou inorganique comportant une solubilité dans l'eau d'au moins 10 grammes par litre de H₂O à 25 degrés C et pouvant être lié par covalence à du graphène vierge ou dans l'eau fixé électrostatiquement de manière stable à celui-ci,
ledit composé organique et/ou inorganique étant fixé à une nanofeuille de graphène vierge pour former ladite couche de revêtement,
ledit graphène vierge desdites au moins deux nanofeuilles de graphène vierge comportant une teneur en carbone d'au moins 99 %, une teneur en oxygène inférieure à 1 % et étant pur, de sorte que le graphène vierge soit exempt de substances étrangères et d'impuretés,
ladite nanofeuille de graphène vierge et/ou ledit composé organique et/ou inorganique de la couche de revêtement étant conçus pour présenter un groupe fonctionnel pouvant se lier à un groupe fonctionnel correspondant dans une solution aqueuse.

2. Particule centrale de graphène selon la revendication 1 :
(a) ladite particule centrale de graphène comportant une taille moyenne de 10 nm à 5000 nm, et ladite couche de revêtement recouvrant au moins 85 % de la surface de la particule de graphène ;
(b) ladite particule centrale de graphène comprenant 2 à 100 feuilles de graphène vierge empilées et ladite particule comportant une taille moyenne inférieure ou égale à 900 nm, de préférence inférieure ou égale à 350 nm, plus préférablement inférieure ou égale à 250 nm ; ou
(c) ladite particule centrale de graphène comprenant 2 à 100 feuilles de graphène vierge empilées et ladite couche de revêtement recouvrant au moins 90 % de la surface de la particule de graphène.

3. Particule centrale de graphène selon la revendication 1 ou 2 :
(i) ladite particule centrale de graphène comprenant 5 à 25 feuilles de graphène vierge empilées, éventuellement 5 à 7 feuilles de graphène vierge empilées ;
(ii) lesdites nanofeuilles de graphène vierge empilées formant une particule comportant une taille moyenne de 150 nm ;
(iii) ladite couche de revêtement comprenant un polymère organique, éventuellement ledit polymère organique comprenant un ou plusieurs polymères parmi la polyéthylèneimine, la polycaprolactone, le polyéthylène glycol, l'alcool polyvinylique, la polyvinylpyrrolidone, les polymères de (méth)acrylate et de (méth)acrylamide, le polystyrène-acide carboxylique et le polystyrène-amine ;
(iv) ladite couche de revêtement recouvrant au moins 99 % de la surface des feuilles de graphène ;
(v) ladite couche de revêtement comportant une épaisseur inférieure ou égale à 18 nm pour permettre un minimum de 2 nm pour une séquence peptidique fixée qui présente une capacité de ciblage ; et/ou
(vi) ladite couche de revêtement comportant une épaisseur de 8 à 17 nm, de 2 à 18 nm, ou de 5 à 15 nm.

4. Biocapteur de graphène conçu pour détecter un biomarqueur cible, le biocapteur de graphène comprenant :
une particule selon l'une quelconque des revendications 1 à 3, et un groupement détectable pouvant émettre un signal fluorescent, le groupement détectable étant lié par covalence à la particule centrale de graphène par l'intermédiaire d'une liaison peptidique comprenant une séquence de reconnaissance spécifique au biomarqueur cible,
ladite couche de revêtement et ladite liaison peptidique étant conçues pour fixer une distance de désactivation entre les nanofeuilles de graphène et le groupement détectable dans lequel les nanofeuilles de graphène désactivent le signal fluorescent du groupement détectable,
ladite liaison peptidique étant en outre conçue pour fixer, lors de la reconnaissance par le biomarqueur cible de la séquence de reconnaissance, une distance d'émission entre les nanofeuilles de graphène et le groupement détectable, lesdites nanofeuilles de graphène ne désactivant pas le signal fluorescent du groupement détectable, et
ladite liaison peptidique spécifique au biomarqueur cible et ledit groupement détectable formant ainsi un composant détectable spécifique cible conçu pour émettre un signal fluorescent détectable lors de la modification de la liaison peptidique par le biomarqueur cible, et éventuellement :
(i) ladite séquence de reconnaissance étant spécifique à un biomarqueur choisi dans le groupe constitué par les protéases, les kinases, les arginases, les cytokines/chimiokines, les dioxygénases, les estérases et leurs combinaisons, et éventuellement ladite protéase étant choisie dans le groupe constitué par la sérine, l'aspartyle, la cystéine, la métalloprotéase, la caspase, l'urokinase, la cathepsine, la caspase et leurs combinaisons ;
(ii) ledit biomarqueur indiquant un état choisi dans le groupe constitué par une activité cellulaire cancéreuse ou précancéreuse, une activité bactérienne, une infection, une inflammation et leurs combinaisons.

5. Biocapteur de graphène selon la revendication 4, ledit groupement détectable étant un chromophore/luminophore choisi dans le groupe constitué par les colorants organiques, les colorants inorganiques, les fluorophores, les phosphophores, les particules absorbant la lumière, les points quantiques, leurs combinaisons et leurs complexes métallés, éventuellement :
(a) ledit chromophore/luminophore étant un colorant organique choisi dans le groupe constitué par les coumarines, le pyrène, les cyanines, les colorants BODIPY, les benzènes, le N-méthylcarbazole, l'érythrosine B, le N-acétyl-L-tryptophanamide, le 2,5-diphenyloxazole, le rubrène et le N-(3-sulfopropyl)acridinium ;
(b) ledit chromophore/luminophore étant un colorant inorganique choisi dans le groupe constitué par les porphyrines, les phtalocyanines, les chlorines et les chromophores métallés, éventuelllement en outre lesdites porphyrines étant choisies dans le groupe constitué par la tétracarboxy-phényl-porphyrine (TCPP) et la Zn-TCPP ;
(c) ledit chromophore/luminophore étant un fluorophore ou phosphore choisi dans le groupe constitué par les colorants phosphorescents, les fluorescéines, les rhodamines et les anthracènes ; ou
(d) ledit point quantique étant choisi dans le groupe constitué par les points quantiques noyau/enveloppe de CdSe/ZnS, les points quantiques noyau/enveloppe de CdTe/CdSe, les points quantiques noyau/enveloppe de CdSe/ZnTe et les points quantiques semi-conducteurs alliés.

6. Biocapteur de graphène selon la revendication 4 ou 5 :
(i) ladite couche de revêtement comprenant une monocouche de polymère choisie dans le groupe constitué par la polyéthylèneimine, la polycaprolactone, l'alcool polyvinylique, la polyvinylpyrrolidone, les polymères de (méth)acrylate et de (méth)acrylamide, le polystyrène-acide carboxylique, le polystyrène-amine, le dextrane, le pullulane, le chitosane, l'alginate, la cellulose et l'acide hyaluronique, et leurs mélanges ou co-polymères ; et/ou
(ii) ledit biocapteur de graphène comprenant au moins deux groupements détectables différents fixés à ladite particule porteuse centrale par l'intermédiaire des liaisons peptidiques respectives qui comportent chacune une séquence de reconnaissance spécifique aux différents biomarqueurs cibles.

7. Biocapteur de graphène selon l'une quelconque des revendications 4 à 6 :
(a) ladite particule centrale de graphène comportant une taille d'environ 50 nm à environ 500 nm ; ou
(b) ledit biocapteur de graphène comprenant une pluralité de composants détectables fixés à la particule centrale de graphène par l'intermédiaire de liaisons peptidiques respectives.

8. Biocapteur de graphène multiplex, comprenant : la particule centrale de graphène selon l'une quelconque de revendications 1 à 3 et une pluralité de composants détectables spécifiques cibles fixés à la particule centrale de graphène,
chaque composant détectable spécifique cible étant formé par un groupement détectable pouvant émettre un signal fluorescent, ledit groupement détectable fixé à une liaison peptidique comprenant une séquence de reconnaissance spécifique à un biomarqueur cible,
chaque composant détectable spécifique cible la liaison peptidique étant conçue pour fournir en combinaison avec la couche de revêtement de la particule centrale de graphène, une distance de désactivation entre le groupement détectable et la particule centrale de graphène, ladite nanofeuille de graphène désactivant le signal fluorescent du groupement détectable, et
ladite séquence de reconnaissance pour le biomarqueur cible de chaque composant détectable étant conçue pour détecter spécifiquement un biomarqueur cible et ladite pluralité de composants détectables étant choisie pour détecter spécifiquement une pluralité de différents biomarqueurs cibles, et éventuellement :
(a) ledit biocapteur de graphène multiplex comprenant jusqu'à 10 composants détectables spécifiques cibles, chaque composant détectable présentant un groupement détectable, détectable sur toute la plage du spectre UV/Vis/NIR ; et/ou
(b) ledit biocapteur de graphène multiplex comprenant des oligopeptides de composants détectables spécifiques cibles à une densité de 1,6 ± 0,2 x 10⁻⁵ moles par gramme de biocapteur.

9. Composition comprenant une ou plusieurs particules centrales de graphène selon l'une quelconque des revendications 1 à 3 et/ou un ou plusieurs biocapteurs de graphène selon l'une quelconque des revendications 4 à 8 en combinaison avec un agent auxiliaire approprié, et éventuellement ladite composition étant une composition pharmaceutique et ledit agent auxiliaire étant un agent auxiliaire pharmaceutiquement acceptable.

10. Méthode de détection de l'activité d'un biomarqueur dans un échantillon biologique comprenant :
la mise en contact d'un échantillon biologique avec le biocapteur de graphène selon l'une quelconque des revendications 4 à 8 pour créer une solution réactionnelle ; et
la détection d'un changement dans ladite solution réactionnelle ;
et éventuellement :
(a) ladite méthode comprenant en outre :
l'exposition de ladite solution réactionnelle à une source de lumière d'excitation ; et
la détection d'un changement dans un spectre d'absorption ou d'émission du groupement détectable en fonction de l'activité dudit biomarqueur dans ledit échantillon ;
(b) ledit changement comprenant un décalage vers le bleu du maximum d'absorption ou d'émission dudit groupement détectable après ladite mise en contact par rapport au spectre d'absorption ou d'émission avant ladite mise en contact ;
(c) ledit changement comprenant l'aspect d'une nouvelle bande de couleur visible ou de luminescence par rapport au spectre d'absorption ou d'émission dudit groupement détectable avant ladite mise en contact ;
(d) ladite mise en contact comprenant l'incubation dudit échantillon avec ledit biocapteur pendant une période de temps inférieure à 90 minutes, de préférence inférieure à 60 minutes ;
(e) ladite mise en contact comprenant la fourniture d'une microplaque comprenant une pluralité de micropuits en son sein, un ou plusieurs desdits micropuits comprenant une pluralité desdits biocapteurs répartis en leur sein, et l'ajout dudit échantillon biologique dans lesdits micropuits pour créer des solutions réactionnelles respectives dans chacun desdits micropuits ; et/ou
(f) ladite détection étant précédée par un chauffage de l'échantillon et/ou de la solution de test.

11. Système permettant la détection de l'activité d'un biomarqueur dans un échantillon biologique, le système comprenant un biocapteur selon l'une quelconque des revendications 4 à 8 et des réactifs pour une utilisation combinée ou séquentielle simultanée dans une méthode de détection de l'activité d'un biomarqueur dans un échantillon biologique selon la revendication 10.

12. Méthode permettant la détection multiplexée de l'activité d'une pluralité de biomarqueurs dans un échantillon biologique, la méthode comprenant :
la mise en contact d'un échantillon biologique avec un biocapteur multiplex selon la revendication 8 conçu pour présenter des composants détectables spécifiques à la pluralité des biomarqueurs, la mise en contact étant effectuée pour créer une solution réactionnelle ; et
la détection d'un changement dans ladite solution réactionnelle et/ou
la mise en contact de l'échantillon biologique avec de multiples biocapteurs selon l'une quelconque des revendications 4 à 6 présentant chacun une liaison peptidique spécifique à un biomarqueur de la pluralité de biomarqueurs ;
et éventuellement :
(a) ladite détection étant précédée par un chauffage de l'échantillon et/ou de la solution de test ; et/ou
(b) ledit échantillon comprenant une pluralité d'échantillons et ladite mise en contact étant effectuée avec la pluralité d'échantillons en parallèle.

13. Système permettant la détection multiplexée de l'activité d'une pluralité de biomarqueurs dans un échantillon biologique, le système comprenant
un biomarqueur multiplex selon la revendication 8 et/ou un biomarqueur présentant chacun une liaison peptidique spécifique à un biomarqueur de la pluralité des biomarqueurs ainsi qu'éventuellement une pluralité de réactifs pour une utilisation combinée ou séquentielle simultanée dans une méthode de détection multiplexée de l'activité d'un biomarqueur dans un échantillon biologique selon la revendication 12.

14. Méthode permettant la fabrication de la particule centrale de graphène selon l'une quelconque des revendications 1 à 3, la méthode comprenant
la fourniture d'au moins deux nanofeuilles de graphène vierge comportant une surface de graphène vierge empilées l'une sur l'autre pour former un agrégat de graphène vierge ; et
le revêtement d'une surface de graphène avec une couche de revêtement de molécules organiques et/ou inorganiques.

15. Méthode permettant la fabrication d'un biocapteur selon l'une quelconque des revendications 4 à 6, la méthode comprenant la fourniture de la particule centrale de graphène selon l'une quelconque des revendications 1 à 3 et la fixation sur la surface de graphène d'un groupement détectable par l'intermédiaire d'une liaison peptidique, et éventuellement ladite fixation étant précédée par la dispersion de la particule centrale de graphène selon l'une quelconque des revendications 1 à 3, et la fixation étant effectuée par la mise en contact de la particule centrale de graphène dispersée avec le composant détectable comprenant le groupement détectable fixé à la liaison peptique.

16. Système permettant la fabrication d'un biocapteur selon l'une quelconque des revendications 4, 5, 7 ou d'une caractéristique (ii) selon la revendication 6, le système comprenant une particule centrale de graphène selon l'une quelconque des revendications 1 à 3, une liaison peptidique, un groupement détectable et des réactifs permettant de fixer le groupement détectable à une surface de la particule par l'intermédiaire de la liaison peptidique.
